# EUROPEAN PATENT APPLICATION

(11) **EP 2 481 736 A1**
(43) Date of publication of application: **01.08.2012**
(21) Application number: 12153098.4
(22) Date of filing: 18.04.2008
(51) Int. Cl.: C07D 401/14, C07D 413/14, C07D 417/14, A61K 31/519, A61K 31/444, A61P 35/00

(54) **Kinase inhibitors useful for the treatment of myleoproliferative diseases and other proliferative diseases**

(30) Priority: 20.04.2007 US 91321607 P
(62) Divisional of application: 08746333.7
(71) Applicant: Deciphera Pharmaceuticals, LLC., Lawrence KS 66047 (US)
(72) Inventor: Flynn, Daniel, L., Lawrence, KS 66047 (US); Petillo, Peter, A., Lawrence, KS 66046 (US); Kaufman, Michael, D., Lawrence, KS 66047 (US)
(74) Representative: Harris, Jennifer Lucy

(57) **Abstract**

Compounds of the present invention find utility in the treatment of mammalian cancers and especially human cancers including but not limited to malignant melanomas, solid tumors, glioblastomas, ovarian cancer, pancreatic cancer, prostate cancer, lung cancers, breast cancers, kidney cancers, hepatic cancers, cervical carcinomas, metastasis of primary tumor sites, myeloproliferative diseases, chronic myelogenous leukemia, leukemias, papillary thyroid carcinoma, non-small cell lung cancer, mesothelioma, hypereosinophilic syndrome, gastrointestinal stromal tumors, colonic cancers, ocular diseases characterized by hyperproliferation leading to blindness including various retinopathies, diabetic retinopathy, rheumatoid arthritis, asthma, chronic obstructive pulmonary disease, mastocytosis, mast cell leukemia, a disease caused by c-Abl kinase, oncogenic forms thereof, aberrant fusion proteins thereof and polymorphs thereof, a disease caused by c-Kit kinase, oncogenic forms thereof, aberrant fusion proteins thereof and polymorphs thereof, a disease caused by c-Met kinase, oncogenic forms thereof, aberrant fusion proteins thereof and polymorphs thereof, or a disease caused by a Raf kinase, oncogenic forms thereof, aberrant fusion proteins thereof and polymorphs thereof.

## Description

### Cross-Reference to Related Applications

This application claims the benefit of Provisional Application 60/913,216 filed April 20th, 2007. This provisional application is incorporated by reference herein in its entirety.

### Field of the Invention

The present invention relates to novel kinase inhibitors and modulator compounds useful for the treatment of various diseases. More particularly, the invention is concerned with such compounds, kinase/compound adducts, methods of treating diseases, and methods of synthesis of the compounds. Preferrably, the compounds are useful for the modulation of kinase activity of C-Abl, c-Kit, the HER family, FGFR, VEGFR, PDGFR, Flt-3, the Raf kinase family and disease causing polymorphs thereof.

### Background of the invention

Several members of the protein kinase family have been clearly implicated in the pathogenesis of various proliferative and myleoproliferative diseases and thus represent important targets for treatment of these diseases. Some of the proliferative diseases relevant to this invention include cancer, rheumatoid arthritis, atherosclerosis, and retinopathies. Important examples of kinases which have been shown to cause or contribute to the pathogensis of these diseases include C-Abl kinase and the oncogenic fusion protein bcr-Abl kinase; c-Kit kinase, FGFR, PDGF receptor kinase; VEGF receptor kinases; Flt-3 kinase, the HER family and the Raf kinase family.

C-Abl kinase is an important non-receptor tyrosine kinase involved in cell signal transduction. This ubiquitously expressed kinase--- upon activation by upstream signaling factors including growth factors, oxidative stress, integrin stimulation, and ionizing radiation---localizes to the cell plasma membrane, the cell nucleus, and other cellular compartments including the actin cytoskeleton (Van Etten, Trends Cell Biol. (1999) 9: 179). There are two normal isoforms of Abl kinase: Abl-1A and Abl-1B. The N-terminal half of c-Abl kinase is important for autoinhibition of the kinase domain catalytic activity (Pluk et al, Cell (2002) 108: 247). Details of the mechanistic aspects of this autoinhibition have recently been disclosed (Nagar et al, Cell (2003) 112: 859). The N-terminal myristolyl amino acid residue of Abl-1B has been shown to intramolecularly occupy a hydrophobic pocket formed from alpha-helices in the C-lobe of the kinase domain. Such intramolecular binding induces a novel binding area for intramolecular docking of the SH2 domain and the SH3 domain onto the kinase domain, thereby distorting and inhibiting the catalytic activity of the kinase. Thus, an intricate intramolecular negative regulation of the kinase activity is brought about by these N-terminal regions of c-Abl kinase. An aberrant dysregulated form of c-Abl is formed from a chromosomal translocation event, referred to as the Philadelphia chromosome (P.C. Nowell et al, Science (1960) 132: 1497; J.D. Rowley, Nature (1973) 243: 290). This abnormal chromosomal translocation leads aberrant gene fusion between the Abl kinase gene and the breakpoint cluster region (BCR) gene, thus encoding an aberrant protein called bcr-Abl (G. Q. Daley et al, Science (1990) 247: 824; M. L. Gishizky et al, Proc. Natl. Acad. Sci. USA (1993) 90: 3755; S. Li et al, J. Exp. Med. (1999) 189: 1399). The bcr-Abl fusion protein does not include the regulatory myristolylation site (B. Nagar et al, Cell (2003) 112: 859) and as a result functions as an oncoprotein which causes chronic myeloid leukemia (CML). CML is a malignancy of pluripotent hematopoietic stem cells. The p210 form of bcr-Abl is seen in 95% of patients with CML, and in 20% of patients with acute lymphocytic leukemia and is exemplified by sequences such as e14a2 and e13a2. The corresponding p190 form, exemplified by the sequence e1a2 has also been identified. A p185 form has also been disclosed and has been linked to being causative of up to 10% of patients with acute lymphocytic leukemia. It will be appreciated by one skilled in the art that "p210 form", "p190 form" and "p185 form" each describe a closely related group of fusion proteins, and that Sequence ID's used herein are merely representative of each form and are not meant to restrict the scope solely to those sequences.

C-KIT (Kit, CD117, stem cell factor receptor) is a 145 kDa transmembrane tyrosine kinase protein that acts as a type-III receptor (Pereira et al. J Carcin. (2005), 4: 19). The c-KIT proto-oncogene, located on chromosome 4q11-21, encodes the c-KIT receptor, whose ligand is the stem cell factor (SCF, steel factor, kit ligand, mast cell growth factor, Morstyn G, et al. Oncology (1994) 51(2):205. Yarden Y, et al. Embo J (1987) 6(11):3341). The receptor has tyrosine-protein kinase activity and binding of the ligands leads to the autophosphorylation of KIT and its association with substrates such as phosphatidylinositol 3-kinase (Pi3K). Tyrosine phosphorylation by protein tyrosine kinases is of particular importance in cellular signalling and can mediate signals for major cellular processes, such as proliferation, differentiation, apoptosis, attachment, and migration. Defects in KIT are a cause of piebaldism, an autosomal dominant genetic developmental abnormality of pigmentation characterized by congenital patches of white skin and hair that lack melanocytes. Gain-of-function mutations of the c-KIT gene and the expression of phosphorylated KIT are found in most gastrointestinal stromal tumors and mastocytosis. Further, almost all gonadal seminomas/dysgerminomas exhibit KIT membranous staining, and several reports have clarified that some (10-25%) have a c-KIT gene mutation (Sakuma, Y. et al. Cancer Sci (2004) 95:9, 716). KIT defects have also been associated with testicular tumors including germ cell tumors (GCT) and testicular germ cell tumors (TGCT).

The role of c-kit expression has been studied in hematologic and solid tumours, such as acute leukemias (Cortes J. et al. Cancer (2003) 97(11):2760) and gastrointestinal stromal tumors (GIST, Fletcher C.D. et al. Hum Pathol (2002) 33(5):459). The clinical importance of c-kit expression in malignant tumors relies on studies with Gleevec^{®} (imatinib mesylate, STI571, Novartis Pharma AG Basel, Switzerland) that specifically inhibits tyrosine kinase receptors (Lefevre G. et al. J Biol Chem (2004) 279(30):31769). Moreover, a clinically relevant breakthrough has been the finding of anti-tumor effects of this compound in GIST, a group of tumors regarded as being generally resistant to conventional chemotherapy (de Silva CM, Reid R: Pathol Oncol Res (2003) 9(1):13-19). GIST most often become Gleevec resistant and molecularly targeted small therapies that target c-KIT mutations remain elusive.

c-MET is a unique receptor tyrosine kinase (RTK) located on chromosome 7p and activated via its natural ligand hepatocyte growth factor. c-MET is found mutated in a variety of solid tumors (Ma P.C. et al. Cancer Metastasis (2003) 22:309). Mutations in the tyrosine kinase domain are associated with hereditary papillary renal cell carcinomas (Schmidt L et al. Nat. Genet. (1997) 16:68; Schmidt L, et al. Oncogene (1999) 18:2343), whereas mutations in the sema and juxtamembrane domains are often found in small cell lung cancers (SCLC; Ma P.C. et al. Cancer Res (2003) 63:6272). Many activating mutations are also found in breast cancers (Nakopoulou et al. Histopath (2000) 36(4): 313). The panoply of tumor types for which c-Met mediated growth has been implicated suggests this is a target ideally suited for modulation by specific c-MET small molecule inhibitors.

The *TPR-MET* oncogene is a transforming variant of the c-MET RTK and was initially identified after treatment of a human osteogenic sarcoma cell line transformed by the chemical carcinogen *N*-methyl-*N*'-nitro-*N*-nitrosoguanidine (Park M. et al. Cell (1986) 45:895). The TPR-MET fusion oncoprotein is the result of a chromosomal translocation, placing the *TPR3* locus on chromosome 1 upstream of a portion of the *c-MET* gene on chromosome 7 encoding only for the cytoplasmic region. Studies suggest that TPR-MET is detectable in experimental cancers (e.g. Yu J. et al. Cancer (2000) 88:1801). Dimerization of the *M*ᵣ 65,000 TPR-MET oncoprotein through a leucine zipper motif encoded by *TPR* leads to constitutive activation of the c-MET kinase (Zhen Z. et al. Oncogene (1994) 9:1691). TPR-MET acts to activated wild-type c-MET RTK and can activate crucial cellular growth pathways, including the Ras pathway (Aklilu F. et al. Am J Physiol (1996) 271:E277) and the phosphatidylinositol 3-kinase (PI3K)/AKT pathway (Ponzetto C. et al. Mol Cell Biol (1993) 13:4600). Conversely, in contrast to c-MET RTK, TPR-MET is ligand independent, lacks the CBL binding site in the juxtamembrane region in c-MET, and is mainly cytoplasmic. c-Met immunohistochemical expression seems to be associated with abnormal β-catenin expression, and provides good prognostic and predictive factors in breast cancer patients.

The majority of small molecule kinase inhibitors that have been reported have been shown to bind in one of three ways. Most of the reported inhibitors interact with the ATP binding domain of the active site and exert their effects by competing with ATP for occupancy. Other inhibitors have been shown to bind to a separate hydrophobic region of the protein known as the "DFG-in-conformation" pocket wherein such a binding mode by the inhibitor causes the kinase to adopt the "DFG-out" conformation, and still others have been shown to bind to both the ATP domain and the "DFG-in-conformation" pocket again causing the kinase to adopt the "DGF-out" conformation. Examples specific to inhibitors of Raf kinases can be found in Lowinger et al, Current Pharmaceutical Design (2002) 8: 2269; Dumas, J. et al., Current Opinion in Drug Discovery & Development (2004) 7: 600; Dumas, J. et al, WO 2003068223 A1 (2003); Dumas, J., et al, WO 9932455 A1 (1999), and Wan, P.T.C., et al, Cell (2004) 116: 855.

Physiologically, kinases are regulated by a common activation/deactivation mechanism wherein a specific activation loop sequence of the kinase protein binds into a specific pocket on the same protein which is referred to as the switch control pocket. Such binding occurs when specific amino acid residues of the activation loop are modified for example by phosphorylation, oxidation, or nitrosylation. The binding of the activation loop into the switch pocket results in a conformational change of the protein into its active form (Huse, M. and Kuriyan, J. Cell (109) 275)

### Summary of the Invention

Compounds of the present invention find utility in the treatment of mammalian cancers and especially human cancers including but not limited to malignant, melanomas, glioblastomas, ovarian cancer, pancreatic cancer, prostate cancer, lung cancers, breast cancers, kidney cancers, cervical carcinomas, metastasis of primary tumor sites, myeloproliferative diseases, leukemias, papillary thyroid carcinoma, non small cell lung cancer, mesothelioma, hypereosinophilic syndrome, gastrointestinal stromal tumors, colonic cancers, ocular diseases characterized by hyperproliferation leading to blindness including various retinopathies, rheumatoid arthritis, asthma, chronic obstructive pulmonary disorder, mastocyctosis, mast cell leukemia, a disease caused by c-Abl kinase, oncogenic forms thereof, aberrant fusion proteins thereof and polymorphs thereof, or a disease caused by a Raf kinase, oncogenic forms thereof, aberrant fusion proteins thereof and polymorphs thereof.

### Section 1 - Description of the Preferred Embodiments

The following descriptions refer to various compounds and moieties thereof.
Cycloalkyl refers to monocyclic saturated carbon rings taken from cyclopropyl, cyclobutyl, cyclopentyl, cyclohexyl, cycloheptanyl and cyclooctanyl;
Aryl refers to monocyclic or fused bicyclic ring systems characterized by delocalized π electrons (aromaticity) shared among the ring carbon atoms of at least one carbocyclic ring; preferred aryl rings are taken from phenyl, naphthyl, tetrahydronaphthyl, indenyl, and indanyl;
Heteroaryl refers to monocyclic or fused bicyclic ring systems characterized by delocalized π electrons (aromaticity) shared among the ring carbon or heteroatoms including nitrogen, oxygen, or sulfur of at least one carbocyclic or heterocyclic ring; heteroaryl rings are taken from, but not limited to, pyrrolyl, furyl, thienyl, oxazolyl, thiazolyl, isoxazolyl, isothiazolyl, imidazolyl, pyrazolyl, oxadiazolyl, thiadiazolyl, triazolyl, tetrazolyl, pyridinyl, pyrimidinyl, pyrazinyl, pyridazinyl, triazinyl, indolyl, indolinyl, isoindolyl, isoindolinyl, indazolyl, benzofuranyl, benzothienyl, benzothiazolyl, benzothiazolonyl, benzoxazolyl, benzoxazolonyl, benzisoxazolyl, benzisothiazolyl, benzimidazolyl, benzimidazolonyl, benztriazolyl, imidazopyridinyl, pyrazolopyridinyl, imidazolonopyridinyl, thiazolopyridinyl, thiazolonopyridinyl, oxazolopyridinyl, oxazolonopyridinyl, isoxazolopyridinyl, isothiazolopyridinyl, triazolopyridinyl, imidazopyrimidinyl, pyrazolopyrimidinyl, imidazolonopyrimidinyl, thiazolopyridiminyl, thiazolonopyrimidinyl, oxazolopyridiminyl, oxazolonopyrimidinyl, isoxazolopyrimidinyl, isothiazolopyrimidinyl, triazolopyrimidinyl, dihydropurinonyl, pyrrolopyrimidinyl, purinyl, pyrazolopyrimidinyl, phthalimidyl, phthalimidinyl, pyrazinylpyridinyl, pyridinopyrimidinyl, pyrimidinopyrimidinyl, cinnolinyl, quinoxalinyl, quinazolinyl, quinolinyl, isoquinolinyl, phthalazinyl, benzodioxyl, benzisothiazoline-1,1,3-trionyl, dihydroquinolinyl, tetrahydroquinolinyl, dihydroisoquinolyl, tetrahydroisoquinolinyl, benzoazepinyl, benzodiazepinyl, benzoxapinyl, and benzoxazepinyl;
Heterocyclyl refers to monocyclic rings containing carbon and heteroatoms taken from oxygen, nitrogen, or sulfur and wherein there is not delocalized π electrons (aromaticity) shared among the ring carbon or heteroatoms; heterocyclyl rings include, but are not limited to, oxetanyl, azetadinyl, tetrahydrofuranyl, pyrrolidinyl, oxazolinyl, oxazolidinyl, thiazolinyl, thiazolidinyl, pyranyl, thiopyranyl, tetrahydropyranyl, dioxalinyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, and homotropanyl;
Poly-aryl refers to two or more monocyclic or fused aryl bicyclic ring systems characterized by delocalized π electrons (aromaticity) shared among the ring carbon atoms of at least one carbocyclic ring wherein the rings contained therein are optionally linked together;
Poly-heteroaryl refers to two or more monocyclic or fused bicyclic systems characterized by delocalized π electrons (aromaticity) shared among the ring carbon or heteroatoms including nitrogen, oxygen, or sulfur of at least one carbocyclic or heterocyclic ring wherein the rings contained therein are optionally linked together, wherein at least one of the monocyclic or fused bicyclic rings of the poly-heteroaryl system is taken from heteroaryl as defined broadly above and the other rings are taken from either aryl, heteroaryl, or heterocyclyl as defined broadly above;
Poly-heterocyclyl refers to two or more monocyclic or fused bicyclic ring systems containing carbon and heteroatoms taken from oxygen, nitrogen, or sulfur and wherein there is not delocalized π electrons (aromaticity) shared among the ring carbon or heteroatoms wherein the rings contained therein are optionally linked, wherein at least one of the monocyclic or fused bicyclic rings of the poly-heteroaryl system is taken from heterocyclyl as defined broadly above and the other rings are taken from either aryl, heteroaryl, or heterocyclyl as defined broadly above;
Alkyl refers to straight or branched chain C1-C6alkyls;
Halogen refers to fluorine, chlorine, bromine, and iodine;
Alkoxy refers to -O-(alkyl) wherein alkyl is defined as above;
Alkoxylalkyl refers to -(alkyl)-O-(alkyl) wherein alkyl is defined as above;
Alkoxylcarbonyl refers to -C(O)O-(alkyl) wherein alkyl is defined as above;
CarboxylC1-C6alkyl refers to-(C1-C6alkyl)CO₂H wherein alkyl is defined as above;
Substituted in connection with a moiety refers to the fact that a further substituent may be attached to the moiety to any acceptable location on the moiety.

The term salts embraces pharmaceutically acceptable salts commonly used to form alkali metal salts of free acids and to form addition salts of free bases. The nature of the salt is not critical, provided that it is pharmaceutically-acceptable. Suitable pharmaceutically-acceptable acid addition salts may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, arylaliphatic, and heterocyclyl containing carboxylic acids and sulfonic acids, examples of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, stearic, salicylic, p-hydroxybenzoic, phenylacetic, mandelic, embonic (pamoic), methanesulfonic, ethanesulfonic, benzenesulfonic, pantothenic, toluenesulfonic, 2-hydroxyethanesulfonic, sulfanilic, cyclohexylaminosulfonic, algenic, 3-hydroxybutyric, galactaric and galacturonic acid. Suitable pharmaceutically-acceptable salts of free acid-containing compounds of Formula I include metallic salts and organic salts. More preferred metallic salts include, but are not limited to appropriate alkali metal (group Ia) salts, alkaline earth metal (group IIa) salts and other physiological acceptable metals. Such salts can be made from aluminum, calcium, lithium, magnesium, potassium, sodium and zinc. Preferred organic salts can be made from primary amines, secondary amines, tertiary amines and quaternary ammonium salts, including in part, tromethamine, diethylamine, tetra-N-methylammonium, N,N'-dibenzylethylenediamine, chloroprocaine, choline, diethanolamine, ethylenediamine, meglumine (N-methylglucamine) and procaine.

The term prodrug refers to derivatives of active compounds which revert *in vivo* into the active form. For example, a carboxylic acid form of an active drug may be esterified to create a prodrug, and the ester is subsequently converted *in vivo* to revert to the carboxylic acid form. See Ettmayer et. al, J. Med. Chem (2004) 47: 2393 and Lorenzi et. al, J. Pharm. Exp. Therpeutics (2005) 883 for reviews.

Structural, chemical and stereochemical definitions are broadly taken from IUPAC recommendations, and more specifically from Glossary of Terms used in Physical Organic Chemistry (IUPAC Recommendations 1994) as summarized by P. Müller, Pure Appl. Chem., 66, 1077-1184 (1994) and Basic Terminology of Stereochemistry (IUPAC Recommendations 1996) as summarized by G.P. Moss Pure and Applied Chemistry, 68, 2193-2222 (1996). Specific definitions are as follows:
Atropisomers are defined as a subclass of conformers which can be isolated as separate chemical species and which arise from restricted rotation about a single bond.
Regioisomers or structural isomers are defined as isomers involving the same atoms in different arrangements.
Enatiomers are defined as one of a pair of molecular entities which are mirror images of each other and non-superimposable.
Diastereomers or diastereoisomers are defined as stereoisomers other than enantiomers. Diastereomers or diastereoisomers are stereoisomers not related as mirror images. Diastereoisomers are characterized by differences in physical properties, and by some differences in chemical behavior towards achiral as well as chiral reagents.
Tautomerism is defined as isomerism of the general form

   G-X-Y=Z ≒ X=Y-Z-G

   where the isomers (called tautomers) are readily interconvertible; the atoms connecting the groups X,Y,Z are typically any of C, H, O, or S, and G is a group which becomes an electrofuge or nucleofuge during isomerization. The commonest case, when the electrofuge is H⁺, is also known as "prototropy".

Tautomers are defined as isomers that arise from tautomerism, independent of whether the isomers are isolable.

### 1. First aspect of the invention - Compounds, Methods, Preparations and Adducts

The invention includes compounds of the formula Ia: 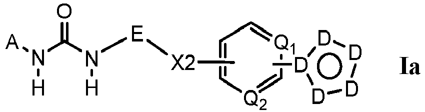 wherein Q1 and Q2 are each individually and independently selected from the group consisting of N and CH and wherein at least one of Q1 and Q2 is N;
and wherein the ring containing Q1 and Q2 may be optionally substituted with one or more R20 moieties;
each D is individually taken from the group consisting of C, CH, C-R20, N-Z3, N, O and S, such that the resultant ring is taken from the group consisting of pyrazolyl, triazolyl, isoxazolyl, isothiazolyl, oxazolyl, and thiadiazolyl;
wherein E is selected from the group consisting of phenyl, pyridyl, and pyrimidinyl;
when Q1 and Q2 are both N, the A ring is selected from the group consisting of cyclopentyl, cyclohexyl, G1, G2, and G3;
when only one of Q1 and Q2 is N, the A ring is selected from the group consisting of cyclopentyl, cyclohexyl, G1, G2, G3 and G4;
G1 is a heteroaryl taken from the group consisting of pyrrolyl, furyl, thienyl, oxazolyl, thiazolyl, isoxazolyl-4-yl, isoxazolyl-5-yl, isothiazolyl, imidazolyl, pyrazolyl, oxadiazolyl, thiadiazolyl, triazolyl, and tetrazolyl;
G2 is a fused bicyclic heteroaryl taken from the group consisting of indolyl, indolinyl, isoindolyl, isoindolinyl, indazolyl, benzofuranyl, benzothienyl, benzothiazolyl, benzothiazolonyl, benzoxazolyl, benzoxazolonyl, benzisoxazolyl, benzisothiazolyl, benzimidazolyl, benzimidazolonyl, benztriazolyl, imidazopyridinyl, pyrazolopyridinyl, imidazolonopyridinyl, thiazolopyridinyl, thiazolonopyridinyl, oxazolopyridinyl, oxazolonopyridinyl, isoxazolopyridinyl, isothiazolopyridinyl, triazolopyridinyl, imidazopyrimidinyl, pyrazolopyrimidinyl, imidazolonopyrimidinyl, thiazolopyridiminyl, thiazolonopyrimidinyl, oxazolopyridiminyl, oxazolonopyrimidinyl, isoxazolopyrimidinyl, isothiazolopyrimidinyl, triazolopyrimidinyl, dihydropurinonyl, pyrrolopyrimidinyl, purinyl, pyrazolopyrimidinyl, phthalimidyl, phthalimidinyl, pyrazinylpyridinyl, pyridinopyrimidinyl, pyrimidinopyrimidinyl, cinnolinyl, quinoxalinyl, quinazolinyl, quinolinyl, isoquinolinyl, phthalazinyl, benzodioxyl, benzisothiazoline-1,1,3-trionyl, dihydroquinolinyl, tetrahydroquinolinyl, dihydroisoquinolyl, tetrahydroisoquinolinyl, benzoazepinyl, benzodiazepinyl, benzoxapinyl, and benzoxazepinyl;
G3 is a heterocyclyl taken from the group consisting of oxetanyl, azetadinyl, tetrahydrofuranyl, pyrrolidinyl, oxazolinyl, oxazolidinyl, imidazolonyl, pyranyl, thiopyranyl, tetrahydropyranyl, dioxalinyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, and homotropanyl;
G4 is selected from the group consisting of phenyl, naphthyl, pyrazinyl, pyridazinyl, triazinyl, pyridinyl, and pyrimidinyl;
the A ring is substituted with one -(X1-A1) moiety or one A1 moiety, and may be optionally substituted with one or two R2 moieties;
when A is phenyl and E is phenyl, then Z1 cannot be -O(CH₂)_{q}R5, -(CH₂)ₚ-G3, or - (CH₂)ₚR5;
A1 is selected from the group consisting of phenyl, G1, G3 and G4;
X1 is selected from the group consisting of a direct bond linking the A and A1 rings, - (CH₂)ₙ(O)ᵣ(CH₂)ₙ-, -(CH₂)ₙ(NR3)ᵣ(CH₂)ₙ-, -(CH₂)ₙ(S)ᵣ(CH₂)ₙ-, -(CH₂)ₙ(C(O))ᵣ(CH₂)ₙ-, - (CH₂)ₙ(C(O)NR3)ᵣ(CH₂)ₙ-, and -(CH₂)ₙ(SO₂NR3)ᵣ(CH₂)ₙ-, wherein any of the alkylenes may be straight or branched chain;
when X1 is a direct bond, E is substituted with one or two R16 moieties;
when X1 is not a direct bond, then E is optionally substituted with one or two R16 moieties;
X2 is selected from the group consisting of -O-, -S(CH₂)ₙ-, -N(R3)(CH₂)ₙ-, -(CH₂)ₚ-, and wherein the carbon atoms of -(CH₂)ₙ-, -(CH₂)ₚ-, of X2 may be further substituted by oxo or one or more C1-C6alkyl moieties;
when A, A1, G1, G2 G3 or G4 has one or more substitutable sp2-hybridized carbon atoms, each respective sp2 hybridized carbon atom may be optionally substituted with a Z1 substituent;
when A, A1, G1, G2 or G3 has one or more substitutable sp3-hybridized carbon atoms, each respective sp3 hybridized carbon atom may be optionally substituted with a Z2 substituent;
when A, A1, G1, G2 or G3 has one or more substitutable nitrogen atoms, each respective nitrogen atom may be optionally substituted with a Z4 substituent;
each Z1 is independently and individually selected from the group consisting of C1-6alkyl, branched C3-C7alkyl, C3-C8cycloalkyl, halogen, fluoroC1-C6alkyl wherein the alkyl moiety can be partially or fully fluorinated, cyano, C1-C6alkoxy, fluoroC1-C6alkoxy wherein the alkyl moiety can be partially or fully fluorinated, -(CH₂)ₙOH, oxo, C1-C6alkoxyC1-C6alkyl, (R4)₂N(CH)ₙ-, (R3)₂N(CH)ₙ-, (R4)₂N(CH₂)_{q}N(R4)(CH₂)ₙ-, (R4)₂N(CH₂)_{q}O(CH₂)ₙ-, (R3)₂NC(O)-, (R4)₂NC(O)-, (R4)₂NC(O)C1-C6alkyl-, - (R4)NC(O)R8, C1-C6alkoxycarbonyl-, -carboxyC1-C6alkyl, C1-C6alkoxycarbonylC1-C6alkyl-, (R3)₂NSO₂-, -SOR3, (R4)₂NSO₂-, -N(R4)SO₂R5, -N(R4)SO₂R8, - O(CH₂)_{q}OC1-C6alkyl, -O(CH₂)_{q}R5, -SO₂R3, -SOR4, -S(O)₂R5, -(CH₂)ₙC(O)R5, - C(O)R8, -C(O)R6, -C(=NOH)R6, -C(=NOR3)R6, -(CH₂)ₙN(R4)C(O)R8, -(CH₂)ₙ-G1, - (CH₂)ₙ-G4, phenoxy, -(CH₂)ₙO(CH₂)ₙ-G1, -(CH₂)ₚO(CH₂)ₙ-G4, -N(R3)(CH₂)_{q}O-alkyl, - N(R3)(CH₂)_{q}N(R4)₂, -N(R3)(CH₂)_{q}R5, -(CH₂)ₙN(R3)(CH₂)ₙ-aryl, -(CH₂)ₙN(R3)(CH₂)ₙ-G1, -(CH₂)ₙN(R3)(CH₂)ₙ-G4, nitro, -(CH₂)ₙNHC(O)(CH₂)ₙR5, - (CH₂)ₙNHS(O)₂(CH₂)ₙR5, -(CH₂)ₙC(O)NH(CH₂)_{q}R5, -(CH₂)ₙOC(O)R5, - CH(OH)(CH₂)ₚR5, -CH(OH)CH(OH)R4, -(CH₂)ₙR5, -C(=NH)R5, -C(=NH)N(R4)₂, - C(=NOR3)R5, -C(=NOR3)N(R4)₂, and -NHC(=NH)R8;
in the event that Z1 contains an alkyl or alkylene moiety, such moieties may be further substituted with one or more C1-C6alkyls;
each Z2 is independently and individually selected from the group consisting of aryl, C1-C6alkyl, C3-C8cycloalkyl, branched C3-C7alkyl, hydroxyl, hydroxyC1-C6alkyl-, cyano, (R3)₂N-, (R4)₂N-, (R4)₂NC1-C6alkyl-, (R4)₂NC2-C6alkylN(R4)(CH₂)ₙ-, (R4)₂NC2-C6alkylO(CH₂)ₙ-, (R3)₂NC(O)-, (R4)₂NC(O)-, (R4)₂NC(O)-C1-C6alkyl-, carboxyl, - carboxyC1-C6alkyl, C -C6alkoxycarbonyl-, C1-C6alkoxycarbonylC1-C6alkyl-, (R3)₂NSO₂-, (R4)₂NSO₂-, -SO₂R5 -SO₂R8, -(CH₂)ₙN(R4)C(O)R8, -C(O)R8, =O, =NOH, =N(OR6), -(CH₂)ₙ-G1, -(CH₂)ₙ-G4, -(CH₂)ₙO(CH₂)ₙ-G1, -(CH₂)ₙO(CH₂)ₙ-G4, - (CH₂)ₙN(R3)(CH₂)ₙ-aryl, -(CH₂)ₙN(R3)(CH₂)ₙ-G1, -(CH₂)ₙN(R3)(CH₂)ₙ-G4, - (CH₂)ₙNHC(O)(CH₂)ₙR5, -(CH₂)ₙNHS(O)₂(CH₂)ₙR5, -(CH₂)ₙC(O)NH(CH₂)_{q}R5, - (CH₂)ₙC(O)R5, -(CH₂)ₙOC(O)R5, and -(CH₂)ₙR5;
in the event that Z2 contains an alkyl or alkylene moiety, such moieties may be further substituted with one or more C1-C6alkyls; each Z3 is independently and individually selected from the group consisting of H, C1-C6alkyl, branched C3-C7alkyl, C3-C8cycloalkyl, fluoroC1-C6alky wherein the alkyl moiety can be partially or fully fluorinated, hydroxyC2-C6alkyl-, C1-C6alkoxycarbonyl-, -C(O)R8, R5C(O)(CH₂)ₙ-, (R4)₂NC(O)-, (R4)₂NC(O)C1-C6alkyl-, R8C(O)N(R4)(CH₂)_{q}-, (R3)₂NSO₂-, (R4)₂NSO₂-, -(CH₂)_{q}N(R3)₂, and -(CH₂)_{q}N(R4)₂;
each Z4 is independently and individually selected from the group consisting of C1-C6alkyl, branched C3-7alkyl, hydroxyC2-C6alkyl-, C1-C6alkoxyC2-C6alkyl-, (R4)₂N-C2-C6alkyl-, (R4)₂N-C2-C6alkylN(R4)-C2-C6alkyl-, (R4)₂N-C2-C6alkyl-O-C2-C6alkyl- (R4)₂NC(O)C1-C6alkyl-, carboxyC1-C6alkyl, C1-C6alkoxycarbonylC1-C6alkyl-, -C2-C6alkylN(R4)C(O)R8, R8-C(=NR3)-, -SO₂R8, -COR8, -(CH₂)ₙ-G1, - (CH₂)ₙ-G4, -(CH₂)_{q}O(CH₂)ₙ-G1, -(CH₂)_{q}O(CH₂)ₙ-G4, -(CH₂)_{q}NR3(CH₂)ₙ-G1, - (CH₂)_{q}NR3(CH₂)ₙ-G4, -(CH₂)_{q}NHC(O)(CH₂)ₙR5, -(CH₂)_{q}C(O)NH(CH₂)_{q}R5, - (CH₂)_{q}C(O)R5, -(CH₂)_{q}OC(O)R5, -(CH₂)_{q}R5, -(CH₂)_{q}NR4(CH₂)_{q}R5, and - (CH₂)_{q}O(CH₂)_{q}R5;
in the event that Z4 contains an alkyl or alkylene moiety, such moieties may be further substituted with one or more C1-C6alkyls;
each R2 is selected from the group consisting of H, R17-substituted aryl-, R17-substituted G1, R17-substituted G4, C1-C6alkyl, branched C3-C8alkyl, R19 substituted C3-C8cycloalkyl-, fluoroC1-C6alkyl- wherein the alkyl is fully or partially fluorinated, halogen, cyano, C1-C6alkoxy-, and fluoroC1-C6alkoxy- wherein the alkyl group is fully or partially fluorinated, hydroxyl substituted C1-C6alkyl-, hydroxyl substituted branched C3-C8alkyl-, cyano substituted C1-C6alkyl-, cyano substituted branched C3-C8alkyl-, (R3)₂NC(O)C1-C6alkyl-, (R3)₂NC(O)C3-C8 branched alkyl-;
wherein each R3 is independently and individually selected from the group consisting of H, C1-C6alkyl, branched C3-C7alkyl, and C3-C8cycloalkyl;
each R4 is independently and individually selected from the group consisting of H, C1-C6alkyl, hydroxyC1-C6alkyl-, dihydroxyC1-C6alkyl-, C1-C6alkoxyC1-C6alkyl-, branched C3-C7alkyl, branched hydroxyC1-C6alkyl-, branched C1-C6alkoxyC1-C6alkyl-, branched dihydroxyC1-C6alkyl-, -(CH₂)ₚN(R7)₂, -(CH₂)ₚR5, - (CH₂)ₚC(O)N(R7)₂, -(CH₂)ₙC(O)R5, -(CH₂)ₙC(O)OR3, R19 substituted C3-C8cycloalkyl-;
each R5 is independently and individually selected from the group consisting of 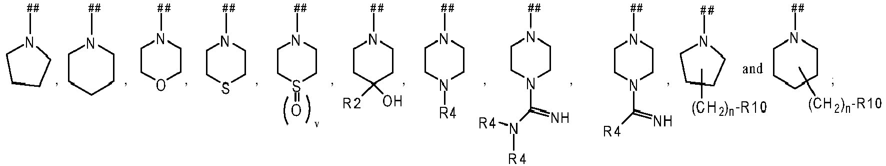 and wherein the symbol (##) is the point of attachment to respective R4, R7, R8, Z2, Z3 or Z4 moieties containing a R5 moiety;
each R6 is independently and individually selected from the group consisting of C1-C6alkyl, branched C3-C7alkyl, R19 substituted C3-C8cycloalkyl-, phenyl, G1, and G3;
each R7 is independently and individually selected from the group consisting of H, C1-C6alkyl, hydroxyC2-C6alkyl-, dihydroxyC2-C6alkyl-, C1-C6alkoxyC2-C6alkyl-, branched C3-C7alkyl, branched hydroxyC2-C6alkyl-, branched C1-C6alkoxyC2-C6alkyl-, branched dihydroxyC2-C6alkyl-, -(CH₂)_{q}R5, -(CH₂)ₙC(O)R5, - (CH₂)ₙC(O)OR3, R19 substituted C3-C8cycloalkyl- and -(CH₂)ₙR17;
each R8 is independently and individually selected from the group consisting of C1-C6alkyl, branched C3-C7alkyl, fluoroC1-C6alkyl- wherein the alkyl moiety is partially or fully fluorinated, R19 substituted C3-C8cycloalkyl-, phenyl, phenylC1-C6alkyl-, G1, G1-C1-C6alkyl-, G4-(CH₂)ₙ-, OH, C1-C6alkoxy, -N(R3)₂, -N(R4)₂, and R5; each R10 is independently and individually selected from the group consisting of -CO₂H, -CO₂C1-C6alkyl, -C(O)N(R4)₂, OH, C1-C6alkoxy, and -N(R4)₂;
each R16 is independently and individually selected from the group consisting of C1-C6alkyl, branched C3-C7alkyl, R19 substituted C3-C8cycloalkyl-, halogen, fluoroC1-C6alkyl- wherein the alkyl moiety can be partially or fully fluorinated, cyano, hydroxyl, C1-C6alkoxy, fluoroC1-C6alkoxy- wherein the alkyl moiety can be partially or fully fluorinated, -N(R3)₂, -N(R4)₂, R3 substituted C2-C3alkynyl- and nitro;
each R17 is independently and individually selected from the group consisting of H, C1-C6alkyl, branched C3-C7alkyl, R19 substituted C3-C8cycloalkyl-, halogen, fluoroC1-C6alkyl- wherein the alkyl moiety can be partially or fully fluorinated, cyano, hydroxyl, C1-C6alkoxy, fluoroC1-C6alkoxy- wherein the alkyl moiety can be partially or fully fluorinated, -N(R3)₂, -N(R4)₂, and nitro;
each R19 is independently and individually selected from the group consisting of H, OH and C1-C6alkyl;
each R20 is independently and individually selected from the group consisting of C1-C6alkyl, branched C3-C7alkyl, R19 substituted C3-C8cycloalkyl-, halogen, fluoroC1-C6alkyl- wherein the alkyl moiety can be partially or fully fluorinated, cyano, hydroxyl, C1-C6alkoxy, fluoroC1-C6alkoxy- wherein the alkyl moiety can be partially or fully fluorinated, -N(R3)₂, -N(R4)₂, -N(R3)C(O)R3, -C(O)N(R3)₂ and nitro;
wherein two R4 moieties independently and individually taken from the group consisting of C1-C6alkyl, branched C3-C6alkyl, hydroxyalkyl-, and alkoxyalkyl and attached to the same nitrogen heteroatom may cyclize to form a C3-C7 heterocyclyl ring;
k is 0 or 1; n is 0-6; p is 1-4; q is 2-6; r is 0 or 1; t is 1-3; v is 1 or 2; x is 0-2;
stereo-, regioisomers and tautomers of such compounds;
and with the proviso if A1 is G3, imidazolyl or pyrazolyl then X1 is not a direct bond.

### 1.1 Compounds of Formula Ia which exemplify preferred

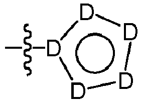

In a preferred embodiment of compounds of formula Ia, said compounds have preferred 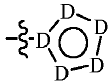 which is selected from the group consisting of 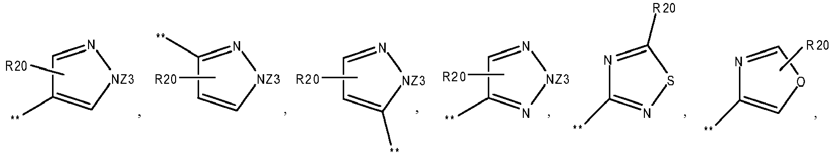 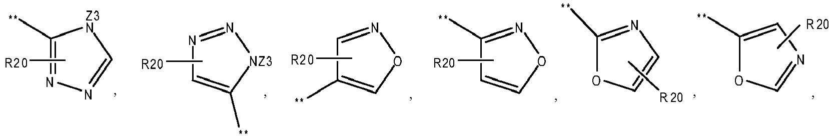 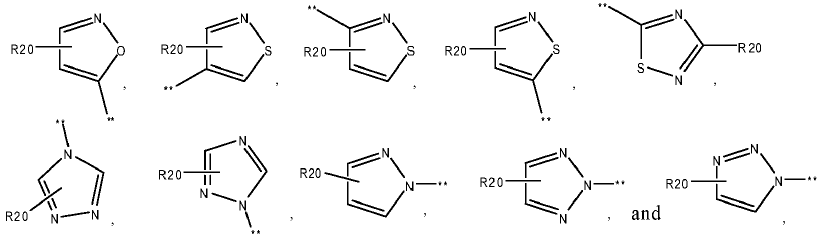 wherein the symbol (**) indicates the point of attachment to the aromatic ring.

### 1.1.1 Compounds of Formula Ia which exemplify preferred E1 and X3 moieties

In a preferred embodiment of compounds of formula Ia, said compounds have structures of formula Ib 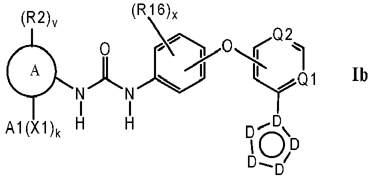 wherein A is any possible isomer of pyrazole.

### 1.1.2 Compounds of Formula Ib which exemplify preferred A, D ring and R16 moieties

In a preferred embodiment of compounds of formula Ib, said compounds have structures of formula Ic

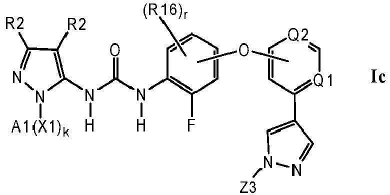

### 1.1.3 Compounds of Formula Ib which exemplify preferred A, D ring and R16 moieties In a preferred embodiment of compounds of formula Ib, said compounds have structures of formula Id

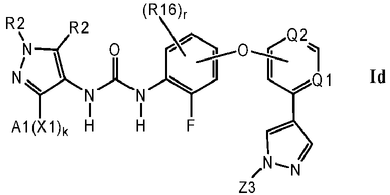

### 1.1.4 Compounds of Formula Ib which exemplify preferred A, D ring and R16 moieties

In a preferred embodiment of compounds of formula Ib, said compounds have structures of formula Ie

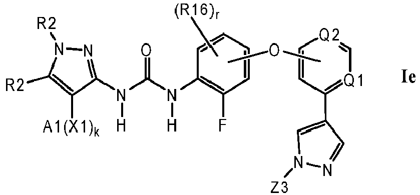

### 1.1.5 Compounds of Formula Ia which exemplify preferred A moieties

In a preferred embodiment of compounds of formula Ia, said compounds have structures of formula If 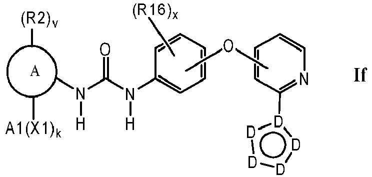 wherein A is selected from the group consisting of any possible isomer of phenyl, pyridine and pyrimidine.

### 1.1.6 Compounds of Formula If which exemplify preferred A, D ring and R16 moieties

In a preferred embodiment of compounds of formula If, said compounds have structures of formula Ig

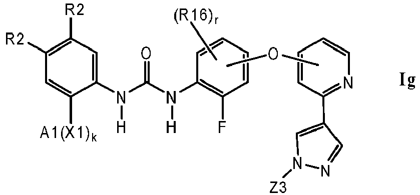

### 1.1.7 Compounds of Formula If which exemplify preferred A, D ring and R16 moieties

In a preferred embodiment of compounds of formula If, said compounds have structures of formula Ih

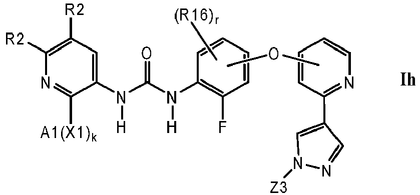

### 1.1.8 Compounds of Formula If which exemplify preferred A, D ring and R16 moieties

In a preferred embodiment of compounds of formula If, said compounds have structures of formula Ii

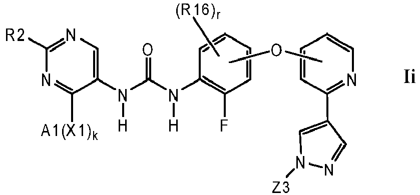

### 1.1.29 Most preferred compounds of formula Ia

1-(3-tert-butyl-1-phenyl-1H-pyrazol-5-yl)-3-(2-fluoro-4-(2-(1-methyl-1H-pyrazol-4-yl)pyridin-4-yloxy)phenyl)urea, 1-(1-(3-cyanophenyl)-3-isopropyl-1H-pyrazol-5-yl)-3-(2-fluoro-4-(2-(1-methyl-1H-pyrazol-4-yl)pyridin-4-yloxy)phenyl)urea, 1-(4-(2-(1H-pyrazol-4-yl)pyridin-4-yloxy)-2-fluorophenyl)-3-(3-tert-butyl-1-(3 -cyanophenyl)-1H-pyrazol-5-yl)urea, 4-(3-tert-butyl-5-(3-(2-fluoro-4-(2-(1-methyl-1H-pyrazol-4-yl)pyridin-4-yloxy)phenyl)ureido)-1H-pyrazol-1-yl)benzyl acetate, 1-(3-tert-butyl-1-(3-cyanophenyl)-1H-pyrazol-5-yl)-3-(2-fluoro-4-(2-(1-methyl-1H-pyrazol-4-yl)pyridin-4-yloxy)phenyl)urea, 1-(3-tert-butyl-1-(4-fluorophenyl)-1H-pyrazol-5-yl)-3-(2-fluoro-4-(2-(1-methyl-1H-pyrazol-4-yl)pyridin-4-yloxy)phenyl)urea, 1-(1-(3-cyano-4-fluorophenyl)-3-isopropyl-1H-pyrazol-5-yl)-3-(2-fluoro-4-(2-(1-methyl-1H-pyrazol-4-yl)pyridin-4-yloxy)phenyl)urea, 1-(3-tert-butyl-1-(4-cyanophenyl)-1H-pyrazol-5-yl)-3-(2-fluoro-4-(2-(1-methyl-1H-pyrazol-4-yl)pyridin-4-yloxy)phenyl)urea, 1-(1-(4-cyanophenyl)-3-isopropyl-1H-pyrazol-5-yl)-3-(2-fluoro-4-(2-(1-methyl-1H-pyrazol-4-yl)pyridin-4-yloxy)phenyl)urea, ethyl 3-(3-tert-butyl-5-(3-(2-fluoro-4-(2-(1-methyl-1H-pyrazol-4-yl)pyridin-4-yloxy)phenyl)ureido)-1H-pyrazol-1-yl)benzoate, 1-(3-tert-butyl-1-(3-(hydroxymethyl)phenyl)-1H-pyrazol-5-yl)-3-(2-fluoro-4-(2-(1-methyl-1H-pyrazol-4-yl)pyridin-4-yloxy)phenyl)urea, 3-(3-tert-butyl-5-(3-(2-fluoro-4-(2-(1-methyl-1H-pyrazol-4-yl)pyridin-4-yloxy)phenyl)ureido)-1H-pyrazol-1-yl)benzoic acid, 1-(3-tert-butyl-1-(4-(hydroxymethyl)phenyl)-1H-pyrazol-5-yl)-3-(2-fluoro-4-(2-(1-methyl-1H-pyrazol-4-yl)pyridin-4-yloxy)phenyl)urea, 1-(1-(3-cyanophenyl)-3-ethyl-1H-pyrazol-5-yl)-3-(2-fluoro-4-(2-(1-methyl-1H-pyrazol-4-yl)pyridin-4-yloxy)phenyl)urea, 1-(2-tert-butyl-4-phenylpyrimidin-5-yl)-3-(2-fluoro-4-(2-(1-methyl-1H-pyrazol-4-yl)pyridin-4-yloxy)phenyl)urea, 1-(1-(3-cyano-4-methylphenyl)-3-isopropyl-1H-pyrazol-5-yl)-3-(2-fluoro-4-(2-(1-methyl-1H-pyrazol-4-yl)pyridin-4-yloxy)phenyl)urea, 1-(1-(3-cyanophenyl)-3-isopropyl-1H-pyrazol-5-yl)-3-(2-fluoro-3-methyl-4-(2-(1-methyl-1H-pyrazol-4-yl)pyridin-4-yloxy)phenyl)urea, 1-(1-(3-cyanophenyl)-3-isopropyl-1H-pyrazol-5-yl)-3-(2,3-difluoro-4-(2-(1-methyl-1H-pyrazol-4-yl)pyridin-4-yloxy)phenyl)urea, 1-(2-fluoro-4-(2-(1-methyl-1H-pyrazol-4-yl)pyridin-4-yloxy)phenyl)-3-(2-methyl-4-phenylpyrimidin-5-yl)urea, 1-(1-(3-cyanophenyl)-3-ethyl-1H-pyrazol-5-yl)-3-(2-fluoro-4-(2-(1-propyl-1H-pyrazol-4-yl)pyridin-4-yloxy)phenyl)urea, 1-(1-(3-cyanophenyl)-3-isopropyl-1H-pyrazol-5-yl)-3-(3-fluoro-4-(2-(1-methyl-1H-pyrazol-4-yl)pyridin-4-yloxy)phenyl)urea, 1-(1-(3-cyanophenyl)-3-ethyl-1H-pyrazol-5-yl)-3-(2-fluoro-4-(2-(1-isopropyl-1H-pyrazol-4-yl)pyridin-4-yloxy)phenyl)urea, 1-(1-(3-cyanophenyl)-3-ethyl-1H-pyrazol-5-yl)-3-(4-(2-(1-ethyl-1H-pyrazol-4-yl)pyridin-4-yloxy)-2-fluorophenyl)urea, 1-(3-ethyl-1-(4-(hydroxymethyl)phenyl)-1H-pyrazol-5-yl)-3-(2-fluoro-4-(2-(1-methyl-1H-pyrazol-4-yl)pyridin-4-yloxy)phenyl)urea, 1-(2-fluoro-4-(2-(1-methyl-1H-pyrazol-4-yl)pyridin-4-yloxy)phenyl)-3-(1-isopropyl-5-phenyl-1H-pyrazol-4-yl)urea, 1-(2-fluoro-4-(2-(1-methyl-1H-pyrazol-4-yl)pyridin-4-yloxy)phenyl)-3-(1-isopropyl-3-phenyl-1H-pyrazol-4-yl)urea, 1-(1-ethyl-5-phenyl-1H-pyrazol-4-yl)-3-(2-fluoro-4-(2-(1-methyl-1H-pyrazol-4-yl)pyridin-4-yloxy)phenyl)urea, 1-(1-ethyl-3-phenyl-1H-pyrazol-4-yl)-3-(2-fluoro-4-(2-(1-methyl-1H-pyrazol-4-yl)pyridin-4-yloxy)phenyl)urea, 1-(3-ethyl-1-(3-(hydroxymethyl)phenyl)-1H-pyrazol-5-yl)-3-(2-fluoro-4-(2-(1-methyl-1H-pyrazol-4-yl)pyridin-4-yloxy)phenyl)urea, 1-(1-(3-cyanophenyl)-3-methyl-1H-pyrazol-5-yl)-3-(2-fluoro-4-(2-(1-methyl-1H-pyrazol-4-yl)pyridin-4-yloxy)phenyl)urea, 1-(3-(3-cyanophenyl)-1-isopropyl-1H-pyrazol-4-yl)-3-(2-fluoro-4-(2-(1-methyl-1H-pyrazol-4-yl)pyridin-4-yloxy)phenyl)urea, 1-(2-fluoro-4-(2-(1-methyl-1H-pyrazol-4-yl)pyridin-4-yloxy)phenyl)-3-(1-(3-fluorophenyl)-3-isopropyl-1H-pyrazol-5-yl)urea, 1-(3-tert-butyl-1-phenyl-1H-pyrazol-5-yl)-3-(2,3-difluoro-4-(2-(1-methyl-1H-pyrazol-4-yl)pyridin-4-yloxy)phenyl)urea, 1-(2,3-difluoro-4-(2-(1-methyl-1H-pyrazol-4-yl)pyridin-4-yloxy)phenyl)-3-(3-(2-fluorophenyl)-1-methyl-1H-pyrazol-5-yl)urea, 1-(1,3-diphenyl-1H-pyrazol-5-yl)-3-(2-fluoro-4-(2-(1-methyl-1H-pyrazol-4-yl)pyridin-4-yloxy)phenyl)urea, 1-(2,3-difluoro-4-(2-(1-methyl-1H-pyrazol-4-yl)pyridin-4-yloxy)phenyl)-3-(1,3-diphenyl-1H-pyrazol-5-yl)urea, and 1-(5-chloro-2-phenylphenyl)-3-(2-fluoro-4-(2-(1-methyl-1H-pyrazol-4-yl)pyridin-4-yloxy)phenyl)urea.

### 1.2 Methods

### 1.2a Methods of Protein Modulation

The invention includes methods of modulating kinase activity of a variety of kinases, e.g. C-Abl kinase, bcr-Abl kinase, VEGFR-2 kinase mutants, c-Met, the HER family of kinases, FGFR, Flt-3, c-Kit, PDGFRα, PDGFRβ and the Raf family of kinases. The kinases may be wildtype kinases, oncogenic forms thereof, aberrant fusion proteins thereof or polymorphs of any of the foregoing. The method comprises the step of contacting the kinase species with compounds of the invention and especially those set forth in sections section 1. The kinase species may be activated or unactivated, and the species may be modulated by phosphorylations, sulfation, fatty acid acylations glycosylations, nitrosylation, cystinylation (i.e. proximal cysteine residues in the kinase react with each other to form a disulfide bond) or oxidation. The kinase activity may be selected from the group consisting of catalysis of phospho transfer reactions, inhibition of phosphorylation, oxidation or nitrosylation of said kinase by another enzyme, enhancement of dephosphorylation, reduction or denitrosylation of said kinase by another enzyme, kinase cellular localization, and recruitment of other proteins into signaling complexes through modulation of kinase conformation.

### 1.2b Treatment Methods

The methods of the invention also include treating individuals suffering from a condition selected from the group consisting of cancer, hyperproliferative diseases, metabolic diseases, neurodegenerative diseases or diseases characterized by angiogenesis. These methods comprise administering to such individuals compounds of the invention, and especially those of section 1, said diseases including, but not limited to, solid tumors, malignant melanomas, glioblastomas, ovarian cancer, pancreatic cancer, prostate cancer, lung cancers, breast cancers, kidney cancers, hepatic cancers, cervical carcinomas, metastasis of primary tumor sites, myeloproliferative diseases, chronic myelogenous leukemia, leukemias, papillary thyroid carcinoma, non-small cell lung cancer, mesothelioma, hypereosinophilic syndrome, gastrointestinal stromal tumors, colonic cancers, ocular diseases characterized by hyperproliferation leading to blindness including various retinopathies, diabetic retinopathy and age-related macular degeneration and hyperosinophilic syndrome, rheumatoid arthritis, asthma, chronic obstructive pulmonary disorder, mastocytosis, mast cell leukemia, a disease caused by c-Abl kinase, oncogenic forms thereof, aberrant fusion proteins thereof and polymorphs thereof, a disease caused by a c-Kit kinase, oncogenic forms thereof, aberrant fusion proteins thereof and polymorphs thereof, a disease caused by a c-Met kinase, oncogenic forms thereof, aberrant fusion proteins thereof and polymorphs thereof or a disease caused by a Raf kinase, oncogenic forms thereof, aberrant fusion proteins thereof and polymorphs thereof. The administration method is not critical, and may be from the group consisting of oral, parenteral, inhalation, and subcutaneous.

### 1.3 Pharmaceutical Preparations

The compounds of the invention, especially those of Section 1 may form a part of a pharmaceutical composition by combining one or more such compounds with a pharamaceutically acceptable carrier. Additionally, the compositions may include an additive selected from the group consisting of adjuvants, excipients, diluents, and stablilizers.

### Section 2. Synthesis of compounds of the present invention

The compounds of the invention are available by the procedures and teachings of WO 2006/071940, incorporated by reference, and by the general synthetic methods illustrated in the Schemes below and the accompanying examples.

As indicated in Scheme 1, ureas of general formula 1 can be readily prepared by the union of amines of general formula 2 with isocyanates 3 or isocyanate surrogates 4 (trichloroethyl carbamates) or 5 (isopropenyl carbamates). Preferred conditions for the preparation of compounds of general formula 1 involve heating a solution of 4 or 5 with 2 in the presence of a tertiary base such as diisopropylethylamine, triethylamine or N-methylpyrrolidine in a solvent such as dimethylformamide, dimethylsulfoxide, tetrahydrofuran or 1,4-dioxane at a temperature between 50 and 100 °C for a period of time ranging from 1 hour to 2 days.

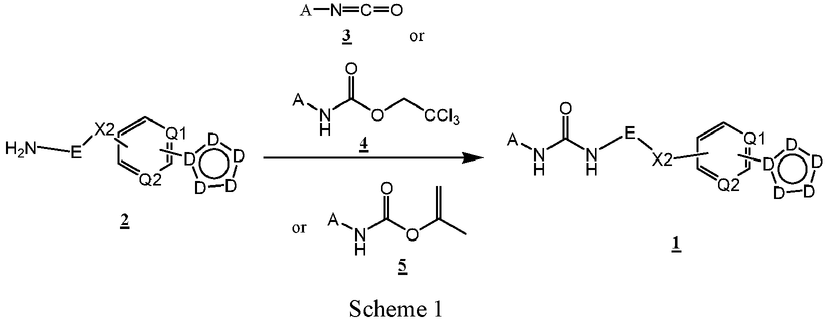

As shown in Scheme 2, isocyanates 3 can be prepared from amines A-NH₂ 6 with phosgene, or a phosgene equivalent such as diphosgene, triphosgene, or N,N-dicarbonylimidazole. Trichloroethyl carbamates 4 and isopropenyl carbamates 5 are readily prepared from amines A-NH₂ (6) by acylation with trichloroethyl chloroformate or isopropenyl chloroformate by standard conditions familiar to those skilled in the art. Preferred conditions for the preparation of 4 and 5 include include treatment of compound 6 with the appropriate chloroformate in the presence of pyridine in an aprotic solvent such as dichloromethane or in the presence of aqueous hydroxide or carbonate in a biphasic aqueous/ethyl acetate solvent system.

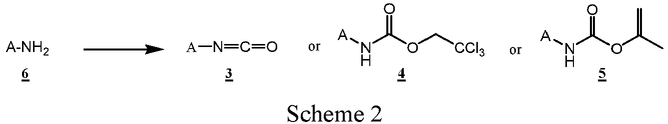

Additionally, compounds of formula 1 can also be prepared from carboxylic acids 7 by the intermediacy of in-situ generated acyl azides (Curtius rearrangement) as indicated in Scheme 3. Preferred conditions for Scheme 3 include the mixing of acid 7 with amine 2 and diphenylphosphoryl azide in a solvent such as 1,4-dioxane or dimethylformamide in the presence of base, such as triethylamine, and raising the temperature of the reaction to about 80-120 °C to affect the Curtius rearrangement.

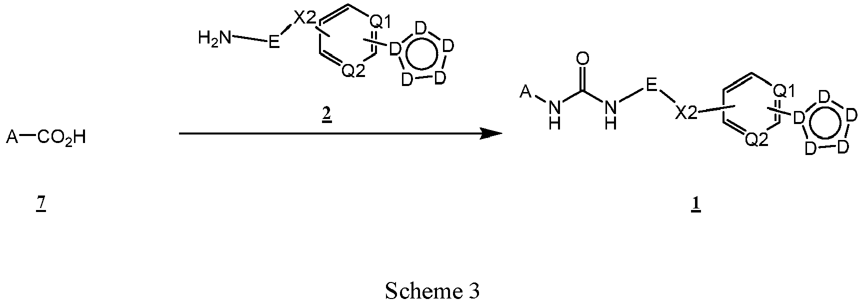

Many methods exist for the preparation of amines A-NH₂ 6 and acids A-CO₂H 7, depending on the nature of the A-moiety. Many such methods have been described in detail in WO 2006/071940, and are incorporated by reference here. Preferred synthetic methods are outlined in the following Schemes for the non-limiting examples wherein A is a 1-substituted-pyrazole (optionally substituted by R2) or A and A1 are linked by C-C bond.

As illustrated in Scheme 4, A1-substituted, pyrazole amines 10 (a preferred aspect of A-NH2 6, Scheme 2) are available by the condensation of hydrazines 8 and beta-keto nitriles 9. Preferred conditions for this transformation are by heating in ethanolic HCl. Hydrazines 8 are in turn available by the diazotization of amines 11 followed by reduction or, alternately from the hydrolysis of hydrazones 13 obtained by the palladium mediated coupling of benzophenone hydrazone with compounds of formula A1-X 12, wherein X represents a halogen or triflate moiety.

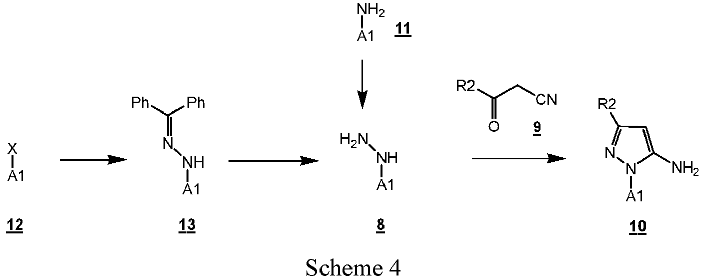

Another preferred method for constructing A1-substituted pyrazoles is illustrated by the general preparation of pyrazole acid 16 (Scheme 5), an aspect of A-CO₂H 7 (Scheme 3). As indicated in Scheme 5, the union of a pyrazole 5-carboxylic ester 14 with A1-X 12, wherein X represents a halide, triflate, or boronic acid suitable for direct transition metal-catalyzed couplings with pyrazoles 14, provides A1-substituted pyrazole esters 15. Preferred conditions for such transformations involve mixing a boronic acid 12 [X= B(OH)₂] and ethyl esters, benzyl esters, or tert-butyl esters 14 in dichloromethane with copper acetate and pyridine in the presence of crushed molecular sieves, with or without heating. The esters 15 in turn can be converted to acids 16 using conditions familiar to those skilled in the art.

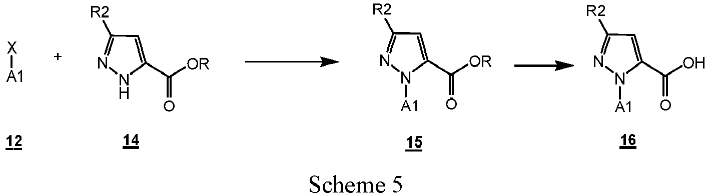

A non-limiting illustration of general Scheme 5 is found in Scheme 6, wherein pyrazole ester 17 is combined with boronic acid 18 in the presence of copper(II) acetate and pyridine to provide the N-aryl pyrazole 19. Saponification of ester 19 using lithium hydroxide or other equivalent methods provides acid 20, an example of general intermediate 7, above.

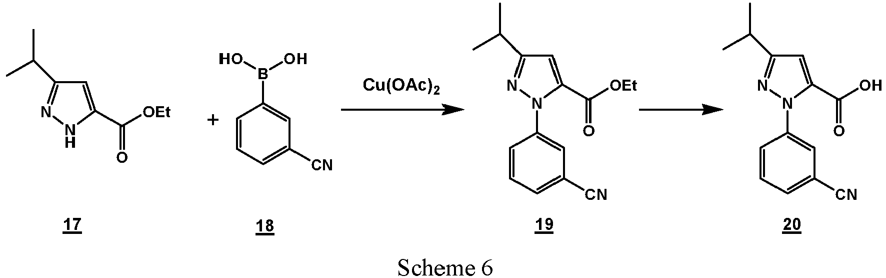

The general synthesis of intermediates useful for the construction of compounds of formula 1 wherein A and A1 are linked by a C-C bond is shown in Scheme 7. In this case, palladium catalyzed reactions (for example, Suzuki or Stille reactions) of A1-X 12 with a complementary component 21 or 22 provides compounds 23 or 24, examples of general intermediates A-NH₂ 6 or A-CO₂H 7, respectively. In this synthetic sequence, the X- groups on the reactants 12 and 21 or 22 are moieties that undergo transition metal catalyzed cross coupling reactions, such as halides or triflates and boronic acids or esters, stannanes, silanes, organozincs or other organometallic moieties known by those skilled in the art to be suitable substrates for such processes. The X-groups in Scheme 7 are complementary moieties for cross coupling processes such that when A1-X 12 is a halide or triflate, A-X 21 or A-X 22 will be a complementary organometallic, such as a stannane or the like or a boronic acid or ester. Likewise, if A1-X 12 is an organometallic reagent or a boronic acid or ester, A-X will be a halide or triflate.

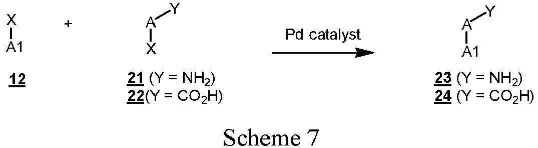

Within Scheme 7, it will be understood by those skilled in the art that there are additional synthetic equivalents for the Y-groups of 21 and 22 that can be used interchangeably with NH₂ and CO₂H with the addition of additional transforming steps. For example, the Y group of 21 might also be a protected amino group such as N-Boc or a surrogate amino group such as nitro that would give rise to compounds of formula 23 after acidic hydrolysis or reduction respectively. Similarly, it will be recognized that the Y group of 22 might also be an ester which could be hydrolyzed to an acid of formula 24 by standard synthetic methods.

A non limiting example of Scheme 7 is illustrated by the preparation of compound 28, an example of general intermediate A-CO₂H 7, above. Thus, iodoester 25 can be combined with phenylboronic acid 26 in the presence of a palladium catalyst to provide compound 27. Saponification of ester 27 provides acid 28, an example of general intermediate A-CO₂H 7, above.

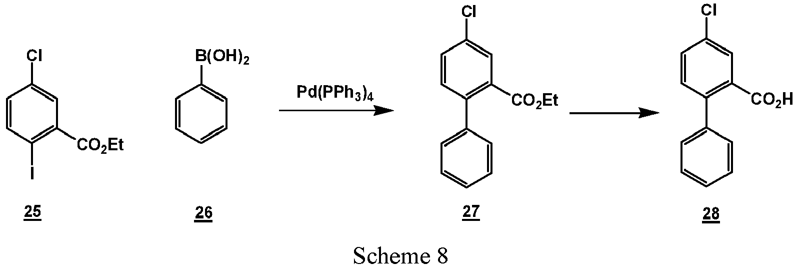

By analogy to Schemes 1 and 2 above, it will be recognized by those skilled in the art that the compounds of formula 1 can also be prepared by the union of amines A-NH₂ 6 with isocyanates 29 (Scheme 9). Isocyanates 29 can be prepared from general amines 2 by standard synthetic methods. Suitable methods for example, include reaction of 2 with phosgene, or a phosgene equivalent such as diphosgene, triphosgene, or N,N-dicarbonylimidazole. In addition to the methods above for converting amines 2 into isocynates 29, the isocyanates 29 can also be prepared *in situ* by the Curtius rearrangement and variants thereof. Those skilled in the art will further recognize that isocycanates 29 need not be isolated, but may be simply generated in situ. Accordingly, acids 30 can be converted to compounds of formula 1 either with or without isolation of 29. Preferred conditions for the direct conversion of acids 30 to compounds of formula 1 involve the mixing of acid 30, amine A-NH₂ 6, diphenylphosphoryl azide and a suitable base, for example diisopropylethylamine, in an aprotic solvent, for example dioxane. Heating said mixture to a temperature of between 80 and 120 °C provides the compounds of formula 1.

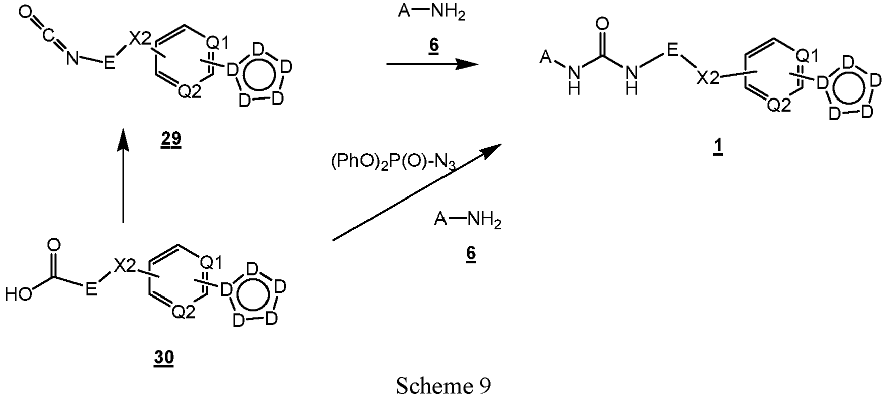

Additionally, compounds of formula 1 can also be prepared from amines 2 by first preparing stable isocyanate equivalents, such as carbamates (Scheme 10). Especially preferred carbamates include trichloroethyl carbamates (31) and isopropenyl carbamates (32) which are readily prepared from amine 2 by acylation with trichloroethyl chloroformate or isopropenyl chloroformate respectively using standard conditions familiar to those skilled in the art. Further reaction of carbamates 31 or 32 with amines A-NH₂ 6 provides compounds of formula 1. Those skilled in the art will further recognize that certain carbamates can also be prepared from acids 30 by Curtius rearrangement and trapping with an alcoholic co-solvent. For example, treatment of acids 30 (Scheme 9) with diphenylphosphoryl azide and trichloroethanol at elevated temperature provide carbamates of formula 31.

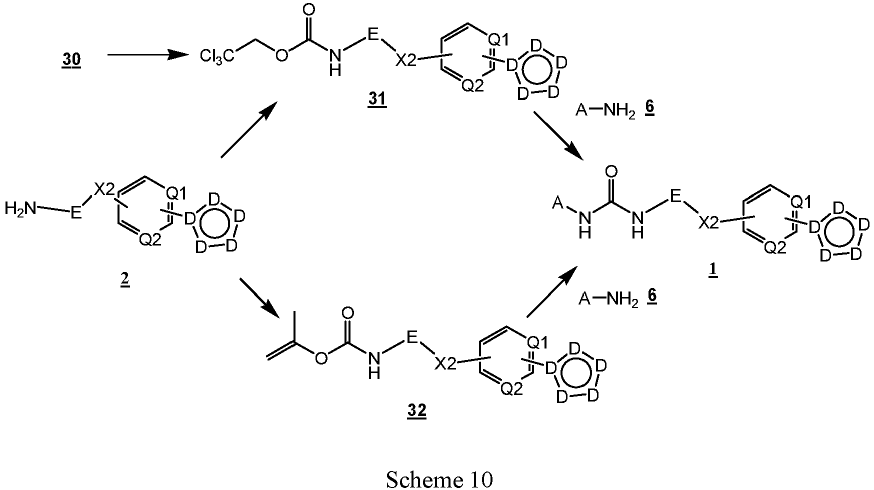

Amines 2 useful for the invention can be synthesized according to methods commonly known to those skilled in the art. Amines of general formula 2 contain three rings and can be prepared by the stepwise union of three monocyclic subunits as illustrated in the following non-limiting Schemes. Scheme 11 illustrates one mode of assembly in which an E-containing subunit 33 is combined with the central six-membered-ring-containing subunit 34 to provide the bicyclic intermediate 35. In one aspect of Scheme 11, the "M" moiety of 33 represents a hydrogen atom of a heteroatom on the X2 linker that participates in a nucleophilic aromatic substitution reaction with monocycle 34. Such reactions may be facilitated by the presence of bases (for example, potassium tert-butoxide), thus M may also represent a suitable counterion (for example potassium, sodium, lithium, or cesium) within an alkoxide, sulfide or amide moiety. Alternately, the "M" group can represent a metallic species (for example, copper, boron, tin, zirconium, aluminum, magnesium, lithium, silicon, etc.) on a carbon atom of the X2 moiety that can undergo a transition-metal-mediated coupling with monocycle 34.

The "Y" group of monocyclic species 33 is an amine or an amine surrogate, such as an amine masked by a protecting group ("P" in formula 36), a nitro group, or a carboxy acid or ester that can be used to prepare an amine via known rearrangement. Examples of suitable protecting groups "P" include but are not limited to tert-butoxycarbonyl (Boc), benzyloxycarbonyl (Cbz), and acetamide. In the instances wherein the "Y"-group of intermediate 33 is not an amine, the products of Scheme 11 will be amine surrogates such as 36 or 37 that can be converted to amine 2 by a deprotection, reduction or rearrangement (for example, Curtius rearrangement) familiar to those skilled in the art.

In these instances, the "LG" of monocycle 34 represents a moiety that can either be directly displaced in a nucleophilic substitution reaction (with or without additional activation) or can participate in a transition-mediated union with fragment 33. The W group of monocycle 34 or bicycle 35 represents a moiety that allows the attachment of a 5-membered heterocycle. In one aspect, the "W" group represents a halogen atom that will participate in a transition-metal-mediated coupling with a pre-formed heterocyclic reagent (for example a boronic acid or ester, or heteroaryl stannane) to give rise to amine 2. In another aspect, the "W" group of 34 and 35 represents a functional group that can be converted to a five-membered heterocycle by an annulation reaction. Non-limiting examples of such processes would include the conversion of a cyano, formyl, carboxy, acetyl, or alkynyl moiety into a five-membered heterocycle. It will be understood by those skilled in the art that such annulations may in fact be reaction sequences and that the reaction arrows in Scheme 11 may represent either a single reaction or a reaction sequence. Additionally, the "W" group of 35 may represent a leaving group (halogen or triflate) that can be displaced by a nucleophilic nitrogen atom of a pyrazole or triazole ring.

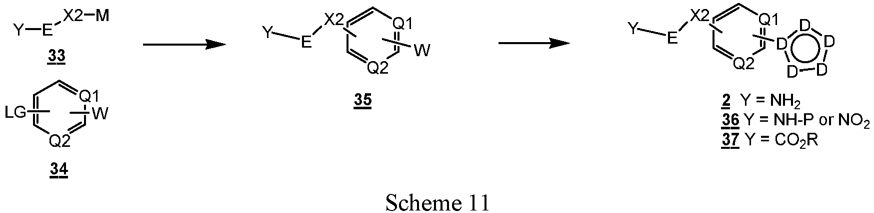

Some non-limiting examples of general Scheme 11 are illustrated in the Schemes below. Scheme 12 illustrates the preparation of pyrazole 42, an example of general amine 2. In Scheme 12, commercially available 3-fluoro-4-aminophenol is reacted with potassium tert-butoxide and 2,4-dichloropyridine 39 to provide chloropyridine 40. The preferred solvent for this transformation is dimethylacetamide at a temperature between 80 and 100 °C. Subsequent union of chloropyridine 40 with commercially available pyrazole-4-boronic acid pinacol ester 41 in the presence of a palladium catalyst, preferably palladium tetrakis(triphenylphosphine), provides amine 42.

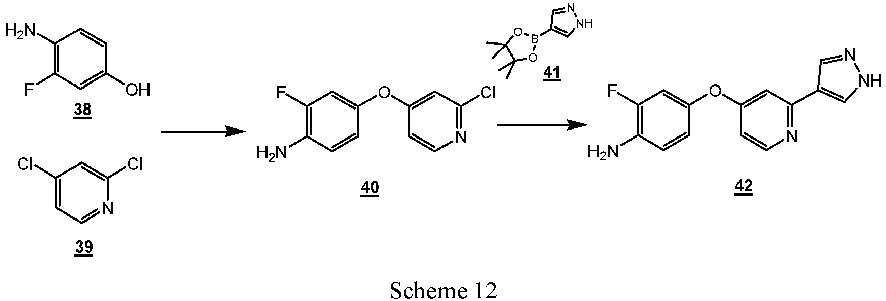

Scheme 13 illustrates the preparation of amine 48, also an example of general amine 2. In this example, the chloropyridine 43 is representative of general intermediate 34 (Scheme 11) wherein the "W" group of 34 is a tert-butoxycarbonyl moiety. The union of chloropyridine 43 with phenol 38 provides ether 44, an example of general intermediate 35 (Scheme 11). Introduction of a benzylcarbamate (Cbz) protecting group provides compound 45. The ester of 45 can be reduced using diisobutylaluminum hydride to provide aldehyde 46, also an example of general intermediate 35 (Scheme 11). Conversion of aldehyde 46 into isoxazole 47 is accomplished by treatment with tosylmethyl isocyanide and potassium carbonate. Isoxazole 47 is an example of general intermediate 36 (wherein the "Y" group is -NH-Cbz). Hydrogenation of 47 provides amine 48, an example of general amine 2.

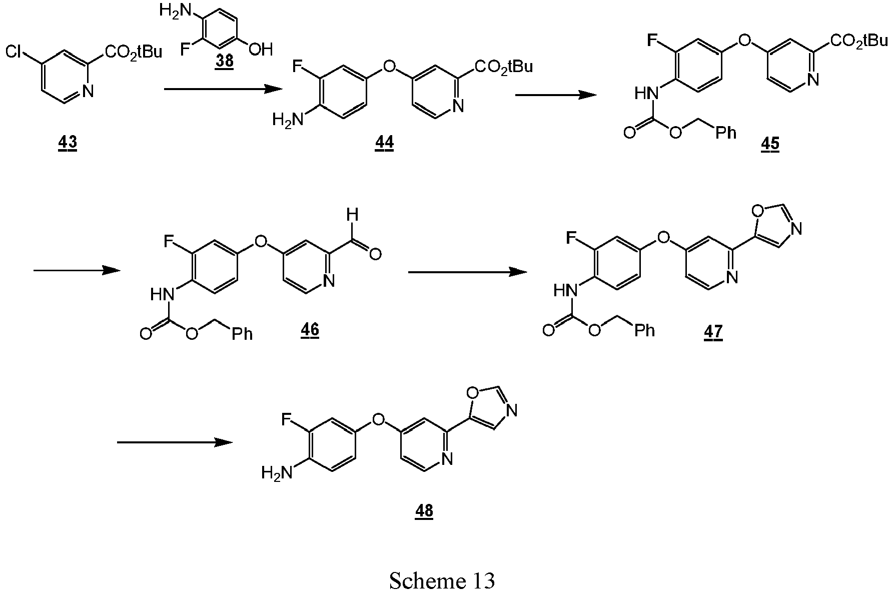

Scheme 14 illustrates another general method of preparing amines 2 by first attaching the 5-membered heterocycle to the central 6-membered ring (34). As before, the "LG" of monocycle 34 represents a moiety that can either be directly displaced in a nucleophilic substitution reaction (with or without additional activation) or can participate in a transition-mediated union with fragment 33. The "W" group of monocycle 34 represents a moiety that allows the attachment of a 5-membered heterocycle. In one aspect, the "W" group represents a halogen atom that will participate in a transition-metal-mediated coupling with a pre-formed heterocyclic reagent (for example, a boronic acid or ester, or heteroaryl stannane) to give rise to amine 2. In another aspect, the "W" group of 34 represents a functional group that can be converted to a five-membered heterocycle by an annulation reaction. After conversion of 34 to 49, the "LG" moiety can be replaced with an "X2" linkage to the E-sub-unit to provide the tricylic amine 2, as described above in Scheme 11. Those skilled in the art will recognize that amines 2 may be accessed directly from the union of 49 and 33 or may arise indirectly via the intermediacy of 36 or 37, as described previously.

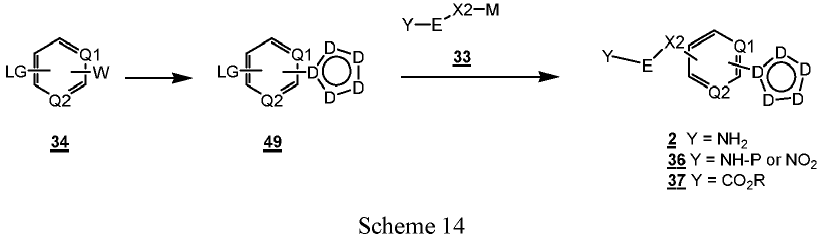

A specific example of Scheme 14 is illustrated by the preparation of amine 55 in Scheme 15. Thus, commercially available pyrimidine 50, an example of general intermediate 34, undergoes a palladium-catalyzed coupling with the commercially available pyrazole boronate 51 to provide the bicycle 52, an example of general intermediate 49. Oxidation of the sulfide moiety of 52 (The "LG" group of general intermediate 49) with m-chloroperbenzoic acid further activates this moiety toward nucleophilic displacement and gives rise to intermediate 53. Treatment of sulfone 53 with phenol 54 in the presence of a base provides tricylic amine 55, an example of general amine 2. Preferred bases for the later transformation include potassium carbonate and potassium tert-butoxide in polar aprotic solvents such as dimethylformamide or dimethylacetamide.

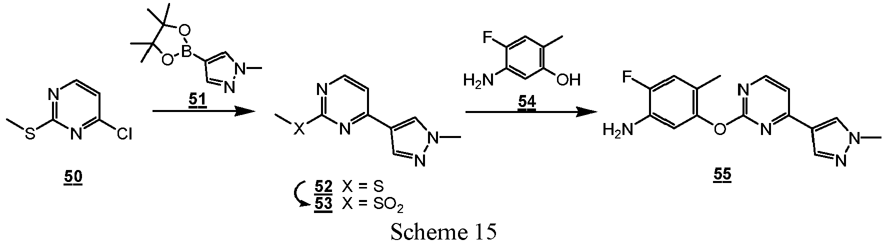

Additional examples of general amines of formula 2 can be prepared as illustrated in Scheme 16. Thus, by analogy to Scheme 12, the general intermediate 40 can be converted by palladium-mediated Stille-coupling into oxazoles 57 or 59 by reaction with the tributylstannanes 56 (see: Cheng et al., *Biorg. Med. Chem Lett.,* 2006, 2076) or 58 (commercially available, Aldrich). In a similar fashion, commercially available boronic acid 60 (Anichem) can be converted into oxazole 61 via Suzuki reaction employing standard conditions familiar to those skilled in the art. Similarly, isoxazoles 63 and 65 can be obtained by the palladium-catalyzed reaction of 40 with 4-isoxazoleboronic acid pinacol ester 62 (commercially available, Frontier Scientific) or tributylstannane 64 (see: Sakamoto, et al. Tetrahedron, 1991, 5111).

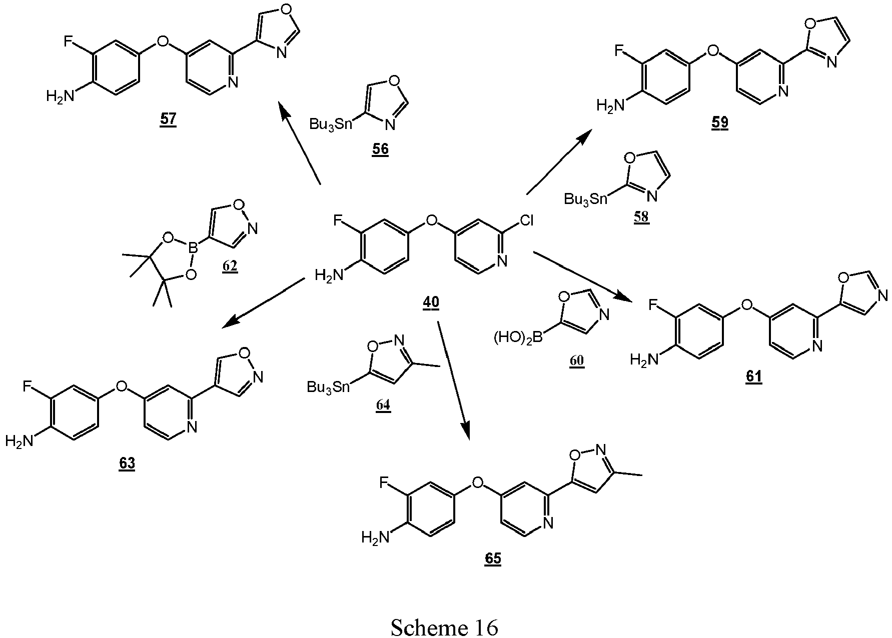

As an extension of Schemes 11, 12 and 16, amines of general formula 2 containing an isothiazole ring can also be prepared by the methods described above. Scheme 17 shows a non-limiting example wherein a palladium-catalyzed Stille reaction of trimethylstannane 66 (see: Wentland, et al. J. Med. Chem., 1993, 1580) with 40 can provide isothiazole 67. In a similar fashion, palladium-catalyzed Suzuki-cross coupling between 40 and the boronate ester 68 (see: Blackaby, et al., US 7030128) gives rise to isothiazole amine 69.

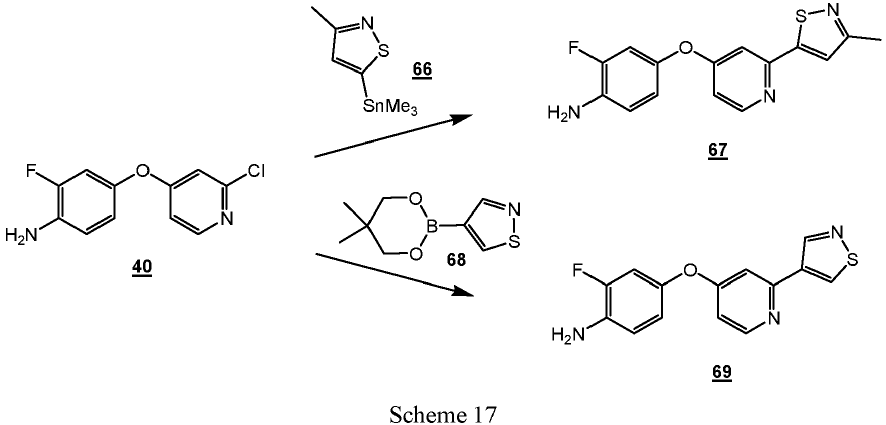

Scheme 18 illustrates non-limiting examples of Scheme 11 wherein the "W" group is a leaving group for nucleophilic aromatic substitution. Thus, some amines of general formula 2 in which the 5-membered heterocycle is a nitrogen-linked pyrazole (73) or triazole (74-77) can be prepared from general intermediate 40 by reaction with pyrazole (70) or triazoles (71 and 72). Preferred conditions for such transformations include the use of polar aprotic solvents such as 1-methyl-2-pyrrolidinone, dimethylacetamide, or dimethylsulfoxide in the presence of non-nucleophilic bases such as potassium carbonate, sodium hydride, 1,8-diaza-bicyclo[5.4.0]undec-7-ene (DBU), and the like. Preferred temperatures for such transformations are from ambient temperature up to about 250 °C and may optionally include the use of microwave irradiation or sonication.

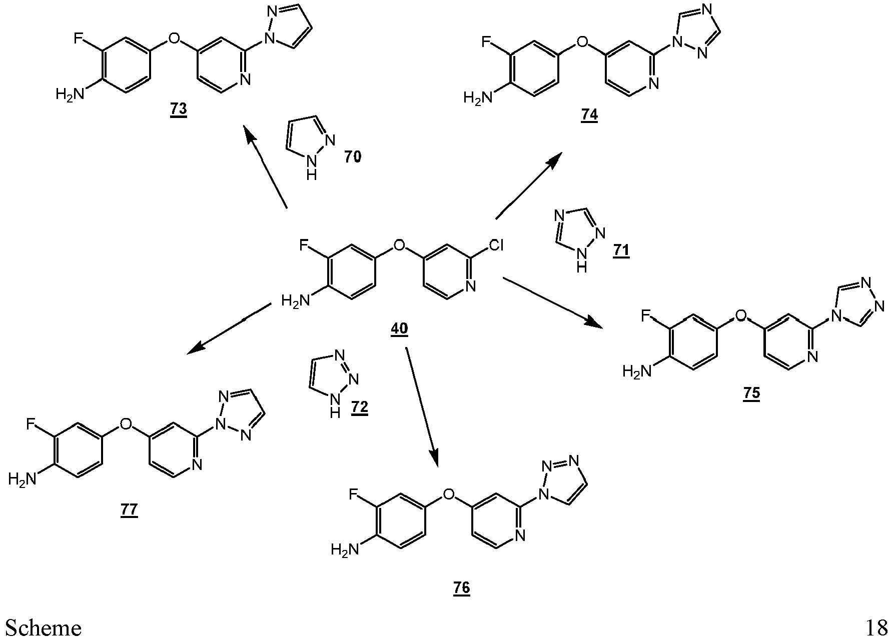

Scheme 19 illustrates the preparation of amine 79, a non-limiting example of a general amine of formula 2 bearing a C-linked 1,2,3-triazole. Conversion of chloropyridine 40 into alkyne 78 can be accomplished by Sonogashira cross-coupling with trimethylsilylacetylene, followed by aqueous hydrolysis of the trimethylsilyl group. Reaction of alkyne 78 with trimethylsilyl azide at elevated temperature affords the triazole 79 after aqueous work-up (see for example, Kallander, et. al, J Med. Chem., 2005, 5644, and references therein).

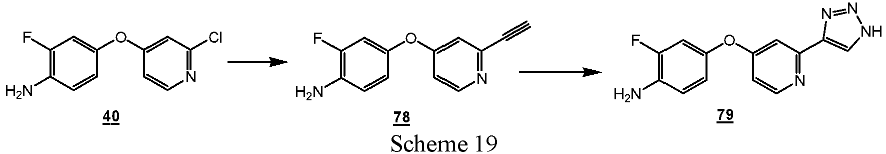

Those skilled in the art will recognize that the specific methods disclosed in Schemes 16-19 for the conversion of intermediate 40 to general amine 2 will be applicable to the conversion of intermediate 50 to additional examples of general amine 2 by the sequence of Scheme 15. Additional synthetic methods for the preparation of compounds of formula 1 are found in the following examples.

### Section 3. Examples

General method A: To a solution of the starting pyrazole amine (1 eq) in EtOAc were added 2,2,2-trichloroethylchloroformate (1.1 eq) and saturated NaHCO₃ (2-3 eq) at 0 °C. After stirring for 3h at RT, the layers were separated and the aqueous layer extracted with EtOAc. The combined organic extracts were washed with brine, dried (Na₂SO₄) and concentrated under vacuum to yield the crude TROC carbamate of the pyrazole amine.

To the carbamate (1 eq) in DMSO were added diisopropylethylamine (2 eq), the appropriate amine (2 eq) and the mixture was stirred at 60 °C for 16h or until all the starting carbamate was consumed. Water was added to the mixture and the product was extracted with EtOAc (2x25 mL). The combined organic extracts were washed with brine solution, dried (Na₂SO₄) and concentrated to yield crude product, which was purified by column chromatography to yield the target compound.

General method B: To a suspension of the amine (usually 0.67 mmol) in EtOAc (2 mL) was added aqueous 1N NaOH. The reaction mixture was cooled to 0 °C and treated with isopropenyl chloroformate (0.1 mL, 0.94 mmol) over 30 sec. The reaction mixture was stirred for 15 min at 0 °C and 1h at RT. The reaction was poured into THF-EtOAc (1:1; 40 mL) and washed with H₂OO (2x10 mL) and brine (2x10 mL). The organics were dried (Na₂SO₄), concentrated and the residue purified via column chromatography or recrystallization to provide the target (prop-1-en-2-yl)carbamate. To the carbamate (usually 0.26 mmol) was added the appropriate amine (usually 0.26 mmol) in THF (2 mL) and 1-methylpyrrolidine (catalytic amount) at 60 °C for 18h. The mixture was diluted with CH₂Cl₂ (2 mL) and hexane (0.5 mL) solution, and stirred for 10 min. The resultant solid was filtered and dried.

General method C: To a stirring solution of the carboxylic acid (0.24 mmol) and TEA (1.2 mmol) in 1,4-dioxane (4.5 mL) at RT was added DPPA (0.29 mmol). After stirring for 0.5 h at RT, the appropriate amine (0.71 mmol) was added and the reaction was stirred with heating at 100 °C for 2h. The reaction was cooled to RT, diluted with brine (15 mL) and extracted with EtOAc (3x30 mL). The combined organic layers were dried (MgSO₄) and concentrated. The residue was purified by chromatography to afford the target compound.

General Method D: To a solution of amine (6.53 mmol) in ethyl acetate (20 mL) at RT was added a solution of sodium bicarbonate (11.90 mmol) in water (20 mL) and isopropenyl chloroformate (9.79 mmol). The resultant mixture was stirred for 3 h at RT. The organic layer was separated. The aqueous layer was extracted once with ethyl acetate. The combined organic extracts were washed with brine, dried (MgSO₄) and concentrated in vacuo. The residue was used without further purification or purified via recrystallization or chromatography to provide the corresponding prop-1-en-2-yl carbamate.

Example A1: A suspension of 3-fluoro-4-aminophenol (8.0 g, 63.0 mmol) in dimethylacetamide (80 mL) was de-gassed in vacuo and treated with potassium tert-butoxide (7.3 g, 65 mmol). The resultant mixture was stirred at RT for 30 min. 2,4-Dichloropyridine (8 g, 54 mmol) was added and the mixture was heated to 80 °C for 12 h. The solvent was removed under reduced pressure to give a residue which was partitioned between water and EtOAc (3 x 100 mL). The organic layers were washed with saturated brine, dried (MgSO₄), concentrated *in vacuo* and purified by silica gel column chromatography to give 4-(2-chloro-pyridin-4-yloxy)-2-fluoro-phenylamine (11 g, 86% yield). ¹H NMR (300 MHz, DMSO-d6), δ 8.24 (d, J = 5.7 Hz, 1 H), 7.00 (dd, J = 9.0, 2.7 Hz, 1 H), 6.89-6.73 (m, 4 H), 5.21 (br s, 2 H); MS (ESI) m/z: 239.2 (M+H+).

A solution of 4-(2-chloropyridin-4-yloxy)-2-fluorobenzenamine (3 g, 12.6 mmol), 1-methyl-3-(4,4,5,5-tetramethyl-[1,3,2]dioxaborolan-2-yl)-1H-pyrazole (5.2 g, 25.2 mmol), and Na₂CO₃ (2.7 g, 25.2 mmol) in DME (18 mL) and water (6 mL) was sparged with nitrogen for 20 min. Pd(PPh₃)₄ (729 mg, 0.63 mmol) was added and the resulting mixture was heated to 100 °C for 16 h. The solvent was removed under reduced pressure and the crude product was suspended in water and extracted with EtOAc. The organic layer was washed with brine, dried (Na₂SO₄), filtered and concentrated *in vacuo.* The residue was purified via silica gel chromatography to give 2-fluoro-4-(2-(1-methyl-1H-pyrazol-4-yl)pyridin-4-yloxy)benzenamine (2 g, 56 % yield). ¹H NMR (300 MHz, DMSO-d₆), δ 8.31 (d ,J = 5.7 Hz, 1 H), 8.21 (s, 1 H), 7.92 (s, 1 H), 7.12 (d, J = 2.4 Hz, 1 H), 6.96 (m, 1 H), 6.85-6.72 (m, 2 H), 6.56 (m, 1 H), 5.15 (s, 2 H), 3.84 (s, 3H); MS (ESI) m/z: 285.0 (M+H⁺).

Example A2: A solution of 1,3-difluoro-2-methyl-benzene (15 g, 0.12 mol) in H₂SO₄ (100 mL) was treated drop wise with 65% HNO₃ (11.4 g, 0.12 mol) at -10 °C and the resultant mixture was stirred for about 30 min. The mixture was poured into ice-water and extracted with ethyl acetate (3 x 200 mL). The combined organic layers were washed with brine, dried (Na₂SO₄) and concentrated *in vacuo* to give 1,3-difluoro-2-methyl-4-nitro-benzene (16 g, 78% yield) ¹H NMR (400MHz, CDCl₃): δ 7.80 (m, 1 H), 6.95 (m, 1 H), 2.30 (s, 3 H).

1,3-Difluoro-2-methyl-4-nitro-benzene (16 g, 0.092 mol), benzyl alcohol (10 g, 0.092 mol) and K₂CO₃ (25.3 g, 0.18 mol), were combined in DMF (300 mL) and heated to 100 °C overnight. The mixture was poured into water and extracted with ethyl acetate (3 x 200 mL). The combined organic layers were washed with brine, dried (Na₂SO₄), concentrated *in vacuo* and purified by silica gel chromatography to give 1-benzyloxy-3-fluoro-2-methyl-4-nitro-benzene (8 g, 33% yield). ¹H NMR (400MHz, DMSO-d₆): δ 8.04 (t, J = 8.8 Hz, 1 H), 7.30-7.46 (m, 5 H), 7.08 (d, J = 9.2 Hz, 1 H), 5.28 (s, 2 H), 2.13 (s, 3 H).

Using a procedure analogous to Example A3, 1-benzyloxy-3-fluoro-2-methyl-4-nitro-benzene (8 g, 0.031 mol) was hydrogenated to give 4-amino-3-fluoro-2-methylphenol (4.2 g, 96% yield). ¹H NMR (300MHz, DMSO-d₆): δ 8.61 (s, 1 H), 6.36 (m, 2 H), 4.28 (s, 2 H), 1.96 (s, 3 H); MS (ESI) m/z: 142.1 [M+H]⁺.

Potassium tert-butoxide (3.5 g, 31 mmol) was added to a solution of 4-amino-3-fluoro-2-methyl-phenol (4.2 g, 30 mmol) in dimethylacetamide. The mixture was stirred at RT for 30 min. A solution of 2,4-dichloropyridine (4.38 g, 30 mmol) in dimethylacetamide was added and the mixture was heated at 100 °C overnight. The reaction mixture was concentrated *in vacuo* and the residue was dissolved in ethyl acetate (200 mL) and filtered through silica gel. The filter cake was washed with ethyl acetate and the combined filtrates were concentrated *in vacuo* and purified by silica gel chromatography to give 4-(2-chloro-pyridin-4-yloxy)-2-fluoro-3-methyl-phenylamine (3.2 g, 42% yield). ¹H NMR (400MHz, DMSO-d₆): δ 8.21 (d, J = 6.4 Hz, 1 H), 6.84 (d, J = 2.0 Hz, 1 H), 6.81 (dd, J = 5.6, 2.4 Hz, 1 H), 6.67-6.65 (m, 2 H), 5.13 (s, 2 H), 1.91 (s, 3 H); MS (ESI): m/z 253.2 [M+H]⁺.

Using a procedure analogous to Example A3, 4-(2-chloro-pyridin-4-yloxy)-2-fluoro-3-methyl-phenylamine (1.0 g, 3.3 mmol), 1-methyl-4-(4,4,5,5-tetramethyl-[1,3,2]dioxaborolan-2-yl)-1H-pyrazole (1 g, 4.8 mmol), Na₂CO₃ (0.84 g, 6.6 mmol) and Pd(PPh₃)₄ (0.25 g, 0.2 mmol) were combined to give 2-fluoro-3-methyl-4-[2-(1-methyl-1H-pyrazol-4-yl)-pyridin-4-yloxy]-phenylamine (0.74 g, 75% yield). ¹H NMR (400 MHz, DMSO-d₆): δ 8.27 (d, J = 6.4 Hz, 1 H), 8.18 (s, 1 H), 7.90 (s, 1 H), 7.07 (s, 1 H), 6.68-6.61 (m, 2 H), 6.45 (dd, J = 5.6, 2.4 Hz, 1 H), 5.06 (s, 2 H), 3.82 (s, 3 H), 1.95 (s, 3H); MS (ESI) m/z: 299.2 [M+H]⁺.

Example A3: 1,2,3-Trifluoro-4-nitro-benzene (30 g, 0.17 mol), benzyl alcohol (18.4 g, 0.17 mol) and K₂CO₃ (35 g, 0.25 mol) were combined in DMF (300 mL) and were stirred at RT for 8 h. Water (300 mL) was added, and the mixture was extracted with EtOAc (3x500 mL). The combined organic layers were washed with brine, dried (MgSO₄), concentrated *in vacuo* and purified by column chromatography on silica gel to give 1-benzyloxy-2,3-difluoro-4-nitro-benzene (16 g, 36% yield). ¹HNMR (400 MHz, DMSO-*d₆): δ* 8.06 (m, 1 H), 7.49-7.30 (m, 6 H), 5.37 (s, 2 H).

A solution of 1-benzyloxy-2,3-difluoro-4-nitro-benzene (14 g, 52.8 mmol) in MeOH (200 mL) was stirred with Pd/C (10%, 1.4 g, 1.3 mmol) under a hydrogen atmosphere (30 psi) for 2 h. The catalyst was removed by filtration, and the filtrate was concentrated in vacuo to afford 4-amino-2,3-difluorophenol (7 g, 92.1% yield). ¹H NMR (400 MHz, DMSO-d₆): δ 9.05 (s, 1 H), 6.45 (t, J = 8.8 Hz, 1 H), 6.34 (t, J = 9.2 Hz, 1 H), 4.67 (s, 2 H); MS (ESI) m/z: 146.1[M+H]⁺.

4-amino-2,3-difluorophenol (6 g, 41.4 mmol) and potassium tert-butoxide (4.9 g, 43.5 mmol) were suspended in DMAc (200 mL) and stirred at RT for 30 min under Ar atmosphere. 2,4-Dichloropyridine (6.1 g, 41.4 mmol) was added, and the resulting mixture was heated to 70 °C for 8 h. The reaction mixture was filtered, concentrated in *vacuo* and purified by silica gel chromatography to afford 4-(2-chloro-pyridin-4-yloxy)-2,3-difluoro-phenylamine (7 g, 66% yield). ¹H NMR (400 MHz, DMSO-d₆): δ 8.27 (d, J = 6.0 Hz, 1 H), 7.05 (s, 1 H), 6.95 (m, 1 H), 6.92 (m, 1 H), 6.62 (m, 1 H), 5.60 (s, 2 H); MS (ESI) m/z: 257.1 [M+H]⁺.

Nitrogen was bubbled though a solution of 4-(2-chloro-pyridin-4-yloxy)-2,3-difluoro-phenylamine (2 g, 7.8 mmol), 1-methyl-4-(4,4,5,5-tetramethyl-[1,3,2]dioxaborolan-2-yl)-1H-pyrazole (1.6 g, 7.8 mmol) and Na₂CO₃ (1.65 g, 15.6 mmol) in DME (12 mL) and H₂O (4 mL) for 20 min. Pd(PPh₃)₄ (450 mg, 0.4 mmol), was added and then resulting mixture was degassed *in vacuo,* blanketed with nitrogen and heated to 70 °C for 16 h. The reaction was concentrated to dryness under reduced pressure. The crude product was suspended in water and extracted with EtOAc (3 x 10 mL). The organic layer was washed with brine, dried (Na₂SO₄), concentrated *in vacuo* and purified by silica gel chromatography to give 2,3-difluoro-4-[2-(1-methyl-1H-pyrazol-4-yl)-pyridin-4-yloxy]-phenylamine (1.3 g, 55% yield). ¹H NMR (400 MHz, DMSO-*d₆*): *δ* 8.40 (d, *J* = 6.0 Hz, 1 H), 8.32 (s, 1 H), 8.02 (s, 1 H), 7.26 (s, 1 H), 6.96 (t, *J* = 8.8 Hz, 1 H), 6.71-6.68 (m, 2 H), 5.62 (s, 2 H), 3.92 (s, 3 H); MS (ESI) m/z: 303.2[M+H]⁺.

Example A4: Following the procedure of Lohse, et al. (Synlett. 1999, 1, 45-48), 4-(2-chloropyridin-4-yloxy)-2-fluorobenzenamine from Example A1 (0.842 g, 3.53 mmol), 1-propyl-4-(4,4,5,5-tetramethyl-1,3,2-dioxaborolan-2-yl)-1H-pyrazole (1.00 g, 4.24 mmol), K₂CO₃ (1.317 g, 9.53 mmol) and Pd(PPh₃)₄ (0.204 g, 0.176 mmol) were combined in DME (4.0 ml)/H₂O (4.80 ml). The headspace was degassed with Ar for 10 min. The reaction was then heated with stirring at 90 °C overnight. The completed reaction was cooled to RT, diluted with EtOAc and filtered through Celite® to remove Pd. The filtrate was washed with brine (2x), dried (MgSO₄), concentrated *in vacuo* and purified by flash column chromatography (100% hexanes to 100% EtOAc) to afford 2-fluoro-4-(2-(1-propyl-1H-pyrazol-4-yl)pyridin-4-yloxy)benzenamine (1.21 g, 110% yield) as a thick oil contaminated with EtOAc but used as obtained. ¹H NMR (400 MHz, DMSO-*d₆*): δ 8.32-8.31 (m, 1H), 8.27 (s, 1H), 7.95 (brs, 1H), 7.64-7.52 (m, 2H), 7.14 (brs, 1H), 6.98-6.95 (m 1H), 6.84-6.79 (m, 1H), 6.76-6.73 (m, 1H), 6.58-6.56 (m, 1H), 5.17 (s, 2H), 4.05-3.99 (m, 2H), 1.83-1.74 (m, 2H), 0.83-0.79 (m, 3H); MS (ESI) m/z: 313.2 (M+H⁺).

Example A5: Using a procedure analogous to Example A1, 2-fluoro-4-aminophenol (2.6 g, 24 mmol) and 2,4-dichloropyridine (2.88 g, 20 mol) were combined to provide 4-(2-chloropyridin-4-yloxy)-3-fluoroaniline (3.2 g, 67% yield). ¹HNMR (400 MHz, DMSO-*d*₆): δ 8.25 (d, *J* = 5.6Hz, 1 H), 6.99 (m, 1 H), 6.90 (m, 2 H), 6.50 (d, *J* = 1.6 Hz, 1 H), 6.41 (d, *J*= 10.4Hz, 1 H), 5.51 (s, 2 H); MS (ESI) m/z: 239.1 (M+H⁺).

Using a procedure analogous to Example A1, 4-(2-chloropyridin-4-yloxy)-3-fluoroaniline (3 g, 11.6 mmol), 1-methyl-4-(4,4,5,5-tetramethyl-1,3,2-dioxaborolan-2-yl)-1H-pyrazole (3.4 g, 16.4 mmol), Na₂CO₃ (2.7 g, 25.2 mmol) and Pd(Ph₃)₄ (1.5 g, 0.1 eq) were combined to give 3-fluoro-4-(2-(1-methyl-1H-pyrazol-4-yl)pyridin-4-yloxy)aniline (1.1 g, 34% yield). ¹H NMR (400 MHz, DMSO-*d₆*): *δ* (8.31 (d, *J*= 5.6 Hz, 1 H), 8.22 (s, 1 H), 7.93 (s, 1 H), 7.14 (s, 1 H), 6.98 (m, 1 H), 6.55-6.49 (m, 2 H), 6.42 (d, J = 7.2 Hz, 1 H), 5.44 (s, 2 H), 3.86 (s, 3 H); MS (ESI) m/z: (M+H⁺): 285.2.

Example A6: Pyrazole-4-boronic acid pinacol ester (0.85 g, 4.38 mmol), powdered K₂CO₃ (1.816 g, 13.14 mmol) and 2-iodopropane (0.547 ml, 5.48 mmol) were combined in MeCN (100 ml) and stirred with heating at 80 °C. After 18 h, the reaction was treated with additional 2-iodopropane (0.547 ml, 5.48 mmol) and heating at 80 °C continued. After another 48 h, the reaction was cooled to RT and filtered through a glass frit to remove the solids. The filtrate was concentrated to a residue, which was diluted with EtOAc, filtered again on a glass frit and concentrated. The crude product was purified by flash column chromatography (EtOAc/hexanes) to afford 1-isopropyl-4-(4,4,5,5-tetramethyl-1,3,2-dioxaborolan-2-yl)-1H-pyrazole (0.37 g, 36% yield) as a waxy solid. ¹H NMR (400 MHz, DMSO-*d₆*): δ 7.95 (s, 1H), 7.56 (s, 1H), 4.53-4.46 (m, 1H), 1.39-1.37 (m, 6H), 1.23 (s, 12H); MS (ESI) m/z: 237.3 (M+H⁺).

Using a procedure analogous to Example A1, 4-(2-chloropyridin-4-yloxy)-2-fluorobenzenamine from Example A1 (0.310 g, 1.299 mmol), 1-isopropyl-4-(4,4,5,5-tetramethyl-1,3,2-dioxaborolan-2-yl)-1H-pyrazole (0.368 g, 1.559 mmol), K₂CO₃ (0.485 g, 3.51 mmol) and Pd(PPh₃)₄ 0.075 g, 0.065 mmol) were combined and purified by flash column chromatography (EtOAc/CH₂Cl₂) to afford 2-fluoro-4-(2-(1-isopropyl-1H-pyrazol-4-yl)pyridin-4-yloxy)benzenamine (0.38 g, 94% yield) as an oil contaminated with EtOAc. ¹H NMR (400 MHz, DMSO-*d*₆): δ 8.32-8.30 (m, 2H), 7.94 (s, 1H), 7.18-7.17 (m, 1H), 6.98-6.94 (m, 1H), 6.84-6.80 (m, 1H), 6.76-6.75 (m, 1H), 6.57-6.55 (m, 1H), 5.15 (s, 1H), 4.54-4.47 (m, 1H), 1.43-1.41 (m, 6H); MS (ESI) m/z: 313.2 (M+H⁺).

Example A7: Using a procedure analogous to Example A1, 4-(2-chloropyridin-4-yloxy)-2-fluoroaniline from Example A1 (2 g, 8.4 mmol), 1-ethyl-4 -(4,4,5,5-tetramethyl-1,3,2-dioxaborolan-2-yl)-1H-pyrazole (3.7 g, 16.8 mmol), Na₂CO₃ (1.8 g, 16.8 mmol) and Pd(PPh₃)₄ (0.48 g) were combined to give 4-(2-(1-ethyl-1H-pyrazol-4-yl)pyridin-4-yloxy)-2-fluoroaniline (2.1 g, 84 % yield). ¹H NMR (400 MHz, DMSO-*d₆*): *δ* 8.37 (d, J = 6.0 Hz, 1 H), 8.33 (s, 1 H), 8.00 (s, 1 H), 7.21 (s, 1 H), 7.02 (dd, J = 12.0, 2.8 Hz, 1 H), 6.91-6.79 (m, 2 H), 6.63 (dd, J = 5.6, 2.4 Hz, 1 H), 5.22 (s, 2 H), 4.19 (q, J = 7.2 Hz, 2 H), 1.44 (t, J = 7.2 Hz, 3 H); MS (ESI) m/z: 299.1 [M + H]⁺.

Example A8: To a biphasic mixture of Example A2 (1.5 g, 4.96 mmol) in ethyl acetate (20 ml) and water (20.00 ml) was added NaHCO₃ (1.3 g, 15 mmol) and isopropenyl chloroformate (0.6 mL). The reaction was stirred at RT for 1 hour. The organic layer was separated and washed with brine, dried and evaporated to provide prop-1-en-2-yl 2-fluoro-4-(2-(1-methyl-1H-pyrazol-4-yl)pyridin-4-yloxy)phenylcarbamate which was used for the next reaction (0.92 g, 48% yield). MS (ESI) m/z: 387.1 (M+H⁺).

Example A9: 4-(2-Chloro-pyridin-4-yloxy)-2-fluorophenylamine from Example A1 (0.597 g, 2.5 mmol), 4-(4,4,5,5-tetramethyl-1,3,2-dioxaborolan-2-yl)-1H-pyrazole (0.728g, 3.75 mmol), Cs₂CO₃ (3.10 g, 9.5 mmol) and Pd(PPh₃)₄ (0.289 g, 0.25 mmol) were combined in DMF/H₂O (20 mL). The reaction mixture was degassed, blanketed with N₂ and heated at 90 °C overnight. The completed reaction was diluted with H₂O (5 mL) and extracted with EtOAc (3 x 50 mL). The combined organics were washed with brine (20 mL), dried (MgSO₄), concentrated *in vacuo* and purified by chromatography to afford 4-(2-(1H-pyrazol-4-yl)pyridin-4-yloxy)-2-fluorobenzenamine (0.56 g, 83% yield) as a light yellow solid. ¹H NMR (400 MHz, DMSO-*d₆*) *δ* 13.01 (s, 1 H), 8.38 (d, *J* = 5.6 Hz, 1 H), 8.35 (s, 1 H), 8.06 (s, 1 H), 7.29 (d, *J* =2.4 Hz, 1 H), 7.03 (dd, *J* = 11. 6, 2.4 Hz, 1 H), 6.89 (t, J = 8.8 Hz, 1 H), 6.84 (m, *J* = 8.4 Hz, 1 H), 6.60 (m, 1 H), 5.20 (s, 2 H); MS (ESI) *m*/*z*: 271.0 (M+H⁺).

Example A10: Methyl chloroformate (77.3 g, 0.82 mol) was added dropwise to a -10 °C solution of 2-chloro-4-fluorophenol (100g, 0.68 mol) and sodium hydroxide (32.8 g, 0.82 mol) in water (550 mL). After complete addition, the precipitated solid was collected by filtration and washed with water to give 2-chloro-4-fluorophenyl methyl carbonate (110 g, 79 % yield). ¹H NMR (300 MHz, DMSO-*d*₆): δ 7.62 (dd, *J*= 8.1, 2.7 Hz, 1 H), 7.50 (dd, *J* = 9.0, 5.4 Hz, 1 H), 7.30 (td, *J* = 8.1, 3.0 Hz, 1 H), 3.86 (s, 3 H); MS (ESI) m/z: 205.2 (M+H⁺).

To a suspension of 2-chloro-4-fluorophenyl methyl carbonate (110 g, 0.54 mol) in conc. H₂SO₄ (50 mL) was slowly added a mixture comprised of conc. H₂SO₄ (40 mL) and fuming HNO₃ (40.8 mL, 0.89 mol). The resultant mixture was stirred for 30 min at 0 °C. The reaction mixture was poured into ice water and the precipitated solid was collected by filtation and washed with water to give 2-chloro-4-fluoro-5-nitrophenyl methyl carbonate (120 g, 90% yield). ¹H NMR (400 MHz, DMSO-d₆): δ 8.45 (d, *J* = 7.2 Hz, 1 H), 8.12 (d, *J*= 10.8 Hz, 1 H), 3.89 (s, 3 H); MS (ESI) m/z: 250.1 (M+H⁺).

2-Chloro-4-fluoro-5-nitrophenyl methyl carbonate (120g 0.48mol) was combined with a solution of sodium hydroxide (22.7 g, 0.57 mol) in water (300 mL) and the resultant mixture was refluxed for 4h. The insoluble solids were removed by filtration and the filtrate was acidified with dilute HCl. The precipitated solid was collected by filtration and washed with water to give 2-chloro-4-fluoro-5-nitrophenol (90 g, 98% yield). ¹H NMR (400 MHz, DMSO-*d*₆): δ 11.18 (s ,1 H), 8.10 (d, *J* = 10.4 Hz, 1 H), 7.62 (d, *J* =7.2 Hz, 1 H); MS (ESI) m/z: 192.1 (M+H⁺).

2-chloro-4-fluoro-5-nitrophenol (85 g, 0.45 mol) and 10% Pd/C (25g, 0.023 mol) were combined in EtOH and hydrogenated (50 psi) for 12h. The reaction mixture was filtered. The filtrate was concentrated *in vacuo* and purified by silica gel chromatography to provide 3-amino-4-fluorophenol (40 g 70%, yield). ¹H NMR (400 MHz, DMSO-*d*₆): δ 8.87 (s, 1 H), 6.70 (dd, *J* = 11.2, 8.8 Hz, 1 H), 6.14 (dd, *J* = 7.8, 2.4 Hz, 1 H), 5.84 (m, 1 H), 4.92 (s, 2 H); MS (ESI) m/z: 128.2 (M+H⁺).

4-Chloro-2-methylsulfanyl-pyrimidine (1.4 g, 8.8 mmol), 1-methyl-4-(4,4,5,5-tetramethyl-[1,3,2]dioxaborolan-2-yl)-1H-pyrazole (2.0 g, 1.1 eq), Na₂CO₃ (2.8 g, 3 eq) and Pd(PPh₃)₄ (500 mg, 0.43 mmol) were combined in a solvent comprised of toluene/EtOH/H₂O (4/4/1, 20 mL). The reaction mixture was purged with argon and heated to 100 °C overnight. The reaction was filtered to remove insolubles and the filtrate was concentrated *in vacuo.* The residue was purified by silica gel chromatography to provide 4-(1-methyl-1H-pyrazol-4-yl)-2-(methylthio)pyrimidine contaminated with triphenylphoshine oxide (2.0 g, >100% yield). ¹H NMR (300 MHz, DMSO-*d*₆): δ 8.49 (d, *J* = 5.1 Hz, 1 H), 8.46 (s, 1 H), 8.12 (s, 1 H), 7.38 (d, *J* = 5.1 Hz, 1 H), 3.89 (s, 3 H), 2.52 (s, 3 H).

A solution of 4-(1-methyl-1H-pyrazol-4-yl)-2-methylsulfanyl-pyrimidine (2.0 g crude, 8.8 mmol) in dichloromethane (20 mL) was treated with m-CPBA (3.0 g, 17.4 mmol) portionwise at RT. The reaction was stirred 2h and was quenched with saturated aqueous NaS₂SO₃ (3 mL). The mixture was partitioned with saturated aq Na₂CO₃ and the organics were washed with brine, dried (Na₂SO₄), and concentrated to provide a mixture (2.0 g) of 2-methanesulfonyl-4-(1-methyl-1H-pyrazol-4-yl)-pyrimidine and 2-methanesulfinyl-4-(1-methyl-1H-pyrazol-4-yl)-pyrimidine with a molar ratio of 1:0.3. ¹H NMR (400 MHz, DMSO-*d*₆): δ 8.83 (d, *J* = 5.2 Hz, 1 H), 8.82 (d, *J* = 5.2 Hz, 0.24 H), 8.57 (s, 1 H), 8.57 (s, 0.24 H), 8.21 (s, 1 H), 8.21 (s, 0.23 H), 7.80 (d, *J* = 5.6 Hz, 1 H), 7.80 (d, *J*= 5.6 Hz, 0.25 H), 3.48 (s, 3 H), 2.88 (s, 0.7 H).

The above mixture of 2-methanesulfonyl-4-(1-methyl-1H-pyrazol-4-yl)-pyrimidine and 2-methanesulfinyl-4-(1-methyl-1H-pyrazol-4-yl)-pyrimidine (1 g, 4.2 mmol), 4-amino-3-fluoro-phenol (1.1 g, 8.6 mmol) and K₂CO₃ (1.2 g, 8.6 mmol) in DMF (10 mL) was heated at 100 °C for 12h. The reaction was partitioned between H₂O and EtOAc (3 x 50 mL). The combined organics were dried (Na₂SO4), concentrated *in vacuo* and chromatographed to 2-fluoro-5-(4-(1-methyl-1H-pyrazol-4-yl)pyrimidin-2-yloxy)benzenamine (402 mg). ¹H NMR (400 MHz, DMSO-d₆): δ 8.44 (d, *J*= 5.2 Hz, 1 H), 8.39 (s, 1 H), 8.07 (s, 1 H), 7.41 (d, *J* = 5.2 Hz, 1 H), 6.98 (t, *J* = 9.6 Hz, 1 H), 6.53 (dd, *J* = 5.6, 2.0 Hz, 1 H), 6.28 (d, *J* = 8.4 Hz, 1 H), 5.25 (br s, 2 H), 3.88 (s, 3 H). MS (ESI) m/z: 286.2 (M+H⁺).

Example A11: 4-Fluoro-2-methyl-phenol (25 g, 0.2 mol) was added to a solution of sodium hydroxide (9.7 g, 0.24 mol) in water (160 mL) and the resultant solution was cooled to 0 °C. Methyl chloroformate (24.2 g, 0.26 mol) was added dropwise at 0 °C. At the completion of the reaction, the pH was adjusted to pH 8 with saturated aqueous Na₂CO₃ and then mixture was extracted with ethyl acetate (3 x 300 mL). The combined organic extracts were washed with water and brine, dried (MgSO₄) and were concentrated under reduced pressure to provide carbonic acid 4-fluoro-2-methyl-phenyl ester methyl ester (30 g, 82% yield). ¹H NMR (300 MHz, DMSO-*d₆*): δ 7.22-7.13 (m, 2 H), 7.05 (m, 1 H), 3.81 (s, 3 H), 2.12 (s, 3 H).

To a solution of carbonic acid 4-fluoro-2-methyl-phenyl ester methyl ester (15 g, 81.5 mmol) in conc. sulfuric acid (100 mL) at 0 °C was added powdered KNO₃ (8.3 g, 82.2 mmol) in several portions. The reaction mixture was stirred for 1 hour at 0 °C and was then poured into ice water and extracted with ethyl acetate (3 x 100 mL). The extracts were washed with water and brine, dried (MgSO₄), concentrated *in vacuo* and purified by silica gel chromatography to provide carbonic acid 4-fluoro-2-methyl-5-nitrophenyl ester methyl ester (2.0 g, 11% yield). ¹H NMR (300 MHz, DMSO-*d*₆): δ 8.14 (d, *J* = 6.9, 1 H), 7.60 (d, *J* = 12.0 Hz, 1 H), 3.86 (s, 3 H), 2.25 (s, 3 H).

To a solution of aqueous sodium hydroxide (1.2 N, 20 mL, 24 mmol) was added 4-fluoro-2-methyl-5-nitro-phenyl ester methyl ester (2.0 g, 8.7 mmol), then the resultant mixture was refluxed for 2 hours. The reaction was cooled to RT and partitioned between EtOAc and water. The organic layer was washed with water and brine, dried (MgSO₄), and concentrated *in vacuo* to provide 4-fluoro-2-methyl-5-nitro-phenol (1.4 g, 93 % yield). ¹H NMR (300 MHz, DMSO-*d*₆) δ 10.33 (s, 1 H), 7.45 (d, *J* = 6.6, 1 H), 7.32 (d, *J* = 12.3 Hz, 1 H), 2.19 (s, 3 H).

A mixture of 4-fluoro-2-methyl-5-nitro-phenol (1.4 g, 8.2 mmol) and 10% Pd/C (0.3 g, 20%/w) in MeOH (80 mL) was stirred under H₂ (30 psi) for 2h. The Pd/C was removed by filtration and the filtrate was concentrated to give 5-amino-4-fluoro-2-methyl-phenol (0.68 g, 62% yield). ¹H NMR (300 MHz, DMSO-*d*₆) δ 8.75 (s, 1 H), 6.62 (d, *J* = 12.0 Hz, 1 H), 6.21 (d, *J* = 8.1 Hz, 1 H), 4.69 (s, 2 H), 1. 93 (s, 3 H).

The mixture of 2-methanesulfonyl-4-(1-methyl-1H-pyrazol-4-yl)-pyrimidine and 2-methanesulfinyl-4-(1-methyl-1H-pyrazol-4-yl)-pyrimidine from Example A10 (1 g, 4.2 mmol), 5-amino-4-fluoro-2-methylphenol (1.2 g, 8.5 mmol) and K₂CO₃ (1.2 g, 8.6 mmol) were combined in DMF (10 mL) using a procedure analogous to Example A10 to provide 2-fluoro-4-methyl-5-(4-(1-methyl-1H-pyrazol-4-yl)pyrimidin-2-yloxy)benzenamine (420 mg). ¹H NMR (400 MHz, DMSO-*d*₆): δ 8.42 (d, *J*= 5.2 Hz, 1 H), 8.39 (s, 1 H), 8.07 (s, 1 H), 7.40 (d, *J* = 5.2 Hz, 1 H), 6.90 (d, *J* = 9.6 Hz, 1 H), 6.47 (d, *J* = 8.4 Hz, 1 H), 5.02 (br s, 2 H), 3.88 (s, 3 H), 1.88 (s, 3 H); MS (ESI) m/z: 300.2 (M+H⁺).

Example B1: Phenyl hydrazine and 4,4-dimethyl-3-oxopentanenitrile were combined according to literature procedures to yield 3-t-butyl-1-phenyl-1H-pyrazol-5-amine. See WO 2006/071940.

Example B2: Sodium metal (13.8 g, 0.5 mol) was added portion-wise to ice-cold anhydrous EtOH (700 mL). After complete dissolution of the Na, a mixture of 3-methylbutan-2-one (100 g, 1.16 mol) and oxalic acid diethyl ester (77 ml, 0.5 mol) was added drop-wise. The reaction mixture was stirred in an ice-salt bath and until TLC indicated completion of the reaction. Acetic acid (38.1 ml, 0.5 mol) was added and the mixture was stirred at RT for about 30 min. The reaction mixture was cooled in an ice-salt bath and treated with hydrazine hydrate (29.4 g, 0.5 mol). After complete addition, the mixture was warmed to RT and stirred until judged complete by TLC. The reaction mixture was concentrated under reduced pressure and re-dissolved in EtOAc. The EtOAc solution was washed with NaHCO₃, brine and water, dried (MgSO₄) and concentrated in vacuo. The resultant solid was washed with cold petroleum ether to give ethyl 3-isopropyl-1H-pyrazole-5-carboxylate (90 g, 42% yield, two steps) as an off-white solid. ¹H NMR (300 MHz, CDCl₃): δ 12.00 (s, 1 H), 6.57 (s, 1 H), 4.30 (q, *J* = 7.2 Hz, 2 H), 3.00 (m, 1 H), 1.46 (t, *J* = 7.2 Hz, 3 H), 1.28 (d, *J* = 6.8 Hz, 6 H); MS (ESI) m/z: 183.3 (M+H⁺).

A mixture of 3-cyanophenylboronic acid (0.423 g, 2.88 mmol), Cu(OAc)₂ (0.498 g, 2.74 mmol) and ethyl 3-isopropyl-1H-pyrazole-5-carboxylate (0.500 g, 2.74 mmol) in pyridine (4.0 ml) was heated at 80 °C for 4.5 h. The reaction was quenched with H₂O (30 mL) and extracted with EtOAc (3x40 mL). The combined organic layers were washed with brine (30 mL), dried (MgSO₄) and concentrated. The crude product was purified by chromatography to afford ethyl 1-(3-cyanophenyl)-3-isopropyl-1H-pyrazole-5-carboxylate (0.3267 g, 42% yield) as a white solid. MS (ESI) m/z: 284.0 (M+H⁺)

To a stirring solution of ethyl 1-(3-cyanophenyl)-3-isopropyl-1H-pyrazole-5-carboxylate (0.3267 g, 1.15 mmol) in 1:1:1 THF/EtOH/H2O (18 ml) at 22 °C was added LiOH·H₂O (0.242 g, 5.765 mmol). The reaction was stirred at RT for 4h. Solvent was removed under reduced pressure. The residue was diluted with H₂O (3 ml) and washed with Et₂O (5 mL). The pH was adjusted to ∼4 with 3M HCl. The aqueous solution was extracted with EtOAc (2x30 mL). The combined organic layers were washed with brine (15 mL), dried (MgSO₄) and concentrated to afford 1-(3-cyanophenyl)-3-isopropyl-1H-pyrazole-5-carboxylic acid (0.275g, 94% yield) as a white solid. MS (ESI) m/z: 256.0 (M+H⁺).

Example B3: Sodium metal (13.8 g, 0.5 mol) was added portionwise to ice-cold anhydrous EtOH (700 mL). After complete dissolution of the Na, a mixture of 3,3-dimethylbutan-2-one (50 g, 0.5 mol) and oxalic acid diethyl ester (77 ml, 0.5 mol) was added drop-wise. The reaction mixture was stirred in ice-salt bath and until TLC indicated completion of the reaction. Acetic acid (38.1 ml, 0.5 mol) was added and the mixture was stirred at RT for 30 min. The reaction mixture was cooled in an ice-salt bath and treated with hydrazine hydrate (29.4 g, 0.5 mol). After complete addition, the mixture was warmed to RT and stirred until judged complete by TLC. The reaction mixture was concentrated under reduced pressure and re-dissolved in EtOAc. The EtOAc solution was washed with NaHCO₃, brine and water, dried (MgSO₄) and concentrated *in vacuo.* The resultant solid was washed with cold petroleum ether to give ethyl 3-tert-butyl-1H-pyrazole-5-carboxylate (49 g, 50% yield over two steps) as a white solid. ¹H NMR (400 MHz, CDCl₃): δ 6.65 (s, 1 H), 4.38 (q, *J*= 6.8 Hz, 2 H), 1.39 (t, *J*= 6.8 Hz, 3 H), 1.35 (s, 1 H); MS (ESI) m/z: 197.2 (M+H⁺).

Following the procedure of Example B5, 3-cyanophenylboronic acid (5.0 g, 34 mmol, 1.00 eq), ethyl 3-t-butyl-1H-pyrazole-5-carboxylate (6.68 g, 34 mmol, 1.00 eq), Cu(OAc)₂ (6.18 g, 34 mmol, 1.00 eq), 4Å MS (10 g) and pyridine (34 mmol, 1.00 eq, 2.80 mL) were combined in CH₂Cl₂ (300 mL) to afford ethyl 5-tert-butyl-2-(3-cyanophenyl)-2H-pyrazole-3-carboxylic acid ethyl ester (3.5 g, 35% yield). ¹H NMR (400 MHz, DMSO-*d₆*): *δ* 8.05 (m, 1H), 7.92 (m, 1 H), 7.83 (m, 1H), 7.68 (m, 1H), 7.06 (s, 1H), 4.20 (m, 2 H), 1.30 (s, 9H), 1.19 (m, 3H); MS (ESI) *m*/*z*: 298.0 [M+H]⁺.

Following the procedure of Example B5, ethyl 3-t-butyl-1-(3-cyanophenyl)-1H-pyrazole-5-carboxylate (3.5 g, 11.8 mmol, 1.00 eq) and LiOH·H₂O (2.5 g, 60 mmol, 5.00 eq) were combined to provide 5-tert-butyl-2-(3-cyano-phenyl)-2H-pyrazole-3-carboxylic acid (2.9 g, 91% yield). ¹H NMR (400 MHz, DMSO-*d₆*): *δ* 8.02 (m, 1H), 7.89 (m, 1H), 7.82 (m, 1H), 7.66 (t, J = 8.0 Hz, 1H), 7.00 (s, 1H), 1.30 (s, 9H); MS (ESI) *m*/*z*: 270.2 [M+H]⁺.

Example B4: 5-Bromo-2-fluoro-benzonitrile (5 g, 25 mmol), 1-(diphenylmethylene)hydrazine (5 g, 25 mmol), Pd(OAc)₂ (0.2 g, 0.9 mmol), dppf (0.69 g, 1.25 mmol), and Cs₂CO₃ (16 g, 50 mmol) were combined in toluene (500 mL). The resulting mixture was degassed under vacuum, blanketed with nitrogen and then heated to reflux overnight. The cooled reaction mixture was filtered through silica gel, and the filter cake was washed with EtOAc (100 mL). The combined filtrates were concentrated *in vacuo* to give 5-(2-(diphenylmethylene)hydrazinyl)-2-fluorobenzonitrile (6.7 g, 86% yield). ¹H NMR (300 MHz, DMSO): *δ* 9.12 (s, 1 H), 7.60 (m, 5 H), 7.46 (m, 2 H), 7.35 (m, 6 H).

To a solution of 5-(2-(diphenylmethylene)hydrazinyl)-2-fluorobenzonitrile (3.5 g, 11 mmol) in EtOH (50 mL) was added 4-methyl-3-oxopentanenitrile (1.22 g, 11 mmol) and conc HCl (5 mL, 60 mmol). The resulting mixture was heated to reflux overnight. The reaction mixture was concentrated under reduced pressure, the residue poured into water and washed with EtOAc (4x 100 mL). The aqueous layer was neutralized with sodium carbonate solution to pH 8, and the resultant suspension was extracted with EtOAc (3 x 100 mL). The combined organic layers were concentrated *in vacuo* and purified by silica gel chromatography to provide 5-(5-amino-3-isopropyl-1H-pyrazol-1-yl)-2-fluorobenzonitrile (2.3 g, 86% yield). ¹H NMR (400 MHz, DMSO-d₆): *δ* 8.02 (m, 1 H), 7.94 (m, 1 H), 7.57 (t, *J* = 6.6 Hz, 1 H), 5.57 (s, 2 H), 5.44 (s, 1 H), 2.74 (m, 1 H), 1.14 (d, J = 6.8 Hz, 6 H); MS (ESI) m/z: 245.2 [M+H]⁺.

Example B5: A mixture of 4-cyanophenylboronic acid (5.0 g, 34 mmol, 1.00 eq), ethyl 3-t-butyl-1H-pyrazole-5-carboxylate from Example B3 (6.68 g, 34 mmol), Cu(OAc)₂ (6.18 g, 34 mmol), 4Å MS (10 g) and pyridine (2.80 mL, 34 mmol) were combined in CH₂Cl₂ (300 mL) and stirred at RT for 48 h open to the air. The solids were removed by filtration and the filtrate was sequentially washed with H₂O, 1M aq HCl, satd aq NaHCO₃, and brine. The organics were dried (MgSO₄), concentrated *in vacuo* and purified by silica gel chromatography to provide ethyl 3-tert-butyl-1-(4-cyanophenyl)-1H-pyrazole-5-carboxylate (4.0 g, 40 % yield). ¹H NMR (400 MHz, DMSO-*d₆*): δ 7.98-7.95 (m, 2 H), 7.72 -7.70 (m, 2 H), 7.06 (s, 1H), 4.20 (m, 2 H), 1.30 (s, 9 H), 1.19 (m, 3 H); MS (ESI) *m*/*z*: 298.0 [M+H]⁺.

A solution of ethyl 3-tert-butyl-1-(4-cyanophenyl)-1H-pyrazole-5-carboxylate (4.0 g, 13.5 mmol) in a mixed solvent of THF/EtOH/H₂O (100 mL, 1:1:1) was treated with LiOH·H₂O (2.85 g, 68.4 mmol) and the resultant mixture was stirred at ambient temp for 3 h. The reaction mixture was diluted with 1M aq HCl until pH 3 and then the mixture was extracted with EtOAc (2 x 50 mL). The combined organics were washed with brine, dried (MgSO₄), and concentrated *in vacuo* to afford 3-tert-butyl-1-(4-cyanophenyl)-1H-pyrazole-5-carboxylic acid (3.3 g, 92%). ¹H NMR (400 MHz, DMSO-*d₆):* δ 7.95-7.92 (m, 2H), 7.69-7.67 (m, 2H), 7.03 (s, 1H), 1.30 (s, 9H); MS (ESI) *m*/*z*: 270.16 [M+H]⁺.

Example B6: To a solution of m-aminobenzoic acid (200 g, 1.46 mmol) in conc. HCl (200 mL) was added an aqueous solution (250 mL) of NaNO₂ (102 g, 1.46 mmol) at 0 °C and the reaction mixture was stirred for 1h. A solution of SnCl₂·2H₂O (662 g, 2.92 mmol) in conc. HCl (2 L) was then added at 0 °C. The reaction solution was stirred for an additional 2h at RT. The precipitate was filtered and washed with EtOH and ether to give 3-hydrazinobenzoic acid hydrochloride as a white solid, which was used for the next reaction without further purification. ¹H NMR (400 MHz, DMSO-d₆): δ 10.8 (s, 3H), 8.46 (s, 1H), 7.53 (s, 1H), 7.48 (d, *J* = 7.6 Hz, 1H), 7.37 (m, 1H), 7.21 (d, *J* = 7.6 Hz, 1H).

A mixture of 3-hydrazinobenzoic acid hydrochloride (200 g, 1.06 mol) and 4,4-dimethyl-3-oxopentanenitrile (146 g, 1.167 mol) in EtOH (2 L) was heated at reflux overnight. The reaction solution was evaporated under reduced pressure. The residue was purified by column chromatography to give 3-(5-amino-3-t-butyl-pyrazol-1-yl)-benzoic acid ethyl ester (116 g, 40% yield) as a white solid together with 3-(5-amino-3-t-butyl-pyrazol-1-yl)benzoic acid (93 g, 36% yield). 3-(5-amino-3-t-butyl-pyrazol-1-yl)benzoic acid and ethyl ester. ¹H NMR (400 MHz, DMSO-*d*₆): δ 8.14 (s, 1H), 7.86-7.80 (m, 2 H), 7.57 (t, *J* = 8.0 Hz, 1H), 5.40 (s, 1H), 5.26 (s, 2 H), 4.33 (q, *J* = 7.2 Hz, 2H), 1.31 (t, *J*= 7.2 Hz, 3H), 1.20 (s, 9H); MS (ESI) *m*/*z*: 288.0 [M+H]⁺.

Example B7: Using a procedure analogous to Example B16, ethyl 4-(3-t-butyl-5-amino-1H-pyrazol-1-yl)benzoate (3.67 mmol) was prepared from ethyl 4-hydrazinobenzoate and pivaloylacetonitrile. ¹H NMR (400 MHz, DMSO-d₆): δ 799 (d, J = 8.8 Hz, 2 H), 7.76 (d, J = 8.8 Hz, 2 H), 5.43 (s, 1 H), 5.38 (br s, 2 H), 4.30 (q, J = 7.2 Hz, 2 H), 1.31 (t, J = 7.2 Hz, 3 H), 1.20 (s, 9 H); MS (ESI) *m*/*z*: 288.3 [M+H]⁺.

Example B8: A mixture of ethyl propionate (50 g, 0.49 mol) and acetonitrile (30 mL, 0.59 mol) in THF (50 mL) was added drop-wise to a mixture of NaH (29.4 g, 0.74 mol) in THF (800 mL). After complete addition, the resulting mixture was heated at reflux for 10 h. The reaction was concentrated *in vacuo,* diluted with water and partitioned with EtOAc (3 x 400 mL). The organic phase was washed with brine, dried (Na₂SO₄) and concentrated to give 3-oxo-pentanenitrile (39 g, 82% yield). ¹H NMR (400 Hz, DMSO-d6): *δ* 3.99 (s, 2 H), 2.50 (q, *J* = 7.2 Hz, 2 H), 0.93 (t, *J* = 7.2 Hz, 3 H).

To a suspension of 3-bromo-benzonitrile (25 g, 138 mmol) in toluene (200 mL) was added 1-(diphenylmethylene)hydrazine (30 g, 153 mmol), Pd(OAc)₂ (1 g, 4.46 mmol), dppf (3.5 g, 6.3 mmol), and CsCO₃ (80 g, 245 mmol). The mixture was purged of air, back filled with N₂ and then heated at reflux overnight. The reaction mixture was cooled to RT and filtrated through silica gel. The filter cake was washed with EtOAc (200 mL) and the combined filtrates were concentrated to provide 3-(2-(diphenylmethylene)hydrazinyl)benzonitrile (40 g, 98% yield). ¹H NMR (400 MHz, DMSO-*d₆*): *δ* 9.24 (s, 1 H), 7.54-7.59 (m, 5 H), 7.42-7.43 (m, 2 H), 7.12-7.37 (m, 6H), 7.12 (d, *J* = 6.4 Hz, 1 H).

Conc HCl (8 mL, 96 mmol) was added to a solution of 3-(2-(diphenylmethylene)hydrazinyl)benzonitrile (5.0 g, 16.8 mmol) and 3-oxo-pentanenitrile (2.5 g, 25.2 mmol) in ethanol (80 mL). The resultant mixture was heated at reflux for 10 h under N₂ atmosphere. The solvent was removed *in vacuo* and the residue was suspended in water. The alkalinity was adjusted to pH 8 with saturated NaHCO₃ solution, and the mixture was extracted with EtOAc. The combined organics were washed with brine, dried (Na₂SO₄), concentrated *in vacuo* and purified by silica gel chromatography to afford 3-(5-amino-3-ethyl-1H-pyrazol-1-yl)benzonitrile (2 g, 56% yield). ¹H NMR (400 Hz, DMSO-*d₆*): *δ* 7.98 (s, 1 H), 7.92 (d, *J* = 8.8 Hz, 1 H), 7.68 (d, *J* = 7.6 Hz, 1 H), 7.61 (t, *J*= 7.6 Hz, 1 H), 5.45 (s, 2 H), 5.37 (s, 1 H), 2.42 (q, *J*= 7.6 Hz, 2 H), 1.12 (t, *J* = 7.6 Hz, 3 H); MS (ESI) *m*/*z*: 213.2[M+H]⁺.

Example B9: In a mixture of saturated sodium bicarbonate:toluene:ethanol (1:2:1) (20 mL) was dissolved methyl 2-tert-butyl-4-chloropyrimidine-5-carboxylate (2.71 g, 11.85 mmol) and phenylboronic acid (2.88 g, 23.7 mmol) and to this was added tetrakis-(triphenylphosphine)palladium(0) (300 mg). The reaction stirred at 75 °C, under Ar, overnight. After dilution with ethyl acetate (75 mL) and water (75 mL), the mixture was filtered through Celite^{®} and the organic phase separated. The organic phase was washed with 5% citric acid (75 mL) and brine (75 mL), dried (Na₂SO₄) and evaporated at reduced pressure to give a semi solid/oil. The solid was purified by chromatography (ethyl acetate/Hexanes) to give a clear thick oil, which solidified to a white solid identified as methyl 2-tert-butyl-4-phenylpyrimidine-5-carboxylate (2.58 g, 81% yield). ¹H NMR (300 MHz, DMSO-*d*₆), δ 1.39 (s, 9 H), 3.70 (s, 3 H), 7.49-7.52 (m, 3 H), 7.61-7.63 (m, 2 H), 9.04 (s, 1 H); MS (ESI) m/z: 271.3 (M+H⁺).

In a 1:1:1 mixture of methanol:dioxane:water (15 mL) was placed methyl 2-tert-butyl-4-phenylpyrimidine-5-carboxylate (2.58 g, 9.54 mmol) and lithium hydroxide hydrate (1.20 g, 28.6 mmol). The solution was stirred at RT overnight. The mix was diluted with ethyl acetate (70 mL) and washed with 5% citric acid (100 mL). The organic phase washed with brine, dried (Na₂SO₄) and evaporated at reduced pressure to give a white solid, identified as 2-tert-butyl-4-phenylpyrimidine-5-carboxylic acid (2.31 g, 94% yield). ¹H NMR (300 MHz, DMSO-*d*₆): δ 1.38 (s, 9 H), 7.48-7.50 (m, 3 H), 7.64-7.67 (m, 2 H), 9.01 (s, 1 H), 12.75 (s, 1 H); MS (ESI) m/z: 257.3 (M+H⁺).

Example B10: A suspension of 5-amino-2-methyl-benzonitrile (3 g, 22.7 mmol) in conc HCl (60 mL) was cooled to 0 °C and treated with a solution of sodium nitrite (1.6 g, 22.7 mmol) in water (10 mL) at such a rate that the reaction temperature never rose above 5°. Stirring was continued for 30 min. To this mixture was added drop-wise a solution of SnCl₂ (10.2 g, 45.4 mmol) in conc HCl (10 mL) also at such a rate that the reaction temperature never rose above 5°C. After the addition, the resulting mixture was stirred at 0 °C for 1 h, and at RT for an additional 1 h. The precipitate was collected by filtration, washed with ethyl ether, and dried *in vacuo* to provide 5-hydrazinyl-2-methylbenzonitrile hydrochloride (3.6 g, 87 % yield), which was used without purification in next step.

A solution of 5-hydrazinyl-2-methylbenzonitrile hydrochloride (3.6 g, 19.7 mmol), 4-methyl-3-oxo-pentanenitrile (2.6 g, 23.6 mmol), and conc HCl (5 mL, 60 mmol) in EtOH (50 mL) was heated at 80 °C for 8 h under N₂. The solvent was removed *in vacuo* and the crude solid was washed with ethyl ether. The solid was suspended in H₂O, and the mixture was neutralized with satd NaHCO₃ solution and was extracted with EtOAc. The organic extracts were washed with brine, dried (Na₂SO₄) and concentrated *in vacuo* to afford 5-(5-amino-3-isopropyl-1H-pyrazol-1-yl)-2-methylbenzonitrile (3.4 g, 72 % yield). ¹H NMR (400 Hz, DMSO-*d₆*): *δ* 7.92 (d, J = 2.4 Hz, 1 H), 7.81 (dd, *J* = 8.4, 2.4 Hz, 1 H), 7.52 (d, *J*= 8.8 Hz, 1 H), 5.44 (br s, 2 H), 5.38 (s, 1 H), 2.75 (m, 1 H), 2.49 (s, 3 H), 1.16 (d, *J* = 6.8 Hz, 6 H); MS (ESI) *m*/*z*: 241.2[M+H]⁺.

Example B11: A suspension of 3-(2-(diphenylmethylene)hydrazinyl)benzonitrile from Example B8 (20 g, 67 mmol) in EtOH (500 mL) was treated with conc HCl (5.5 mL, 66 mmol) and 4-methyl-3-oxo-pentanenitrile (11 g, 0.1 mol). The reaction mixture was heated at reflux overnight. The reaction mixture was concentrated under reduced pressure, dissolved in water (300 mL) and washed with EtOAc (3 x 300 mL). The aqueous layer was neutralized with NaHCO₃ solution to pH = 8 and was extracted with EtOAc (2 x 300 mL). The combined organic layers were washed brine, dried (Na₂SO₄), and evaporated to give 3-(5-amino-3-isopropyl-1H-pyrazol-1-yl)benzonitrile (8.2 g, 54% yield) ¹H-NMR (400 MHz, DMSO-*d₆*): *δ* 7.99 (s, 1 H), 7.92 (m, 1 H), 7.69 (d, *J*= 6.4 Hz 1 H), 7.60 (t, *J* = 7.2 Hz, 1 H), 5.44 (s, 2 H), 5.38 (s, 1 H), 2.74 (m, 1 H), 1.16 (d, *J* = 7.6 Hz, 6 H); MS (ESI) m/z: 227.2 [M + H]⁺.

Example B12: Benzoylacetate (2.88 g, 15.0 mmol) and triethylorthoformate (4.51 g, 30 45 mmol) were stirred into acetic anhydride (10 mL) and warmed to 130 °C for 3 hrs. The reaction was concentrated *in vacuo* and azeotroped with toluene (2 x 10 mL) to afford (Z)-ethyl 2-benzoyl-3-ethoxyacrylate.

21% Sodium ethoxide in ethanol (4.86 g, 14.98 mmol) was added to a solution of acetamidine hydrochloride (2.047 g, 14.98 mmol) in ethanol (20 mL) and the reaction was stirred at RT for 15 min. To this was added crude (Z)-ethyl 2-benzoyl-3-ethoxyacrylate (3.72 g, 14.98 mmol) in ethanol (20 mL). After stirring overnight at RT, the reaction was warmed to reflux for 1 hr, cooled to RT and concentrated *in vacuo.* The residue was diluted with water (50 mL) and acidified with acetic acid (pH∼=3) with formation of a precipate. The mixture was extracted with ethyl acetate (2 x 50 mL). The combined organic phases were washed with brine, dried (Na₂SO₄) and concentrated *in vacuo* to give a semi-solid sludge. This was triturated with ether (40 mL), filtered to remove solids and the ether phase concentrated *in vacuo* to afford ethyl 2-methyl-4-phenylpyrimidine-5-carboxylate (∼70% purity). MS (ESI) m/z: 243.0 (M+H⁺).

A solution of crude ethyl 2-methyl-4-phenylpyrimidine-5-carboxylate (2.46 g, 7.11 mmol) and lithium hydroxide hydrate (1.193 mg, 28.4 mmol) in ethanol:water:dioxane (1:1:1, 24 mL) was stirred at RT overnight. The mixture was diluted with water (40 mL), 1N NaOH (5 mL) and ether (40 mL). The aqueous phase made acidic with 6N HCl (pH∼=3) and extracted with ethyl acetate (2 x 25 mL). The combined organic layers were washed with brine, dried (Na₂SO₄) and concentrated invacuo to 2-methyl-4-phenylpyrimidine-5-carboxylic acid (1.47 g, 97% yield) as a yellow solid. ¹H NMR (400 MHz, DMSO-*d₆*): δ 2.69 (s, 3 H), 7.44-7.50 (m, 3 H), 7.60 (d, 2 H), 8.97 (s, 1 H), 13.49 (s, 1 H); MS (ESI) m/z: 215.0 (M+H⁺).

Example B13: To a solution of 4-hydrazinobenzoic acid (10 g, 65.8 mmol) in EtOH (200 mL) was added conc. HCl (12 M, 54.8 mL, 658 mmol) and 3-oxo-pentanenitrile from Example B8 (12.7 g, 0.13 mol). The resulting mixture was heated to reflux overnight. The reaction mixture was concentrated *in vacuo,* the residue was dissolved in water and washed with EtOAc (4 x 200 mL). The aqueous layer was made alkaline (pH 8) with satd aq NaHCO₃ solution, and was extracted with EtOAc (2 x 200 mL). The organic extracts were washed with brine, dried (Na₂SO₄) and concentrated *in vacuo* to give 4-(5-amino-3-ethyl-pyrazol-1-yl)-benzoic acid ethyl ester (9.0 g, 53% yield). ¹H-NMR (400 MHz, DMSO-*d₆*): *δ* 7.98 (d, *J* = 7.2 Hz, 2 H), 7.73 (d, *J* = 7.2 Hz, 2 H), 5.42 (s, 2 H), 5.36 (s, 1 H), 4.29 (q, *J* = 7.2 Hz, 2 H), 2.41 (q, *J* = 7.6 Hz, 2 H), 1.30 (t, *J* = 7.2 Hz, 3 H), 1.11 (t, *J* = 7.6 Hz, 3 H); MS (ESI) *m*/*z:* 260.1 [M+H]⁺.

Example B14: A solution of ethyl 3-oxo-3-phenylpropanoate (3.00 g , 15.61 mmol) and triethylorthoformate (3.89 mL) in acetic anhydride (4.42 mL) was warmed to 120 °C for 4 h and concentrated *in vacuo* to give (E)-ethyl 2-benzoyl-3-ethoxyacrylate (3.40 g, 88% yield). MS (ESI) m/z: 217.0 (M+H⁺).

A solution of hydrazine hydrate (823 mg, 16.4 mmol) in ethanol (5 mL) was added dropwise to a solution (E)-ethyl 2-benzoyl-3-ethoxyacrylate (3.40 g, 13.7 mmol) in ethanol (20 mL) at 0 °C. The mix was allowed to warm to RT, stir for 24 h and then concentrated *in vacuo* to give a three component mixture. The mixture was dissolved in ethyl acetate (30 mL), washed with 5% citric acid (25 mL), saturated sodium bicarbonate (25 mL) and brine (25 mL), dried (Na₂SO₄), concentrated *in vacuo* and purified by column chromatography (ethyl acetate/hexanes) to give a light yellow solid (1.37 g,). LC and LCMS show a 1:1 mix of methyl and ethyl esters. MS (ESI) m/z: 203.0 and 217.0 (M+H⁺).

1N Lithium hexamethyldisilazane (5.09 mL, 5.09 mmol) was added to a solution of the mixture of esters (1.00 g, ∼4.62 mmol) in THF (5 mL) at 0 °C. After 0.5h isopropyliodide (2.358 g, 13.87 mmol) was added and the reaction allowed to slowly warm to RT. After 3 h the reaction was warmed to reflux and stirred overnight. Isopropyliodide (1.5 mL) and cesium carbonate (1 g) were added and warming continued overnight. The reaction is treated with additional isopropyliodide (1.5 mL) and stirred at 80 °C overnight. The reaction is treated with additional isopropyliodide (1.5 mL) and stirred at 80 °C overnight. The reaction is treated with additional isopropyliodide (1.5 mL) and stirred at 80 °C overnight. The reaction is cooled to RT and filtered free of insolubles. The filtrate was diluted with ethyl acetate (50 mL), washed with 5% citric acid (40 mL), saturated sodium bicarbonate (40 mL) and brine (40 mL), dried (Na₂SO₄), concentrated *in vacuo* and purified by column chromatography (ethyl acetate/hexanes) to give ethyl 1-isopropyl-3-phenyl-1H-pyrazole-4-carboxylate and ethyl 1-isopropyl-5-phenyl-1H-pyrazole-4-carboxylate as a mixture of methyl and ethyl esters (470 mg) as an oil. MS (ESI) m/z: 259.0 and 245.0 (M+H⁺).

A solution of the above esters (470 mg, ∼1.819 mmol) and lithium hydroxide (305 mg, 7.28 mmol) in ethanol:water:dioxane (1:1:1, 6 mL) was warmed to 40 °C and stirred overnight. The reaction was cooled to RT and diluted with water (30 mL) and ether (20 mL). The ether was discarded and the aqueous phase made acidic with 6N HCl (pH∼=2, wet litmus) and extracted with ethyl acetate (2 x 25 mL). The combined ethyl acetate layers were washed with brine (25 mL), dried (Na₂SO₄) and concentrated *in vacuo* to give a white solid, 1-isopropyl-3-phenyl-1H-pyrazole-4-carboxylic acid (major isomer) and 1-isopropyl-5-phenyl-1H-pyrazole-4-carboxylic acid (381 mg, 91% yield). ¹H NMR (300 MHz, DMSO-*d*₆, major isomer): δ 1.50 (d, 6 H), 3.38 (s, 3 H), 4.61 (hp, 1 H), 7.39-7.45 (m, 3 H), 7.77-7.79 (m, 2 H), 8.38 (s, 1 H), 12.21 (s, 1 H); MS (ESI) m/z: 231.0 (M+H⁺).

Using General Method C, 1-isopropyl-3-phenyl-1H-pyrazole-4-carboxylic acid and 1-isopropyl-5-phenyl-1H-pyrazole-4-carboxylic acid (381 mg, 1.655 mmol), triethylamine (193 mg, 1.903 mmol), 2,2,2-trichloroethanol (494 mg, 3.31 mmol) and DPPA (524 mg, 1.903 mmol) were combined and purified by column chromatography (ethyl acetate/hexanes) to afford 2,2,2-trichloroethyl 1-isopropyl-3-phenyl-1H-pyrazol-4-ylcarbamate (faster elution, 340 mg) as a thick oil, ¹H NMR (300 MHz, DMSO-*d*₆): δ 1.51 (d, 6 H), 4.55 (hp, 1 H), 4.95 (s, 2 H), 7.35-7.46 (m, 3 H), 7.75-7.77 (m, 2 H), 7.95 (s, 1 H), 9.35 (s, 1 H); MS (ESI) m/z: 378.0 (M+H⁺), and 2,2,2-trichloroethyl 1-isopropyl-5-phenyl-1H-pyrazol-4-ylcarbamate (slower elution, 79 mg) as a foam, ¹H NMR (300 MHz, DMSO-*d*₆): δ 1.40 (d, 6 H), 4.42 (hp, 1 H), 4.88 (s, 2 H), 7.43-7.60 (m, 6 H), 9.15 (s, 1 H); MS (ESI) m/z: 378.0 (M+H⁺).

Example B15: For the preparation of 2,2,2-trichloroethyl 1-isopropyl-3-phenyl-1H-pyrazol-4-ylcarbamate see Example B14.

Example B16: A slurry of ethyl 3-phenyl-1H-pyrazole-4-carboxylate from Example B14 (1.051 g, 4.86 mmol), powdered potassium carbonate (5.37 g, 38.9 mmol) and ethyl iodide (2.274 g, 14.58 mmol) in acetonitrile (20 mL) was warmed to reflux for 5h and then cooled to RT. The reaction was filtered and the solids washed with acetonitrile (2 x 20 mL). The filtrate was concentrated in vacuo and partitioned between ethyl acetate (30 mL) and water (30 mL). The organic phase was washed with brine, dried (Na₂SO₄) and concentrated *in vacuo* to give an oil. LC, LCMS and NMR are consistent with a 2:1 mix of the ethyl isomers (1.066 g, 90% yield). MS (ESI) m/z: 245.0 (M+H⁺).

Using a procedure analogous to Example B14, the 2:1 mix of isomeric esters (1.066 g, 4.36 mmol) and lithium hydroxide (732 mg, 17.45 mmol) were combined to give the carboxylic acids as a 2:1 mixture of the ethyl isomers as a thick oil. LC and LCMS are consistent with assigned structures. MS (ESI) m/z: 217.0 (M+H⁺).

Using General Method C, the mixture of isomeric acids (877 mg, 4.06 mmol), triethylamine (472 mg, 4.66 mmol), 2,2,2-trichloroethanol (1.515 g, 10.14 mmol) and DPPA (1.284 g, 4.66 mmol) were combined and purified by chromatography (ethyl acetate/hexane) to give 2,2,2-trichloroethyl 1-ethyl-3-phenyl-1H-pyrazol-4-ylcarbamate (faster elution, 261 mg, >99% purity), ¹H NMR (300 MHz, DMSO-*d*₆): δ 1.53 (t, 3 H), 4.26 (q, 2 H), 5.01 (s, 2 H), 7.40-7.52 (m, 3 H), 7.81-7.83 (m, 2 H), 7.99 (s, 1 H), 9.43 (s, 1 H); MS (ESI) m/z: 364.0 (M+H⁺), and 2,2,2-trichloroethyl 1-ethyl-5-phenyl-1H-pyrazol-4-ylcarbamate (slower elution, 261 mg, >99% purity), ¹H NMR (300 MHz, DMSO-d₆): δ 1.37 (t, 3 H), 4.13 (q, 2 H), 4.94 (s, 2 H), 7.52-7.64 (m, 6 H), 9.24 (s, 1 H); MS (ESI) m/z: 364.0 (M+H⁺).

Example B17: For the preparation of 2,2,2-trichloroethyl 1-ethyl-3-phenyl-1H-pyrazol-4-ylcarbamate see Example B16.

Example B18: A mixture of 3-fluorophenylboronic acid (0.5 g, 3.57 mmol), Cu(OAc)₂ (1.0 g, 5.36 mmol) and ethyl 3-isopropyl-1H-pyrazole-5-carboxylate from Example B2 (0.71 g,3.93 mmol) was stirred at RT in pyridine (15 mL) for 36 hours. The reaction was diluted with water (40 mL) and extracated with ethyl acetate (3x20 ml). The combined organic layers were washed, dried, concentrated *in vacuo* and purified by chromatography (ethyl acetate/hexanes) to provide ethyl 1-(3-fluorophenyl)-3-isopropyl-1H-pyrazole-5-carboxylate as a colorless oil (0.42 g,43% yield). ¹H NMR (400 MHz, DMSO-d₆)δ 7.50 (m ,1H), 7.40(m, 1H), 7.31 (m, 2H), 6.98 (s, 1H), 4.20(q, J=6Hz, 2H), 3.00 (m, 1H), 1.25 (d, J = 6 Hz, 6H), 1.18 (d, J = 6 Hz, 3H); m/z: 277.0 (M+H⁺).

A mixture ethyl 1-(3-fluorophenyl)-3-isopropyl-1H-pyrazole-5-carboxylate (0.50 g, 1.8 mmol) and lithium hydroxide monohydrate (0.37 g, 8.8 mmol) was stirred in (THF: ethanol: water-2:1:1, 20 ml) for 1 hr. The volatiles from the reaction mixture were evaporated and the residue diluted with water (50 mL). The solution was acidified with HCl to pH ∼2 with stirring. The solids were filtered, washed with water and dried to give 1-(3-fluorophenyl)-3-isopropyl-1H-pyrazole-5-carboxylic acid as a white crystalline solid (0.35 g, 78% yield). ¹H NMR (400 MHz, DMSO-d₆): δ 7.50 (m ,1H), 7.37 (dd, J - 10.2, 2.4 Hz, 1H), 7.31-7.25 (m, 2H), 6.92 (s, 1H), 2.96 (m, 1H), 1.24 (d, J = 7 Hz, 6H); m/z: 249 (M+H⁺).

Example B19: To a flask charged with KOtBu (4 g, 36 mmol) and ether (100 mL, dry) was added dropwise a mixture of 2-fluorobenzonitrile (2.1 g, 17.5 mmol) and MeCN (0.738 g, 18 mmol) at 0 °C. After addition, the mixture was stirred at RT for 2 days. Water was added and the reaction and extracted with ether (3×100 mL). The organic layers were combined, washed with brine, dried (Na₂SO₄) and concentrated *in vacuo* to afford a yellow oil, which was dissolved in CH₂Cl₂ and the solution was acidified with 3M HCl. After stirring the solution at RT for 2 hours, the solution was extracted with dichloromethane (3×200 mL). The organic layers were combined, washed with brine and dried (Na₂SO₄) and concentrated *in vacuo* to give 3-(2-fluoro-phenyl)-3-oxo-propionitrile (2 g, 70% yield) as a yellow solid. ¹H NMR (300 MHz, DMSO-*d₆*): δ 7.99-7.92 (m, 1 H), 7.70-7.58 (m, 1 H), 7.35-7.14 (m, 2 H), 4.09 (m, 2 H).

To a solution of 3-(2-fluorophenyl)-3-oxopropanenitrile (0.501 g, 3.07 mmol) in EtOH (5 mL) added methylhydrazine (0.141 g, 3.07 mmol) and Conc. HCl (2 drops) and mixture was stirred at 80 °C for 16h. Solvents were removed; Sat. NaHCO₃ solution (35 mL) was added and the product was extracted with EtOAc (2x30 mL). The combined organics were washed with brine, dried (Na₂SO₄) and concentrated to afford crude product which was purified column chromatography (methanol/DCM)to afford 3-(2-fluorophenyl)-1-methyl-1H-pyrazol-5-amine as a thick residue (0.3 g, 52% yield). ¹H NMR (400 MHz, DMSO-*d*₆): δ 7.83 (t, *J*= 7.2 Hz, 1H), 7.19-7.13 (m, 3H), 5.75 (d, *J* = 4.4 Hz, 1H), 5.27 (s, 2H), 3.56 (s, 3H); MS (ESI) m/z: 192.1 (M+H⁺).

Example 1: Using general method B, Example B1 (0.0898 g, 0.30 mmol), THF (1.0 mL), Example A1 (0.0853 g, 0.30 mmol) and 1-methyl pyrrolidine (0.00312 mL) were combined and the resultant product purified via column chromatography to yield 1-(3-t-butyl-1-phenyl-1H-pyrazol-5-yl)-3-(2-fluoro-4-(2-(1-methyl-1H-pyrazol-4-yl)pyridin-4-yloxy)phenyl)urea (0.125 g, 79% yield) as a white foam. ¹H NMR (DMSO-*d₆*): δ 9.10 (d, *J* = 1.6 Hz, 1H), 8.89 (s, 1H), 8.60-8.57 (m, 2H), 8.28-8.23 (m, 2H), 7.68 (d, *J* = 2.0 Hz, 1H), 7.56-7.49 (m, 3H), 7.44-7.38 (m, 2H), 7.17-7.11 (m, 2H), 6.39 (s, 1H), 3.92 (s, 3H), 2.31 (s, 3H), 1.26 (s, 9H); MS (ESI) m/z: 526.2 (M+H⁺).

Example 2: Using general method C, Example B2 (0.060 g, 0.24 mmol) and Example A1 (0.200 g, 0.71 mmol) were combined and the resultant product purified via column chromatography to yield 1-(1-(3-cyanophenyl)-3-isopropyl-1H-pyrazol-5-yl)-3-(2-fluoro-4-(2-(1-methyl-1H-pyrazol-4-yl)pyridin-4-yloxy)phenyl)urea (0.077 g, 61% yield) as a white solid. ¹H NMR (DMSO-*d₆*): δ 9.06 (d, *J* = 1.6 Hz, 1H), 8.95 (s, 1H), 8.55-8.54 (m, 2H), 8.22 (s, 1H), 8.18 (t, *J* = 9.2 Hz, 1H), 8.03 (t, *J* = 1.2 Hz, 1H), 7.91-7.86 (m, 2H), 7.73 (t, *J* = 8.0 Hz, 1H), 7.61 (s, 1H), 7.39 (dd, *J* = 11.2, 2.4 Hz, 1H), 7.12-7.07 (m, 2H), 6.38 (s, 1H), 3.90 (s, 3H), 2.89 (m, 1H), 2.29 (s, 3H), 1.22 (d, *J* = 6.4 Hz, 6H); MS (ESI) m/z: 537.3 (M+H⁺).

Example 3: Using General Method C, Example B3 (100 mg, 0.370 mmol), triethylamine (43 mg, 0.426 mmol), Example A9 (100 mg, 0.370 mmol) and DPPA (117 mg, 0.426 mmol) were combined and purified by chromatography (methanol/ethyl acetate) to give a residue which was treated with 4N HCl/dioxane (0.4 mL) and stirred for 5 min. The solid was collected by filtration and identified as 1-(4-(2-(1H-pyrazol-4-yl)pyridin-4-yloxy)-2-fluorophenyl)-3-(3-tert-butyl-1-(3-cyanophenyl)-1H-pyrazol-5-yl)urea hydrochloride (13 mg, 6% yield). ¹H NMR (300 MHz, DMSO-*d*₆): δ 1.27 (s, 9 H), 6.42 (s, 1 H), 7.00-7.12 (m, 2 H), 7.38 (d, 1 H), 7.50-7.60 (m, 2 H), 7.73 (t, 2 H), 7.86-7.91 (m, 2 H), 8.03 (s, 1 H), 8.17 (t, 1 H), 8.50-8.53 (m, 2 H), 9.01 (s, 1 H), 9.10 (s, 1 H); MS (ESI) m/z: 537.3 (M+H⁺).

Example 4: Using General Method C, Example B3 (152 mg, 0.563 mmol), triethylamine (66 mg, 0.648 mmol), Example A1 (150 mg, 0.563 mmol) and DPPA (178 mg, 0.648 mmol) were combined and purified by column chromatography (methanol/ethyl acetate) to give a white foam. The foam was dissolved in acetonitrile (4 mL), treated with 4N HCl/dioxane (0.3 mL), concentrated *in vacuo* and lyophilized to afford 1-(3-tert-butyl-1-(3-cyanophenyl)-1H-pyrazol-5-yl)-3-(2-fluoro-4-(2-(1-methyl-1H-pyrazol-4-yl)pyridin-4-yloxy)phenyl)urea hydrochloride (129 mg, 41% yield). ¹H NMR (300 MHz, DMSO-*d₆):* δ 1.27 (s, 9 H), 3.92 (s, 3 H), 6.41 (s, 1 H), 7.12-7.14 (m, 2 H), 7.42-7.45 (m, 1 H), 7.50-7.60 (m, 1 H), 7.70-7.74 (m, 2 H), 7.84-7.92 (m, 2 H), 8.04 (s, 1 H), 8.15-8.20 (m, 1 H), 8.49 (s, 1 H), 8.52-8.53 (m, 1 H), 8.81 (s, 1 H), 9.27 (s, 1 H), 9.31 (s, 1 H); MS (ESI) m/z: 551.2 (M+H⁺).

Example 5: A solution of Example B4 (249 mg, 1.02 mmol) in THF (2 mL) was cooled to -78 °C and treated with a solution of lithium bis(trimethylsilyl)amide (1.0 M in THF) (2.12 mL, 2.12 mmol). The resultant solution was stirred 20 min at -78 °C. Isopropenyl chloroformate (0.12 mL, 1.11 mmol) was added rapidly and the reaction was stirred 10 min at -78 °C and quenched by addition of 2 M aq HCl (1.2 mL, 2.4 mmol). The mixture was warmed to RT and partitioned between EtOAc and water. The organics were washed with brine, dried (Na₂SO₄) and concentrated *in vacuo* to provide prop-1-en-2-yl 1-(3-cyano-4-fluorophenyl)-3-isopropyl-1H-pyrazol-5-ylcarbamate (341 mg, 100% yield). ¹H NMR (400 MHz, DMSO-*d*₆): δ 9.89 (br s, 1 H), 8.04 (dd, J = 5.6, 2.8 Hz, 1 H), 7.88 (ddd, J = 9.2, 4.8, 2.8 Hz, 1), 7.68 (t, J = 9.2 Hz, 1 H), 6.30 (s, 1 H), 4.70 (br s, 1 H), 4.62 (br s, 1 H), 2.88 (m, 1 H), 1.81 (br s, 3 H), 1.21 (d, J = 7.2 Hz, 6 H); MS (ESI) *m*/*z*: 329.0 (M+H⁺).

Prop-1-en-2-yl 1-(3-cyano-4-fluorophenyl)-3-isopropyl-1H-pyrazol-5-ylcarbamate (95 mg, 0.29 mmol), Example A1 (80 mg, 0.28 mmol) and N-methylpyrrolidine (2.5 mg, 0.029 mmol) were combined in THF (2 mL) and heated to 55 °C in a capped flask for 60 h. The reaction mixture was purifed by silca gel chromatography and recrystallization (acetone-acetonitrile) to provide 1-(1-(3-cyano-4-fluorophenyl)-3-isopropyl-1H-pyrazol-5-yl)-3-(2-fluoro-4-(2-(1-methyl-1H-pyrazol-4-yl)pyridin-4-yloxy)phenyl)urea (49 mg, 30% yield). ¹H NMR (400 MHz, DMSO-*d*₆): δ 8.93 (s, 1 H), 8.89 (s, 1 H), 8.36 (d, J = 5.7 Hz, 1 H), 8.25 (s, 1 H), 8.16 (dd, J = 5.8, 2.8 Hz, 1 H), 8.09 (t, J = 9.1 Hz, 1 H), 7.98-7.93 (m, 2 H), 7.71 (t, J = 9.0 Hz, 1 H), 7.26-7.21 (m, 2 H), 6.99 (m, 1 H), 6.65 (dd, J = 5.7, 2.6 Hz, 1 H), 6.37 (s, 1 H), 3.84 (s, 3 H), 2.88 (m, 1 H), 1.22 (d, J = 6.8 Hz, 6 H); MS (ESI) *m*/*z*: 555.0 (M+H⁺).

Example 6: Using General Method C, Example B5 (0.100 g, 0.371 mmol), TEA (0.259 mL, 1.857 mmol), DPPA (0.10 mL, 0.464 mmol) and Example A1 (0.211 g, 0.743 mmol) were combined and was purified by chromatography to afford 1-(3-tert-butyl-1-(4-cyanophenyl)-1H-pyrazol-5-yl)-3-(2-fluoro-4-(2-(1-methyl-1H-pyrazol-4-yl)pyridin-4-yloxy)phenyl)urea (0.116g, 57% yield) as a white foam. It was converted to corresponding mesylate salt by reacting with MsOH (1.0 eq.). ¹H NMR (400 MHz, DMSO-d₆): δ 9.10 (s, 1H), 9.01 (s, 1H), 8.60-8.57 (m, 2H), 8.27 (s, 1H), 8.18 (t, J = 9.6 Hz, 1H), 8.01 (d, *J*= 8.4 Hz, 2H), 7.78 (d, *J*= 8.8 Hz, 2H), 7.68 (d, *J*= 2.0 Hz, 1H), 7.42 (dd, *J* = 11.6, 2.8 Hz, 2H), 7.16-7.10 (m, 2H), 6.44 (s, 1H), 3.92 (s, 3H), 2.32 (s, 3H), 1.28 (s, 9H); MS (ESI) m/z: 551.2 (M+H⁺).

Example 7: Using General Method A, Example B6 (1.50 g, 5.22 mmol) was converted to ethyl 3-(3-tert-butyl-5-((2,2,2-trichloroethoxy)carbonyl)-1H-pyrazol-1-yl)benzoate (2.79 g, 116% yield) as a thick oil. ¹H NMR (300 MHz, DMSO-*d*₆): δ 1.26 (s, 9 H), 1.32 (t, 3 H), 4.32 (q, 2 H), 4.82 (s, 2 H), 6.32 (s, 1 H), 7.59-7.63 (m, 1 H), 7.72-7.74 (m, 1 H), 7.89-7.92 (m, 1 H), 8.01 (s, 1 H), 10.10 (s, 1 H); MS (ESI) m/z: 464.0 (M+H⁺).

Using General Method A, Example A1 (300 mg, 1.055 mmol), diisopropylethylamine base (682 mg, 5.28 mmol) and ethyl 3-(3-tert-butyl-5-((2,2,2-trichloroethoxy)carbonyl)-1H-pyrazol-1-yl)benzoate (488 mg, 1.055 mmol) were combined and purified by column chromatography (methanol/ethyl acetate) to afford ethyl 3-(3-tert-butyl-5-(3-(2-fluoro-4-(2-(1-methyl-1H-pyrazol-4-yl)pyridin-4-yloxy)phenyl)ureido)-1H-pyrazol-1-yl)benzoate (250 mg, 39% yield) as a white foam. ¹H NMR (300 MHz, DMSO-*d*₆): δ 1.27 (s, 9 H), 1.31 (t, 3 H), 3.84 (s, 3 H), 4.31 (q, 2 H), 6.40 (s, 1 H), 6.65 (m, 1 H), 6.97-7.00 (m, 1 H), 7.21 (s, 1 H), 7.24 (s, 1 H), 6.68 (t, 1 H), 7.81-7.84 (m, 1 H), 7.95-8.12 (m, 4 H), 8.24 (s, 1 H), 8.35 (d, 1 H), 8.86 (s, 1 H), 8.93 (s, 1 H); MS (ESI) m/z: 598.2 (M+H⁺).

In tetrahydrofuran (5 mL) was placed ethyl 3-(3-tert-butyl-5-(3-(2-fluoro-4-(2-(1-methyl-1H-pyrazol-4-yl)pyridin-4-yloxy)phenyl)ureido)-1H-pyrazol-1-yl)benzoate (192 mg, 0.321 mmol) and cooled to ∼0C. To this was added 1M LiAlH₄/THF (0.321 mL, 0.321 mmol). After 1 hr at ∼0C the reaction was carefully quenched with water (10 mL) and diluted with ethyl acetate (20 mL). The organic phase was washed with brine (10 mL), dried (Na₂SO₄) and concentrated *in vacuo* to give a foam. LC analysis showed starting material. The foam was dissolved in THF (5 mL) and cooled to 0 °C. This was treated with 1M LiAlH₄/THF (321 µL) and stirred at ∼0 °C for 1 hr. The reaction was then quenched with water (10 mL), diluted with ethyl acetate (20 mL), washed with brine (10 mL), dried (Na₂SO₄) and concentrated *in vacuo* to afford 1-(3-tert-butyl-1-(3-(hydroxymethyl)phenyl)-1H-pyrazol-5-yl)-3-(2-fluoro-4-(2-(1-methyl-1H-pyrazol-4-yl)pyridin-4-yloxy)phenyl)urea (146 mg, 82% yield) as a foam. The foam was dissolved in ethanol (4 mL), treated with p-toluenesulfonic acid (50 mg, 0.263 mmol), concentrated *in vacuo,* triturated with ether (10 mL) and filtered to give a solid identified as 1-(3-tert-butyl-1-(3-(hydroxymethyl)phenyl)-1H-pyrazol-5-yl)-3-(2-fluoro-4-(2-(1-methyl-1H-pyrazol-4-yl)pyridin-4-yloxy)phenyl)urea p-toluenesulfonic acid (133 mg). ¹H NMR (300 MHz, DMSO-*d*₆): δ 1.26 (s, 9 H), 2.27 (s, 3 H), 3.90 (s, 3 H), 4.58 (s, 2 H), 6.38 (s, 1 H), 7.07-7.10 (m, 4 H), 7.35-7.39 (m, 3 H), 7.44-7.48 (m, 4 H), 7.55 (br s, 1 H), 8.20-8.25 (m, 2 H), 8.53-8.55 (m, 2 H), 8.87 (s, 1 H), 9.07 (s, 1 H), OH and SO3H are missing; MS (ESI) m/z: 556.3 (M+H⁺).

Example 8: Using General Method D, Example B7 (0.31 g, 1.079 mmol) was converted to ethyl 4-(3-tert-butyl-5-((prop-1-en-2-yloxy)carbonyl)-1H-pyrazol-1-yl)benzoate (0.35 g, 87% yield) as an off-white solid. ¹H NMR (400 MHz, Acetone-*d*₆): δ 8.12 (d, *J* = 8.4 Hz, 2H), 6.76 (d, *J* = 8.4 Hz, 2H), 6.38 (s, 1H), 4.62 (brs, 2H), 4.37 (q, *J* = 7.2 Hz, 2H), 1.96 (brs, 3H), 1.37 (t, *J*= 7.2 Hz, 3H), 1.33 (s, 9H); MS (ESI) m/z: 372.3 (M+H⁺).

To a solution of ethyl 4-(3-tert-butyl-5-((prop-1-en-2-yloxy)carbonyl)-1H-pyrazol-1-yl)benzoate (0.201 g, 0.541 mmol) in THF (2 ml) added Example A1 (0.154 g, 0.541 mmol) and catalytic amount of 1-methyl pyrrolidine and the mixture was stirred for 2h at 60 °C. Solvents were removed and the crude product was purified by column chromatography (ethyl acetate/hexanes) to afford ethyl 4-(3-tert-butyl-5-(3-(2-fluoro-4-(2-(1-methyl-1H-pyrazol-4-yl)pyridin-4-yloxy)phenyl)ureido)-1H-pyrazol-1-yl)benzoate (0.176 g, 54% yield) as a colorless foam. ¹H NMR (400 MHz, DMSO-d₆): δ 8.96 (s, 1H), 8.93 (s, 1H), 8.36 (d, *J*= 5.6 Hz, 1H), 8.25 (s, 1H), 8.10-8.07 (m, 3H), 7.95 (s, 1H), 7.71 (d, *J* = 8.8 Hz, 2H), 7.25-7.71 (m, 2H), 6.98 (dt, *J* = 9.2 Hz, 1.2Hz, 1H), 6.66 (dd, *J* = 5.6 Hz, 2.4Hz, 1H), 6.43 (s, 1H), 4.33 (q, *J*= 6.8 Hz, 2H), 3.84 (s, 3H), 1.32 (t, *J*= 6.8 Hz, 3H), 1.27 (s, 9H); MS (ESI) m/z: 598.2 (M+H⁺).

To a solution of ethyl 4-(3-tert-butyl-5-(3-(2-fluoro-4-(2-(1-methyl-1H-pyrazol-4-yl)pyridin-4-yloxy)phenyl)ureido)-1H-pyrazol-1-yl)benzoate (0.175 g, 0.293 mmol) in THF (2 ml) was added 1M LiAlH₄/THF (0.9 ml, 0.878 mmol) at 0 °C and mixture was stirred for 2h at RT. Sat. Na₂SO₄ solution was added carefully to quench the reaction mixture and the resulting solution was stirred for 6h at RT. The quenched mixture was filtered through a Celite^{®} pad, washing with EtOAc (2x3 mL) and the combined filtrate was concentrated *in vacuo* and purified by column chromatography (methanol/CH₂Cl₂) to afford 1-(3-tert-butyl-1-(4-(hydroxymethyl)phenyl)-1H-pyrazol-5-yl)-3-(2-fluoro-4-(2-(1-methyl-1H-pyrazol-4-yl)pyridin-4-yloxy)phenyl)urea (0.086, 53% yield) as a white solid. This was converted to the corresponding tosylate salt. ¹H NMR (400 MHz, DMSO-d₆): δ 9.07 (s, 1H), 8.85 (s, 1H), 8.52-8.49 (m, 2H), 8.24 (t, *J* = 5.2 Hz, 1H), 8.18 (s, 1H), 7.54 (s, 1H), 7.48-7.43 (m, 6H), 7.35 (dd, *J*= 11.6 Hz, 2.4 Hz, 1H), 7.09 (d, *J*= 8.0 Hz, 3H), 7.01 (brs, 1H), 6.38 (s, 1H), 4.56 (s, 2H), 3.89 (s, 3H), 2.27 (s, 3H), 1.26 (s, 9H); MS (ESI) m/z: 556.3 (M+H⁺).

Example 9: Using General Method D, Example B8 (0.250 g, 1.18 mmol) was converted to prop-1-en-2-yl 1-(3-cyanophenyl)-3-ethyl-1H-pyrazol-5-ylcarbamate and purified via recrystallization (hexanes) to isolate a colorless semi-solid (0.350g, 100% yield) H NMR(400 MHz, DMSO-d₆): δ 9.87(brs, 1H), 7.9-7.65(m,4H), 6.26(s, 1H), 4.61(m, 2H), 2.53(q, J = 6Hz, 2H), 1.76(s, 3H), 1.14(t, J = 6Hz, 3H); MS (ESI) m/z: 297.0 (M+H⁺).

2,2,2-Trichloroethyl 1-(3-cyanophenyl)-3-ethyl-1H-pyrazol-5-ylcarbamate (0.300 g, 0.774 mmol) was reacted with Example A1 (0.220 g, 0.774 mmol) in presence of N-methylpyrrolidine (0.013 g, 0.155 mmol) in dioxane (5 ml) at 70 °C for 14 hours. The solvent was completely removed and the residue recrystallized (acetonitrile) to provide 1-(1-(3-cyanophenyl)-3-ethyl-1H-pyrazol-5-yl)-3-(2-fluoro-4-(2-(1-methyl-1H-pyrazol-4-yl)pyridin-4-yloxy)phenyl)urea (0.303g, 75% yield). ¹H NMR (400 MHz, DMSO-d₆) δ 8.91 (s, 1H), 8.88 (m, 2H), 8.48 (d, J = 9Hz, 1H), 8.31 (d, J = 5.5Hz, 1H), 8.2 (s, 1H), 8.04 (t, J = 9Hz, 1H), 7.99 (m, 1H), 7.91 (s, 1H), 7.84 (m, 1H), 7.69 (t, J = 8Hz, 1H), 7.19(dd, J = 12.0, 2.6Hz, 1H), 7.17 (d, J = 2.60Hz, 1H), 6.95 (brd, J = 9Hz, 1H), 6.61 (d, J = 5.5, 2.5Hz, 1H), 6.32 (s, 1H), 3.80 (s, 3H), 2.52 (q, J = 6Hz, 2H), 1.15 (d, J = 6Hz, 3H); MS(ESI) m/z : 523.0 (M+H⁺).

Example 10: Using General Method C, Example B9 (60 mg, 0.23 mmol) and Example A1 (67 mg, 0.23 mmol) in presence of DPPA (57 µL, 0.23 mmol) and (36 µL, 0.23 mmol) were combined and the resultant product purified via column chromatography (EtOAc/hexanes) to afford 1-(2-tert-butyl-4-(pyridin-3-yl)pyrimidin-5-yl)-3-(2-fluoro-5-(8-methyl-2-(methylamino)-7-oxo-7,8-dihydropyrido[2,3-d]pyrimidin-6-yl)phenyl)urea (68 mg, 54% yield). ¹H NMR (400 MHz, DMSO-d₆, major isomer): δ 9.05 (s, 1H), 9.02 (brs, 1H), 8.58 (s, 1H), 8.34 (d, *J* = 5.6 Hz, 1H), 8.23 (s, 1H), 8.15 (t, *J* = 9.2 Hz, 1H), 7.94 (s, 1H), 7.74 (m, 2H), 7.53 (m, 3H), 7.22 (dd, *J* = 2.4, and 11.6 Hz, 1H), 7.19 (d, J = 2.4Hz, 1H), 6.99 (m, 1H), 6.65 (dd, *J* = 2.4, and 5.6 Hz, 1H), 3.83 (s, 3H), 1.36 (s, 9H); MS (ESI) m/z: 538.3 (M+H⁺).

Example 11: To a solution of Example B10 (0.15 g, 0.624 mmol) in CH₂Cl₂ (10 mL) were added pyridine (0.102 ml, 1.248 mmol) and 2,2,2-trichloroethyl carbonochloridate (0.103 ml, 0.749 mmol) at 0 °C. After stirring for 2h at RT, 2M HCl was added, the organic layer was separated and aqueous layer was extracted with CH₂Cl₂ (1x20 mL). The combined organics were washed with brine, dried (Na₂SO₄) and concentrated to afford a white foam.

To this foam was added Example A1 (0.177 g, 0.624 mmol), DIPEA (0.081 g, 0.624 mmol) and DMSO (2 mL) and the mixture was heated to 65 °C for 5h. Water (40 mL) was added and product was extracted with EtOAc (2x35 ml). The combined organics were washed with brine, dried (Na₂SO₄) and concentrated to afford crude product which was purified by column chromatography (EtOAc/CH₂Cl₂) to afford 1-(1-(3-cyano-4-methylphenyl)-3-isopropyl-1H-pyrazol-5-yl)-3-(2-fluoro-4-(2-(1-methyl-1H-pyrazol-4-yl)pyridin-4-yloxy)phenyl)urea (71 mg, 20% 2 step yield) as an off-white solid. ¹H NMR (400 MHz, DMSO-*d*₆): δ 8.96 (s, 1H), 8.88 (s, 1H), 8.38 (d, *J*= 6.0 Hz, 1H), 8.26 (s, 1H), 8.12 (t, *J*= 9.2 Hz, 1H), 7.97 (s, 2H), 7.80-7.77 (m, 1H), 7.64 (d, *J*= 8.4 Hz, 1H), 7.27-7.23 (m, 2H), 7.01 (d, *J* = 8.4 Hz, 1H), 6.68 (dd, *J* = 6.0 Hz, 2.0 Hz, 1H), 6.38 (s, 1H), 3.86 (s, 3H), 2.93-2.86 (m, 1H), 2.55 (s, 3H), 1.24 (d, *J*= 6.4 Hz, 6H); MS (ESI) m/z: 551.0 (M+H⁺).

Example 12: Prop-1-en-2-yl 1-(3-cyanophenyl)-3-isopropyl-1H-pyrazol-5-ylcarbamate (63 mg, 0.20 mmol), prepared from Example B11 according to General Method D, Example A2 (57 mg, 0.19 mmol) and N-methylpyrrolidine (1.627 mg, 0.019 mmol) were combined in THF (0.4 mL) and heated to 55 °C overnight. The reaction mixture was purified by chromatography to provide 1-(1-(3-cyanophenyl)-3-isopropyl-1H-pyrazol-5-yl)-3-(2-fluoro-3-methyl-4-(2-(1-methyl-1H-pyrazol-4-yl)pyridin-4-yloxy)phenyl)urea (58 mg, 55% yield. ¹H NMR (400 MHz, DMSO-*d*₆), δ 8.96 (s, 2 H), 8.36 (d, J = 5.8 Hz, 1 H), 8.27 (s, 1 H), 8.06 (s, 1 H), 8.00-7.88 (m, 4 H), 7.76 (t, J = 8.0 Hz, 1 H), 7.20 (d, J = 1.9 Hz, 1 H), 6.97 (d, J = 9.2 Hz, 1 H), 6.59 (dd, J = 5.6, 2.1 Hz, 1 H), 6.40 (s, 1 H), 3.87 (s, 3 H), 2.91 (m, 1 H), 2.07 (s, 3 H), 1.25 (d, J = 7.1 Hz, 6 H); MS (ESI) *m*/*z*: 551.2 (M+H⁺).

Example 13: A solution of prop-1-en-2-yl 1-(3-cyanophenyl)-3-isopropyl-1H-pyrazol-5-ylcarbamate (73 mg, 0.235 mmol), prepared from Example B11 according to General Method D, Example A3 (68 mg, 0.225 mmol) and N-methylpyrrolidine (1.9 mg, 0.022 mmol) were combined in THF (0.6 mL) and heated to 55 °C overnight. The reaction mixture was purified by chromatography to provide 1-(1-(3-cyanophenyl)-3-isopropyl-1H-pyrazol-5-yl)-3-(2,3-difluoro-4-(2-(1-methyl-1H-pyrazol-4-yl)pyridin-4-yloxy)phenyl)urea (77 mg, 62% yield). ¹H NMR (400 MHz, DMSO-*d*₆), δ 9.18 (s, 1 H), 8.99 (s, 1 H), 8.39 (d, J = 5.6 Hz, 1 H), 8.29 (s, 1 H), 8.05 (m, 1 H), 7.99 (s, 1 H), 7.94-7.87 (m, 3 H), 7.74 (t, J = 8.0 Hz, 1 H), 7.28 (d, J = 2.4 Hz, 1 H), 7.18 (m, 1 H), 6.74 (dd, J = 5.8, 2.2 Hz, 1 H), 6.40 (s, 1 H), 3.85 (s, 3 H), 2.90 (m, 1 H), 1.23 (d, J = 7.0 Hz, 6 H); MS (ESI) *m*/*z*: 555.2 (M+H⁺).

Example 14: Following General Method C, Example B12 (75 mg, 0.352 mmol), triethylamine (41 mg, 0.405 mmol), Example A1 (100 mg, 0.352 mmol) and DPPA (111 mg, 0.405 mmol) were combined and purified by column chromatography (methanol/ethyl acetate) to afford 1-(2-fluoro-4-(2-(1-methyl-1H-pyrazol-4-yl)pyridin-4-yloxy)phenyl)-3-(2-methyl-4-phenylpyrimidin-5-yl)urea (41 mg, 23% yield) as a white foam. ¹H NMR (300 MHz, DMSO-*d*₆): δ 2.62 (s, 3 H), 3.86 (s, 3 H), 6.65 (s, 1 H), 6.99 (d, 1 H), 7.18-7.24 (m, 2 H), 7.50-7.53 (m, 3 H), 7.66-7.69 (m, 2 H), 7.94 (s, 1 H), 8.13-8.18 (m, 1 H), 8.23 (s, 1 H), 8.30-8.35 (m, 1 H), 8.55 (br s, 1 H), 9.03-9.05 (m, 2 H); MS (ESI) m/z: 496.0 (M+H⁺).

Example 15: To a solution of Example B8 (0.300 g, 1.413 mmol) and Troc-Cl (0.233 ml, 1.696 mmol) in EtOAc (7 ml) at 0 °C was added 3M NaOH (0.707 ml, 2.120 mmol). The reaction was stirred at 0 °C for 30 min and then at RT for 2h. The reaction was treated with satd. NaHCO₃ (2-3 ml) and more Troc-Cl (0.1 ml) and stirred at RT. After 90 min, the layers of the biphasic reaction were separated and the organic layer washed with H₂O (2x), brine (1x), dried (MgSO₄), concentrated *in vacuo* and purified by flash column chromatography (EtOAc/hexanes) to afford 2,2,2-trichloroethyl 1-(3-cyanophenyl)-3-ethyl-1H-pyrazol-5-ylcarbamate (0.47 g, 86% yield) as a colorless oil. ¹H NMR (400 MHz, DMSO-*d*₆): δ 10.18 (brs, 1H), 7.92 (s, 1H), 7.85-7.82 (m, 2H), 7.70-7.66 (m, 1H), 6.29 (s, 1H), 4.86 (s, 2H), 2.61-2.56 (m, 2H), 1.21-1.18 (m, 3H); MS (ESI) m/z: 387.0 (M+H⁺), 389.0 (M+2+H⁺).

2,2,2-Trichloroethyl 1-(3-cyanophenyl)-3-ethyl-1H-pyrazol-5-ylcarbamate (0.156 g, 0.402 mmol), Example A4 and iPr₂NEt (0.154 ml, 0.885 mmol) were combined in DMSO (4 ml) and stirred with heating at 70 °C. After 18 h, the completed reaction was cooled to RT, diluted with brine and extracted with EtOAc (3x). The combined organics were washed with brine (2x), dried (MgSO₄), concentrated in vacuo and purified by reverse phase chromatography to afford 1-(1-(3-cyanophenyl)-3-ethyl-1H-pyrazol-5-yl)-3-(2-fluoro-4-(2-(1-propyl-1H-pyrazol-4-yl)pyridin-4-yloxy)phenyl)urea (60 mg, 27% yield) as a white solid following lyophilization. ¹H NMR (400 MHz, DMSO-*d*₆): δ 9.03 (s, 1H), 8.96 (s, 1H), 8.51 (brs, 2H), 8.18-8.13 (m, 2H), 8.04 (brs, 1H), 7.92-7.87 (m, 2H), 7.87-7.74 (m 1H), 7.52 (brs, 1H), 7.37-7.36 (m, 1H), 7.09-7.07 (m, 1H), 6.96 (brs, 1H), 6.37 (s, 1H), 4.13-4.09 (m, 2H), 2.61-2.55 (m, 2H), 1.83-1.77 (m, 2H), 1.24-1.18 (m, 3H), 0.84-0.81 (m, 3H); MS (ESI) m/z: 551.2 (M+H⁺).

Example 16: A solution of the prop-1-en-2-yl 1-(3-cyanophenyl)-3-isopropyl-1H-pyrazol-5-yl (90 mg, 0.290 mmol), prepared from of Example B11 using General Method D, Example A5 (78 mg, 0.273 mmol) and N-methylpyrrolidine (2.3 mg, 0.027 mmol) were combined in THF (0.6 mL) and heated to 55 °C for 40 h. The reaction mixture was purified by chromatography and the obtained solid was recrystallized (acetone-hexanes) to provide 1-(1-(3-cyanophenyl)-3-isopropyl-1H-pyrazol-5-yl)-3-(3-fluoro-4-(2-(1-methyl-1H-pyrazol-4-yl)pyridin-4-yloxy)phenyl)urea (93 mg, 63% yield). ¹H NMR (400 MHz, DMSO-*d*₆), δ 9.40 (s, 1 H), 8.71 (s, 1 H), 8.40 (d, J = 5.6 Hz, 1 H), 8.31 (s, 1 H), 8.08 (t, J = 1.8 Hz, 1 H), 8.01 (d, J = 0.5 Hz, 1 H), 7.96 (ddd, J = 8.2, 2.2, 1.1 Hz, 1 H), 7.92 (dt, J = 8.0, 1.2 Hz, 1 H), 7.78 (t, J = 8.0 Hz, 1 H), 7.69 (dd, J = 13.3, 2.4 Hz, 1 H), 7.34 (t, J = 9.1 Hz, 1 H), 7.26 (d, J = 2.6 Hz, 1 H), 7.24 (m, 1 H), 6.66 (dd, J = 5.8, 2.6 Hz, 1 H), 6.43 (s, 1 H), 3.90 (s, 3 H), 2.96 (m, 1 H), 1.29 (d, J = 6.9 Hz, 6 H); MS (ESI) *m*/*z*: 537.3 (M+H⁺).

Example 17: 2,2,2-Trichloroethyl 1-(3-cyanophenyl)-3-ethyl-1H-pyrazol-5-ylcarbamate from Example 15 (0.156 g, 0.402 mmol), Example A6 (0.126 g, 0.402 mmol) and iPr₂NEt (0.154 ml, 0.885 mmol) were combined in DMSO (4 ml) and stirred with heating at 70 °C. After 18 h, the completed reaction was cooled to RT and purified directly by reverse phase chromatography to afford 1-(1-(3-cyanophenyl)-3-ethyl-1H-pyrazol-5-yl)-3-(2-fluoro-4-(2-(1-isopropyl-1H-pyrazol-4-yl)pyridin-4-yloxy)phenyl)urea (87 mg, 39% yield) as a white solid following lyophilization. ¹H NMR (400 MHz, DMSO-*d*₆): δ 9.10 (brs, 1H), 9.03 (s, 1H), 8.66 (s, 1H), 8.60-8.58 (m, 1H), 8.27-8.20 (m, 2H), 8.10 (brs, 1H), 7.98-7.93 (m, 2H), 7.82-7.80 (m, 1H), 7.66 (s, 1H), 7.45-7.42 (m, 1H), 7.17-7.15 (m, 1H), 7.08-7.07 (m, 1H), 6.43 (s, 1H), 4.63-4.60 (m, 1H), 2.67-2.61 )m, 2H), 1.50-1.49 (m, 6H), 1.28-1.25 (m, 3H); MS (ESI) m/z: 551.2 (M+H⁺).

Example 18: 2,2,2-Trichloroethyl 1-(3-cyanophenyl)-3-ethyl-1H-pyrazol-5-ylcarbamate from Example 15 (0.156 g, 0.402 mmol), Example A7 (0.120 g, 0.402 mmol) and iPr₂NEt (0.154 ml, 0.885 mmol) were combined in DMSO (4 ml) and stirred with heating at 70 °C. After 18 h, the completed reaction was cooled to RT and the crude product was purified directly by reverse phase chromatography to afford 1-(1-(3-cyanophenyl)-3-ethyl-1H-pyrazol-5-yl)-3-(4-(2-(1-ethyl-1H-pyrazol-4-yl)pyridin-4-yloxy)-2-fluorophenyl)urea (78 mg, 36% yield) as a white solid following lyophilization. ¹H NMR (400 MHz, DMSO-*d*₆): δ 9.05 (s, 1H), 8.97 (s, 1H), 8.54-8.51 (m, 2H), 8.19-8.16 (m, 2H), 8.04 (brs, 1H), 7.92-7.87 (m, 2H), 7.76-7.72 (m, 1H), 7.55 (s, 1H), 7.39-7.38 (m, 1H), 7.36-7.35 (m, 1H), 7.00 (brs, 1H), 6.37 (s, 1H), 4.22-4.17 (m, 2H), 2.59-2.53 (m, 2H), 1.41-1.38 (m, 3H), 1.22-1.19 (m, 3H); MS (ESI) m/z: 537.3 (M+H)⁺.

Example 19: Using General Method D, Example B13 (0.301 g, 1.161 mmol) was converted to ethyl 4-(3-ethyl-5-((prop-1-en-2-yloxy)carbonyl)-1H-pyrazol-1-yl)benzoate (0.4 g, 100%) as a thick residue. ¹H NMR (400 MHz, DMSO-*d*₆): δ 9.99 (brs, 1H), 8.12 (d, *J* = 8.4 Hz, 2H), 7.73 (d, *J* = 8.4 Hz, 2H), 6.35 (s, 1H), 4.72 (brs, 2H), 4.39 (q, *J* = 7.2 Hz, 2H), 2.65 (q, *J* = 7.6 Hz, 2H), 1.87 (brs, 3H), 1.39 (t, *J* = 7.2 Hz, 3H), 1.26 (t, *J* = 7.6 Hz, 3H); MS (ESI) m/z: 344.0 (M+H⁺).

A THF solution of ethyl 4-(3-ethyl-5-((prop-1-en-2-yloxy)carbonyl)-1H-pyrazol-1-yl)benzoate (0.2 g, 0.58 mmol) and Example A1 (0.16 g, 0.58 mmol) in the presence of DBU (a drop) was combined to afford ethyl 4-(3-ethyl-5-(3-(2-fluoro-4-(2-(1-methyl-1H-pyrazol-4-yl)pyridin-4-yloxy)phenyl)ureido)-1H-pyrazol-1-yl)benzoate (0.117 g, 35% yield) as a white solid. ¹H NMR (400 MHz, DMSO-*d*₆): δ 9.05 (brs, 2H), 8.46 (d, *J* = 5.6 Hz, 1H), 8.34 (s, 1H), 8.22-8.17 (m, 3H), 8.05 (s, 1H), 7.81 (d, *J* = 8.4 Hz, 2H), 7.35-7.31 (m, 2H), 7.09 (d, *J* = 8.8 Hz, 1H), 6.76 (dd, *J* = 5.6 Hz, 2.4 Hz, 1H), 6.47 (s, 1H), 4.43 (q, *J* = 7.2 Hz, 2H), 3.94 (s, 3H), 2.68 (q, *J* = 7.6 Hz, 2H), 1.42 (t, *J* = 7.2 Hz, 3H), 1.30 (t, *J* = 7.6 Hz, 3H); MS (ESI) m/z: 570.2 (M+H⁺).

Using a procedure analogous to Example 8, ethyl 4-(3-ethyl-5-(3-(2-fluoro-4-(2-(1-methyl-1H-pyrazol-4-yl)pyridin-4-yloxy)phenyl)ureido)-1H-pyrazol-1-yl)benzoate (0.115 g, 0.202 mmol) was converted to 1-(3-ethyl-1-(4-(hydroxymethyl)phenyl)-1H-pyrazol-5-yl)-3-(2-fluoro-4-(2-(1-methyl-1H-pyrazol-4-yl)pyridin-4-yloxy)phenyl)urea (0.096 g, 90% yield) and isolated as a white solid following purification via column chromatography (methanol/CH₂Cl₂). ¹H NMR (400 MHz, DMSO-*d*₆): δ 9.07 (s, 1H), 8.92 (s, 1H), 8.45 (d, *J* = 5.6 Hz, 1H), 8.33 (s, 1H), 8.24 (t, *J* = 9.2 Hz, 1H), 8.04 (s, 1H), 7.56-7.52 (m, 4H), 7.33-7.29 (m, 2H), 7.09 (dt, *J*= 8.8 Hz, 1.6Hz, 1H), 6.75 (dd, *J*= 5.6 Hz, 2.4 Hz, 1H), 6.41 (s, 1H), 5.41 (t, *J* = 5.6 Hz, 1H), 4.65 (d, *J* = 5.6 Hz, 2H), 3.93 (s, 3H), 2.64 (q, *J* = 7.6 Hz, 2H), 1.27 (t, *J* = 7.6 Hz, 3H); MS (ESI) m/z: 528.3 (M+H⁺).

Example 20: A solution of Example B14 (79 mg, 0.210 mmol), DIEA (81 mg, 0.629 mmol) and Example A1 (60 mg, 0.210 mmol) in DMSO (2 mL) was warmed to 80 °C overnight and then 110 °C overnight. The reaction was cooled to RT, diluted with water (30 mL) and extracted with ethyl acetate (30 mL). The combined organic phases were washed with brine (30 mL), dried (Na₂SO₄), concentrated *in vacuo* and purified by column chromatography (ethyl acetate/hexane→methanol/ethyl acetate) to give an impure foam (21 mg). This foam was purified by reverse phase chromatography (acetonitrile/water/0.1% TFA), lyophilized, and dissolved a mixture of water (5 mL), saturated sodium bicarbonate (5 mL) and ethyl acetate (15 mL). The organic phase was separated, washed with brine (10 mL), dried (Na₂SO₄), concentrated *in vacuo,* dissolved in acetonitrile/water and lyophilized to afford 1-(2-fluoro-4-(2-(1-methyl-1H-pyrazol-4-yl)pyridin-4-yloxy)phenyl)-3-(1-isopropyl-5-phenyl-1H-pyrazol-4-yl)urea (11 mg, 10% yield). ¹H NMR (300 MHz, DMSO-*d*₆): δ 1.45 (d, 6 H), 3.97 (s, 3 H), 4.41 (hp, 1 H), 6.77-6.79 (m, 1 H), 7.08-7.11 (m, 1 H), 7.30-7.34 (m, 2 H), 7.53-7.55 (m, 2 H), 7.64-7.73 (m, 3 H), 7.94 (s, 1 H), 8.08 (s, 1 H), 8.27 (s, 1 H), 8.32-8.37 (m, 2 H), 8.47 (d, 1 H), 8.84 (s, 1 H); MS (ESI) m/z: 512.2 (M+H⁺).

Example 21: In a manner analogous to Example 20, Example B15 (200 mg, 0.531 mmol), DIEA (206 mg, 1.593 mmol) and Example A1 (151 mg, 0.531 mmol) were combined and purified by column chromatography (ethyl acetate/hexane) and reverse phase chromatography (acetonitrile/water/0.1% TFA) to afford 1-(2-fluoro-4-(2-(1-methyl-1H-pyrazol-4-yl)pyridin-4-yloxy)phenyl)-3-(1-isopropyl-3-phenyl-1H-pyrazol-4-yl)urea (38 mg, 13% yield). ¹H NMR (300 MHz, DMSO-*d*₆): δ 1.56 (d, 6 H), 3.97 (s, 3 H), 4.61 (hp, 1 H), 6.79 (m, 1 H), 7.09-7.12 (m, 1 H), 7.33 (s, 1 H), 7.34-7.37 (m, 1 H), 7.47-7.49 (m, 1 H), 7.56-7.60 (m, 2 H), 7.76-7.78 (m, 2 H), 8.08 (s, 1 H), 8.13 (s, 1 H), 8.35-8.37 (m, 2 H), 8.48 (d, 1 H), 8.52 (s, 1 H), 9.00 (br s, 1 H); MS (ESI) m/z: 512.2 (M+H⁺).

Example 22: A solution of Example B16 (261 mg, 0.720 mmol), DIEA (372 mg, 2.88 mmol) and Example A1 (205 mg, 0.720 mmol) in DMSO (5 mL) was warmed to 75 °C and stirred overnight. The mixture was extracted with ethyl acetate (30 mL). The organic phase was washed with brine (30 mL), dried (Na₂SO₄), concentrated *in vacuo* and purified by column chromatography (methanol/dichloromethane) to give a film. The film was subjected to 4N HCl/dioxane to afford 1-(1-ethyl-5-phenyl-1H-pyrazol-4-yl)-3-(2-fluoro-4-(2-(1-methyl-1H-pyrazol-4-yl)pyridin-4-yloxy)phenyl)urea hydrochloride (29 mg, 8% yield). ¹H NMR (300 MHz, DMSO-*d*₆): δ 1.21 (t, 3 H), 3.93 (s, 3 H), 3.98 (q, 2 H), 7.05-7.08 (m, 2 H), 7.35-7.60 (m, 6 H), 7.75 (s, 1 H), 7.80 (s, 1 H), 8.30-8.30 (m, 2 H), 8.47 (s, 1 H), 8.51 (d, 1 H), 8.80 (s, 1 H), 8.90 (br s, 1 H); MS (ESI) m/z: 498.2 (M+H⁺).

Example 23: A solution of Example B17 (250 mg, 0.689 mmol), DIEA (356 mg, 2.76 mmol) and Example A1 (196 mg, 0.689 mmol) in DMSO (5 mL) was warmed to 75 °C and stirred overnight. The temperature was increased to 100 °C and stirring continued overnight. The mixture was cooled to RT and diluted with ethyl acetate (25 mL) and water (25 mL). The organic phase was washed with brine (20 mL), dried (Na₂SO₄), concentrated *in vacuo* and purified by column chromatography (methanol/dichloromethane) to give a film. The film was subjected to 4N HCl/dioxane to afford 1-(1-ethyl-3-phenyl-1H-pyrazol-4-yl)-3-(2-fluoro-4-(2-(1-methyl-1H-pyrazol-4-yl)pyridin-4-yloxy)phenyl)urea hydrochloride (95 mg, 27% yield). ¹H NMR (300 MHz, DMSO-*d*₆): δ 1.40 (t, 3 H), 3.93 (s, 3 H), 4.15 (q, 2 H), 7.10-7.18 (m, 2 H), 7.31-7.48 (m, 4 H), 7.62-7.68 (m, 2 H), 7.75 (s, 1 H), 7.99 (s, 1 H), 8.32 (m, 1 H), 8.48 (s, 1 H), 8.54 (d, 1 H), 8.59 (s, 1 H), 8.80 (s, 1 H), 9.10 (br s, 1 H); MS (ESI) m/z: 498.2 (M+H⁺).

Example 24: To a stirring solution of Example B18 (0.09 g, 0.363 mmol) and TEA (0.076 mL, 0.544 mmol) in dioxane (3.0 mL) was added DPPA (0.117 mL, 0.544 mmol). After stirring for 0.5h at RT, Example A1 (0.103 g, 0.363 mmol) was added and the reaction was heated at 100 °C for 3h. Water (15 mL) was added to reaction mixture and the resulting precipitate was collected by filtration. The crude product was purified by chromatography to afford 1-(2-fluoro-4-(2-(1-methyl-1H-pyrazol-4-yl)pyridin-4-yloxy)phenyl)-3-(1-(3-fluorophenyl)-3-isopropyl-1H-pyrazol-5-yl)urea (0.097 g, 50% yield) as a white solid. ¹H NMR (400 MHz, DMSO-d6): δ 9.00 (s, 1H), 8.92 (s, 1H), 8.38 (d, *J* = 5.6 Hz, 1H), 8.27 (s, 1H), 8.14 (t, *J* = 9.2 Hz, 1H), 7.97 (s, 1H), 7.59 (q, *J* = 6.8 Hz, 1H), 7.45-7.41 (m, 2H), 7.31-7.23 (m, 3H), 7.01 (d, *J* = 8.8 Hz, 1H), 6.68 (dd, *J* = 5.6, 2.4 Hz, 1H), 6.38 (s, 1H), 3.86 (s, 3H), 2.90 (m, 1H), 1.24 (d, *J* = 6.8 Hz, 6H); MS (ESI) m/z: 530.2 (M+H⁺).

Example 25: Using general method B, Example A8 (0.083 g, 0.21 mmol) and Example B19 (0.04 g, 0.21 mmol) in presence of N-methyl pyrrolidine (cat amount) were combined to afford 1-(2,3-difluoro-4-(2-(1-methyl-1H-pyrazol-4-yl)pyridin-4-yloxy)phenyl)-3-(3-(2-fluorophenyl)-1-methyl-1H-pyrazol-5-yl)urea as white solid (0.059 g, 53% yield). ¹H NMR (400 MHz, DMSO-*d*₆): δ 9.16 (s, 1H), 9.12 (s, 1H), 8.38 (d, *J* = 5.6 Hz, 1H), 8.28 (s, 1H), 8.02-7.98 (m, 2H), 7.90 (t, *J* = 8.0 Hz, 1H), 7.34-7.18 (m, 5H), 6.74 (d, *J*= 5.6 Hz, 1H), 6.65-6.64 (m, 1H), 3.84 (s, 3H), 3.77 (s, 3H); MS (ESI) m/z: 520.1 (M+H⁺).

Example 26: Using General Method C, Example B3 (0.085 g, 0.31 mmol), Example A10 (0.075 g, 0.26 mmol) in presence of triethylamine (0.08 g, 0.79 mmol) and DPPA (0.14 g, 0.52 mmol) were combined to afford 1-(3-tert-butyl-1-(3-cyanophenyl)-1H-pyrazol-5-yl)-3-(2-fluoro-5-(4-(1-methyl-1H-pyrazol-4-yl)pyrimidin-2-yloxy)phenyl)urea as a white solid (0.099 g, 68% yield). ¹H NMR (400 MHz, DMSO-*d*₆): δ 9.06 (d, J = 2.0 Hz, 1H), 8.97 (s, 1H), 8.46 (d, J= 5.2 Hz, 1H), 8.42 (s, 1H), 8.08 (s, 1H), 8.02 (t, J= 2.0 Hz, 1H), 7.96 (dd, J = 6.8 Hz, 2.8 Hz, 1H), 7.89-7.84 (m, 2H), 7.72 (t, J = 8.0 Hz, 1H), 7.45 (d, *J* = 5.2 Hz, 1H), 7.28 (dd, *J* = 10.8 Hz, 8.8 Hz, 1H), 6.87-6.83 (m, 1H), 6.39 (s, 1H), 3.87 (s, 3H), 1.24 (s, 9H); MS (ESI) m/z: 552.3 (M+H⁺).

Example 27: Using General Method C, Example B3 (0.088 g, 0.32 mmol), Example A11 (0.075 g, 0.25 mmol), triethylamine (0.076 g, 0.75 mmol) and DPPA (0.14 g, 0.5 mmol) were combined to afford 1-(3-tert-butyl-1-(3-cyanophenyl)-1H-pyrazol-5-yl)-3-(2-fluoro-4-methyl-5-(4-(1-methyl-1H-pyrazol-4-yl)pyrimidin-2-yloxy)phenyl)urea as a white solid (0.108 g, 76% yield). ¹H NMR (400 MHz, DMSO-*d*₆): δ 8.96 (d, *J* = 1.6 Hz, 1H), 8.91 (s, 1H), 8.44 (d, *J* = 5.2 Hz, 1H), 8.42 (s, 1H), 8.08 (s, 1H), 8.02 (t, *J* = 1.6 Hz, 1H), 7.88-7.82 (m, 3H), 7.71 (t, *J*= 8.0 Hz, 1H), 7.44 (d, *J*= 4.8 Hz, 1H), 7.21 (d, *J*= 12.0 Hz, 1H), 6.38 (s, 1H), 3.87 (s, 3H), 2.01 (s, 3H), 1.24 (s, 9H); MS (ESI) m/z: 566.3 (M+H⁺).

Using the synthetic procedures and methods described herein and methods known to those skilled in the art, the following compounds were made: 1-(5-chloro-2-phenylphenyl)-3-(2-fluoro-4-(2-(1-methyl-1H-pyrazol-4-yl)pyridin-4-yloxy)phenyl)urea, 4-(3-tert-butyl-5-(3-(2-fluoro-4-(2-(1-methyl-1H-pyrazol-4-yl)pyridin-4-yloxy)phenyl)ureldo)-1H-pyrazol-1-yl)benzyl acetate, 1-(3-tert-butyl-1-(4-fluorophenyl)-1H-pyrazol-5-yl)-3 -(2-fluoro-4-(2-(1-methyl-1H-pyrazol-4-yl)pyridin-4-yloxy)phenyl)urea, 1-(1-(4-cyanophenyl)-3-isopropyl-1H-pyrazol-5-yl)-3-(2-fluoro-4-(2-(1-methyl-1H-pyrazol-4-yl)pyridin-4-yloxy)phenyl)urea, ethyl 3-(3-tert-butyl-5-(3-(2-fluoro-4-(2-(1-methyl-1H-pyrazol-4-yl)pyridin-4-yloxy)phenyl)ureido)-1H-pyrazol-1-yl)benzoate, 3-(3-tert-butyl-5-(3-(2-fluoro-4-(2-(1-methyl-1H-pyrazol-4-yl)pyridin-4-yloxy)phenyl)ureido)-1H-pyrazol-1-yl)benzoic acid, 1-(3-ethyl-1-(3-(hydroxymethyl)phenyl)-1H-pyrazol-5-yl)-3-(2-fluoro-4-(2-(1-methyl-1H-pyrazol-4-yl)pyridin-4-yloxy)phenyl)urea, 1-(1-(3-cyanophenyl)-3-methyl-1H-pyrazol-5-yl)-3-(2-fluoro-4-(2-(1-methyl-1H-pyrazol-4-yl)pyridin-4-yloxy)phenyl)urea, 1-(3-(3-cyanophenyl)-1-isopropyl-1H-pyrazol-4-yl)-3-(2-fluoro-4-(2-(1-methyl-1H-pyrazol-4-yl)pyridin-4-yloxy)phenyl)urea, 1-(3-tert-butyl-1-phenyl-1H-pyrazol-5-yl)-3-(2,3-difluoro-4-(2-(1-methyl-1H-pyrazol-4-yl)pyridin-4-yloxy)phenyl)urea, 1-(1,3-diphenyl-1H-pyrazol-5-yl)-3-(2-fluoro-4-(2-(1-methyl-1H-pyrazol-4-yl)pyridin-4-yloxy)phenyl)urea, and 1-(2,3-difluoro-4-(2-(1-methyl-1H-pyrazol-4-yl)pyridin-4-yloxy)phenyl)-3-(1,3-diphenyl-1H-pyrazol-5-yl)urea.

Using the synthetic procedures and methods described herein and methods known to those skilled in the art, the following compounds are made:
1-(4-(2-(1H-pyrazol-4-yl)pyridin-4-yloxy)-2-fluorophenyl)-3-(3-tert-butyl-1-(4-cyano-3-fluorophenyl)-1H-pyrazol-5-yl)urea, 1-(3-tert-butyl-1-(4-cyano-3-fluorophenyl)-1H-pyrazol-5-yl)-3-(2-fluoro-4-(2-(1-methyl-1H-pyrazol-4-yl)pyridin-4-yloxy)phenyl)urea, 1-(4-(2-(1H-pyrazol-4-yl)pyridin-4-yloxy)-3-methylphenyl)-3-(3-tert-butyl-1-(4-cyano-3-fluorophenyl)-1H-pyrazol-5-yl)urea, 1-(3-tert-butyl-1-(4-cyano-3-fluorophenyl)-1H-pyrazol-5-yl)-3-(3-methyl-4-(2-(1-methyl-1H-pyrazol-4-yl)pyridin-4-yloxy)phenyl)urea, 1-(4-(2-(1H-pyrazol-4-yl)pyridin-4-yloxy)-2-fluorophenyl)-3-(3-tert-butyl-1-(4-cyanophenyl)-1H-pyrazol-5-yl)urea, 1-(3-tert-butyl-1-(4-cyanophenyl)-1H-pyrazol-5-yl)-3-(2-fluoro-4-(2-(1-methyl-1H-pyrazol-3-yl)pyridin-4-yloxy)phenyl)urea, 1-(4-(2-(1H-pyrazol-3-yl)pyridin-4-yloxy)-2-fluorophenyl)-3-(3-tert-butyl-1-(4-cyanophenyl)-1H-pyrazol-5-yl)urea, 1-(3-tert-butyl-1-(4-cyanophenyl)-1H-pyrazol-5-yl)-3-(2-fluoro-4-(2-(1-methyl-1H-pyrazol-5-yl)pyridin-4-yloxy)phenyl)urea, 1-(4-(2-(1H-pyrazol-5-yl)pyridin-4-yloxy)-2-fluorophenyl)-3-(3-tert-butyl-1-(4-cyanophenyl)-1H-pyrazol-5-yl)urea, 1-(3-tert-butyl-1-(4-cyanophenyl)-1H-pyrazol-5-yl)-3-(2-fluoro-4-(2-(isoxazol-4-yl)pyridin-4-yloxy)phenyl)urea, 1-(3-tert-butyl-1-(4-cyanophenyl)-1H-pyrazol-5-yl)-3-(2-fluoro-4-(2-(4-methyl-4H-1,2,4-triazol-3-yl)pyridin-4-yloxy)phenyl)urea, 1-(1-tert-butyl-3-(3-cyanophenyl)-1H-pyrazol-4-yl)-3-(2-fluoro-4-(2-(1-methyl-1H-pyrazol-4-yl)pyridin-4-yloxy)phenyl)urea, 1-(3-(3-cyanophenyl)-1-isopropyl-1H-pyrazol-4-yl)-3-(2-fluoro-4-(2-(1-methyl-1H-pyrazol-3-yl)pyridin-4-yloxy)phenyl)urea, 1-(1-tert-butyl-3-(3-cyanophenyl)-1H-pyrazol-4-yl)-3-(2-fluoro-4-(2-(1-methyl-1H-pyrazol-3-yl)pyridin-4-yloxy)phenyl)urea, 1-(3-(3-cyanophenyl)-1-isopropyl-1H-pyrazol-4-yl)-3-(2-fluoro-4-(2-(1-methyl-1H-pyrazol-5-yl)pyridin-4-yloxy)phenyl)urea, 1-(1-tert-butyl-3-(3-cyanophenyl)-1H-pyrazol-4-yl)-3-(2-fluoro-4-(2-(1-methyl-1H-pyrazol-5-yl)pyridin-4-yloxy)phenyl)urea, 1-(3-tert-butyl-1-(3-cyanophenyl)-1H-pyrazol-5-yl)-3-(3-methyl-4-(2-(1-methyl-1H-pyrazol-4-yl)pyridin-4-yloxy)phenyl)urea, 1-(3-tert-butyl-1-(3-cyanophenyl)-1H-pyrazol-5-yl)-3-(3-methyl-4-(2-(1-methyl-1H-pyrazol-5-yl)pyridin-4-yloxy)phenyl)urea, 1-(3-tert-butyl-1-(3-cyanophenyl)-1H-pyrazol-5-yl)-3-(3-methyl-4-(2-(1-methyl-1H-pyrazol-3-yl)pyridin-4-yloxy)phenyl)urea, 1-(3-tert-butyl-1-(3-cyanophenyl)-1H-pyrazol-5-yl)-3-(2-fluoro-4-(2-(2-methyl-2H-1,2,3-triazol-4-yl)pyridin-4-yloxy)phenyl)urea, 1-(3-tert-butyl-1-(3-cyanophenyl)-1H-pyrazol-5-yl)-3-(2-fluoro-4-(2-(1-methyl-1H-pyrazol-3-yl)pyridin-4-yloxy)phenyl)urea, 1-(3-tert-butyl-1-(3-cyanophenyl)-1H-pyrazol-5-yl)-3-(2-fluoro-4-(2-(1-methyl-1H-pyrazol-5-yl)pyridin-4-yloxy)phenyl)urea, 1-(3-tert-butyl-1-(3-cyanophenyl)-1H-pyrazol-5-yl)-3-(2-fluoro-4-(2-(5-methyl-1,2,4-thiadiazol-3-yl)pyridin-4-yloxy)phenyl)urea, 1-(3-tert-butyl-1-(3-cyanophenyl)-1H-pyrazol-5-yl)-3-(2-fluoro-4-(2-(4-methyl-4H-1,2,4-triazol-3-yl)pyridin-4-yloxy)phenyl)urea, 1-(3-tert-butyl-1-(3-cyanophenyl)-1H-pyrazol-5-yl)-3-(2-fluoro-4-(2-(3-methyl-3H-1,2,3-triazol-4-yl)pyridin-4-yloxy)phenyl)urea, 1-(3-tert-butyl-1-(3-cyanophenyl)-1H-pyrazol-5-yl)-3-(2-fluoro-4-(2-(isoxazol-4-yl)pyridin-4-yloxy)phenyl)urea, 1-(3-tert-butyl-1-(3-cyanophenyl)-1H-pyrazol-5-yl)-3-(2-fluoro-4-(2-(isoxazol-3-yl)pyridin-4-yloxy)phenyl)urea, 1-(1-(4-cyanophenyl)-3-isopropyl-1H-pyrazol-5-yl)-3-(2-fluoro-4-(2-(1-methyl-1H-pyrazol-3-yl)pyridin-4-yloxy)phenyl)urea, 1-(1-(4-cyanophenyl)-3-isopropyl-1H-pyrazol-5-yl)-3-(2-fluoro-4-(2-(1-methyl-1H-pyrazol-5-yl)pyridin-4-yloxy)phenyl)urea, 1-(1-tert-butyl-3-(3-(hydroxymethyl)phenyl)-1H-pyrazol-4-yl)-3-(2-fluoro-4-(2-(1-methyl-1H-pyrazol-4-yl)pyridin-4-yloxy)phenyl)urea, 1-(4-(2-(1H-pyrazol-4-yl)pyridin-4-yloxy)-2-fluorophenyl)-3-(1-tert-butyl-3-(3-(hydroxymethyl)phenyl)-1H-pyrazol-4-yl)urea, 1-(1-tert-butyl-3-(3-(hydroxymethyl)phenyl)-1H-pyrazol-4-yl)-3-(2-fluoro-4-(2-(1-methyl-1H-pyrazol-3-yl)pyridin-4-yloxy)phenyl)urea, 1-(4-(2-(1H-pyrazol-3-yl)pyridin-4-yloxy)-2-fluorophenyl)-3-(1-tert-butyl-3-(3-(hydroxymethyl)phenyl)-1H-pyrazol-4-yl)urea, 1-(1-tert-butyl-3-(3-(hydroxymethyl)phenyl)-1H-pyrazol-4-yl)-3-(2-fluoro-4-(2-(1-methyl-1H-pyrazol-5-yl)pyridin-4-yloxy)phenyl)urea, 1-(4-(2-(1H-pyrazol-5-yl)pyridin-4-yloxy)-2-fluorophenyl)-3-(1-tert-butyl-3-(3-(hydroxymethyl)phenyl)-1H-pyrazol-4-yl)urea, 1-(1-tert-butyl-3-(3-(hydroxymethyl)phenyl)-1H-pyrazol-4-yl)-3-(2-fluoro-4-(2-(isoxazol-4-yl)pyridin-4-yloxy)phenyl)urea, 1-(3-tert-butyl-1-(3-(hydroxymethyl)phenyl)-1H-pyrazol-5-yl)-3-(2-fluoro-4-(2-(1-methyl-1H-pyrazol-3-yl)pyridin-4-yloxy)phenyl)urea, 1-(4-(2-(1H-pyrazol-4-yl)pyridin-4-yloxy)-2-fluorophenyl)-3-(3-tert-butyl-1-(3-(hydroxymethyl)phenyl)-1H-pyrazol-5-yl)urea, 1-(4-(2-(1H-pyrazol-3-yl)pyridin-4-yloxy)-2-fluorophenyl)-3-(3-tert-butyl-1-(3-(hydroxymethyl)phenyl)-1H-pyrazol-5-yl)urea, 1-(3-tert-butyl-1-(3-(hydroxymethyl)phenyl)-1H-pyrazol-5-yl)-3-(2-fluoro-4-(2-(1-methyl-1H-pyrazol-5-yl)pyridin-4-yloxy)phenyl)urea, 1-(3-tert-butyl-1-(3-(hydroxymethyl)phenyl)-1H-pyrazol-5-yl)-3-(2-fluoro-4-(2-(isoxazol-4-yl)pyridin-4-yloxy)phenyl)urea, 1-(3-tert-butyl-1-(3-(hydroxymethyl)phenyl)-1H-pyrazol-5-yl)-3-(2-fluoro-4-(2-(isoxazol-3-yl)pyridin-4-yloxy)phenyl)urea, 1-(3-tert-butyl-1-(3-cyanophenyl)-1H-pyrazol-5-yl)-3-(2-fluoro-4-(2-(4-methyloxazol-2-yl)pyridin-4-yloxy)phenyl)urea, 1-(3-tert-butyl-1-(3-cyanophenyl)-1H-pyrazol-5-yl)-3-(2-fluoro-4-(2-(3-methyl-1,2,4-thiadiazol-5-yl)pyridin-4-yloxy)phenyl)urea, 1-(3-tert-butyl-1-(3-cyanophenyl)-1H-pyrazol-5-yl)-3-(2-fluoro-4-(2-(isoxazol-5-yl)pyridin-4-yloxy)phenyl)urea, 1-(4-(2-(4H-1,2,4-triazol-4-yl)pyridin-4-yloxy)-2-fluorophenyl)-3-(3-tert-butyl-1-(3-cyanophenyl)-1H-pyrazol-5-yl)urea, 1-(3-tert-butyl-1-(3-cyanophenyl)-1H-pyrazol-5-yl)-3-(2-fluoro-4-(2-(isothiazol-4-yl)pyridin-4-yloxy)phenyl)urea,1-(3-tert-butyl-1-(3-cyanophenyl)-1H-pyrazol-5-yl)-3-(2-fluoro-4-(2-(3-methyl-1H-1,2,4-triazol-1-yl)pyridin-4-yloxy)phenyl)urea, 1-(3-tert-butyl-1-(3-cyanophenyl)-1H-pyrazol-5-yl)-3-(2-fluoro-4-(2-(isothiazol-3-yl)pyridin-4-yloxy)phenyl)urea, 1-(3-tert-butyl-1-(3-cyanophenyl)-1H-pyrazol-5-yl)-3-(2-fluoro-4-(2-(3-methyl-1H-pyrazol-1-yl)pyridin-4-yloxy)phenyl)urea, 1-(3-tert-butyl-1-(3-cyanophenyl)-1H-pyrazol-5-yl)-3-(2-fluoro-4-(2-(isothiazol-5-yl)pyridin-4-yloxy)phenyl)urea, 1-(3-tert-butyl-1-(3-cyanophenyl)-1H-pyrazol-5-yl)-3-(2-fluoro-4-(2-(4-methyl-2H-1,2,3-triazol-2-yl)pyridin-4-yloxy)phenyl)urea, 1-(1-(3-cyanophenyl)-3-isopropyl-1H-pyrazol-5-yl)-3-(2-fluoro-4-methyl-5-(4-(1-methyl-1H-pyrazol-4-yl)pyrimidin-2-yloxy)phenyl)urea, 1-(1-(4-cyanophenyl)-3-isopropyl-1H-pyrazol-5-yl)-3-(2-fluoro-4-methyl-5-(4-(1-methyl-1H-pyrazol-4-yl)pyrimidin-2-yloxy)phenyl)urea, 1-(2-fluoro-4-methyl-5-(4-(1-methyl-1H-pyrazol-4-yl)pyrimidin-2-yloxy)phenyl)-3-(1-(4-(hydroxymethyl)phenyl)-3-isopropyl-1H-pyrazol-5-yl)urea, 1-(2-fluoro-4-methyl-5-(4-(1-methyl-1H-pyrazol-4-yl)pyrimidin-2-yloxy)phenyl)-3-(1-(3-(hydroxymethyl)phenyl)-3-isopropyl-1H-pyrazol-5-yl)urea, 1-(1-(3-cyano-4-methylphenyl)-3-isopropyl-1H-pyrazol-5-yl)-3-(2-fluoro-4-methyl-5-(4-(1-methyl-1H-pyrazol-4-yl)pyrimidin-2-yloxy)phenyl)urea, 1-(1-(3-cyano-4-fluorophenyl)-3-isopropyl-1H-pyrazol-5-yl)-3-(2-fluoro-4-methyl-5-(4-(1-methyl-1H-pyrazol-4-yl)pyrimidin-2-yloxy)phenyl)urea, 1-(1-(3-cyanophenyl)-3-isopropyl-1H-pyrazol-5-yl)-3-(2-fluoro-5-(4-(1-methyl-1H-pyrazol-4-yl)pyrimidin-2-yloxy)phenyl)urea, 1-(1-(4-cyanophenyl)-3-isopropyl-1H-pyrazol-5-yl)-3-(2-fluoro-5-(4-(1-methyl-1H-pyrazol-4-yl)pyrimidin-2-yloxy)phenyl)urea, 1-(2-fluoro-5-(4-(1-methyl-1H-pyrazol-4-yl)pyrimidin-2-yloxy)phenyl)-3-(1-(4-(hydroxymethyl)phenyl)-3-isopropyl-1H-pyrazol-5-yl)urea, 1-(2-fluoro-5-(4-(1-methyl-1H-pyrazol-4-yl)pyrimidin-2-yloxy)phenyl)-3-(1-(3-(hydroxymethyl)phenyl)-3-isopropyl-1H-pyrazol-5-yl)urea, 1-(1-(3-cyano-4-methylphenyl)-3-isopropyl-1H-pyrazol-5-yl)-3-(2-fluoro-5-(4-(1-methyl-1H-pyrazol-4-yl)pyrimidin-2-yloxy)phenyl)urea, 1-(1-(3-cyano-4-fluorophenyl)-3-isopropyl-1H-pyrazol-5-yl)-3-(2-fluoro-5-(4-(1-methyl-1H-pyrazol-4-yl)pyrimidin-2-yloxy)phenyl)urea, 1-(5-(4-(1H-pyrazol-4-yl)pyrimidin-2-yloxy)-2-fluoro-4-methylphenyl)-3-(3-tert-butyl-1-(3-cyano-4-methylphenyl)-1H-pyrazol-5-yl)urea,1-(5-(4-(1H-pyrazol-4-yl)pyrimidin-2-yloxy)-2-fluoro-4-methylphenyl)-3-(3-tert-butyl-1-(3-cyano-4-fluorophenyl)-1H-pyrazol-5-yl)urea, 1-(3-tert-butyl-1-(3-cyano-4-methylphenyl)-1H-pyrazol-5-yl)-3-(2-fluoro-5-(4-(1-methyl-1H-pyrazol-4-yl)pyrimidin-2-yloxy)phenyl)urea, 1-(5-(4-(1H-pyrazol-4-yl)pyrimidin-2-yloxy)-2-fluorophenyl)-3-(3-tert-butyl-1-(3-cyano-4-methylphenyl)-1H-pyrazol-5-yl)urea, 1-(5-(4-(1H-pyrazol-4-yl)pyrimidin-2-yloxy)-2-fluorophenyl)-3-(3-tert-butyl-1-(3-cyano-4-fluorophenyl)-1H-pyrazol-5-yl)urea, 1-(3-tert-butyl-1-(3-cyano-4-fluorophenyl)-1H-pyrazol-5-yl)-3-(2-fluoro-5-(4-(1-methyl-1H-pyrazol-4-yl)pyrimidin-2-yloxy)phenyl)urea, 1-(1-(3-cyanophenyl)-3-(1-hydroxy-2-methylpropan-2-yl)-1H-pyrazol-5-yl)-3-(2-fluoro-4-(2-(1-methyl-1H-pyrazol-4-yl)pyridin-4-yloxy)phenyl)urea, 1-(1-(3-cyanophenyl)-3-(1-hydroxyethyl)-1H-pyrazol-5-yl)-3-(2-fluoro-4-(2-(1-methyl-1H-pyrazol-4-yl)pyridin-4-yloxy)phenyl)urea, 1-(1-(3-cyanophenyl)-3-(2-hydroxypropan-2-yl)-1H-pyrazol-5-yl)-3-(2-fluoro-4-(2-(1-methyl-1H-pyrazol-4-yl)pyridin-4-yloxy)phenyl)urea, 1-(3-tert-butyl-1-(4-cyanophenyl)-1H-pyrazol-5-yl)-3-(2-fluoro-4-(2-(oxazol-5-yl)pyridin-4-yloxy)phenyl)urea, 1-(3-tert-butyl-1-(3-cyanophenyl)-1H-pyrazol-5-yl)-3-(2-fluoro-4-(2-(oxazol-4-yl)pyridin-4-yloxy)phenyl)urea, 1-(3-tert-butyl-1-(4-(hydroxymethyl)phenyl)-1H-pyrazol-5-yl)-3-(2-fluoro-4-(2-(oxazol-4-yl)pyridin-4-yloxy)phenyl)urea, 1-(3-tert-butyl-1-(3-(hydroxymethyl)phenyl)-1H-pyrazol-5-yl)-3-(2-fluoro-4-(2-(oxazol-5-yl)pyridin-4-yloxy)phenyl)urea, 1-(3-benzyl-1-phenyl-1H-pyrazol-5-yl)-3-(2-fluoro-4-(2-(1-methyl-1H-pyrazol-4-yl)pyridin-4-yloxy)phenyl)urea, 1-(2,3-difluoro-4-(2-(1-methyl-1H-pyrazol-4-yl)pyridin-4-yloxy)phenyl)-3-(3-phenylisoxazol-5-yl)urea, 1-(2,3-difluoro-4-(2-(1-methyl-1H-pyrazol-4-yl)pyridin-4-yloxy)phenyl)-3-(1-methyl-3-phenyl-1H-pyrazol-5-yl)urea, 1-(2,3-difluoro-4-(2-(1-methyl-1H-pyrazol-4-yl)pyridin-4-yloxy)phenyl)-3-(5-phenylpyridin-3-yl)urea, 1-(3-tert-butyl-1-(4-cyano-3-fluorophenyl)-1H-pyrazol-5-yl)-3-(2-fluoro-4-(2-(isothiazol-4-yl)pyridin-4-yloxy)phenyl)urea, 1-(3-tert-butyl-1-(4-cyano-3-fluorophenyl)-1H-pyrazol-5-yl)-3-(2-fluoro-4-(2-(isothiazol-3-yl)pyridin-4-yloxy)phenyl)urea, 1-(4-(2-(1H-pyrazol-4-yl)pyridin-4-yloxy)-2-fluorophenyl)-3-(3-tert-butyl-1-(4-cyano-3-fluorophenyl)-1H-pyrazol-5-yl)urea, 1-(3-tert-butyl-1-(4-cyano-3-fluorophenyl)-1H-pyrazol-5-yl)-3-(2-fluoro-4-(2-(1-methyl-1H-pyrazol-4-yl)pyridin-4-yloxy)phenyl)urea, 1-(4-(2-(1H-pyrazol-4-yl)pyridin-4-yloxy)-3 -methylphenyl)-3-(3 -tert-butyl-1-(4-cyano-3-fluorophenyl)-1H-pyrazol-5-yl)urea, 1-(3-tert-butyl-1-(4-cyano-3-fluorophenyl)-1H-pyrazol-5-yl)-3-(3-methyl-4-(2-(1-methyl-1H-pyrazol-4-yl)pyridin-4-yloxy)phenyl)urea, 1-(4-(2-(1H-pyrazol-4-yl)pyridin-4-yloxy)-2-fluorophenyl)-3-(3-tert-butyl-1-(4-cyanophenyl)-1H-pyrazol-5-yl)urea, 1-(3-tert-butyl-1-(4-cyanophenyl)-1H-pyrazol-5-yl)-3-(2-fluoro-4-(2-(1-methyl-1H-pyrazol-3-yl)pyridin-4-yloxy)phenyl)urea, 1-(4-(2-(1H-pyrazol-3-yl)pyridin-4-yloxy)-2-fluorophenyl)-3-(3-tert-butyl-1-(4-cyanophenyl)-1H-pyrazol-5-yl)urea, 1-(3-tert-butyl-1-(4-cyanophenyl)-1H-pyrazol-5-yl)-3-(2-fluoro-4-(2-(1-methyl-1H-pyrazol-5-yl)pyridin-4-yloxy)phenyl)urea, 1-(4-(2-(1H-pyrazol-5-yl)pyridin-4-yloxy)-2-fluorophenyl)-3-(3-tert-butyl-1-(4-cyanophenyl)-1H-pyrazol-5-yl)urea, 1-(3-tert-butyl-1-(4-cyanophenyl)-1H-pyrazol-5-yl)-3-(2-fluoro-4-(2-(isoxazol-4-yl)pyridin-4-yloxy)phenyl)urea, 1-(3-tert-butyl-1-(4-cyanophenyl)-1H-pyrazol-5-yl)-3-(2-fluoro-4-(2-(4-methyl-4H-1,2,4-triazol-3-yl)pyridin-4-yloxy)phenyl)urea, 1-(1-tert-butyl-3-(3-cyanophenyl)-1H-pyrazol-4-yl)-3-(2-fluoro-4-(2-(1-methyl-1H-pyrazol-4-yl)pyridin-4-yloxy)phenyl)urea, 1-(3-(3-cyanophenyl)-1-isopropyl-1H-pyrazol-4-yl)-3-(2-fluoro-4-(2-(1-methyl-1H-pyrazol-3-yl)pyridin-4-yloxy)phenyl)urea, 1-(1-tert-butyl-3-(3-cyanophenyl)-1H-pyrazol-4-yl)-3-(2-fluoro-4-(2-(1-methyl-1H-pyrazol-3-yl)pyridin-4-yloxy)phenyl)urea, 1-(3-(3-cyanophenyl)-1-isopropyl-1H-pyrazol-4-yl)-3-(2-fluoro-4-(2-(1-methyl-1H-pyrazol-5-yl)pyridin-4-yloxy)phenyl)urea, 1-(1-tert-butyl-3-(3-cyanophenyl)-1H-pyrazol-4-yl)-3-(2-fluoro-4-(2-(1-methyl-1H-pyrazol-5-yl)pyridin-4-yloxy)phenyl)urea, 1-(3-tert-butyl-1-(3-cyanophenyl)-1H-pyrazol-5-yl)-3-(3-methyl-4-(2-(1-methyl-1H-pyrazol-4-yl)pyridin-4-yloxy)phenyl)urea, 1-(3-tert-butyl-1-(3-cyanophenyl)-1H-pyrazol-5-yl)-3-(3-methyl-4-(2-(1-methyl-1H-pyrazol-5-yl)pyridin-4-yloxy)phenyl)urea, 1-(3-tert-butyl-1-(3-cyanophenyl)-1H-pyrazol-5-yl)-3-(3-methyl-4-(2-(1-methyl-1H-pyrazol-3-yl)pyridin-4-yloxy)phenyl)urea, 1-(3-tert-butyl-1-(3-cyanophenyl)-1H-pyrazol-5-yl)-3-(2-fluoro-4-(2-(2-methyl-2H-1,2,3-triazol-4-yl)pyridin-4-yloxy)phenyl)urea, 1-(3-tert-butyl-1-(3-cyanophenyl)-1H-pyrazol-5-yl)-3-(2-fluoro-4-(2-(1-methyl-1H-pyrazol-3-yl)pyridin-4-yloxy)phenyl)urea, 1-(3-tert-butyl-1-(3-cyanophenyl)-1H-pyrazol-5-yl)-3-(2-fluoro-4-(2-(1-methyl-1H-pyrazol-5-yl)pyridin-4-yloxy)phenyl)urea, 1-(3-tert-butyl-1-(3-cyanophenyl)-1H-pyrazol-5-yl)-3-(2-fluoro-4-(2-(5-methyl-1,2,4-thiadiazol-3-yl)pyridin-4-yloxy)phenyl)urea, 1-(3-tert-butyl-1-(3-cyanophenyl)-1H-pyrazol-5-yl)-3-(2-fluoro-4-(2-(4-methyl-4H-1,2,4-triazol-3-yl)pyridin-4-yloxy)phenyl)urea, 1-(3-tert-butyl-1-(3-cyanophenyl)-1H-pyrazol-5-yl)-3-(2-fluoro-4-(2-(3-methyl-3H-1,2,3-triazol-4-yl)pyridin-4-yloxy)phenyl)urea, 1-(3-tert-butyl-1-(3-cyanophenyl)-1H-pyrazol-5-yl)-3-(2-fluoro-4-(2-(isoxazol-4-yl)pyridin-4-yloxy)phenyl)urea, 1-(3-tert-butyl-1-(3-cyanophenyl)-1H-pyrazol-5-yl)-3-(2-fluoro-4-(2-(isoxazol-3-yl)pyridin-4-yloxy)phenyl)urea, 1-(1-(4-cyanophenyl)-3-isopropyl-1H-pyrazol-5-yl)-3-(2-fluoro-4-(2-(1-methyl-1H-pyrazol-3-yl)pyridin-4-yloxy)phenyl)urea, 1-(1-(4-cyanophenyl)-3-isopropyl-1H-pyrazol-5-yl)-3-(2-fluoro-4-(2-(1-methyl-1H-pyrazol-5-yl)pyridin-4-yloxy)phenyl)urea, 1-(1-tert-butyl-3-(3-(hydroxymethyl)phenyl)-1H-pyrazol-4-yl)-3-(2-fluoro-4-(2-(1-methyl-1H-pyrazol-4-yl)pyridin-4-yloxy)phenyl)urea, 1-(4-(2-(1H-pyrazol-4-yl)pyridin-4-yloxy)-2-fluorophenyl)-3-(1-tert-butyl-3-(3-(hydroxymethyl)phenyl)-1H-pyrazol-4-yl)urea, 1-(1-tert-butyl-3-(3-(hydroxymethyl)phenyl)-1H-pyrazol-4-yl)-3-(2-fluoro-4-(2-(1-methyl-1H-pyrazol-3-yl)pyridin-4-yloxy)phenyl)urea, 1-(4-(2-(1H-pyrazol-3-yl)pyridin-4-yloxy)-2-fluorophenyl)-3-(1-tert-butyl-3-(3-(hydroxymethyl)phenyl)-1H-pyrazol-4-yl)urea, 1-(1-tert-butyl-3-(3-(hydroxymethyl)phenyl)-1H-pyrazol-4-yl)-3-(2-fluoro-4-(2-(1-methyl-1H-pyrazol-5-yl)pyridin-4-yloxy)phenyl)urea, 1-(4-(2-(1H-pyrazol-5-yl)pyridin-4-yloxy)-2-fluorophenyl)-3-(1-tert-butyl-3-(3-(hydroxymethyl)phenyl)-1H-pyrazol-4-yl)urea, 1-(1-tert-butyl-3-(3-(hydroxymethyl)phenyl)-1H-pyrazol-4-yl)-3-(2-fluoro-4-(2-(is oxazol-4-yl)pyridin-4-yloxy)phenyl)urea, 1-(3-tert-butyl-1-(3-(hydroxymethyl)phenyl)-1H-pyrazol-5-yl)-3-(2-fluoro-4-(2-(1-methyl-1H-pyrazol-3-yl)pyridin-4-yloxy)phenyl)urea, 1-(4-(2-(1H-pyrazol-4-yl)pyridin-4-yloxy)-2-fluorophenyl)-3-(3-tert-butyl-1-(3-(hydroxymethyl)phenyl)-1H-pyrazol-5-yl)urea, 1-(4-(2-(1H-pyrazol-3-yl)pyridin-4-yloxy)-2-fluorophenyl)-3-(3-tert-butyl-1-(3-(hydroxymethyl)phenyl)-1H-pyrazol-5-yl)urea, 1-(3-tert-butyl-1-(3-(hydroxymethyl)phenyl)-1H-pyrazol-5-yl)-3-(2-fluoro-4-(2-(1-methyl-1H-pyrazol-5-yl)pyridin-4-yloxy)phenyl)urea, 1-(3-tert-butyl-1-(3-(hydroxymethyl)phenyl)-1H-pyrazol-5-yl)-3-(2-fluoro-4-(2-(isoxazol-4-yl)pyridin-4-yloxy)phenyl)urea, 1-(3-tert-butyl-1-(3-(hydroxymethyl)phenyl)-1H-pyrazol-5-yl)-3-(2-fluoro-4-(2-(isoxazol-3-yl)pyridin-4-yloxy)phenyl)urea, 1-(3-tert-butyl-1-(3-cyanophenyl)-1H-pyrazol-5-yl)-3-(2-fluoro-4-(2-(4-methyloxazol-2-yl)pyridin-4-yloxy)phenyl)urea, 1-(3-tert-butyl-1-(3-cyanophenyl)-1H-pyrazol-5-yl)-3-(2-fluoro-4-(2-(3-methyl-1,2,4-thiadiazol-5-yl)pyridin-4-yloxy)phenyl)urea, 1-(3-tert-butyl-1-(3-cyanophenyl)-1H-pyrazol-5-yl)-3-(2-fluoro-4-(2-(isoxazol-5-yl)pyridin-4-yloxy)phenyl)urea, 1-(4-(2-(4H-1,2,4-triazol-4-yl)pyridin-4-yloxy)-2-fluorophenyl)-3-(3-tert-butyl-1-(3-cyanophenyl)-1H-pyrazol-5-yl)urea, 1-(3-tert-butyl-1-(3-cyanophenyl)-1H-pyrazol-5-yl)-3-(2-fluoro-4-(2-(isothiazol-4-yl)pyridin-4-yloxy)phenyl)urea, 1-(3-tert-butyl-1-(3-cyanophenyl)-1H-pyrazol-5-yl)-3-(2-fluoro-4-(2-(3-methyl-1H-1,2,4-triazol-1-yl)pyridin-4-yloxy)phenyl)urea, 1-(3-tert-butyl-1-(3-cyanophenyl)-1H-pyrazol-5-yl)-3-(2-fluoro-4-(2-(isothiazol-3-yl)pyridin-4-yloxy)phenyl)urea, 1-(3-tert-butyl-1-(3-cyanophenyl)-1H-pyrazol-5-yl)-3-(2-fluoro-4-(2-(3-methyl-1H-pyrazol-1-yl)pyridin-4-yloxy)phenyl)urea, 1-(3-tert-butyl-1-(3-cyanophenyl)-1H-pyrazol-5-yl)-3-(2-fluoro-4-(2-(isothiazol-5-yl)pyridin-4-yloxy)phenyl)urea, 1-(3-tert-butyl-1-(3-cyanophenyl)-1H-pyrazol-5-yl)-3-(2-fluoro-4-(2-(4-methyl-2H-1,2,3-triazol-2-yl)pyridin-4-yloxy)phenyl)urea, 1-(1-(3-cyanophenyl)-3-(1-hydroxyethyl)-1H-pyrazol-5-yl)-3-(2-fluoro-5-(2-(1-methyl-1H-pyrazol-4-yl)pyridin-4-yloxy)phenyl)urea, 1-(1-(3-cyanophenyl)-3-(2-hydroxypropan-2-yl)-1H-pyrazol-5-yl)-3-(2-fluoro-5-(2-(1-methyl-1H-pyrazol-4-yl)pyridin-4-yloxy)phenyl)urea, 1-(3-tert-butyl-1-(3-cyanophenyl)-1H-pyrazol-5-yl)-3-(2-fluoro-5-(2-(1-methyl-1H-pyrazol-4-yl)pyrimidin-4-yloxy)phenyl)urea, 1-(5-(2-(1H-pyrazol-4-yl)pyrimidin-4-yloxy)-2-fluorophenyl)-3-(3-tert-butyl-1-(3-cyanophenyl)-1H-pyrazol-5-yl)urea, 1-(1-(3-cyanophenyl)-3-isopropyl-1H-pyrazol-5-yl)-3-(2-fluoro-5-(2-(1-methyl-1H-pyrazol-4-yl)pyrimidin-4-yloxy)phenyl)urea, 1-(5-(2-(1H-pyrazol-4-yl)pyrimidin-4-yloxy)-2-fluorophenyl)-3-(1-(3-cyanophenyl)-3-isopropyl-1H-pyrazol-5-yl)urea, 1-(5-(4-(1H-pyrazol-4-yl)pyrimidin-2-yloxy)-2-fluorophenyl)-3 -(3-tert-butyl-1-(3-cyanophenyl)-1H-pyrazol-5 - yl)urea, 1-(5-(4-(1H-pyrazol-4-yl)pyrimidin-2-yloxy)-2-fluorophenyl)-3-(1-(3-cyanophenyl)-3-isopropyl-1H-pyrazol-5-yl)urea, 1-(3-fluoro-4-(2-(1-methyl-1H-pyrazol-4-yl)pyridin-4-yloxy)phenyl)-3-(6-(4-fluorophenoxy)benzo[d]thiazol-2-yl)urea, 1-(3-fluoro-4-(2-(1-methyl-1H-pyrazol-4-yl)pyridin-4-yloxy)phenyl)-3-(6-phenoxybenzo[d]thiazol-2-yl)urea, 1-(3-fluoro-4-(2-(1-methyl-1H-pyrazol-4-yl)pyridin-4-yloxy)phenyl)-3-(6-phenoxybenzo[d]oxazol-2-yl)urea, 1-(3-fluoro-4-(2-(1-methyl-1H-pyrazol-4-yl)pyridin-4-yloxy)phenyl)-3-(2-phenoxyquinolin-6-yl)urea, 1-(3-fluoro-4-(2-(1-methyl-1H-pyrazol-4-yl)pyridin-4-yloxy)phenyl)-3-(2-oxo-5-phenoxyindolin-3-yl)urea, 1-(3-fluoro-4-(2-(1-methyl-1H-pyrazol-4-yl)pyridin-4-yloxy)phenyl)-3-(5-phenoxy-1H-indazol-3-yl)urea, 1-(3-fluoro-4-(2-(1-methyl-1H-pyrazol-4-yl)pyridin-4-yloxy)phenyl)-3-(6-phenoxy-1H-indazol-3-yl)urea, 1-(3-fluoro-4-(2-(1-methyl-1H-pyrazol-4-yl)pyridin-4-yloxy)phenyl)-3 -(6-(4-fluorophenoxy)benzo[d]oxazol-2-yl)urea, 1-(3-fluoro-4-(2-(1-methyl-1H-pyrazol-4-yl)pyridin-4-yloxy)phenyl)-3-(2-(4-fluorophenoxy)quinolin-6-yl)urea, 1-(3-fluoro-4-(2-(1-methyl-1H-pyrazol-4-yl)pyridin-4-yloxy)phenyl)-3-(5-(4-fluorophenoxy)-2-oxoindolin-3-yl)urea, 1-(3-fluoro-4-(2-(1-methyl-1H-pyrazol-4-yl)pyridin-4-yloxy)phenyl)-3-(5-(4-fluorophenoxy)-1H-indazol-3-yl)urea, 1-(3-fluoro-4-(2-(1-methyl-1H-pyrazol-4-yl)pyridin-4-yloxy)phenyl)-3-(6-(4-fluorophenoxy)-1H-indazol-3-yl)urea, 1-(4-(2-(1H-pyrazol-4-yl)pyridin-4-yloxy)-3-fluorophenyl)-3-(3-(4-fluorophenoxy)phenyl)urea, 1-(4-(2-(1H-pyrazol-4-yl)pyridin-4-yloxy)-3-fluorophenyl)-3-(3-(4-fluorophenoxy)-5-methylphenyl)urea, 1-(4-(2-(1H-pyrazol-4-yl)pyridin-4-yloxy)-3-fluorophenyl)-3-(3-chloro-5-(4-fluorophenoxy)phenyl)urea, 1-(4-(2-(1H-pyrazol-4-yl)pyridin-4-yloxy)-3-fluorophenyl)-3-(3-(4-fluorophenoxy)-5-(trifluoromethyl)phenyl)urea, 1-(3-fluoro-4-(2-(1-methyl-1H-pyrazol-4-yl)pyridin-4-yloxy)phenyl)-3-(3-(4-fluorophenoxy)phenyl)urea, 1-(3-fluoro-4-(2-(1-methyl-1H-pyrazol-4-yl)pyridin-4-yloxy)phenyl)-3-(3-(4-fluorophenoxy)-5-methylphenyl)urea, 1-(3-chloro-5-(4-fluorophenoxy)phenyl)-3-(3-fluoro-4-(2-(1-methyl-1H-pyrazol-4-yl)pyridin-4-yloxy)phenyl)urea, and 1-(3-fluoro-4-(2-(1-methyl-1H-pyrazol-4-yl)pyridin-4-yloxy)phenyl)-3-(3-(4-fluorophenoxy)-5-(trifluoromethyl)phenyl)urea.

### Section 4. Biological Data

### Abl kinase (Seq.ID no. 1) assay

Activity of Abl kinase (Seq. ID no. 1) was determined by following the production of ADP from the kinase reaction through coupling with the pyruvate kinase/lactate dehydrogenase system (e.g., Schindler, et al. Science (2000) 289, 1938-1942). In this assay, the oxidation of NADH (thus the decrease at A₃₄₀ₙₘ) was continuously monitored spectrophometrically. The reaction mixture (100 µl) contained Abl kinase (1 nM. Abl from deCode Genetics), peptide substrate (EAIYAAPFAKKK, 0.2 mM), MgCl₂ (10 mM), pyruvate kinase (4 units), lactate dehydrogenase (0.7 units), phosphoenol pyruvate (1 mM), and NADH (0.28 mM) in 90 mM Tris buffer containing 0.2 % octyl-glucoside and 3.5 % DMSO, pH 7.5. Test compounds were incubated with Abl (Seq. ID no. 1) and other reaction reagents at 30 °C for 2 h before ATP (500 µM) was added to start the reaction. The absorption at 340 nm was monitored continuously for 2 hours at 30 °C on Polarstar Optima plate reader (BMG). The reaction rate was calculated using the 1.0 to 2.0 h time frame. Percent inhibition was obtained by comparison of reaction rate with that of a control (i.e. with no test compound). IC₅₀ values were calculated from a series of percent inhibition values determined at a range of inhibitor concentrations using software routines as implemented in the GraphPad Prism software package.

### Abl kinase (Seq. ID no. 1)

GTSMDPSSPNYDKWEMERTDITMKHKLGGGQYGEVYEGVWKKYSLTVAVKTLKEDTMEVE
EFLKEAAVMKEIKHPNLVQLLGVCTREPPFYIITEFMTYGNLLDYLRECNRQEVNAVVLL
YMATQISSAMEYLEKKNFIHRDLAARNCLVGENHLVKVADFGLSRLMTGDTYTAHAGAKF
PIKWTAPESLAYNKFSIKSDVWAFGVLLWEIATYGMSPYPGIDLSQVYELLEKDYRMERP
EGCPEKVYELMRACWQWNPSDRPSFAEIHQAFETMFQE

### Abl kinase (Seq. ID no. 2) assay

Activity of T315I Abl kinase (Seq. ID no. 2) was determined by following the production of ADP from the kinase reaction through coupling with the pyruvate kinase/lactate dehydrogenase system (e.g., Schindler, et al. Science (2000) 289, 1938-1942). In this assay, the oxidation of NADH (thus the decrease at A₃₄₀ₙₘ) was continuously monitored spectrophometrically. The reaction mixture (100 µl) contained Abl kinase (4.4 nM. M315I Abl from deCode Genetics), peptide substrate (EAIYAAPFAKKK, 0.2 mM), MgCl₂ (10 mM), pyruvate kinase (4 units), lactate dehydrogenase (0.7 units), phosphoenol pyruvate (1 mM), and NADH (0.28 mM) in 90 mM Tris buffer containing 0.2 % octyl-glucoside and 1 % DMSO, pH 7.5. Test compounds were incubated with T315I Abl (Seq. ID no. 2) and other reaction reagents at 30 °C for 1 h before ATP (500 µM) was added to start the reaction. The absorption at 340 nm was monitored continuously for 2 hours at 30 °C on Polarstar Optima plate reader (BMG). The reaction rate was calculated using the 1.0 to 2.0 h time frame. Percent inhibition was obtained by comparison of reaction rate with that of a control (i.e. with no test compound). IC₅₀ values were calculated from a series of percent inhibition values determined at a range of inhibitor concentrations using software routines as implemented in the GraphPad Prism software package.

### Abl T315I kinase (Seq. ID no. 2)

GTSMDPSSPNYDKWEMERTDITMKHKLGGGQYGEVYEGVWKKYSLTVAVKTLKEDTMEVE
EFLKEAAVMKEIKHPNLVQLLGVCTREPPFYIIIEFMTYGNLLDYLRECNRQEVNAVVLL
YMATQISSAMEYLEKKNFIHRDLAARNCLVGENHLVKVADFGLSRLMTGDTYTAHAGAKF
PIKWTAPESLAYNKFSIKSDVWAFGVLLWEIATYGMSPYPGIDLSQVYELLEKDYRMERP
EGCPEKVYELMRACWQWNPSDRPSFAEIHQAFETMFQE

### BCR-Abl p210-e14a2 (Seq. ID no. 3)

### BCR-Abl p210-e13a2 (Seq. ID no. 4)

MVDPVGFAEAWKAQFPDSEPPRMELRSVGDIEQELERCKASIRRLEQEVNQERFRMIYLQ
TLLAKEKKSYDRQRWGFRRAAQAPDGASEPRASASRPQPAPADGADPPPAEEPEARPDGE
GSPGKARPGTARRPGAAASGERDDRGPPASVAALRSNFERIRKGHGQPGADAEKPFYVNV
EFHHERGLVKVNDKEVSDRISSLGSQAMQMERKKSQHGAGSSVGDASRPPYRGRSSESSC

GVDGDYEDAELNPRFLKDNLIDANGGSRPPWPPLEYQPYQSIYVGGIMEGEGKGPLLRSQ
STSEQEKRLTWPRRSYSPRSFEDCGGGYTPDCSSNENLTSSEEDFSSGQSSRVSPSPTTY
RMFRDKSRSPSQNSQQSFDSSSPPTPQCHKRHRHCPVVVSEATIVGVRKTGQIWPNDDEG
AFHGDADGSFGTPPGYGCAADRAEEQRRHQDGLPYIDDSPSSSPHLSSKGRGSRDALVSG
ALKSTKASELDLEKGLEMRKWVLSGILASEETYLSHLEALLLPMKPLKAAATTSQPVLTS
QQIETIFFKVPELYEIHKESYDGLFPRVQQWSHQQRVGDLFQKLASQLGVYRAFVDNYGV
AMEMAEKCCQANAQFAEISENLRARSNKDAKDPTTKNSLETLLYKPVDRVTRSTLVLHDL
LKHTPASHPDHPLLQDALRISQNFLSSINEEITPRRQSMTVKKGEHRQLLKDSFMVELVE
GARKLRHVFLFTDLLLCTKLKKQSGGKTQQYDCKWYIPLTDLSFQMVDELEAVPNIPLVP
DEELDALKIKISQIKSDIQREKRANKGSKATERLKKKLSEQESLLLLMSPSMAFRVHSRN
GKSYTFLISSDYERAEWRENIREQQKKCFRSFSLTSVELQMLTNSCVKLQTVHSIPLTIN
KEEALQRPVASDFEPQGLSEAARWNSKENLLAGPSENDPNLFVALYDFVASGDNTLSITK
GEKLRVLGYNHNGEWCEAQTKNGQGWVPSNYITPVNSLEKHSWYHGPVSRNAAEYPLSSG
INGSFLVRESESSPSQRSISLRYEGRVYHYRINTASDGKLYVSSESRFNTLAELVHHHST
VADGLITTLHYPAPKRNKPTVYGVSPNYDKWEMERTDITMKHKLGGGQYGEVYEGVWKKY
SLTVAVKTLKEDTMEVEEFLKEAAVMKEIKHPNLVQLLGVCTREPPFYIITEFMTYGNLL
DYLRECNRQEVNAVVLLYMATQISSAMEYLEKKNFIHRDLAARNCLVGENHLVKVADFGL
SRLMTGDTYTAHAGAKFPIKWTAPESLAYNKFSIKSDVWAFGVLLWEIATYGMSPYPGID
RSQVYELLEKDYRMKRPEGCPEKVYELMRACWQWNPSDRPSFAEIHQAFETMFQESSISD
EVEKELGKQGVRGAVTTLLQAPELPTKTRTSRRAAEHRDTTDVPEMPHSKGQGESDPLDH
EPAVSPLLPRKERGPPEGGLNEDERLLPKDKKTNLFSALIKKKKKTAPTPPKRSSSFREM
DGQPERRGAGEEEGRDISNGALAFTPLDTADPAKSPKPSNGAGVPNGALRESGGSGFRSP
HLWKKSSTLTSSRLATGEEEGGGSSSKRFLRSCSVSCVPHGAKDTEWRSVTLPRDLQSTG
RQFDSSTFGGHKSEKPALPRKRAGENRSDQVTRGTVTPPPRLVKKNEEAADEVFKDIMES
SPGSSPPNLTPKPLRRQVTVAPASGLPHKEEAWKGSALGTPAAAEPVTPTSKAGSGAPRG
TSKGPAEESRVRRHKHSSESPGRDKGKLSKLKPAPPPPPAASAGKAGGKPSQRPGQEAAG
EAVLGAKTKATSLVDAVNSDAAKPSQPAEGLKKPVLPATPKPHPAKPSGTPISPAPVPLS
TLPSASSALAGDQPSSTAFIPLISTRVSLRKTRQPPERASGAITKGVVLDSTEALCLAIS
GNSEQMASHSAVLEAGKNLYTFCVSYVDSIQQMRNKFAFREAINKLENNLRELQICPASA
GSGPAATQDFSKLLSSVKEISDIVQR

### BCR-Abl p190-e1a2 (Seq. ID no. 5)

MVDPVGFAEAWKAQFPDSEPPRMELRSVGDIEQELERCKASIRRLEQEVNQERFRMIYLQ
TLLAKEKKSYDRQRWGFRRAAQAPDGASEPRASASRPQPAPADGADPPPAEEPEARPDGE
GSPGKARPGTARRPGAAASGERDDRGPPASVAALRSNFERIRKGHGQPGADAEKPFYVNV
EFHHERGLVKVNDKEVSDRISSLGSQAMQMERKKSQHGAGSSVGDASRPPYRGRSSESSC
GVDGDYEDAELNPRFLKDNLIDANGGSRPPWPPLEYQPYQSIYVGGIMEGEGKGPLLRSQ
STSEQEKRLTWPRRSYSPRSFEDCGGGYTPDCSSNENLTSSEEDFSSGQSSRVSPSPTTY
RMFRDKSRSPSQNSQQSFDSSSPPTPQCHKRHRHCPVVVSEATIVGVRKTGQIWPNDDEG
AFHGDAEALQRPVASDFEPQGLSEAARWNSKENLLAGPSENDPNLFVALYDFVASGDNTL
SITKGEKLRVLGYNHNGEWCEAQTKNGQGWVPSNYITPVNSLEKHSWYHGPVSRNAAEYP
LSSGINGSFLVRESESSPSQRSISLRYEGRVYHYRINTASDGKLYVSSESRFNTLAELVH
HHSTVADGLITTLHYPAPKRNKPTVYGVSPNYDKWEMERTDITMKHKLGGGQYGEVYEGV
WKKYSLTVAVKTLKEDTMEVEEFLKEAAVMKEIKHPNLVQLLGVCTREPPFYIITEFMTY
GNLLDYLRECNRQEVNAVVLLYMATQISSAMEYLEKKNFIHRDLAARNCLVGENHLVKVA
DFGLSRLMTGDTYTAHAGAKFPIKWTAPESLAYNKFSIKSDVWAFGVLLWEIATYGMSPY
PGIDRSQVYELLEKDYRMKRPEGCPEKVYELMRACWQWNPSDRPSFAEIHQAFETMFQES
SISDEVEKELGKQGVRGAVTTLLQAPELPTKTRTSRRAAEHRDTTDVPEMPHSKGQGESD
PLDHEPAVSPLLPRKERGPPEGGLNEDERLLPKDKKTNLFSALIKKKKKTAPTPPKRSSS
FREMDGQPERRGAGEEEGRDISNGALAFTPLDTADPAKSPKPSNGAGVPNGALRESGGSG
FRSPHLWKKSSTLTSSRLATGEEEGGGSSSKRFLRSCSVSCVPHGAKDTEWRSVTLPRDL
QSTGRQFDSSTFGGHKSEKPALPRKRAGENRSDQVTRGTVTPPPRLVKKNEEAADEVFKD
IMESSPGSSPPNLTPKPLRRQVTVAPASGLPHKEEAWKGSALGTPAAAEPVTPTSKAGSG

APRGTSKGPAEESRVRRHKHSSESPGRDKGKLSKLKPAPPPPPAASAGKAGGKPSQRPGQ
EAAGEAVLGAKTKATSLVDAVNSDAAKPSQPAEGLKKPVLPATPKPHPAKPSGTPISPAP
VPLSTLPSASSALAGDQPSSTAFIPLISTRVSLRKTRQPPERASGAITKGVVLDSTEALC
LAISGNSEQMASHSAVLEAGKNLYTFCVSYVDSIQQMRNKFAFREAINKLENNLRELQIC
PASAGSGPAATQDFSKLLSSVKEISDIVQR

### BCR-Ab1 p210-e14a2 T315I (Seq. ID no. 6)

MVDPVGFAEAWKAQFPDSEPPRMELRSVGDIEQELERCKASIRRLEQEVNQERFRMIYLQ
TLLAKEKKSYDRQRWGFRRAAQAPDGASEPRASASRPQPAPADGADPPPAEEPEARPDGE
GSPGKARPGTARRPGAAASGERDDRGPPASVAALRSNFERIRKGHGQPGADAEKPFYVNV
EFHHERGLVKVNDKEVSDRISSLGSQAMQMERKKSQHGAGSSVGDASRPPYRGRSSESSC
GVDGDYEDAELNPRFLKDNLIDANGGSRPPWPPLEYQPYQSIYVGGIMEGEGKGPLLRSQ
STSEQEKRLTWPRRSYSPRSFEDCGGGYTPDCSSNENLTSSEEDFSSGQSSRVSPSPTTY
RMFRDKSRSPSQNSQQSFDSSSPPTPQCHKRHRHCPVVVSEATIVGVRKTGQIWPNDDEG
AFHGDADGSFGTPPGYGCAADRAEEQRRHQDGLPYIDDSPSSSPHLSSKGRGSRDALVSG
ALKSTKASELDLEKGLEMRKWVLSGILASEETYLSHLEALLLPMKPLKAAATTSQPVLTS
QQIETIFFKVPELYEIHKESYDGLFPRVQQWSHQQRVGDLFQKLASQLGVYRAFVDNYGV
AMEMAEKCCQANAQFAEISENLRARSNKDAKDPTTKNSLETLLYKPVDRVTRSTLVLHDL
LKHTPASHPDHPLLQDALRISQNFLSSINEEITPRRQSMTVKKGEHRQLLKDSFMVELVE
GARKLRHVFLFTDLLLCTKLKKQSGGKTQQYDCKWYIPLTDLSFQMVDELEAVPNIPLVP
DEELDALKIKISQIKSDIQREKRANKGSKATERLKKKLSEQESLLLLMSPSMAFRVHSRN
GKSYTFLISSDYERAEWRENIREQQKKCFRSFSLTSVELQMLTNSCVKLQTVHSIPLTIN
KEDDESPGLYGFLNVIVHSATGFKQSSKALQRPVASDFEPQGLSEAARWNSKENLLAGPS
ENDPNLFVALYDFVASGDNTLSITKGEKLRVLGYNHNGEWCEAQTKNGQGWVPSNYITPV
NSLEKHSWYHGPVSRNAAEYPLSSGINGSFLVRESESSPSQRSISLRYEGRVYHYRINTA
SDGKLYVSSESRFNTLAELVHHHSTVADGLITTLHYPAPKRNKPTVYGVSPNYDKWEMER
TDITMKHKLGGGQYGEVYEGVWKKYSLTVAVKTLKEDTMEVEEFLKEAAVMKEIKHPNLV
QLLGVCTREPPFYIIIEFMTYGNLLDYLRECNRQEVNAVVLLYMATQISSAMEYLEKKNF
IHRDLAARNCLVGENHLVKVADFGLSRLMTGDTYTAHAGAKFPIKWTAPESLAYNKFSIK
SDVWAFGVLLWEIATYGMSPYPGIDRSQVYELLEKDYRMKRPEGCPEKVYELMRACWQWN
PSDRPSFAEIHQAFETMFQESSISDEVEKELGKQGVRGAVTTLLQAPELPTKTRTSRRAA
EHRDTTDVPEMPHSKGQGESDPLDHEPAVSPLLPRKERGPPEGGLNEDERLLPKDKKTNL
FSALIKKKKKTAPTPPKRSSSFREMDGQPERRGAGEEEGRDISNGALAFTPLDTADPAKS
PKPSNGAGVPNGALRESGGSGFRSPHLWKKSSTLTSSRLATGEEEGGGSSSKRFLRSCSV
SCVPHGAKDTEWRSVTLPRDLQSTGRQFDSSTFGGHKSEKPALPRKRAGENRSDQVTRGT
VTPPPRLVKKNEEAADEVFKDIMESSPGSSPPNLTPKPLRRQVTVAPASGLPHKEEAWKG
SALGTPAAAEPVTPTSKAGSGAPRGTSKGPAEESRVRRHKHSSESPGRDKGKLSKLKPAP
PPPPAASAGKAGGKPSQRPGQEAAGEAVLGAKTKATSLVDAVNSDAAKPSQPAEGLKKPV
LPATPKPHPAKPSGTPISPAPVPLSTLPSASSALAGDQPSSTAFIPLISTRVSLRKTRQP
PERASGAITKGVVLDSTEALCLAISGNSEQMASHSAVLEAGKNLYTFCVSYVDSIQQMRN
KFAFREAINKLENNLRELQICPASAGSGPAATQDFSKLLSSVKEISDIVQR

### BCR-Abl p210-e13a2 T315I (Seq. ID no. 7)

MVDPVGFAEAWKAQFPDSEPPRMELRSVGDIEQELERCKASIRRLEQEVNQERFRMIYLQ
TLLAKEKKSYDRQRWGFRRAAQAPDGASEPRASASRPQPAPADGADPPPAEEPEARPDGE
GSPGKARPGTARRPGAAASGERDDRGPPASVAALRSNFERIRKGHGQPGADAEKPFYVNV
EFHHERGLVKVNDKEVSDRISSLGSQAMQMERKKSQHGAGSSVGDASRPPYRGRSSESSC
GVDGDYEDAELNPRFLKDNLIDANGGSRPPWPPLEYQPYQSIYVGGIMEGEGKGPLLRSQ
STSEQEKRLTWPRRSYSPRSFEDCGGGYTPDCSSNENLTSSEEDFSSGQSSRVSPSPTTY
RMFRDKSRSPSQNSQQSFDSSSPPTPQCHKRHRHCPVVVSEATIVGVRKTGQIWPNDDEG
AFHGDADGSFGTPPGYGCAADRAEEQRRHQDGLPYIDDSPSSSPHLSSKGRGSRDALVSG
ALKSTKASELDLEKGLEMRKWVLSGILASEETYLSHLEALLLPMKPLKAAATTSQPVLTS
QQIETIFFKVPELYEIHKESYDGLFPRVQQWSHQQRVGDLFQKLASQLGVYRAFVDNYGV

AMEMAEKCCQANAQFAEISENLRARSNKDAKDPTTKNSLETLLYKPVDRVTRSTLVLHDL
LKHTPASHPDHPLLQDALRISQNFLSSINEEITPRRQSMTVKKGEHRQLLKDSFMVELVE
GARKLRHVFLFTDLLLCTKLKKQSGGKTQQYDCKWYIPLTDLSFQMVDELEAVPNIPLVP
DEELDALKIKISQIKSDIQREKRANKGSKATERLKKKLSEQESLLLLMSPSMAFRVHSRN
GKSYTFLISSDYERAEWRENIREQQKKCFRSFSLTSVELQMLTNSCVKLQTVHSIPLTIN
KEEALQRPVASDFEPQGLSEAARWNSKENLLAGPSENDPNLFVALYDFVASGDNTLSITK
GEKLRVLGYNHNGEWCEAQTKNGQGWVPSNYITPVNSLEKHSWYHGPVSRNAAEYPLSSG
INGSFLVRESESSPSQRSISLRYEGRVYHYRINTASDGKLYVSSESRFNTLAELVHHHST
VADGLITTLHYPAPKRNKPTVYGVSPNYDKWEMERTDITMKHKLGGGQYGEVYEGVWKKY
SLTVAVKTLKEDTMEVEEFLKEAAVMKEIKHPNLVQLLGVCTREPPFYIIIEFMTYGNLL
DYLRECNRQEVNAVVLLYMATQISSAMEYLEKKNFIHRDLAARNCLVGENHLVKVADFGL
SRLMTGDTYTAHAGAKFPIKWTAPESLAYNKFSIKSDVWAFGVLLWEIATYGMSPYPGID
RSQVYELLEKDYRMKRPEGCPEKVYELMRACWQWNPSDRPSFAEIHQAFETMFQESSISD
EVEKELGKQGVRGAVTTLLQAPELPTKTRTSRRAAEHRDTTDVPEMPHSKGQGESDPLDH
EPAVSPLLPRKERGPPEGGLNEDERLLPKDKKTNLFSALIKKKKKTAPTPPKRSSSFREM
DGQPERRGAGEEEGRDISNGALAFTPLDTADPAKSPKPSNGAGVPNGALRESGGSGFRSP
HLWKKSSTLTSSRLATGEEEGGGSSSKRFLRSCSVSCVPHGAKDTEWRSVTLPRDLQSTG
RQFDSSTFGGHKSEKPALPRKRAGENRSDQVTRGTVTPPPRLVKKNEEAADEVFKDIMES
SPGSSPPNLTPKPLRRQVTVAPASGLPHKEEAWKGSALGTPAAAEPVTPTSKAGSGAPRG
TSKGPAEESRVRRHKHSSESPGRDKGKLSKLKPAPPPPPAASAGKAGGKPSQRPGQEAAG
EAVLGAKTKATSLVDAVNSDAAKPSQPAEGLKKPVLPATPKPHPAKPSGTPISPAPVPLS
TLPSASSALAGDQPSSTAFIPLISTRVSLRKTRQPPERASGAITKGVVLDSTEALCLAIS
GNSEQMASHSAVLEAGKNLYTFCVSYVDSIQQMRNKFAFREAINKLENNLRELQICPASA
GSGPAATQDFSKLLSSVKEISDIVQR

### BCR-Abl p190-e1a2 (Seq. ID no. 8)

MVDPVGFAEAWKAQFPDSEPPRMELRSVGDIEQELERCKASIRRLEQEVNQERFRMIYLQ
TLLAKEKKSYDRQRWGFRRAAQAPDGASEPRASASRPQPAPADGADPPPAEEPEARPDGE
GSPGKARPGTARRPGAAASGERDDRGPPASVAALRSNFERIRKGHGQPGADAEKPFYVNV
EFHHERGLVKVNDKEVSDRISSLGSQAMQMERKKSQHGAGSSVGDASRPPYRGRSSESSC
GVDGDYEDAELNPRFLKDNLIDANGGSRPPWPPLEYQPYQSIYVGGIMEGEGKGPLLRSQ
STSEQEKRLTWPRRSYSPRSFEDCGGGYTPDCSSNENLTSSEEDFSSGQSSRVSPSPTTY
RMFRDKSRSPSQNSQQSFDSSSPPTPQCHKRHRHCPVVVSEATIVGVRKTGQIWPNDDEG
AFHGDAEALQRPVASDFEPQGLSEAARWNSKENLLAGPSENDPNLFVALYDFVASGDNTL
SITKGEKLRVLGYNHNGEWCEAQTKNGQGWVPSNYITPVNSLEKHSWYHGPVSRNAAEYP
LSSGINGSFLVRESESSPSQRSISLRYEGRVYHYRINTASDGKLYVSSESRFNTLAELVH
HHSTVADGLITTLHYPAPKRNKPTVYGVSPNYDKWEMERTDITMKHKLGGGQYGEVYEGV
WKKYSLTVAVKTLKEDTMEVEEFLKEAAVMKEIKHPNLVQLLGVCTREPPFYIIIEFMTY
GNLLDYLRECNRQEVNAVVLLYMATQISSAMEYLEKKNFIHRDLAARNCLVGENHLVKVA
DFGLSRLMTGDTYTAHAGAKFPIKWTAPESLAYNKFSIKSDVWAFGVLLWEIATYGMSPY
PGIDRSQVYELLEKDYRMKRPEGCPEKVYELMRACWQWNPSDRPSFAEIHQAFETMFQES
SISDEVEKELGKQGVRGAVTTLLQAPELPTKTRTSRRAAEHRDTTDVPEMPHSKGQGESD
PLDHEPAVSPLLPRKERGPPEGGLNEDERLLPKDKKTNLFSALIKKKKKTAPTPPKRSSS
FREMDGQPERRGAGEEEGRDISNGALAFTPLDTADPAKSPKPSNGAGVPNGALRESGGSG
FRSPHLWKKSSTLTSSRLATGEEEGGGSSSKRFLRSCSVSCVPHGAKDTEWRSVTLPRDL
QSTGRQFDSSTFGGHKSEKPALPRKRAGENRSDQVTRGTVTPPPRLVKKNEEAADEVFKD
IMESSPGSSPPNLTPKPLRRQVTVAPASGLPHKEEAWKGSALGTPAAAEPVTPTSKAGSG
APRGTSKGPAEESRVRRHKHSSESPGRDKGKLSKLKPAPPPPPAASAGKAGGKPSQRPGQ
EAAGEAVLGAKTKATSLVDAVNSDAAKPSQPAEGLKKPVLPATPKPHPAKPSGTPISPAP
VPLSTLPSASSALAGDQPSSTAFIPLISTRVSLRKTRQPPERASGAITKGVVLDSTEALC
LAISGNSEQMASHSAVLEAGKNLYTFCVSYVDSIQQMRNKFAFREAINKLENNLRELQIC
PASAGSGPAATQDFSKLLSSVKEISDIVQR

### C-Kit kinase (Seq. ID no. 9) assay

Activity of c-Kit kinase (Seq. ID no. 9) was determined by following the production of ADP from the kinase reaction through coupling with the pyruvate kinase/lactate dehydrogenase system (e.g., Schindler, et al. Science (2000) 289, 1938-1942). In this assay, the oxidation of NADH (thus the decrease at A340nm) was continuously monitored spectrophometrically. The reaction mixture (100 µl) contained c-Kit (cKIT residues T544-V976, from ProQinase, 5.4 nM), polyE4Y (1 mg/ml), MgCl2 (10 mM), pyruvate kinase (4 units), lactate dehydrogenase (0.7 units), phosphoenol pyruvate (1 mM), and NADH (0.28 mM) in 90 mM Tris buffer containing 0.2 % octyl-glucoside and 1 % DMSO, pH 7.5. Test compounds were incubated with C-Met (Seq. ID no. 9) and other reaction reagents at 22 °C for < 2 min before ATP (200 µM) was added to start the reaction. The absorption at 340 nm was monitored continuously for 0.5 hours at 30 °C on Polarstar Optima plate reader (BMG). The reaction rate was calculated using the 0 to 0.5 h time frame. Percent inhibition was obtained by comparison of reaction rate with that of a control (i.e. with no test compound). IC50 values were calculated from a series of percent inhibition values determined at a range of inhibitor concentrations using software routines as implemented in the GraphPad Prism software package.

### c-Kit with N-terminal GST fusion (Seq ID no. 9)

LGYWKIKGLVQPTRLLLEYLEEKYEEHLYERDEGDKWRNKKFELGLEFPNLPYYIDGDVKL
TQSMAIIRYIADKHNMLGGCPKERAEISMLEGAVDIRYGVSRIAYSKDFETLKVDFLSKLP
EMLKMFEDRLCHKTYLNGDHVTHPDFMLYDALDVVLYMDPMCLDAFPKLVCFKKRIEAIPQ
IDKYLKSSKYIWPLQGWQATFGGGDHPPKSDLVPRHNQTSLYKKAGSAAAVLEENLYFQGT
YKYLQKPMYEVQWKVVEEINGNNYVYIDPTQLPYDHKWEFPRNRLSFGKTLGAGAFGKVVE
ATAYGLIKSDAAMTVAVKMLKPSAHLTEREALMSELKVLSYLGNHMNIVNLLGACTIGGPT
LVITEYCCYGDLLNFLRRKRDSFICSKQEDHAEAALYKNLLHSKESSCSDSTNEYMDMKPG
VSYVVPTKADKRRSVRIGSYIERDVTPAIMEDDELALDLEDLLSFSYQVAKGMAFLASKNC
IHRDLAARNILLTHGRITKICDFGLARDIKNDSNYVVKGNARLPVKWMAPESIFNCVYTFE
SDVWSYGIFLWELFSLGSSPYPGMPVDSKFYKMIKEGFRMLSPEHAPAEMYDIMKTCWDAD
PLKRPTFKQIVQLIEKQISESTNHIYSNLANCSPNRQKPVVDHSVRINSVGSTASSSQPL
LVHDDV

### C-Met kinase (Seq. ID no. 10) assay

Activity of C-Met kinase (Seq. ID no. 10) was determined by following the production of ADP from the kinase reaction through coupling with the pyruvate kinase/lactate dehydrogenase system (e.g., Schindler, et al. Science (2000) 289, 1938-1942). In this assay, the oxidation of NADH (thus the decrease at A340nm) was continuously monitored spectrophometrically. The reaction mixture (100 µl) contained C-Met (c-Met residues: 956-1390, from Invitrogen, catalogue #PV3143, 6 nM), polyE4Y (1 mg/ml), MgCl2 (10 mM), pyruvate kinase (4 units), lactate dehydrogenase (0.7 units), phosphoenol pyruvate (1 mM), and NADH (0.28 mM) in 90 mM Tris buffer containing 0.25 mM DTT, 0.2 % octyl-glucoside and 1 % DMSO, pH 7.5. Test compounds were incubated with C-Met (Seq. ID no. 10) and other reaction reagents at 22 °C for 0.5 h before ATP (100 µM) was added to start the reaction. The absorption at 340 nm was monitored continuously for 2 hours at 30 °C on Polarstar Optima plate reader (BMG). The reaction rate was calculated using the 1.0 to 2.0 h time frame. Percent inhibition was obtained by comparison of reaction rate with that of a control (i.e. with no test compound). IC50 values were calculated from a series of percent inhibition values determined at a range of inhibitor concentrations using software routines as implemented in the GraphPad Prism software package.

### cMet Kinase (Seq ID no. 10)

MSYYHHHHHHDYDIPTTENLYFQGAMLVPRGSPWIPFTMKKRKQIKDLGSELVRYDARVHT
PHLDRLVSARSVSPTTEMVSNESVDYRATFPEDQFPNSSQNGSCRQVQYPLTDMSPILTSG
DSDISSPLLQNTVHIDLSALNPELVQAVQHVVIGPSSLIVHFNEVIGRGHFGCVYHGTLLD
NDGKKIHCAVKSLNRITDIGEVSQFLTEGIIMKDFSHPNVLSLLGICLRSEGSPLVVLPYM
KHGDLRNFIRNETHNPTVKDLIGFGLQVAKGMKYLASKKFVHRDLAARNCMLDEKFTVKVA
DFGLARDMYDKEYYSVHNKTGAKLPVKWMALESLQTQKFTTKSDVWSFGVLLWELMTRGAP
PYPDVNTFDITVYLLQGRRLLQPEYCPDPLYEVMLKCWHPKAEMRPSFSELVSRISAIFST
FIGEHYVHVNATYVNVKCVAPYPSLLSSEDNADDEVDTRPASFWETS

### B-Raf(V600E) Kinase Assay

The activity of B-Raf(V600E) kinase (Seq. ID no. 11) was determined by following the formation of ADP from the reaction through coupling with the pyruvate kinase/lactate dehydrogenase system (e.g., Schindler, et al. Science (2000) 289, 1938-1942). In this assay, the oxidation of NADH (thus the decrease at A₃₄₀ₙₘ) was continuously monitored spectrophotometrically. The reaction mixture (100 µl) contained B-Raf(V600E) kinase (0.34 nM nominal concentration, Seq. ID no. 11), unphosphorylated, full-length MEK1 (42 nM, Seq. ID no. 12), MgCl₂ (13 mM), pyruvate kinase (3.5 units), lactate dehydrogenase (5.5 units), phosphoenolpyruvate (1 mM), and NADH (0.28 mM), in 60 mM Tris buffer, containing 0.13% octyl-glucoside and 3.5 % DMSO concentration at pH 7.5. The test compounds were incubated with the reaction mixture at 30 °C for 1h. The reaction was initiated by adding ATP (0.2 mM, final concentration). The absorption at 340 nm was continuously monitored for 3h at 30 °C on a Polarstar Optima plate reader (BMG). The reaction rate was calculated using the 0.5h to 1.5h time frame. Percent inhibition was obtained by comparison of reaction rate with that of a control (i.e. with no test compound). IC₅₀ values were calculated from a series of percent inhibition values determined at a range of inhibitor concentrations using software routines as implemented in the GraphPad Prism software package.

### BRaf V600E kinase (Seq ID no. 11)

MDRGSHHHHHHGSEDRNRMKTLGRRDSSDDWEIPDGQITVGQRIGSGSFGTVYKGKWHGD
VAVKMLNVTAPTPQQLQAFKNEVGVLRKTRHVNILLFMGYSTKPQLAIVTQWCEGSSLYH
HLHIIETKFEMIKLIDIARQTAQGMDYLHAKSIIHRDLKSNNIFLHEDLTVKIGDFGLAT
EKSRWSGSHQFEQLSGSILWMAPEVIRMQDKNPYSFQSDVYAFGIVLYELMTGQLPYSNI
NNRDQIIFMVGRGYLSPDLSKVRSNCPKAMKRLMAECLKKKRDERPLFPQILASIELLAR
SLPKIHR

### MEK1 kinase (Seq ID no. 12)

MELKDDDFEKISELGAGNGGVVFKVSHKPSGLVMARKLIHLEIKPAIRNQIIRELQVLHE
CNSPYIVGFYGAFYSDGEISICMEHMDGGSLDQVLKKAGRIPEQILGKVSIAVIKGLTYL
REKHKIMHRDVKPSNILVNSRGEIKLCDFGVSGQLIDSMANSFVGTRSYMSPERLQGTHY
SVQSDIWSMGLSLVEMAVGRYPIPPPDAKELELMFGCQVEGDAAETPPRPRTPGRPLSSY
GMDSRPPMAIFELLDYIVNEPPPKLPSGVFSLEFQDFVNKCLIKNPAERADLKQLMVHAF
IKRSDAEEVDFAGWLCSTIGLNQPSTPTHAAGV

**Table 1: Biological Data Summary.**

| Biochemical IC₅₀ values of compounds of Formula I. | | | | | |
|---|---|---|---|---|---|
| Example | Abl Enzyme Assay | Abl T315l Enzyme Assay | c-Kit Enzyme Assay | c-Met Enzyme Assay | B-Raf V600E Enzyme Assay |
| 1 | +++ | +++ | n/a | +++ | n/a |
| 2 | +++ | +++ | +++ | +++ | +++ |
| 3 | +++ | +++ | n/a | +++ | +++ |
| 4 | +++ | +++ | n/a | +++ | +++ |
| 5 | +++ | +++ | +++ | ++ | +++ |
| 6 | +++ | +++ | n/a | +++ | +++ |
| 7 | +++ | +++ | n/a | +++ | +++ |
| 8 | +++ | +++ | n/a | +++ | +++ |
| 9 | +++ | +++ | +++ | ++ | ++ |
| 10 | +++ | ‡ | n/a | + | ++ |
| 11 | +++ | +++ | n/a | +++ | +++ |
| 12 | +++ | +++ | +++ | +++ | +++ |
| 13 | +++ | +++ | +++ | +++ | +++ |
| 14 | +++ | ‡ | n/a | + | ‡ |
| 15 | +++ | n/a | n/a | ++ | ++ |
| 16 | +++ | +++ | n/a | +++ | +++ |
| 17 | +++ | +++ | +++ | ++ | + |
| 18 | +++ | n/a | n/a | ++ | ++ |
| 19 | +++ | +++ | +++ | ++ | ++ |
| 20 | +++ | n/a | n/a | n/a | + |
| 21 | +++ | +++ | +++ | ++ | ++ |
| 22 | ++ | n/a | n/a | n/a | + |
| 23 | +++ | n/a | n/a | n/a | + |
| 24 | +++ | +++ | n/a | +++ | +++ |
| 25 | +++ | +++ | n/a | ++ | ++ |
| 26 | n/a | n/a | n/a | n/a | n/a |
| 27 | n/a | n/a | n/a | n/a | n/a |

| | | | | | |
|---|---|---|---|---|---|
| +++ = < 0.1 µM; ++ = < 1.0 µM; + = =< 10 µM; ‡ < 100 µM; n/a = not available | | | | | |

The biochemical IC₅₀ values of other compounds disclosed herein are at least 10 µM against Abl enzyme.

### Cell Culture

BaF3 cells (parental or transfected with the following: wild type p210 BCR-Abl and T315I p210 BCR-Abl was obtained from Professor Richard Van Etten (New England Medical Center, Boston, MA). Briefly, cells were grown in RPMI 1640 supplemented with 10% characterized fetal bovine serum (HyClone, Logan, UT) at 37 degrees Celsius, 5% CO₂, 95% humidity. Cells were allowed to expand until reaching 80% saturation at which point they were subcultured or harvested for assay use.

### Cell Proliferation Assay

A serial dilution of test compound was dispensed into a 96 well black clear bottom plate (Corning, Corning, NY). For each cell line, three thousand cells were added per well in complete growth medium. Plates were incubated for 72 hours at 37 degrees Celsius, 5% CO₂, 95% humidity. At the end of the incubation period Cell Titer Blue (Promega, Madison, WI) was added to each well and an additional 4.5 hour incubation at 37 degrees Celsius, 5% CO₂ 95% humidity was performed. Plates were then read on a BMG Fluostar Optima (BMG, Durham, NC) using an excitation of 544 nM and an emission of 612 nM. Data was analyzed using Prism software (Graphpad, San Diego, CA) to calculate IC50's.

**Table 2: Biological Data Summary. Whole Cell Antiproliferation IC₅₀ values of compounds of Formula I.**

| Example | Ba/F3 p210 whole cell proliferation assay | Ba/F3 p210 T315I whole cell proliferation assay |
|---|---|---|
| 1 | +++ | +++ |
| 2 | +++ | +++ |
| 3 | +++ | +++ |
| 4 | +++ | +++ |
| 5 | +++ | +++ |
| 6 | +++ | +++ |
| 7 | +++ | +++ |
| 8 | +++ | +++ |
| 9 | +++ | +++ |
| 10 | +++ | +++ |
| 11 | +++ | +++ |
| 12 | +++ | +++ |
| 13 | +++ | +++ |
| 14 | +++ | +++ |
| 15 | +++ | +++ |
| 16 | +++ | +++ |
| 17 | +++ | +++ |
| 18 | +++ | +++ |
| 19 | +++ | +++ |
| 20 | +++ | ++ |
| 21 | +++ | +++ |
| 22 | n/a | n/a |
| 23 | n/a | n/a |
| 24 | +++ | +++ |
| 25 | ++ | + |
| 26 | n/a | n/a |
| 27 | n/a | n/a |

| | | |
|---|---|---|
| +++ = 0.1 µM; ++ = <1.0 µM; + = < 10 µM; ‡ < 100 µM; n/a = not available | | |

Aspects and features of the present disclosure are further set out in the following numbered paragraphs:
Compounds of the formula Ia 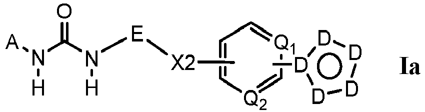 wherein Q1 and Q2 are each individually and independently selected from the group consisting of N and CH and wherein at least one of Q1 and Q2 is N;
and wherein the ring containing Q1 and Q2 may be optionally substituted with one or more R20 moieties;
each D is individually taken from the group consisting of C, CH, C-R20, N-Z3, N, O and S, such that the resultant ring is taken from the group consisting of pyrazolyl, triazolyl, isoxazolyl, isothiazolyl, oxazolyl, and thiadiazolyl;
wherein E is selected from the group consisting of phenyl, pyridyl, and pyrimidinyl;
when Q1 and Q2 are both N, the A ring is selected from the group consisting of cyclopentyl, cyclohexyl, G1, G2, and G3;
when only one of Q1 and Q2 is N, the A ring is selected from the group consisting of cyclopentyl, cyclohexyl, G1, G2, G3 and G4;
G1 is a heteroaryl taken from the group consisting of pyrrolyl, furyl, thienyl, oxazolyl, thiazolyl, isoxazolyl-4-yl, isoxazolyl-5-yl, isothiazolyl, imidazolyl, pyrazolyl, oxadiazolyl, thiadiazolyl, triazolyl, and tetrazolyl;
G2 is a fused bicyclic heteroaryl taken from the group consisting of indolyl, indolinyl, isoindolyl, isoindolinyl, indazolyl, benzofuranyl, benzothienyl, benzothiazolyl, benzothiazolonyl, benzoxazolyl, benzoxazolonyl, benzisoxazolyl, benzisothiazolyl, benzimidazolyl, benzimidazolonyl, benztriazolyl, imidazopyridinyl, pyrazolopyridinyl, imidazolonopyridinyl, thiazolopyridinyl, thiazolonopyridinyl, oxazolopyridinyl, oxazolonopyridinyl, isoxazolopyridinyl, isothiazolopyridinyl, triazolopyridinyl, imidazopyrimidinyl, pyrazolopyrimidinyl, imidazolonopyrimidinyl, thiazolopyridiminyl, thiazolonopyrimidinyl, oxazolopyridiminyl, oxazolonopyrimidinyl, isoxazolopyrimidinyl, isothiazolopyrimidinyl, triazolopyrimidinyl, dihydropurinonyl, pyrrolopyrimidinyl, purinyl, pyrazolopyrimidinyl, phthalimidyl, phthalimidinyl, pyrazinylpyridinyl, pyridinopyrimidinyl, pyrimidinopyrimidinyl, cinnolinyl, quinoxalinyl, quinazolinyl, quinolinyl, isoquinolinyl, phthalazinyl, benzodioxyl, benzisothiazoline-1,1,3-trionyl, dihydroquinolinyl, tetrahydroquinolinyl, dihydroisoquinolyl, tetrahydroisoquinolinyl, benzoazepinyl, benzodiazepinyl, benzoxapinyl, and benzoxazepinyl;
G3 is a heterocyclyl taken from the group consisting of oxetanyl, azetadinyl, tetrahydrofuranyl, pyrrolidinyl, oxazolinyl, oxazolidinyl, imidazolonyl, pyranyl, thiopyranyl, tetrahydropyranyl, dioxalinyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, and homotropanyl;
G4 is selected from the group consisting of phenyl, naphthyl, pyrazinyl, pyridazinyl, triazinyl, pyridinyl, and pyrimidinyl;
the A ring is substituted with one -(X1-A1) moiety or one A1 moiety, and may be optionally substituted with one or two R2 moieties;
when A is phenyl and E is phenyl, then Z1 cannot be -OO(CH₂)_{q}R5, -(CH₂)ₚ-G3, or - (CH₂)ₚR5;
A1 is selected from the group consisting of phenyl, G1, G3 and G4;
X1 is selected from the group consisting of a direct bond linking the A and A1 rings, - (CH₂)ₙ(O)ᵣ(CH₂)ₙ-, -(CH₂)ₙ(NR3)ᵣ(CH₂)ₙ-, -(CH₂)ₙ(S)ᵣ(CH₂)ₙ-, -(CH₂)ₙ(C(O))ᵣ(CH₂)ₙ-, - (CH₂)ₙ(C(O)NR3)ᵣ(CH₂)ₙ-, and -(CH₂)ₙ(SO₂NR3)ᵣ(CH₂)ₙ-, wherein any of the alkylenes may be straight or branched chain;
when X1 is a direct bond, E is substituted with one or two R16 moieties;
when X1 is not a direct bond, then E is optionally substituted with one or two R16 moieties;
X2 is selected from the group consisting of -O-, -S(CH₂)ₙ-, -N(R3)(CH₂)ₙ-, -(CH₂)ₚ-, and wherein the carbon atoms of -(CH₂)ₙ-, -(CH₂)ₚ-, of X2 may be further substituted by oxo or one or more C1-C6alkyl moieties;
when A, A1, G1, G2 G3 or G4 has one or more substitutable sp2-hybridized carbon atoms, each respective sp2 hybridized carbon atom may be optionally substituted with a Z1 substituent;
when A, A1, G1, G2 or G3 has one or more substitutable sp3-hybridized carbon atoms, each respective sp3 hybridized carbon atom may be optionally substituted with a Z2 substituent;
when A, A1, G1, G2 or G3 has one or more substitutable nitrogen atoms, each respective nitrogen atom may be optionally substituted with a Z4 substituent;
each Z1 is independently and individually selected from the group consisting of C1-6alkyl, branched C3-C7alkyl, C3-C8cycloalkyl, halogen, fluoroC1-C6alky wherein the alkyl moiety can be partially or fully fluorinated, cyano, C1-C6alkoxy, fluoroC1-C6alkoxy wherein the alkyl moiety can be partially or fully fluorinated, -(CH₂)ₙOH, oxo, C1-C6alkoxyC1-C6alkyl, (R4)₂N(CH)ₙ-, (R3)₂N(CH)ₙ-, (R4)₂N(CH₂)_{q}N(R4)(CH₂)ₙ-, (R4)₂N(CH₂)_{q}O(CH₂)ₙ-, (R3)₂NC(O)-, (R4)₂NC(O)-, (R4)₂NC(O)C1-C6alkyl-,-(R4)NC(O)R8, C1-C6alkoxycarbonyl-, -carboxyC1-C6alkyl, C1-C6alkoxycarbonylC1-C6alkyl-, (R3)₂NSO₂-, -SOR3, (R4)₂NSO₂-, -N(R4)SO₂R5 -N(R4)SO₂R8, - O(CH₂)_{q}OC1-C6alkyl, -O(CH₂)_{q}R5, -SO₂R3, -SOR4, -S(O)₂R5, -(CH₂)ₙC(O)R5, - C(O)R8, -C(O)R6, -C(=NOH)R6, -C(=NOR3)R6, -(CH₂)ₙN(R4)C(O)R8, -(CH₂)ₙ-G1, - (CH₂)ₙ-G4, phenoxy, -(CH₂)ₙO(CH₂)ₙ-G1, -(CH₂)ₚO(CH₂)ₙ-G4, -N(R3)(CH₂)_{q}O-alkyl, - N(R3)(CH₂)_{q}N(R4)₂, -N(R3)(CH₂)_{q}R5, -(CH₂)ₙN(R3)(CH₂)ₙ-aryl, -(CH₂)ₙN(R3)(CH₂)ₙ-G1, -(CH₂)ₙN(R3)(CH₂)ₙ-G4, nitro, -(CH₂)ₙNHC(O)(CH₂)ₙR5, - (CH₂)ₙNHS(O)₂(CH₂)ₙR5, -(CH₂)ₙC(O)NH(CH₂)_{q}R5, -(CH₂)ₙOC(O)R5, - CH(OH)(CH₂)ₚR5, -CH(OH)CH(OH)R4, -(CH₂)ₙR5, -C(=NH)R5, -C(=NH)N(R4)₂, - C(=NOR3)R5, -C(=NOR3)N(R4)₂, and -NHC(=NH)R8;
in the event that Z1 contains an alkyl or alkylene moiety, such moieties may be further substituted with one or more C1-C6alkyls;
each Z2 is independently and individually selected from the group consisting of aryl, C1-C6alkyl, C3-C8cycloalkyl, branched C3-C7alkyl, hydroxyl, hydroxyC1-C6alkyl-, cyano, (R3)₂N-, (R4)₂N-, (R4)₂NC1-C6alkyl-, (R4)₂NC2-C6alkylN(R4)(CH₂)ₙ-, (R4)₂NC2-C6alkylO(CH₂)ₙ-, (R3)₂NC(O)-, (R4)₂NC(O)-, (R4)₂NC(O)-C1-C6alkyl-, carboxyl, - carboxyC1-C6alkyl, C 1-C6alkoxycarbonyl-, C 1-C6alkoxycarbonylC1-C6alkyl-, (R3)₂NSO₂-, (R4)₂NSO₂-, -SO₂R5, -SO₂R8, -(CH₂)ₙN(R4)C(O)R8, -C(O)R8, =O, =NOH, =N(OR6), -(CH₂)ₙ-G1, -(CH₂)ₙ-G4, -(CH₂)ₙO(CH₂)ₙ-G1, -(CH₂)ₙO(CH₂)ₙ-G4, - (CH₂)ₙN(R3)(CH₂)ₙ-aryl, -(CH₂)ₙN(R3)(CH₂)ₙ-G1, -(CH₂)ₙN(R3)(CH₂)ₙ-G4, - (CH₂)ₙNHC(O)(CH₂)ₙR5, -(CH₂)ₙNHS(O)₂(CH₂)ₙR5, -(CH₂)ₙC(O)NH(CH₂)_{q}R5, - (CH₂)ₙC(O)R5, -(CH₂)ₙOC(O)R5, and -(CH₂)ₙR5;
in the event that Z2 contains an alkyl or alkylene moiety, such moieties may be further substituted with one or more C1-C6alkyls;
each Z3 is independently and individually selected from the group consisting of H, C1-C6alkyl, branched C3-C7alkyl, C3-C8cycloalkyl, fluoroC1-C6alkyl wherein the alkyl moiety can be partially or fully fluorinated, hydroxyC2-C6alkyl-, C1-C6alkoxycarbonyl-, -C(O)R8, RSC(O)(CH₂)ₙ-, (R4)₂NC(O)-, (R4)₂NC(O)C1-C6alkyl-, R8C(O)N(R4)(CH₂)_{q}-, (R3)₂NSO₂-, (R4)₂NSO₂-, -(CH₂)_{q}N(R3)₂, and -(CH₂)_{q}N(R4)₂;
each Z4 is independently and individually selected from the group consisting of C1-C6alkyl, branched C3-7alkyl, hydroxyC2-C6alkyl-, C1-C6alkoxyC2-C6alkyl-, (R4)₂N-C2-C6alkyl-, (R4)₂N-C2-C6alkylN(R4)-C2-C6alkyl-, (R4)₂N-C2-C6alkyl-O-C2-C6alkyl- (R4)₂NC(O)C1-C6alkyl-, carboxyC1-C6alkyl, C1-C6alkoxycarbonylC1-C6alkyl-, -C2-C6alkylN(R4)C(O)R8, R8-C(=NR3)-, -SO₂R8, -COR8, -(CH₂)ₙ-G1, - (CH₂)ₙ-G4, -(CH₂)_{q}O(CH₂)ₙ-G1, -(CH₂)_{q}O(CH₂)ₙ-G4, -(CH₂)_{q}NR3(CH₂)ₙ-G1, - (CH₂)_{q}NR3(CH₂)ₙ-G4, -(CH₂)_{q}NHC(O)(CH₂)ₙR5, -(CH₂)_{q}C(O)NH(CH₂)_{q}R5, - (CH₂)_{q}C(O)R5, -(CH₂)_{q}OC(O)R5, -(CH₂)_{q}R5, -(CH₂)_{q}NR4(CH₂)_{q}R5, and - (CH₂)_{q}O(CH₂)_{q}R5;
in the event that Z4 contains an alkyl or alkylene moiety, such moieties may be further substituted with one or more C1-C6alkyls;
each R2 is selected from the group consisting of H, R17-substituted aryl-, R17-substituted G1, R17-substituted G4, C1-C6alkyl, branched C3-C8alkyl, R19 substituted C3-C8cycloalkyl-, fluoroC1-C6alkyl- wherein the alkyl is fully or partially fluorinated, halogen, cyano, C1-C6alkoxy-, and fluoroC1-C6alkoxy- wherein the alkyl group is fully or partially fluorinated, hydroxyl substituted C1-C6alkyl-, hydroxyl substituted branched C3-C8alkyl-, cyano substituted C1-C6alkyl-, cyano substituted branched C3-C8alkyl-, (R3)₂NC(O)C1-C6alkyl-, (R3)₂NC(O)C3-C8 branched alkyl-;
wherein each R3 is independently and individually selected from the group consisting of H, C1-C6alkyl, branched C3-C7alkyl, and C3-C8cycloalkyl;
each R4 is independently and individually selected from the group consisting of H, C1-C6alkyl, hydroxyC1-C6alkyl-, dihydroxyC1-C6alkyl-, C1-C6alkoxyC1-C6alkyl-, branched C3-C7alkyl, branched hydroxyC1-C6alkyl-, branched C1-C6alkoxyC1-C6alkyl-, branched dihydroxyC1-C6alkyl-, -(CH₂)ₚN(R7)₂, -(CH₂)ₚR5, - (CH₂)ₚC(O)N(R7)₂, -(CH₂)ₙC(O)R5, -(CH₂)ₙC(O)OR3, R19 substituted C3-C8cycloalkyl-;
each R5 is independently and individually selected from the group consisting of 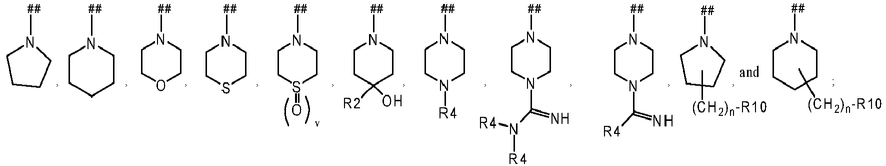 and wherein the symbol (##) is the point of attachment to respective R4, R7, R8, Z2, Z3 or Z4 moieties containing a R5 moiety;
each R6 is independently and individually selected from the group consisting of C1-C6alkyl, branched C3-C7alkyl, R19 substituted C3-C8cycloalkyl-, phenyl, G1, and G3;
each R7 is independently and individually selected from the group consisting of H, C1-C6alkyl, hydroxyC2-C6alkyl-, dihydroxyC2-C6alkyl-, C1-C6alkoxyC2-C6alkyl-, branched C3-C7alkyl, branched hydroxyC2-C6alkyl-, branched C1-C6alkoxyC2-C6alkyl-, branched dihydroxyC2-C6alkyl-, -(CH₂)_{q}R5, -(CH₂)ₙC(O)R5, - (CH₂)ₙC(O)OR3, R19 substituted C3-C8cycloalkyl- and -(CH₂)ₙR17;
each R8 is independently and individually selected from the group consisting of C1-C6alkyl, branched C3-C7alkyl, fluoroC1-C6alkyl- wherein the alkyl moiety is partially or fully fluorinated, R19 substituted C3-C8cycloalkyl-, phenyl, phenylC1-C6alkyl-, G1, G1-C1-C6alkyl-, G4-(CH₂)ₙ-, OH, C1-C6alkoxy, -N(R3)₂, -N(R4)₂, and R5;
each R10 is independently and individually selected from the group consisting of -CO₂H, -CO₂C1-C6alkyl, -C(O)N(R4)₂, OH, C1-C6alkoxy, and -N(R4)₂;
each R16 is independently and individually selected from the group consisting of C1-C6alkyl, branched C3-C7alkyl, R19 substituted C3-C8cycloalkyl-, halogen, fluoroC1-C6alkyl- wherein the alkyl moiety can be partially or fully fluorinated, cyano, hydroxyl,
C1-C6alkoxy, fluoroC1-C6alkoxy- wherein the alkyl moiety can be partially or fully fluorinated, -N(R3)₂, -N(R4)₂, R3 substituted C2-C3alkynyl- and nitro;
each R17 is independently and individually selected from the group consisting of H, C1-C6alkyl, branched C3-C7alkyl, R19 substituted C3-C8cycloalkyl-, halogen, fluoroC1-C6alkyl- wherein the alkyl moiety can be partially or fully fluorinated, cyano, hydroxyl, C1-C6alkoxy, fluoroC1-C6alkoxy- wherein the alkyl moiety can be partially or fully fluorinated, -N(R3)₂, -N(R4)₂, and nitro;
each R19 is independently and individually selected from the group consisting of H, OH and C1-C6alkyl;
each R20 is independently and individually selected from the group consisting of C1-C6alkyl, branched C3-C7alkyl, R19 substituted C3-C8cycloalkyl-, halogen, fluoroC1-C6alkyl- wherein the alkyl moiety can be partially or fully fluorinated, cyano, hydroxyl, C1-C6alkoxy, fluoroC1-C6alkoxy- wherein the alkyl moiety can be partially or fully fluorinated, -N(R3)₂, -N(R4)₂, -N(R3)C(O)R3, -C(O)N(R3)₂ and nitro;
wherein two R4 moieties independently and individually taken from the group consisting of C1-C6alkyl, branched C3-C6alkyl, hydroxyalkyl-, and alkoxyalkyl and attached to the same nitrogen heteroatom may cyclize to form a C3-C7 heterocyclyl ring;
k is 0 or 1; n is 0-6; p is 1-4; q is 2-6; r is 0 or 1; t is 1-3; v is 1 or 2; x is 0-2;
stereo-, regioisomers and tautomers of such compounds;
and with the proviso if A1 is G3, imidazolyl or pyrazolyl then X1 is not a direct bond..
[002] Compounds of paragraph [001] wherein 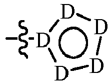 is selected from the group consisting of 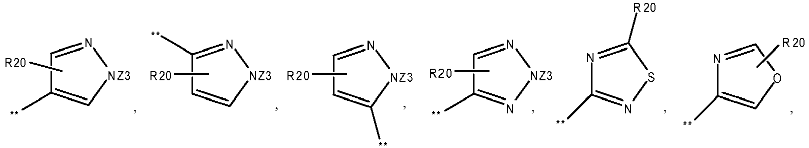 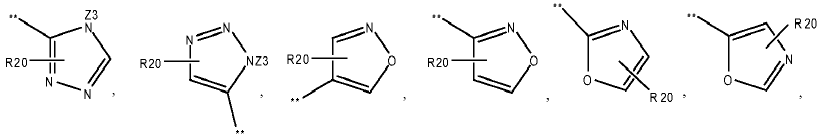 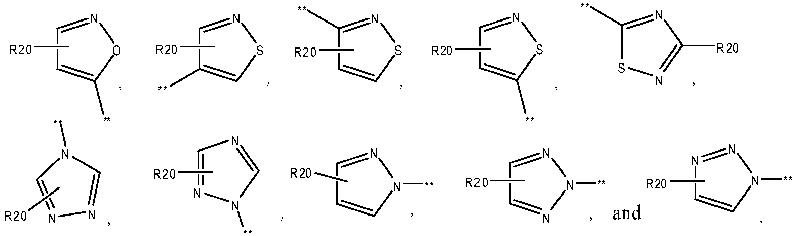 wherein the symbol (**) indicates the point of attachment to the aromatic ring.
[003] Compounds of paragraph [002] having formula Ib 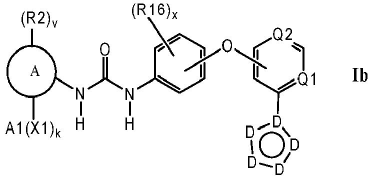 wherein A is any possible isomer of pyrazole.
[004] Compounds of paragraph [003] having formula Ic

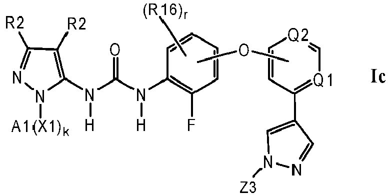

[005] Compounds of paragraph [003] having formula Id 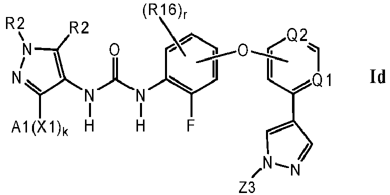 [006] Compounds of paragraph [003] having formula Ie 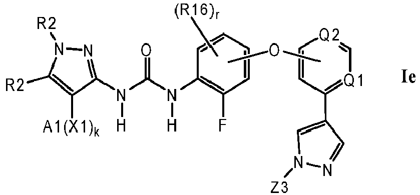 [007] Compounds of paragraph [002] having the formula If 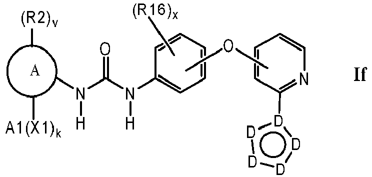 wherein A is selected from the group consisting of any possible isomer of phenyl, pyridine and pyrimidine.
[008] Compounds of paragraph [007] having formula Ig 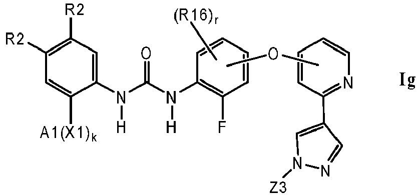 [009] Compounds of paragraph [007] having formula Ih 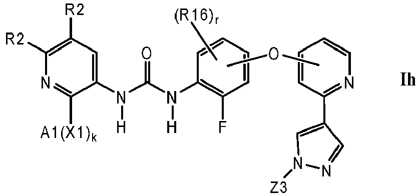 [010] Compounds of paragraph [007] having formula Ii 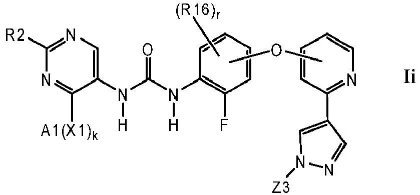 [011] A method of treating mammalian disease wherein the disease etiology or progression is at least partially mediated by the kinase activity of c-Abl kinase, bcr-Abl kinase, Flt-3 kinase, VEGFR-2 kinase mutants, c-Met, HER-1, HER-2, HER-3, HER-4, FGFR, c-Kit, Raf kinase, PDGFRα kinase and PDGFRβ kinase, oncogenic forms thereof, aberrant fusion proteins thereof and polymorphs of any of the foregoing, comprising the step of administering to the mammal a compound of paragraph [001].
[012] A method of paragraph [011] wherein said kinase is selected from the group consisting of bcr-Abl fusion protein kinases p210, bcr-Abl fusion protein kinases p190, bcr-Abl fusion protein kinases bearing the T315I gatekeeper mutant in the Abl kinase domain of p210, bcr-Abl fusion protein kinases bearing the T315I gatekeeper mutant in the Abl kinase domain of p190, and other bcr-Abl polymorphs of any of the foregoing kinases.
[013] The method of paragraph [012], wherein said bcr-Abl fusion protein kinases p210 having Seq. IDs 3 & 4, wherein said bcr-Abl fusion protein kinase p190 has Seq. ID 5, wherein said bcr-Abl fusion protein kinases p210 bearing the T315I mutation in the Abl kinase domain has Seq. IDs 6 & 7, and wherein said bcr-Abl fusion protein kinase p190 bearing the T315I mutation in the Abl kinase domain has Seq. ID 8.
[014] A method of paragraph [011] wherein said kinase is selected from the group consisting of cKit protein kinase, PDGFR-alpha kinase, and any fusion protein, mutation and polymorphs of any of the foregoing.
[015] A method of paragraph [0011] wherein said kinase is selected from the group consisting of B-Raf V600E protein kinase, and any fusion protein, mutation and polymorphs of any of the foregoing.
[016] A method of paragraph [011] wherein said kinase is selected from the group consisting of c-Met protein kinase, and any fusion protein, mutation and polymorphs of any of the foregoing.
[017] A pharmaceutical composition comprising a compound of paragraph [001], together with a pharmaceutically acceptable carrier, optionally containing an additive selected from the group including adjuvants, excipients, diluents, and stabilizers.
[018] A method of treating an individual suffering from a condition selected from the group consisting of cancer, hyperproliferative diseases, metabolic diseases, neurodegenerative diseases, or diseases characterized by angiogenesis, such as solid tumors, melanomas, glioblastomas, ovarian cancer, pancreatic cancer, prostate cancer, lung cancers, breast cancers, renal cancers, hepatic cancers, cervical carcinomas, metastasis of primary tumor sites, myeloproliferative diseases, chronic myelogenous leukemia, leukemias, papillary thyroid carcinoma, non-small cell lung cancer, mesothelioma, hypereosinophilic syndrome, gastrointestinal stromal tumors, colonic cancers, ocular diseases characterized by hyperproliferation leading to blindness including retinopathies, diabetic retinopathy, age-related macular degeneration and hypereosinophilic syndrome, rheumatoid arthritis, asthma, chronic obstructive pulmonary, mastocytosis, mast cell leukemia, or disease a disease caused by c-Kit kinase, oncogenic forms thereof, aberrant fusion proteins thereof and polymorphs thereof, comprising the step of administering to such individual a compound of paragraph [001].
[019] The method of paragraph [018], said compound being administered by a method selected from the group consisting of oral, parenteral, inhalation, and subcutaneous.

## Claims

1. A compound of the formula Ia 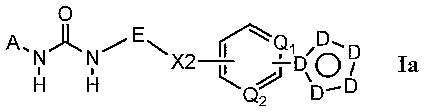 wherein Q1 and Q2 are each individually and independently selected from the group consisting of N and CH and wherein one of Q1 and Q2 is N and the other of Q1 and Q2 is CH;
and wherein the ring containing Q1 and Q2 may be optionally substituted with one or more R20 moieties;
each D is individually taken from the group consisting of C, CH, C-R20, N-Z3, N, O and S, such that the resultant ring is taken from the group consisting of pyrazolyl, triazolyl, isoxazolyl, isothiazolyl, oxazolyl, and thiadiazolyl;
wherein E is selected from the group consisting of phenyl, pyridyl, and pyrimidinyl;
when Q1 and Q2 are both N, the A ring is selected from the group consisting of cyclopentyl, cyclohexyl, G1, G2, and G3;
when only one of Q1 and Q2 is N, the A ring is selected from the group consisting of cyclopentyl, cyclohexyl, G1, G2, G3 and G4;
G1 is a heteroaryl taken from the group consisting of pyrrolyl, furyl, thienyl, oxazolyl, thiazolyl, isoxazolyl-4-yl, isoxazolyl-5-yl, isothiazolyl, imidazolyl, pyrazolyl, oxadiazolyl, thiadiazolyl, triazolyl, and tetrazolyl;
G2 is a fused bicyclic heteroaryl taken from the group consisting of indolyl, indolinyl, isoindolyl, isoindolinyl, indazolyl, benzofuranyl, benzothienyl, benzothiazolyl,
benzothiazolonyl, benzoxazolyl, benzoxazolonyl, benzisoxazolyl, benzisothiazolyl, benzimidazolyl, benzimidazolonyl, benztriazolyl, imidazopyridinyl, pyrazolopyridinyl, imidazolonopyridinyl, thiazolopyridinyl, thiazolonopyridinyl, oxazolopyridinyl, oxazolonopyridinyl, isoxazolopyridinyl, isothiazolopyridinyl, triazolopyridinyl, imidazopyrimidinyl, pyrazolopyrimidinyl, imidazolonopyrimidinyl, thiazolopyridiminyl, thiazolonopyrimidinyl, oxazolopyridiminyl, oxazolonopyrimidinyl, isoxazolopyrimidinyl, isothiazolopyrimidinyl, triazolopyrimidinyl, dihydropurinonyl, pyrrolopyrimidinyl, purinyl, pyrazolopyrimidinyl, phthalimidyl, phthalimidinyl, pyrazinylpyridinyl, pyridinopyrimidinyl, pyrimidinopyrimidinyl, cinnolinyl, quinoxalinyl, quinazolinyl, quinolinyl, isoquinolinyl, phthalazinyl, benzodioxyl, benzisothiazoline-1,1,3-trionyl, dihydroquinolinyl, tetrahydroquinolinyl, dihydroisoquinolyl, tetrahydroisoquinolinyl, benzoazepinyl, benzodiazepinyl, benzoxapinyl, and benzoxazepinyl;
G3 is a heterocyclyl taken from the group consisting of oxetanyl, azetadinyl, tetrahydrofuranyl, pyrrolidinyl, oxazolinyl, oxazolidinyl, imidazolonyl, pyranyl, thiopyranyl, tetrahydropyranyl, dioxalinyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, and homotropanyl;
G4 is selected from the group consisting of phenyl, naphthyl, pyrazinyl, pyridazinyl, triazinyl, pyridinyl, and pyrimidinyl;
the A ring is substituted with one -(X1-A1) moiety or one A1 moiety, and may be optionally substituted with one or two R2 moieties;
when A is phenyl and E is phenyl, then Z1 cannot be -O(CH₂)_{q}R5, -(CH₂)ₚ-G3, or - (CH₂)ₚR5;
A1 is selected from the group consisting of phenyl, G1, G3 and G4;
X1 is selected from the group consisting of a direct bond linking the A and A1 rings, - (CH₂)ₙ(O)ᵣ(CH₂)ₙ-, -(CH₂)ₙ(NR3)ᵣ(CH₂)ₙ-, -(CH₂)ₙ(S)ᵣ(CH₂)ₙ-, -(CH₂)ₙ(C(O))ᵣ(CH₂)ₙ-, - (CH₂)ₙ(C(O)NR3)ᵣ(CH₂)ₙ-, and -(CH₂)ₙ(SO₂NR3)ᵣ(CH₂)ₙ-, wherein any of the alkylenes may be straight or branched chain;
when X1 is a direct bond, E is substituted with one or two R16 moieties;
when X1 is not a direct bond, then E is optionally substituted with one or two R16 moieties;
X2 is selected from the group consisting of -O-, -S(CH₂)ₙ-, -N(R3)(CH₂)ₙ-, -(CH₂)ₚ-, and wherein the carbon atoms of-(CH₂)ₙ-, -(CH₂)ₚ-, of X2 may be further substituted by oxo or one or more C1-C6alkyl moieties;
when A, A1, G1, G2 G3 or G4 has one or more substitutable sp2-hybridized carbon atoms, each respective sp2 hybridized carbon atom may be optionally substituted with a Z1 substituent;
when A, A1, G1, G2 or G3 has one or more substitutable sp3-hybridized carbon atoms, each respective sp3 hybridized carbon atom may be optionally substituted with a Z2 substituent;
when A, A1, G1, G2 or G3 has one or more substitutable nitrogen atoms, each respective nitrogen atom may be optionally substituted with a Z4 substituent;
each Z1 is independently and individually selected from the group consisting of C1-6alkyl, branched C3-C7alkyl, C3-C8cycloalkyl, halogen, fluoroC1-C6alkyl wherein the alkyl moiety can be partially or fully fluorinated, cyano, C1-C6alkoxy, fluoroC1-C6alkoxy wherein the alkyl moiety can be partially or fully fluorinated, -(CH₂)ₙOH, oxo, C1-C6alkoxyC1-C6alkyl, (R4)₂N(CH)ₙ-, (R3)₂N(CH)ₙ-, (R4)₂N(CH₂)_{q}N(R4)(CH₂)ₙ-, (R4)₂N(CH₂)_{q}O(CH₂)ₙ-, (R3)₂NC(O)-, (R4)₂NC(O)-, (R4)₂NC(O)C1-C6alkyl-, - (R4)NC(O)R8, C1-C6alkoxycarbonyl-, -carboxyC1-C6alkyl, C1-C6alkoxycarbonylC1-C6alkyl-, (R3)₂NSO₂-, -SOR3, (R4)₂NSO₂-, -N(R4)SO₂R5, -N(R4)SO₂R8, - O(CH₂)_{q}OC1-C6alkyl, -O(CH₂)_{q}R5, -SO₂R3, -SOR4, -S(O)₂R5, -(CH₂)ₙC(O)R5, - C(O)R8, -C(O)R6, -C(=NOH)R6, -C(=NOR3)R6, -(CH₂)ₙN(R4)C(O)R8, -(CH₂)ₙ-G1, - (CH₂)ₙ-G4, phenoxy, -(CH₂)ₙO(CH₂)ₙ-G1, -(CH₂)ₚO(CH₂)ₙ-G4, -N(R3)(CH₂)_{q}O-alkyl, - N(R3)(CH₂)qN(R4)₂, -N(R3)(CH₂)_{q}R5, -(CH₂)ₙN(R3)(CH₂)ₙ-aryl, -(CH₂)ₙN(R3)(CH₂)ₙ-G1, -(CH₂)ₙN(R3)(CH₂)ₙ-G4, nitro, -(CH₂)ₙNHC(O)(CH₂)ₙR5, - (CH₂)ₙNHS(O)₂(CH₂)ₙR5, -(CH₂)ₙC(O)NH(CH₂)_{q}R5, -(CH₂)ₙOC(O)R5, - CH(OH)(CH₂)ₚR5, -CH(OH)CH(OH)R4, -(CH₂)ₙR5, -C(=NH)R5, -C(=NH)N(R4)₂, - C(=NOR3)R5, -C(=NOR3)N(R4)₂, and -NHC(=NH)R8;
in the event that Z1 contains an alkyl or alkylene moiety, such moieties may be further substituted with one or more C1-C6alkyls;
each Z2 is independently and individually selected from the group consisting of aryl, C1-C6alkyl, C3-C8cycloalkyl, branched C3-C7alkyl, hydroxyl, hydroxyC1-C6alkyl-, cyano, (R3)₂N-, (R4)₂N-, (R4)₂NC1-C6alkyl-, (R4)₂NC2-C6alkylN(R4)(CH₂)ₙ-, (R4)₂NC2-C6alkylO(CH₂)ₙ-, (R3)₂NC(O)-, (R4)₂NC(O)-, (R4)₂NC(O)-C1-C6alkyl-, carboxyl, - carboxyC1-C6alkyl, C1-C6alkoxycarbonyl-, C1-C6alkoxycarbonylC1-C6alkyl-, (R3)₂NSO₂-, (R4)₂NSO₂-, -SO₂R5, -SO₂R8, -(CH₂)ₙN(R4)C(O)R8, -C(O)R8, =O, =NOH, =N(OR6), -(CH₂)ₙ-G1, -(CH₂)ₙ-G4, -(CH₂)ₙO(CH₂)ₙ-G1, -(CH₂)ₙO(CH₂)ₙ-G4, - (CH₂)ₙN(R3)(CH₂)ₙ-aryl, -(CH₂)ₙN(R3)(CH₂)ₙ-G1, -(CH₂)ₙN(R3)(CH₂)ₙ-G4, - (CH₂)ₙNHC(O)(CH₂)ₙR5, -(CH₂)ₙNHS(O)₂(CH₂)ₙR5, -(CH₂)ₙC(O)NH(CH₂)_{q}R5, - (CH₂)ₙC(O)R5, -(CH₂)ₙOC(O)R5, and -(CH₂)ₙR5;
in the event that Z2 contains an alkyl or alkylene moiety, such moieties may be further substituted with one or more C1-C6alkyls;
each Z3 is independently and individually selected from the group consisting of H, C1-C6alkyl, branched C3-C7alkyl, C3-C8cycloalkyl, fluoroC1-C6alkyl wherein the alkyl moiety can be partially or fully fluorinated, hydroxyC2-C6alkyl-, C1-C6alkoxycarbonyl-, -C(O)R8, R5C(O)(CH₂)ₙ-, (R4)₂NC(O)-, (R4)₂NC(O)C1-C6alkyl-, R8C(O)N(R4)(CH₂)_{q}-, (R3)₂NSO₂- (R4)₂NSO₂-, -(CH₂)_{q}N(R3)₂, and -(CH₂)_{q}N(R4)₂;
each Z4 is independently and individually selected from the group consisting of C1-C6alkyl, branched C3-7alkyl, hydroxyC2-C6alkyl-, Cl-C6alkoxyC2-C6alkyl-, (R4)₂N-C2-C6alkyl-, (R4)₂N-C2-C6alkylN(R4)-C2-C6alkyl-, (R4)₂N-C2-C6alkyl-O-C2-C6alkyl- (R4)₂NC(O)C1-C6alkyl-, carboxyC1-C6alkyl, C1-C6alkoxycarbonylC1-C6alkyl-, -C2-C6alkylN(R4)C(O)R8, R8-C(=NR3)-, -SO₂R8, -COR8, -(CH₂)ₙ-G1, - (CH₂)ₙ-G4, -(CH₂)_{q}O(CH₂)ₙ-G1, -(CH₂)_{q}O(CH₂)ₙ-G4, -(CH₂)_{q}NR3(CH₂)ₙ-G1,-(CH₂)_{q}NR3(CH₂)ₙ-G4, -(CH₂)_{q}NHC(O)(CH₂)ₙR5, -(CH₂)_{q}C(O)NH(CH₂)_{q}R5, - (CH₂)_{q}C(O)R5, -(CH₂)_{q}OC(O)R5, -(CH₂)_{q}R5, -(CH₂)_{q}NR4(CH₂)_{q}R5, and - (CH₂)_{q}O(CH₂)_{q}R5;
in the event that Z4 contains an alkyl or alkylene moiety, such moieties may be further substituted with one or more C1-C6alkyls;
each R2 is selected from the group consisting of H, R17-substituted aryl-, R17-substituted G1, R17-substituted G4, Cl-C6alkyl, branched C3-C8alkyl, R19 substituted C3-C8cycloalkyl-, fluoroC1-C6alkyl wherein the alkyl is fully or partially fluorinated, halogen, cyano, C1-C6alkoxy-, and fluoroC1-C6alkoxy- wherein the alkyl group is fully or partially fluorinated, hydroxyl substituted C1-C6alkyl-, hydroxyl substituted branched C3-C8alkyl-, cyano substituted C1-C6alkyl-, cyano substituted branched C3-C8alkyl-, (R3)₂NC(O)C1-C6alkyl-, (R3)₂NC(O)C3-C8 branched alkyl-;
wherein each R3 is independently and individually selected from the group consisting of H, C1-C6alkyl, branched C3-C7alkyl, and C3-C8cycloalkyl;
each R4 is independently and individually selected from the group consisting of H, C1-C6alkyl, hydroxyC1-C6alkyl-, dihydroxyC1-C6alkyl-, C1-C6alkoxyC1-C6alkyl-, branched C3-C7alkyl, branched hydroxyC1-C6alkyl-, branched C1-C6alkoxyC1-C6alkyl-, branched dihydroxyC1-C6alkyl-, -(CH₂)ₚN(R7)₂, -(CH₂)ₚR5, - (CH₂)ₚC(O)N(R7)₂, -(CH₂)ₙC(O)R5, -(CH₂)ₙC(O)OR3, R19 substituted C3-C8cycloalkyl-;
each R5 is independently and individually selected from the group consisting of 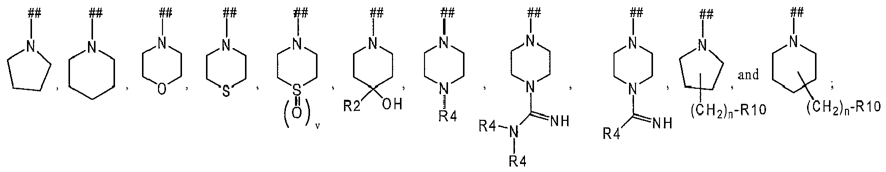 and wherein the symbol (##) is the point of attachment to respective R4, R7, R8, Z2, Z3 or Z4 moieties containing a R5 moiety;
each R6 is independently and individually selected from the group consisting of C1-C6alkyl, branched C3-C7alkyl, R19 substituted C3-C8cycloalkyl-, phenyl, G1, and G3;
each R7 is independently and individually selected from the group consisting of H, C1-C6alkyl, hydroxyC2-C6alkyl-, dihydroxyC2-C6alkyl-, C1-C6alkoxyC2-C6alkyl-, branched C3-C7alkyl, branched hydroxyC2-C6alkyl-, branched C1-C6alkoxyC2-C6alkyl-, branched dihydroxyC2-C6alkyl-, -(CH₂)_{q}R5, -(CH₂)ₙC(O)R5, - (CH₂)ₙC(O)OR3, R19 substituted C3-C8cycloalkyl- and -(CH₂)ₙR17;
each R8 is independently and individually selected from the group consisting of C1-C6alkyl, branched C3-C7alkyl, fluoroC1-C6alkyl- wherein the alkyl moiety is partially or fully fluorinated, R19 substituted C3-C8cycloalkyl-, phenyl, phenylC1-C6alkyl-, G1, G1-C1-C6alkyl-, G4-(CH₂)ₙ-, OH, C1-C6alkoxy, -N(R3)₂, -N(R4)₂, and R5;
each R10 is independently and individually selected from the group consisting of -CO₂H, -CO₂C1-C6alkyl, -C(O)N(R4)₂, OH, C1-C6alkoxy, and -N(R4)₂;
each R16 is independently and individually selected from the group consisting of C1-C6alkyl, branched C3-C7alkyl, R19 substituted C3-C8cycloalkyl-, halogen, fluoroC1-C6alkyl- wherein the alkyl moiety can be partially or fully fluorinated, cyano, hydroxyl, C1-C6alkoxy, fluoroC1-C6alkoxy- wherein the alkyl moiety can be partially or fully fluorinated, -N(R3)₂, -N(R4)₂, R3 substituted C2-C3alkynyl- and nitro;
each R17 is independently and individually selected from the group consisting of H, C1-C6alkyl, branched C3-C7alkyl, R19 substituted C3-C8cycloalkyl-, halogen, fluoroC1-C6alkyl- wherein the alkyl moiety can be partially or fully fluorinated, cyano, hydroxyl, Cl-C6alkoxy, fluoroC1-C6alkoxy- wherein the alkyl moiety can be partially or fully fluorinated, -N(R3)₂, -N(R4)₂, and nitro;
each R19 is independently and individually selected from the group consisting of H, OH and C1-C6alkyl;
each R20 is independently and individually selected from the group consisting of C1-C6alkyl, branched C3-C7alkyl, R19 substituted C3-C8cycloalkyl-, halogen, fluoroC1-C6alkyl- wherein the alkyl moiety can be partially or fully fluorinated, cyano, hydroxyl, C1-C6alkoxy, fluoroCl-C6alkoxy- wherein the alkyl moiety can be partially or fully fluorinated, -N(R3)₂, -N(R4)₂, -N(R3)C(O)R3, -C(O)N(R3)₂ and nitro;
wherein two R4 moieties independently and individually taken from the group consisting of C1-C6alkyl, branched C3-C6alkyl, hydroxyalkyl-, and alkoxyalkyl and attached to the same nitrogen heteroatom may cyclize to form a C3-C7 heterocyclyl ring;
k is 0 or 1; n is 0-6; p is 1-4; q is 2-6; r is 0 or 1; t is 1-3; v is 1 or 2; x is 0-2;
or a pharmaceutically acceptable salt, a stereoisomer, a regioisomer or a tautomer thereof; and with the proviso if A1 is G3, imidazolyl or pyrazolyl then X1 is not a direct bond.

2. The compound of claim 1, wherein each D is individually selected from the group consisting of C, CH, N-Z3, and N, provided that the resultant ring is selected from the group consisting of pyrazolyl.

3. The compound of claim 1 or 2, wherein A is G1 and G1 is selected from the group consisting of pyrazolyl and isoxazolyl-5-yl.

4. The compound of claim 1 or 2, wherein A is G4 and G4 is selected from the group consisting of phenyl, pyridinyl and pyrimidinyl.

5. The compound of claim 2, having formula 1b 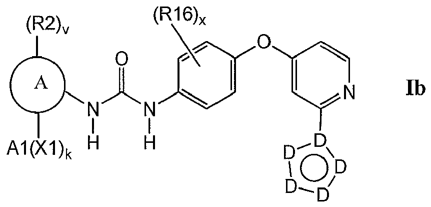 wherein:
each D is individually taken from the group consisting of C, CH, N-Z3, N, provided that the resultant ring is taken from the group consisting of pyrazolyl;
the A ring is selected from the group consisting of G1 and G4;
G1 is a heteroaryl taken from the group consisting of isoxazolyl-5-yl and pyrazolyl;
G4 is selected from the group consisting of phenyl, pyridinyl, and pyrimidinyl;
the A ring is substituted with one A1 moiety, and may be optionally substituted with one or two R2 moieties;
A1 is selected from the group consisting of phenyl;
when A, A1, G1, or G4 has one or more substitutable sp2-hybridized carbon atoms, each respective sp2 hybridized carbon atom may be optionally substituted with a Z1 substituent;
each Z1 is independently and individually selected from the group consisting of C1-6alkyl, branched C3-C7alkyl, C3-C8cycloalkyl, halogen, fluoroC1-C6alkyl wherein the alkyl moiety can be partially or fully fluorinated, cyano, C1-C6alkoxy, -(CH₂)ₙOH, oxo, C1-C6alkoxycarbonyl-, and -C(O)R8,;
when Z1 contains an alkyl or alkylene moiety, each moiety may be further substituted with one or more C1-C6alkyls;
each Z3 is independently and individually selected from the group consisting of H, C1-C6alkyl, branched C3-C7alkyl, C3-C8cycloalkyl, and hydroxyC2-C6alkyl-;
each R2 is selected from the group consisting of H, C1-C6alkyl, branched C3-C8alkyl,
fluoroCl-C6alkyl- wherein the alkyl is fully or partially fluorinated, and halogen;
each R8 is independently and individually selected from the group consisting of C1-C6alkyl, branched C3-C7alkyl, fluoroC1-C6alkyl- wherein the alkyl moiety is partially or
fully fluorinated, OH, and C1-C6alkoxy;
each R16 is independently and individually selected from the group consisting of C1-C6alkyl, branched C3-C7alkyl, and halogen;
k is 0; n is 0-6; r is 0 or 1;v is 1 or 2; x is 1-2;
or a pharmaceutically acceptable salt, a stereoisomer, a regioisomer, or a tautomer thereof.

6. A compound of claim 1 selected from the group consisting of 1-(1-(3-cyanophenyl)-3-isopropyl-1H-pyrazol-5-yl)-3-(2-fluoro-4-(2-(1-methyl-1H-pyrazol-4-yl)pyridin-4-yloxy)phenyl)urea, 1-(3-tert-butyl-1-(3-cyanophenyl)-1H-pyrazol-5-yl)-3-(2-fluoro-4-(2-(1-methyl-1H-pyrazol-4-yl)pyridin-4-yloxy)phenyl)urea, 1-(3-tert-butyl-1-(4-cyanophenyl)-1H-pyrazol-5-yl)-3-(2-fluoro-4-(2-(1-methyl-1H-pyrazol-4-yl)pyridin-4-yloxy)phenyl)urea, 1-(3-tert-butyl-1-(3-(hydroxymethyl)phenyl)-1H-pyrazol-5-yl)-3-(2-fluoro-4-(2-(1-methyl-1H-pyrazol-4-yl)pyridin-4-yloxy)phenyl)urea, 1-(3-tert-butyl-1-(4-(hydroxymethyl)phenyl)-1H-pyrazol-5-yl)-3-(2-fluoro-4-(2-(1-methyl-1H-pyrazol-4-yl)pyridin-4-yloxy)phenyl)urea, 1-(1-(3-cyanophenyl)-3-isopropyl-1H-pyrazol-5-yl)-3-(2-fluoro-3-methyl-4-(2-(1-methyl-1H-pyrazol-4-yl)pyridin-4-yloxy)phenyl)urea, 1-(1-(3-cyanophenyl)-3-isopropyl-1H-pyrazol-5-yl)-3-(2,3-difluoro-4-(2-(1-methyl-1H-pyrazol-4-yl)pyridin-4-yloxy)phenyl)urea, 1-(2,3-difluoro-4-(2-(1-methyl-1H-pyrazol-4-yl)pyridin-4-yloxy)phenyl)-3-(3-(2-fluorophenyl)-1-methyl-1H-pyrazol-5-yl)urea, , 1-(3-tert-butyl-1-phenyl-1H-pyrazol-5-yl)-3-(2-fluoro-4-(2-(1-methyl-1H-pyrazol-4-yl)pyridin-4-yloxy)phenyl)urea, 1-(4-(2-(1H-pyrazol-4-yl)pyridin-4-yloxy)-2-fluorophenyl)-3-(3-tert-butyl-1-(3-cyanophenyl)-1H-pyrazol-5-yl)urea, 1-(1-(3-cyano-4-fluorophenyl)-3-isopropyl-1H-pyrazol-5-yl)-3-(2-fluoro-4-(2-(1-methyl-1H-pyrazol-4-yl)pyridin-4-yloxy)phenyl)urea, 1-(1-(3-cyanophenyl)-3-ethyl-1H-pyrazol-5-yl)-3-(2-fluoro-4-(2-(1-methyl-1H-pyrazol-4-yl)pyridin-4-yloxy)phenyl)urea, 1-(2-tert-butyl-4-phenylpyrimidin-5-yl)-3-(2-fluoro-4-(2-(1-methyl-1H-pyrazol-4-yl)pyridin-4-yloxy)phenyl)urea, 1-(1-(3-cyano-4-methylphenyl)-3-isopropyl-1H-pyrazol-5-yl)-3-(2-fluoro-4-(2-(1-methyl-1H-pyrazol-4-yl)pyridin-4-yloxy)phenyl)urea, 1-(2-fluoro-4-(2-(1-methyl-1H-pyrazol-4-yl)pyridin-4-yloxy)phenyl)-3-(2-methyl-4-phenylpyrimidin-5-yl)urea, 1-(1-(3-cyanophenyl)-3-ethyl-1H-pyrazol-5-yl)-3-(2-fluoro-4-(2-(1-propyl-1H-pyrazol-4-yl)pyridin-4-yloxy)phenyl)urea, 1-(1-(3-cyanophenyl)-3-isopropyl-1H-pyrazol-5-yl)-3-(3-fluoro-4-(2-(1-methyl-1H-pyrazol-4-yl)pyridin-4-yloxy)phenyl)urea, 1-(1-(3-cyanophenyl)-3-ethyl-1H-pyrazol-5-yl)-3-(2-fluoro-4-(2-(1-isopropyl-1H-pyrazol-4-yl)pyridin-4-yloxy)phenyl)urea, 1-(1-(3-cyanophenyl)-3-ethyl-1H-pyrazol-5-yl)-3-(4-(2-(1-ethyl-1H-pyrazol-4-yl)pyridin-4-yloxy)-2-fluorophenyl)urea, 1-(3-ethyl-1-(4-(hydroxymethyl)phenyl)-1H-pyrazol-5-yl)-3-(2-fluoro-4-(2-(1-methyl-1H-pyrazol-4-yl)pyridin-4-yloxy)phenyl)urea, 1-(2-fluoro-4-(2-(1-methyl-1H-pyrazol-4-yl)pyridin-4-yloxy)phenyl)-3-(1-isopropyl-5-phenyl-1H-pyrazol-4-yl)urea, 1-(2-fluoro-4-(2-(1-methyl-1H-pyrazol-4-yl)pyridin-4-yloxy)phenyl)-3-(1-isopropyl-3-phenyl-1H-pyrazol-4-yl)urea, 1-(1-ethyl-5-phenyl-1H-pyrazol-4-yl)-3-(2-fluoro-4-(2-(1-methyl-1H-pyrazol-4-yl)pyridin-4-yloxy)phenyl)urea, 1-(1-ethyl-3-phenyl-1H-pyrazol-4-yl)-3-(2-fluoro-4-(2-(1-methyl-1H-pyrazol-4-yl)pyridin-4-yloxy)phenyl)urea, 1-(2-fluoro-4-(2-(1-methyl-1H-pyrazol-4-yl)pyridin-4-yloxy)phenyl)-3-(1-(3-fluorophenyl)-3-isopropyl-1H-pyrazol-5-yl)urea.

7. The compound of any one of claims 1 to 3 or 5 having formula Ib 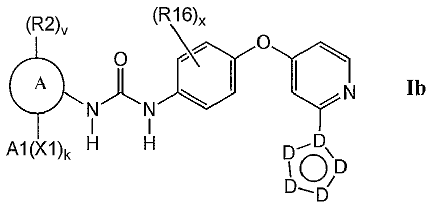 wherein A is pyrazole.

8. The compound of claim 7 having formula Ic, or Id 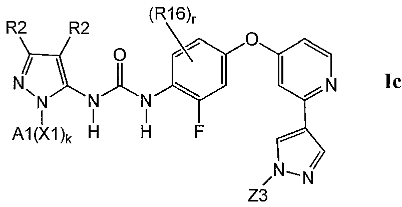 or

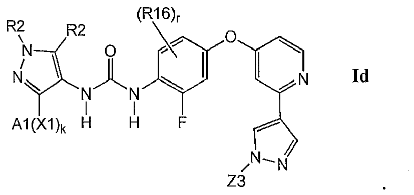

9. The compound of any one of claims 1, 2, 4 or 5 having the formula If 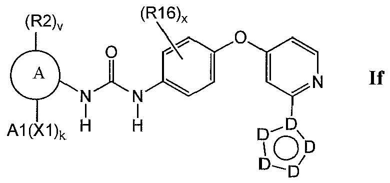 wherein A is selected from the group consisting of phenyl, pyridine, and pyrimidine.

10. The compound of claim 9 having formula Ig, Ih or Ii 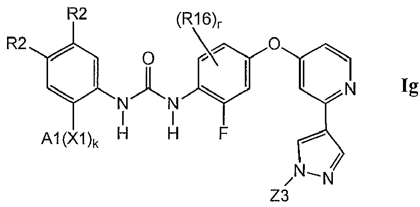 or 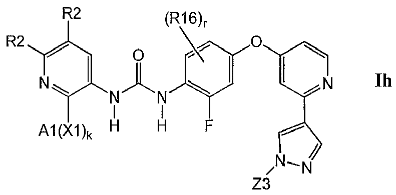 or

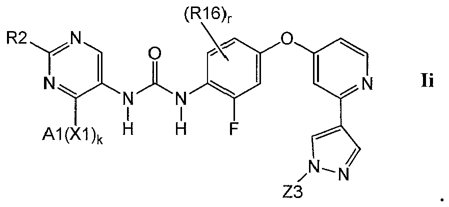

11. A compound of claim 1 selected from the group consisting of 1-(5-chloro-2-phenylphenyl)-3-(2-fluoro-4-(2-(1-methyl-1H-pyrazol-4-yl)pyridin-4-yloxy)phenyl)urea 1-(3-tert-butyl-1-(4-fluorophenyl)-1H-pyrazol-5-yl)-3-(2-fluoro-4-(2-(1-methyl-1H-pyrazol-4-yl)pyridin-4-yloxy)phenyl)urea, 1-(1-(4-cyanophenyl)-3-isopropyl-1H-pyrazol-5-yl)-3-(2-fluoro-4-(2-(1-methyl-1H-pyrazol-4-yl)pyridin-4-yloxy)phenyl)urea, ethyl 3-(3-tert-butyl-5-(3-(2-fluoro-4-(2-(1-methyl-1H-pyrazol-4-yl)pyridin-4-yloxy)phenyl)ureido)-1H-pyrazol-1-yl)benzoate, 1-(3-ethyl-1-(3-(hydroxymethyl)phenyl)-1H-pyrazol-5-yl)-3-(2-fluoro-4-(2-(1-methyl-1H-pyrazol-4-yl)pyridin-4-yloxy)phenyl)urea, 3-(3-tert-butyl-5-(3-(2-fluoro-4-(2-(1-methyl-1H-pyrazol-4-yl)pyridin-4-yloxy)phenyl)ureido)-1H-pyrazol-1-yl)benzoic acid, 1-(1-(3-cyanophenyl)-3-methyl-1H-pyrazol-5-yl)-3-(2-fluoro-4-(2-(1-methyl-1H-pyrazol-4-yl)pyridin-4-yloxy)phenyl)urea, 1-(3-(3-cyanophenyl)-1-isopropyl-1H-pyrazol-4-yl)-3-(2-fluoro-4-(2-(1-methyl-1H-pyrazol-4-yl)pyridin-4-yloxy)phenyl)urea, 1-(3-tert-butyl-1-phenyl-1H-pyrazol-5-yl)-3-(2,3-difluoro-4-(2-(1-methyl-1H-pyrazol-4-yl)pyridin-4-yloxy)phenyl)urea, and 1-(2,3-difluoro-4-(2-(1-methyl-1H-pyrazol-4-yl)pyridin-4-yloxy)phenyl)-3-(5-phenylpyridin-3-yl)urea.

12. A compound of any preceding claim for use in the treatment of mammalian disease wherein the disease etiology or progression is at least partially mediated by the kinase activity of c-Abl kinase, bcr-Abl kinase, VEGFR-2 kinase, c-Kit, oncogenic forms thereof, aberrant fusion proteins thereof and polymorphs of any of the foregoing.

13. The compound for use of claim 12 wherein said kinase is selected from the group consisting of bcr-Abl fusion protein kinases p210, bcr-Abl fusion protein kinases p190, bcr-Abl fusion protein kinases bearing the T315I gatekeeper mutant in the Abl kinase domain of p210, bcr-Abl fusion protein kinases bearing the T315I gatekeeper mutant in the Abl kinase domain of p190, and other bcr-Abl polymorphs of any of the foregoing kinases, preferably wherein said bcr-Abl fusion protein kinases p210 having Seq. IDs 3 & 4, wherein said bcr-Abl fusion protein kinase p190 has Seq. ID 5, wherein said bcr-Abl fusion protein kinases p210 bearing the T315I mutation in the Abl kinase domain has Seq. IDs 6 & 7, and wherein said bcr-Abl fusion protein kinase p190 bearing the T315I mutation in the Abl kinase domain has Seq. ID 8.

14. A pharmaceutical composition comprising a compound of any one of claims 1 to 11, together with a pharmaceutically acceptable carrier, optionally containing an additive selected from the group including adjuvants, excipients, diluents, and stabilizers.

15. A compound of any one of claims 1 to 11 for use in the treatment of a condition selected from the group consisting of cancer, or diseases **characterized by** angiogenesis, solid tumors, melanomas, glioblastomas, ovarian cancer, pancreatic cancer, prostate cancer, lung cancers, breast cancers, renal cancers, hepatic cancers, cervical carcinomas, metastasis of primary tumor sites, myeloproliferative diseases, chronic myelogenous leukemia, leukemias, papillary thyroid carcinoma, non-small cell lung cancer, mesothelioma, hypereosinophilic syndrome, gastrointestinal stromal tumors, colonic cancers, ocular diseases **characterized by** hyperproliferation leading to blindness including retinopathies, diabetic retinopathy, age-related macular degeneration and mastocytosis, mast cell leukemia.


SEQUENCE LISTING

<110>  Deciphera Pharmaceuticals, LLC

<120>  KINASE INHIBITORS USEFUL FOR THE TREATMENT OF MYLEOPROLIFERATIVE
       DISEASES AND OTHER PROLIFERATIVE DISEASES

<130>  P48036EP1

<150>  EP08746333.7
<151>  2008-04-18

<150>  US 60/913,216
<151>  2007-04-20

<160>  12

<170>  PatentIn version 3.3

<210>  1
<211>  278
<212>  PRT
<213>  Homo sapiens

<400>  1

Gly Thr Ser Met Asp Pro Ser Ser Pro Asn Tyr Asp Lys Trp Glu Met
1               5                   10                  15

Glu Arg Thr Asp Ile Thr Met Lys His Lys Leu Gly Gly Gly Gln Tyr
            20                  25                  30

Gly Glu Val Tyr Glu Gly Val Trp Lys Lys Tyr Ser Leu Thr Val Ala
        35                  40                  45

Val Lys Thr Leu Lys Glu Asp Thr Met Glu Val Glu Glu Phe Leu Lys
    50                  55                  60

Glu Ala Ala Val Met Lys Glu Ile Lys His Pro Asn Leu Val Gln Leu
65                  70                  75                  80

Leu Gly Val Cys Thr Arg Glu Pro Pro Phe Tyr Ile Ile Thr Glu Phe
                85                  90                  95

Met Thr Tyr Gly Asn Leu Leu Asp Tyr Leu Arg Glu Cys Asn Arg Gln
            100                 105                 110

Glu Val Asn Ala Val Val Leu Leu Tyr Met Ala Thr Gln Ile Ser Ser
        115                 120                 125

Ala Met Glu Tyr Leu Glu Lys Lys Asn Phe Ile His Arg Asp Leu Ala
    130                 135                 140

Ala Arg Asn Cys Leu Val Gly Glu Asn His Leu Val Lys Val Ala Asp
145                 150                 155                 160

Phe Gly Leu Ser Arg Leu Met Thr Gly Asp Thr Tyr Thr Ala His Ala
                165                 170                 175

Gly Ala Lys Phe Pro Ile Lys Trp Thr Ala Pro Glu Ser Leu Ala Tyr
            180                 185                 190

Asn Lys Phe Ser Ile Lys Ser Asp Val Trp Ala Phe Gly Val Leu Leu
        195                 200                 205

Trp Glu Ile Ala Thr Tyr Gly Met Ser Pro Tyr Pro Gly Ile Asp Leu
    210                 215                 220

Ser Gln Val Tyr Glu Leu Leu Glu Lys Asp Tyr Arg Met Glu Arg Pro
225                 230                 235                 240

Glu Gly Cys Pro Glu Lys Val Tyr Glu Leu Met Arg Ala Cys Trp Gln
                245                 250                 255

Trp Asn Pro Ser Asp Arg Pro Ser Phe Ala Glu Ile His Gln Ala Phe
            260                 265                 270

Glu Thr Met Phe Gln Glu
        275

<210>  2
<211>  278
<212>  PRT
<213>  Homo sapiens

<400>  2

Gly Thr Ser Met Asp Pro Ser Ser Pro Asn Tyr Asp Lys Trp Glu Met
1               5                   10                  15

Glu Arg Thr Asp Ile Thr Met Lys His Lys Leu Gly Gly Gly Gln Tyr
            20                  25                  30

Gly Glu Val Tyr Glu Gly Val Trp Lys Lys Tyr Ser Leu Thr Val Ala
        35                  40                  45

Val Lys Thr Leu Lys Glu Asp Thr Met Glu Val Glu Glu Phe Leu Lys
    50                  55                  60

Glu Ala Ala Val Met Lys Glu Ile Lys His Pro Asn Leu Val Gln Leu
65                  70                  75                  80

Leu Gly Val Cys Thr Arg Glu Pro Pro Phe Tyr Ile Ile Ile Glu Phe
                85                  90                  95

Met Thr Tyr Gly Asn Leu Leu Asp Tyr Leu Arg Glu Cys Asn Arg Gln

            100                 105                 110

Glu Val Asn Ala Val Val Leu Leu Tyr Met Ala Thr Gln Ile Ser Ser
        115                 120                 125

Ala Met Glu Tyr Leu Glu Lys Lys Asn Phe Ile His Arg Asp Leu Ala
    130                 135                 140

Ala Arg Asn Cys Leu Val Gly Glu Asn His Leu Val Lys Val Ala Asp
145                 150                 155                 160

Phe Gly Leu Ser Arg Leu Met Thr Gly Asp Thr Tyr Thr Ala His Ala
                165                 170                 175

Gly Ala Lys Phe Pro Ile Lys Trp Thr Ala Pro Glu Ser Leu Ala Tyr
            180                 185                 190

Asn Lys Phe Ser Ile Lys Ser Asp Val Trp Ala Phe Gly Val Leu Leu
        195                 200                 205

Trp Glu Ile Ala Thr Tyr Gly Met Ser Pro Tyr Pro Gly Ile Asp Leu
    210                 215                 220

Ser Gln Val Tyr Glu Leu Leu Glu Lys Asp Tyr Arg Met Glu Arg Pro
225                 230                 235                 240

Glu Gly Cys Pro Glu Lys Val Tyr Glu Leu Met Arg Ala Cys Trp Gln
                245                 250                 255

Trp Asn Pro Ser Asp Arg Pro Ser Phe Ala Glu Ile His Gln Ala Phe
            260                 265                 270

Glu Thr Met Phe Gln Glu
        275

<210> 3
<211> 2031
<212> PRT
<213> Homo sapiens

<400> 3

<210>  4
<211>  2006
<212>  PRT

<213>  Homo sapiens

<400>  4

Met Val Asp Pro Val Gly Phe Ala Glu Ala Trp Lys Ala Gln Phe Pro
1               5                   10                  15

Asp Ser Glu Pro Pro Arg Met Glu Leu Arg Ser Val Gly Asp Ile Glu
            20                  25                  30

Gln Glu Leu Glu Arg Cys Lys Ala Ser Ile Arg Arg Leu Glu Gln Glu
        35                  40                  45

Val Asn Gln Glu Arg Phe Arg Met Ile Tyr Leu Gln Thr Leu Leu Ala
    50                  55                  60

Lys Glu Lys Lys Ser Tyr Asp Arg Gln Arg Trp Gly Phe Arg Arg Ala
65                  70                  75                  80

Ala Gln Ala Pro Asp Gly Ala Ser Glu Pro Arg Ala Ser Ala Ser Arg
                85                  90                  95

Pro Gln Pro Ala Pro Ala Asp Gly Ala Asp Pro Pro Pro Ala Glu Glu
            100                 105                 110

Pro Glu Ala Arg Pro Asp Gly Glu Gly Ser Pro Gly Lys Ala Arg Pro
        115                 120                 125

Gly Thr Ala Arg Arg Pro Gly Ala Ala Ala Ser Gly Glu Arg Asp Asp
    130                 135                 140

Arg Gly Pro Pro Ala Ser Val Ala Ala Leu Arg Ser Asn Phe Glu Arg
145                 150                 155                 160

Ile Arg Lys Gly His Gly Gln Pro Gly Ala Asp Ala Glu Lys Pro Phe
                165                 170                 175

Tyr Val Asn Val Glu Phe His His Glu Arg Gly Leu Val Lys Val Asn
            180                 185                 190

Asp Lys Glu Val Ser Asp Arg Ile Ser Ser Leu Gly Ser Gln Ala Met
        195                 200                 205

Gln Met Glu Arg Lys Lys Ser Gln His Gly Ala Gly Ser Ser Val Gly
    210                 215                 220

Asp Ala Ser Arg Pro Pro Tyr Arg Gly Arg Ser Ser Glu Ser Ser Cys
225                 230                 235                 240

Gly Val Asp Gly Asp Tyr Glu Asp Ala Glu Leu Asn Pro Arg Phe Leu

                245                 250                 255

Lys Asp Asn Leu Ile Asp Ala Asn Gly Gly Ser Arg Pro Pro Trp Pro
            260                 265                 270

Pro Leu Glu Tyr Gln Pro Tyr Gln Ser Ile Tyr Val Gly Gly Ile Met
        275                 280                 285

Glu Gly Glu Gly Lys Gly Pro Leu Leu Arg Ser Gln Ser Thr Ser Glu
    290                 295                 300

Gln Glu Lys Arg Leu Thr Trp Pro Arg Arg Ser Tyr Ser Pro Arg Ser
305                 310                 315                 320

Phe Glu Asp Cys Gly Gly Gly Tyr Thr Pro Asp Cys Ser Ser Asn Glu
                325                 330                 335

Asn Leu Thr Ser Ser Glu Glu Asp Phe Ser Ser Gly Gln Ser Ser Arg
            340                 345                 350

Val Ser Pro Ser Pro Thr Thr Tyr Arg Met Phe Arg Asp Lys Ser Arg
        355                 360                 365

Ser Pro Ser Gln Asn Ser Gln Gln Ser Phe Asp Ser Ser Ser Pro Pro
    370                 375                 380

Thr Pro Gln Cys His Lys Arg His Arg His Cys Pro Val Val Val Ser
385                 390                 395                 400

Glu Ala Thr Ile Val Gly Val Arg Lys Thr Gly Gln Ile Trp Pro Asn
                405                 410                 415

Asp Asp Glu Gly Ala Phe His Gly Asp Ala Asp Gly Ser Phe Gly Thr
            420                 425                 430

Pro Pro Gly Tyr Gly Cys Ala Ala Asp Arg Ala Glu Glu Gln Arg Arg
        435                 440                 445

His Gln Asp Gly Leu Pro Tyr Ile Asp Asp Ser Pro Ser Ser Ser Pro
    450                 455                 460

His Leu Ser Ser Lys Gly Arg Gly Ser Arg Asp Ala Leu Val Ser Gly
465                 470                 475                 480

Ala Leu Lys Ser Thr Lys Ala Ser Glu Leu Asp Leu Glu Lys Gly Leu
                485                 490                 495

Glu Met Arg Lys Trp Val Leu Ser Gly Ile Leu Ala Ser Glu Glu Thr
            500                 505                 510

Tyr Leu Ser His Leu Glu Ala Leu Leu Leu Pro Met Lys Pro Leu Lys
        515                 520                 525

Ala Ala Ala Thr Thr Ser Gln Pro Val Leu Thr Ser Gln Gln Ile Glu
    530                 535                 540

Thr Ile Phe Phe Lys Val Pro Glu Leu Tyr Glu Ile His Lys Glu Ser
545                 550                 555                 560

Tyr Asp Gly Leu Phe Pro Arg Val Gln Gln Trp Ser His Gln Gln Arg
                565                 570                 575

Val Gly Asp Leu Phe Gln Lys Leu Ala Ser Gln Leu Gly Val Tyr Arg
            580                 585                 590

Ala Phe Val Asp Asn Tyr Gly Val Ala Met Glu Met Ala Glu Lys Cys
        595                 600                 605

Cys Gln Ala Asn Ala Gln Phe Ala Glu Ile Ser Glu Asn Leu Arg Ala
    610                 615                 620

Arg Ser Asn Lys Asp Ala Lys Asp Pro Thr Thr Lys Asn Ser Leu Glu
625                 630                 635                 640

Thr Leu Leu Tyr Lys Pro Val Asp Arg Val Thr Arg Ser Thr Leu Val
                645                 650                 655

Leu His Asp Leu Leu Lys His Thr Pro Ala Ser His Pro Asp His Pro
            660                 665                 670

Leu Leu Gln Asp Ala Leu Arg Ile Ser Gln Asn Phe Leu Ser Ser Ile
        675                 680                 685

Asn Glu Glu Ile Thr Pro Arg Arg Gln Ser Met Thr Val Lys Lys Gly
    690                 695                 700

Glu His Arg Gln Leu Leu Lys Asp Ser Phe Met Val Glu Leu Val Glu
705                 710                 715                 720

Gly Ala Arg Lys Leu Arg His Val Phe Leu Phe Thr Asp Leu Leu Leu
                725                 730                 735

Cys Thr Lys Leu Lys Lys Gln Ser Gly Gly Lys Thr Gln Gln Tyr Asp
            740                 745                 750

Cys Lys Trp Tyr Ile Pro Leu Thr Asp Leu Ser Phe Gln Met Val Asp
        755                 760                 765

Glu Leu Glu Ala Val Pro Asn Ile Pro Leu Val Pro Asp Glu Glu Leu
    770                 775                 780

Asp Ala Leu Lys Ile Lys Ile Ser Gln Ile Lys Ser Asp Ile Gln Arg
785                 790                 795                 800

Glu Lys Arg Ala Asn Lys Gly Ser Lys Ala Thr Glu Arg Leu Lys Lys
                805                 810                 815

Lys Leu Ser Glu Gln Glu Ser Leu Leu Leu Leu Met Ser Pro Ser Met
            820                 825                 830

Ala Phe Arg Val His Ser Arg Asn Gly Lys Ser Tyr Thr Phe Leu Ile
        835                 840                 845

Ser Ser Asp Tyr Glu Arg Ala Glu Trp Arg Glu Asn Ile Arg Glu Gln
    850                 855                 860

Gln Lys Lys Cys Phe Arg Ser Phe Ser Leu Thr Ser Val Glu Leu Gln
865                 870                 875                 880

Met Leu Thr Asn Ser Cys Val Lys Leu Gln Thr Val His Ser Ile Pro
                885                 890                 895

Leu Thr Ile Asn Lys Glu Glu Ala Leu Gln Arg Pro Val Ala Ser Asp
            900                 905                 910

Phe Glu Pro Gln Gly Leu Ser Glu Ala Ala Arg Trp Asn Ser Lys Glu
        915                 920                 925

Asn Leu Leu Ala Gly Pro Ser Glu Asn Asp Pro Asn Leu Phe Val Ala
    930                 935                 940

Leu Tyr Asp Phe Val Ala Ser Gly Asp Asn Thr Leu Ser Ile Thr Lys
945                 950                 955                 960

Gly Glu Lys Leu Arg Val Leu Gly Tyr Asn His Asn Gly Glu Trp Cys
                965                 970                 975

Glu Ala Gln Thr Lys Asn Gly Gln Gly Trp Val Pro Ser Asn Tyr Ile
            980                 985                 990

Thr Pro Val Asn Ser Leu Glu Lys  His Ser Trp Tyr His  Gly Pro Val
        995                 1000                1005

Ser Arg  Asn Ala Ala Glu Tyr  Pro Leu Ser Ser Gly  Ile Asn Gly
    1010                1015                1020

Ser Phe Leu Val Arg Glu Ser Glu Ser Ser Pro Ser Gln Arg Ser
    1025                1030                1035

Ile Ser Leu Arg Tyr Glu Gly Arg Val Tyr His Tyr Arg Ile Asn
    1040                1045                1050

Thr Ala Ser Asp Gly Lys Leu Tyr Val Ser Ser Glu Ser Arg Phe
    1055                1060                1065

Asn Thr Leu Ala Glu Leu Val His His His Ser Thr Val Ala Asp
    1070                1075                1080

Gly Leu Ile Thr Thr Leu His Tyr Pro Ala Pro Lys Arg Asn Lys
    1085                1090                1095

Pro Thr Val Tyr Gly Val Ser Pro Asn Tyr Asp Lys Trp Glu Met
    1100                1105                1110

Glu Arg Thr Asp Ile Thr Met Lys His Lys Leu Gly Gly Gly Gln
    1115                1120                1125

Tyr Gly Glu Val Tyr Glu Gly Val Trp Lys Lys Tyr Ser Leu Thr
    1130                1135                1140

Val Ala Val Lys Thr Leu Lys Glu Asp Thr Met Glu Val Glu Glu
    1145                1150                1155

Phe Leu Lys Glu Ala Ala Val Met Lys Glu Ile Lys His Pro Asn
    1160                1165                1170

Leu Val Gln Leu Leu Gly Val Cys Thr Arg Glu Pro Pro Phe Tyr
    1175                1180                1185

Ile Ile Thr Glu Phe Met Thr Tyr Gly Asn Leu Leu Asp Tyr Leu
    1190                1195                1200

Arg Glu Cys Asn Arg Gln Glu Val Asn Ala Val Val Leu Leu Tyr
    1205                1210                1215

Met Ala Thr Gln Ile Ser Ser Ala Met Glu Tyr Leu Glu Lys Lys
    1220                1225                1230

Asn Phe Ile His Arg Asp Leu Ala Ala Arg Asn Cys Leu Val Gly
    1235                1240                1245

Glu Asn His Leu Val Lys Val Ala Asp Phe Gly Leu Ser Arg Leu
    1250                1255                1260

Met Thr Gly Asp Thr Tyr Thr Ala His Ala Gly Ala Lys Phe Pro

1265                1270                1275

Ile Lys Trp Thr Ala Pro Glu Ser Leu Ala Tyr Asn Lys Phe Ser
    1280                1285                1290

Ile Lys Ser Asp Val Trp Ala Phe Gly Val Leu Leu Trp Glu Ile
    1295                1300                1305

Ala Thr Tyr Gly Met Ser Pro Tyr Pro Gly Ile Asp Arg Ser Gln
    1310                1315                1320

Val Tyr Glu Leu Leu Glu Lys Asp Tyr Arg Met Lys Arg Pro Glu
    1325                1330                1335

Gly Cys Pro Glu Lys Val Tyr Glu Leu Met Arg Ala Cys Trp Gln
    1340                1345                1350

Trp Asn Pro Ser Asp Arg Pro Ser Phe Ala Glu Ile His Gln Ala
    1355                1360                1365

Phe Glu Thr Met Phe Gln Glu Ser Ser Ile Ser Asp Glu Val Glu
    1370                1375                1380

Lys Glu Leu Gly Lys Gln Gly Val Arg Gly Ala Val Thr Thr Leu
    1385                1390                1395

Leu Gln Ala Pro Glu Leu Pro Thr Lys Thr Arg Thr Ser Arg Arg
    1400                1405                1410

Ala Ala Glu His Arg Asp Thr Thr Asp Val Pro Glu Met Pro His
    1415                1420                1425

Ser Lys Gly Gln Gly Glu Ser Asp Pro Leu Asp His Glu Pro Ala
    1430                1435                1440

Val Ser Pro Leu Leu Pro Arg Lys Glu Arg Gly Pro Pro Glu Gly
    1445                1450                1455

Gly Leu Asn Glu Asp Glu Arg Leu Leu Pro Lys Asp Lys Lys Thr
    1460                1465                1470

Asn Leu Phe Ser Ala Leu Ile Lys Lys Lys Lys Lys Thr Ala Pro
    1475                1480                1485

Thr Pro Pro Lys Arg Ser Ser Ser Phe Arg Glu Met Asp Gly Gln
    1490                1495                1500

Pro Glu Arg Arg Gly Ala Gly Glu Glu Glu Gly Arg Asp Ile Ser
    1505                1510                1515

Asn Gly Ala Leu Ala Phe Thr Pro Leu Asp Thr Ala Asp Pro Ala
    1520                1525                1530

Lys Ser Pro Lys Pro Ser Asn Gly Ala Gly Val Pro Asn Gly Ala
    1535                1540                1545

Leu Arg Glu Ser Gly Gly Ser Gly Phe Arg Ser Pro His Leu Trp
    1550                1555                1560

Lys Lys Ser Ser Thr Leu Thr Ser Ser Arg Leu Ala Thr Gly Glu
    1565                1570                1575

Glu Glu Gly Gly Gly Ser Ser Ser Lys Arg Phe Leu Arg Ser Cys
    1580                1585                1590

Ser Val Ser Cys Val Pro His Gly Ala Lys Asp Thr Glu Trp Arg
    1595                1600                1605

Ser Val Thr Leu Pro Arg Asp Leu Gln Ser Thr Gly Arg Gln Phe
    1610                1615                1620

Asp Ser Ser Thr Phe Gly Gly His Lys Ser Glu Lys Pro Ala Leu
    1625                1630                1635

Pro Arg Lys Arg Ala Gly Glu Asn Arg Ser Asp Gln Val Thr Arg
    1640                1645                1650

Gly Thr Val Thr Pro Pro Pro Arg Leu Val Lys Lys Asn Glu Glu
    1655                1660                1665

Ala Ala Asp Glu Val Phe Lys Asp Ile Met Glu Ser Ser Pro Gly
    1670                1675                1680

Ser Ser Pro Pro Asn Leu Thr Pro Lys Pro Leu Arg Arg Gln Val
    1685                1690                1695

Thr Val Ala Pro Ala Ser Gly Leu Pro His Lys Glu Glu Ala Trp
    1700                1705                1710

Lys Gly Ser Ala Leu Gly Thr Pro Ala Ala Ala Glu Pro Val Thr
    1715                1720                1725

Pro Thr Ser Lys Ala Gly Ser Gly Ala Pro Arg Gly Thr Ser Lys
    1730                1735                1740

Gly Pro Ala Glu Glu Ser Arg Val Arg Arg His Lys His Ser Ser
    1745                1750                1755

Glu Ser Pro Gly Arg Asp Lys Gly Lys Leu Ser Lys Leu Lys Pro
    1760                1765                1770

Ala Pro Pro Pro Pro Pro Ala Ala Ser Ala Gly Lys Ala Gly Gly
    1775                1780                1785

Lys Pro Ser Gln Arg Pro Gly Gln Glu Ala Ala Gly Glu Ala Val
    1790                1795                1800

Leu Gly Ala Lys Thr Lys Ala Thr Ser Leu Val Asp Ala Val Asn
    1805                1810                1815

Ser Asp Ala Ala Lys Pro Ser Gln Pro Ala Glu Gly Leu Lys Lys
    1820                1825                1830

Pro Val Leu Pro Ala Thr Pro Lys Pro His Pro Ala Lys Pro Ser
    1835                1840                1845

Gly Thr Pro Ile Ser Pro Ala Pro Val Pro Leu Ser Thr Leu Pro
    1850                1855                1860

Ser Ala Ser Ser Ala Leu Ala Gly Asp Gln Pro Ser Ser Thr Ala
    1865                1870                1875

Phe Ile Pro Leu Ile Ser Thr Arg Val Ser Leu Arg Lys Thr Arg
    1880                1885                1890

Gln Pro Pro Glu Arg Ala Ser Gly Ala Ile Thr Lys Gly Val Val
    1895                1900                1905

Leu Asp Ser Thr Glu Ala Leu Cys Leu Ala Ile Ser Gly Asn Ser
    1910                1915                1920

Glu Gln Met Ala Ser His Ser Ala Val Leu Glu Ala Gly Lys Asn
    1925                1930                1935

Leu Tyr Thr Phe Cys Val Ser Tyr Val Asp Ser Ile Gln Gln Met
    1940                1945                1950

Arg Asn Lys Phe Ala Phe Arg Glu Ala Ile Asn Lys Leu Glu Asn
    1955                1960                1965

Asn Leu Arg Glu Leu Gln Ile Cys Pro Ala Ser Ala Gly Ser Gly
    1970                1975                1980

Pro Ala Ala Thr Gln Asp Phe Ser Lys Leu Leu Ser Ser Val Lys
    1985                1990                1995

Glu Ile Ser Asp Ile Val Gln Arg
    2000                2005

<210> 5
<211> 1530
<212> PRT
<213> Homo sapiens

<400> 5

Met Val Asp Pro Val Gly Phe Ala Glu Ala Trp Lys Ala Gln Phe Pro
1               5                   10                  15

Asp Ser Glu Pro Pro Arg Met Glu Leu Arg Ser Val Gly Asp Ile Glu
            20                  25                  30

Gln Glu Leu Glu Arg Cys Lys Ala Ser Ile Arg Arg Leu Glu Gln Glu
        35                  40                  45

Val Asn Gln Glu Arg Phe Arg Met Ile Tyr Leu Gln Thr Leu Leu Ala
    50                  55                  60

Lys Glu Lys Lys Ser Tyr Asp Arg Gln Arg Trp Gly Phe Arg Arg Ala
65                  70                  75                  80

Ala Gln Ala Pro Asp Gly Ala Ser Glu Pro Arg Ala Ser Ala Ser Arg
                85                  90                  95

Pro Gln Pro Ala Pro Ala Asp Gly Ala Asp Pro Pro Pro Ala Glu Glu
            100                 105                 110

Pro Glu Ala Arg Pro Asp Gly Glu Gly Ser Pro Gly Lys Ala Arg Pro
        115                 120                 125

Gly Thr Ala Arg Arg Pro Gly Ala Ala Ala Ser Gly Glu Arg Asp Asp
    130                 135                 140

Arg Gly Pro Pro Ala Ser Val Ala Ala Leu Arg Ser Asn Phe Glu Arg
145                 150                 155                 160

Ile Arg Lys Gly His Gly Gln Pro Gly Ala Asp Ala Glu Lys Pro Phe
                165                 170                 175

Tyr Val Asn Val Glu Phe His His Glu Arg Gly Leu Val Lys Val Asn
            180                 185                 190

Asp Lys Glu Val Ser Asp Arg Ile Ser Ser Leu Gly Ser Gln Ala Met
        195                 200                 205

Gln Met Glu Arg Lys Lys Ser Gln His Gly Ala Gly Ser Ser Val Gly
    210                 215                 220

Asp Ala Ser Arg Pro Pro Tyr Arg Gly Arg Ser Ser Glu Ser Ser Cys
225                 230                 235                 240

Gly Val Asp Gly Asp Tyr Glu Asp Ala Glu Leu Asn Pro Arg Phe Leu
                245                 250                 255

Lys Asp Asn Leu Ile Asp Ala Asn Gly Gly Ser Arg Pro Pro Trp Pro
            260                 265                 270

Pro Leu Glu Tyr Gln Pro Tyr Gln Ser Ile Tyr Val Gly Gly Ile Met
        275                 280                 285

Glu Gly Glu Gly Lys Gly Pro Leu Leu Arg Ser Gln Ser Thr Ser Glu
    290                 295                 300

Gln Glu Lys Arg Leu Thr Trp Pro Arg Arg Ser Tyr Ser Pro Arg Ser
305                 310                 315                 320

Phe Glu Asp Cys Gly Gly Gly Tyr Thr Pro Asp Cys Ser Ser Asn Glu
                325                 330                 335

Asn Leu Thr Ser Ser Glu Glu Asp Phe Ser Ser Gly Gln Ser Ser Arg
            340                 345                 350

Val Ser Pro Ser Pro Thr Thr Tyr Arg Met Phe Arg Asp Lys Ser Arg
        355                 360                 365

Ser Pro Ser Gln Asn Ser Gln Gln Ser Phe Asp Ser Ser Ser Pro Pro
    370                 375                 380

Thr Pro Gln Cys His Lys Arg His Arg His Cys Pro Val Val Val Ser
385                 390                 395                 400

Glu Ala Thr Ile Val Gly Val Arg Lys Thr Gly Gln Ile Trp Pro Asn
                405                 410                 415

Asp Asp Glu Gly Ala Phe His Gly Asp Ala Glu Ala Leu Gln Arg Pro
            420                 425                 430

Val Ala Ser Asp Phe Glu Pro Gln Gly Leu Ser Glu Ala Ala Arg Trp
        435                 440                 445

Asn Ser Lys Glu Asn Leu Leu Ala Gly Pro Ser Glu Asn Asp Pro Asn
    450                 455                 460

Leu Phe Val Ala Leu Tyr Asp Phe Val Ala Ser Gly Asp Asn Thr Leu
465                 470                 475                 480

Ser Ile Thr Lys Gly Glu Lys Leu Arg Val Leu Gly Tyr Asn His Asn
                485                 490                 495

Gly Glu Trp Cys Glu Ala Gln Thr Lys Asn Gly Gln Gly Trp Val Pro
            500                 505                 510

Ser Asn Tyr Ile Thr Pro Val Asn Ser Leu Glu Lys His Ser Trp Tyr
        515                 520                 525

His Gly Pro Val Ser Arg Asn Ala Ala Glu Tyr Pro Leu Ser Ser Gly
    530                 535                 540

Ile Asn Gly Ser Phe Leu Val Arg Glu Ser Glu Ser Ser Pro Ser Gln
545                 550                 555                 560

Arg Ser Ile Ser Leu Arg Tyr Glu Gly Arg Val Tyr His Tyr Arg Ile
                565                 570                 575

Asn Thr Ala Ser Asp Gly Lys Leu Tyr Val Ser Ser Glu Ser Arg Phe
            580                 585                 590

Asn Thr Leu Ala Glu Leu Val His His His Ser Thr Val Ala Asp Gly
        595                 600                 605

Leu Ile Thr Thr Leu His Tyr Pro Ala Pro Lys Arg Asn Lys Pro Thr
    610                 615                 620

Val Tyr Gly Val Ser Pro Asn Tyr Asp Lys Trp Glu Met Glu Arg Thr
625                 630                 635                 640

Asp Ile Thr Met Lys His Lys Leu Gly Gly Gly Gln Tyr Gly Glu Val
                645                 650                 655

Tyr Glu Gly Val Trp Lys Lys Tyr Ser Leu Thr Val Ala Val Lys Thr
            660                 665                 670

Leu Lys Glu Asp Thr Met Glu Val Glu Glu Phe Leu Lys Glu Ala Ala
        675                 680                 685

Val Met Lys Glu Ile Lys His Pro Asn Leu Val Gln Leu Leu Gly Val
    690                 695                 700

Cys Thr Arg Glu Pro Pro Phe Tyr Ile Ile Thr Glu Phe Met Thr Tyr
705                 710                 715                 720

Gly Asn Leu Leu Asp Tyr Leu Arg Glu Cys Asn Arg Gln Glu Val Asn
                725                 730                 735

Ala Val Val Leu Leu Tyr Met Ala Thr Gln Ile Ser Ser Ala Met Glu
            740                 745                 750

Tyr Leu Glu Lys Lys Asn Phe Ile His Arg Asp Leu Ala Ala Arg Asn
        755                 760                 765

Cys Leu Val Gly Glu Asn His Leu Val Lys Val Ala Asp Phe Gly Leu
    770                 775                 780

Ser Arg Leu Met Thr Gly Asp Thr Tyr Thr Ala His Ala Gly Ala Lys
785                 790                 795                 800

Phe Pro Ile Lys Trp Thr Ala Pro Glu Ser Leu Ala Tyr Asn Lys Phe
                805                 810                 815

Ser Ile Lys Ser Asp Val Trp Ala Phe Gly Val Leu Leu Trp Glu Ile
            820                 825                 830

Ala Thr Tyr Gly Met Ser Pro Tyr Pro Gly Ile Asp Arg Ser Gln Val
        835                 840                 845

Tyr Glu Leu Leu Glu Lys Asp Tyr Arg Met Lys Arg Pro Glu Gly Cys
    850                 855                 860

Pro Glu Lys Val Tyr Glu Leu Met Arg Ala Cys Trp Gln Trp Asn Pro
865                 870                 875                 880

Ser Asp Arg Pro Ser Phe Ala Glu Ile His Gln Ala Phe Glu Thr Met
                885                 890                 895

Phe Gln Glu Ser Ser Ile Ser Asp Glu Val Glu Lys Glu Leu Gly Lys
            900                 905                 910

Gln Gly Val Arg Gly Ala Val Thr Thr Leu Leu Gln Ala Pro Glu Leu
        915                 920                 925

Pro Thr Lys Thr Arg Thr Ser Arg Arg Ala Ala Glu His Arg Asp Thr
    930                 935                 940

Thr Asp Val Pro Glu Met Pro His Ser Lys Gly Gln Gly Glu Ser Asp
945                 950                 955                 960

Pro Leu Asp His Glu Pro Ala Val Ser Pro Leu Leu Pro Arg Lys Glu
                965                 970                 975

Arg Gly Pro Pro Glu Gly Gly Leu Asn Glu Asp Glu Arg Leu Leu Pro
            980                 985                 990

Lys Asp Lys Lys Thr Asn Leu Phe Ser Ala Leu Ile Lys Lys Lys Lys

        995                 1000                1005

Lys Thr Ala Pro Thr Pro Pro Lys Arg Ser Ser Ser Phe Arg Glu
    1010                1015                1020

Met Asp Gly Gln Pro Glu Arg Arg Gly Ala Gly Glu Glu Glu Gly
    1025                1030                1035

Arg Asp Ile Ser Asn Gly Ala Leu Ala Phe Thr Pro Leu Asp Thr
    1040                1045                1050

Ala Asp Pro Ala Lys Ser Pro Lys Pro Ser Asn Gly Ala Gly Val
    1055                1060                1065

Pro Asn Gly Ala Leu Arg Glu Ser Gly Gly Ser Gly Phe Arg Ser
    1070                1075                1080

Pro His Leu Trp Lys Lys Ser Ser Thr Leu Thr Ser Ser Arg Leu
    1085                1090                1095

Ala Thr Gly Glu Glu Glu Gly Gly Gly Ser Ser Ser Lys Arg Phe
    1100                1105                1110

Leu Arg Ser Cys Ser Val Ser Cys Val Pro His Gly Ala Lys Asp
    1115                1120                1125

Thr Glu Trp Arg Ser Val Thr Leu Pro Arg Asp Leu Gln Ser Thr
    1130                1135                1140

Gly Arg Gln Phe Asp Ser Ser Thr Phe Gly Gly His Lys Ser Glu
    1145                1150                1155

Lys Pro Ala Leu Pro Arg Lys Arg Ala Gly Glu Asn Arg Ser Asp
    1160                1165                1170

Gln Val Thr Arg Gly Thr Val Thr Pro Pro Pro Arg Leu Val Lys
    1175                1180                1185

Lys Asn Glu Glu Ala Ala Asp Glu Val Phe Lys Asp Ile Met Glu
    1190                1195                1200

Ser Ser Pro Gly Ser Ser Pro Pro Asn Leu Thr Pro Lys Pro Leu
    1205                1210                1215

Arg Arg Gln Val Thr Val Ala Pro Ala Ser Gly Leu Pro His Lys
    1220                1225                1230

Glu Glu Ala Trp Lys Gly Ser Ala Leu Gly Thr Pro Ala Ala Ala
    1235                1240                1245

Glu Pro Val Thr Pro Thr Ser Lys Ala Gly Ser Gly Ala Pro Arg
    1250                1255                1260

Gly Thr Ser Lys Gly Pro Ala Glu Glu Ser Arg Val Arg Arg His
    1265                1270                1275

Lys His Ser Ser Glu Ser Pro Gly Arg Asp Lys Gly Lys Leu Ser
    1280                1285                1290

Lys Leu Lys Pro Ala Pro Pro Pro Pro Pro Ala Ala Ser Ala Gly
    1295                1300                1305

Lys Ala Gly Gly Lys Pro Ser Gln Arg Pro Gly Gln Glu Ala Ala
    1310                1315                1320

Gly Glu Ala Val Leu Gly Ala Lys Thr Lys Ala Thr Ser Leu Val
    1325                1330                1335

Asp Ala Val Asn Ser Asp Ala Ala Lys Pro Ser Gln Pro Ala Glu
    1340                1345                1350

Gly Leu Lys Lys Pro Val Leu Pro Ala Thr Pro Lys Pro His Pro
    1355                1360                1365

Ala Lys Pro Ser Gly Thr Pro Ile Ser Pro Ala Pro Val Pro Leu
    1370                1375                1380

Ser Thr Leu Pro Ser Ala Ser Ser Ala Leu Ala Gly Asp Gln Pro
    1385                1390                1395

Ser Ser Thr Ala Phe Ile Pro Leu Ile Ser Thr Arg Val Ser Leu
    1400                1405                1410

Arg Lys Thr Arg Gln Pro Pro Glu Arg Ala Ser Gly Ala Ile Thr
    1415                1420                1425

Lys Gly Val Val Leu Asp Ser Thr Glu Ala Leu Cys Leu Ala Ile
    1430                1435                1440

Ser Gly Asn Ser Glu Gln Met Ala Ser His Ser Ala Val Leu Glu
    1445                1450                1455

Ala Gly Lys Asn Leu Tyr Thr Phe Cys Val Ser Tyr Val Asp Ser
    1460                1465                1470

Ile Gln Gln Met Arg Asn Lys Phe Ala Phe Arg Glu Ala Ile Asn
    1475                1480                1485

Lys Leu Glu Asn Asn Leu Arg Glu Leu Gln Ile Cys Pro Ala Ser
    1490                1495                1500

Ala Gly Ser Gly Pro Ala Ala Thr Gln Asp Phe Ser Lys Leu Leu
    1505                1510                1515

Ser Ser Val Lys Glu Ile Ser Asp Ile Val Gln Arg
    1520                1525                1530

<210> 6
<211> 2031
<212> PRT
<213> Homo sapiens

<400> 6

Met Val Asp Pro Val Gly Phe Ala Glu Ala Trp Lys Ala Gln Phe Pro
1               5                   10                  15

Asp Ser Glu Pro Pro Arg Met Glu Leu Arg Ser Val Gly Asp Ile Glu
            20                  25                  30

Gln Glu Leu Glu Arg Cys Lys Ala Ser Ile Arg Arg Leu Glu Gln Glu
        35                  40                  45

Val Asn Gln Glu Arg Phe Arg Met Ile Tyr Leu Gln Thr Leu Leu Ala
    50                  55                  60

Lys Glu Lys Lys Ser Tyr Asp Arg Gln Arg Trp Gly Phe Arg Arg Ala
65                  70                  75                  80

Ala Gln Ala Pro Asp Gly Ala Ser Glu Pro Arg Ala Ser Ala Ser Arg
                85                  90                  95

Pro Gln Pro Ala Pro Ala Asp Gly Ala Asp Pro Pro Pro Ala Glu Glu
            100                 105                 110

Pro Glu Ala Arg Pro Asp Gly Glu Gly Ser Pro Gly Lys Ala Arg Pro
        115                 120                 125

Gly Thr Ala Arg Arg Pro Gly Ala Ala Ala Ser Gly Glu Arg Asp Asp
    130                 135                 140

Arg Gly Pro Pro Ala Ser Val Ala Ala Leu Arg Ser Asn Phe Glu Arg
145                 150                 155                 160

Ile Arg Lys Gly His Gly Gln Pro Gly Ala Asp Ala Glu Lys Pro Phe
                165                 170                 175

Tyr Val Asn Val Glu Phe His His Glu Arg Gly Leu Val Lys Val Asn

180                 185                 190

Asp Lys Glu Val Ser Asp Arg Ile Ser Ser Leu Gly Ser Gln Ala Met
        195                 200                 205

Gln Met Glu Arg Lys Lys Ser Gln His Gly Ala Gly Ser Ser Val Gly
    210                 215                 220

Asp Ala Ser Arg Pro Pro Tyr Arg Gly Arg Ser Ser Glu Ser Ser Cys
225                 230                 235                 240

Gly Val Asp Gly Asp Tyr Glu Asp Ala Glu Leu Asn Pro Arg Phe Leu
                245                 250                 255

Lys Asp Asn Leu Ile Asp Ala Asn Gly Gly Ser Arg Pro Pro Trp Pro
            260                 265                 270

Pro Leu Glu Tyr Gln Pro Tyr Gln Ser Ile Tyr Val Gly Gly Ile Met
        275                 280                 285

Glu Gly Glu Gly Lys Gly Pro Leu Leu Arg Ser Gln Ser Thr Ser Glu
    290                 295                 300

Gln Glu Lys Arg Leu Thr Trp Pro Arg Arg Ser Tyr Ser Pro Arg Ser
305                 310                 315                 320

Phe Glu Asp Cys Gly Gly Gly Tyr Thr Pro Asp Cys Ser Ser Asn Glu
                325                 330                 335

Asn Leu Thr Ser Ser Glu Glu Asp Phe Ser Ser Gly Gln Ser Ser Arg
            340                 345                 350

Val Ser Pro Ser Pro Thr Thr Tyr Arg Met Phe Arg Asp Lys Ser Arg
        355                 360                 365

Ser Pro Ser Gln Asn Ser Gln Gln Ser Phe Asp Ser Ser Ser Pro Pro
    370                 375                 380

Thr Pro Gln Cys His Lys Arg His Arg His Cys Pro Val Val Val Ser
385                 390                 395                 400

Glu Ala Thr Ile Val Gly Val Arg Lys Thr Gly Gln Ile Trp Pro Asn
                405                 410                 415

Asp Asp Glu Gly Ala Phe His Gly Asp Ala Asp Gly Ser Phe Gly Thr
            420                 425                 430

Pro Pro Gly Tyr Gly Cys Ala Ala Asp Arg Ala Glu Glu Gln Arg Arg
        435                 440                 445

His Gln Asp Gly Leu Pro Tyr Ile Asp Asp Ser Pro Ser Ser Ser Pro
    450                 455                 460

His Leu Ser Ser Lys Gly Arg Gly Ser Arg Asp Ala Leu Val Ser Gly
465                 470                 475                 480

Ala Leu Lys Ser Thr Lys Ala Ser Glu Leu Asp Leu Glu Lys Gly Leu
                485                 490                 495

Glu Met Arg Lys Trp Val Leu Ser Gly Ile Leu Ala Ser Glu Glu Thr
            500                 505                 510

Tyr Leu Ser His Leu Glu Ala Leu Leu Leu Pro Met Lys Pro Leu Lys
        515                 520                 525

Ala Ala Ala Thr Thr Ser Gln Pro Val Leu Thr Ser Gln Gln Ile Glu
    530                 535                 540

Thr Ile Phe Phe Lys Val Pro Glu Leu Tyr Glu Ile His Lys Glu Ser
545                 550                 555                 560

Tyr Asp Gly Leu Phe Pro Arg Val Gln Gln Trp Ser His Gln Gln Arg
                565                 570                 575

Val Gly Asp Leu Phe Gln Lys Leu Ala Ser Gln Leu Gly Val Tyr Arg
            580                 585                 590

Ala Phe Val Asp Asn Tyr Gly Val Ala Met Glu Met Ala Glu Lys Cys
        595                 600                 605

Cys Gln Ala Asn Ala Gln Phe Ala Glu Ile Ser Glu Asn Leu Arg Ala
    610                 615                 620

Arg Ser Asn Lys Asp Ala Lys Asp Pro Thr Thr Lys Asn Ser Leu Glu
625                 630                 635                 640

Thr Leu Leu Tyr Lys Pro Val Asp Arg Val Thr Arg Ser Thr Leu Val
                645                 650                 655

Leu His Asp Leu Leu Lys His Thr Pro Ala Ser His Pro Asp His Pro
            660                 665                 670

Leu Leu Gln Asp Ala Leu Arg Ile Ser Gln Asn Phe Leu Ser Ser Ile
        675                 680                 685

Asn Glu Glu Ile Thr Pro Arg Arg Gln Ser Met Thr Val Lys Lys Gly
    690                 695                 700

Glu His Arg Gln Leu Leu Lys Asp Ser Phe Met Val Glu Leu Val Glu
705                 710                 715                 720

Gly Ala Arg Lys Leu Arg His Val Phe Leu Phe Thr Asp Leu Leu Leu
                725                 730                 735

Cys Thr Lys Leu Lys Lys Gln Ser Gly Gly Lys Thr Gln Gln Tyr Asp
            740                 745                 750

Cys Lys Trp Tyr Ile Pro Leu Thr Asp Leu Ser Phe Gln Met Val Asp
        755                 760                 765

Glu Leu Glu Ala Val Pro Asn Ile Pro Leu Val Pro Asp Glu Glu Leu
    770                 775                 780

Asp Ala Leu Lys Ile Lys Ile Ser Gln Ile Lys Ser Asp Ile Gln Arg
785                 790                 795                 800

Glu Lys Arg Ala Asn Lys Gly Ser Lys Ala Thr Glu Arg Leu Lys Lys
                805                 810                 815

Lys Leu Ser Glu Gln Glu Ser Leu Leu Leu Leu Met Ser Pro Ser Met
            820                 825                 830

Ala Phe Arg Val His Ser Arg Asn Gly Lys Ser Tyr Thr Phe Leu Ile
        835                 840                 845

Ser Ser Asp Tyr Glu Arg Ala Glu Trp Arg Glu Asn Ile Arg Glu Gln
    850                 855                 860

Gln Lys Lys Cys Phe Arg Ser Phe Ser Leu Thr Ser Val Glu Leu Gln
865                 870                 875                 880

Met Leu Thr Asn Ser Cys Val Lys Leu Gln Thr Val His Ser Ile Pro
                885                 890                 895

Leu Thr Ile Asn Lys Glu Asp Asp Glu Ser Pro Gly Leu Tyr Gly Phe
            900                 905                 910

Leu Asn Val Ile Val His Ser Ala Thr Gly Phe Lys Gln Ser Ser Lys
        915                 920                 925

Ala Leu Gln Arg Pro Val Ala Ser Asp Phe Glu Pro Gln Gly Leu Ser
    930                 935                 940

Glu Ala Ala Arg Trp Asn Ser Lys Glu Asn Leu Leu Ala Gly Pro Ser
945                 950                 955                 960

Glu Asn Asp Pro Asn Leu Phe Val Ala Leu Tyr Asp Phe Val Ala Ser
                965                 970                 975

Gly Asp Asn Thr Leu Ser Ile Thr Lys Gly Glu Lys Leu Arg Val Leu
            980                 985                 990

Gly Tyr Asn His Asn Gly Glu Trp  Cys Glu Ala Gln Thr  Lys Asn Gly
        995                 1000                 1005

Gln Gly  Trp Val Pro Ser Asn  Tyr Ile Thr Pro Val  Asn Ser Leu
    1010                 1015                 1020

Glu Lys  His Ser Trp Tyr His  Gly Pro Val Ser Arg  Asn Ala Ala
    1025                 1030                 1035

Glu Tyr  Pro Leu Ser Ser Gly  Ile Asn Gly Ser Phe  Leu Val Arg
    1040                 1045                 1050

Glu Ser  Glu Ser Ser Pro Ser  Gln Arg Ser Ile Ser  Leu Arg Tyr
    1055                 1060                 1065

Glu Gly  Arg Val Tyr His Tyr  Arg Ile Asn Thr Ala  Ser Asp Gly
    1070                 1075                 1080

Lys Leu  Tyr Val Ser Ser Glu  Ser Arg Phe Asn Thr  Leu Ala Glu
    1085                 1090                 1095

Leu Val  His His His Ser Thr  Val Ala Asp Gly Leu  Ile Thr Thr
    1100                 1105                 1110

Leu His  Tyr Pro Ala Pro Lys  Arg Asn Lys Pro Thr  Val Tyr Gly
    1115                 1120                 1125

Val Ser  Pro Asn Tyr Asp Lys  Trp Glu Met Glu Arg  Thr Asp Ile
    1130                 1135                 1140

Thr Met  Lys His Lys Leu Gly  Gly Gly Gln Tyr Gly  Glu Val Tyr
    1145                 1150                 1155

Glu Gly  Val Trp Lys Lys Tyr  Ser Leu Thr Val Ala  Val Lys Thr
    1160                 1165                 1170

Leu Lys  Glu Asp Thr Met Glu  Val Glu Glu Phe Leu  Lys Glu Ala
    1175                 1180                 1185

Ala Val  Met Lys Glu Ile Lys  His Pro Asn Leu Val  Gln Leu Leu
    1190                 1195                 1200

Gly Val  Cys Thr Arg Glu Pro  Pro Phe Tyr Ile Ile  Ile Glu Phe

1205                1210                1215

Met Thr Tyr Gly Asn Leu Leu Asp Tyr Leu Arg Glu Cys Asn Arg
    1220                1225                1230

Gln Glu Val Asn Ala Val Val Leu Leu Tyr Met Ala Thr Gln Ile
    1235                1240                1245

Ser Ser Ala Met Glu Tyr Leu Glu Lys Lys Asn Phe Ile His Arg
    1250                1255                1260

Asp Leu Ala Ala Arg Asn Cys Leu Val Gly Glu Asn His Leu Val
    1265                1270                1275

Lys Val Ala Asp Phe Gly Leu Ser Arg Leu Met Thr Gly Asp Thr
    1280                1285                1290

Tyr Thr Ala His Ala Gly Ala Lys Phe Pro Ile Lys Trp Thr Ala
    1295                1300                1305

Pro Glu Ser Leu Ala Tyr Asn Lys Phe Ser Ile Lys Ser Asp Val
    1310                1315                1320

Trp Ala Phe Gly Val Leu Leu Trp Glu Ile Ala Thr Tyr Gly Met
    1325                1330                1335

Ser Pro Tyr Pro Gly Ile Asp Arg Ser Gln Val Tyr Glu Leu Leu
    1340                1345                1350

Glu Lys Asp Tyr Arg Met Lys Arg Pro Glu Gly Cys Pro Glu Lys
    1355                1360                1365

Val Tyr Glu Leu Met Arg Ala Cys Trp Gln Trp Asn Pro Ser Asp
    1370                1375                1380

Arg Pro Ser Phe Ala Glu Ile His Gln Ala Phe Glu Thr Met Phe
    1385                1390                1395

Gln Glu Ser Ser Ile Ser Asp Glu Val Glu Lys Glu Leu Gly Lys
    1400                1405                1410

Gln Gly Val Arg Gly Ala Val Thr Thr Leu Leu Gln Ala Pro Glu
    1415                1420                1425

Leu Pro Thr Lys Thr Arg Thr Ser Arg Arg Ala Ala Glu His Arg
    1430                1435                1440

Asp Thr Thr Asp Val Pro Glu Met Pro His Ser Lys Gly Gln Gly
    1445                1450                1455

Glu Ser Asp Pro Leu Asp His Glu Pro Ala Val Ser Pro Leu Leu
    1460                1465                1470

Pro Arg Lys Glu Arg Gly Pro Pro Glu Gly Gly Leu Asn Glu Asp
    1475                1480                1485

Glu Arg Leu Leu Pro Lys Asp Lys Lys Thr Asn Leu Phe Ser Ala
    1490                1495                1500

Leu Ile Lys Lys Lys Lys Lys Thr Ala Pro Thr Pro Pro Lys Arg
    1505                1510                1515

Ser Ser Ser Phe Arg Glu Met Asp Gly Gln Pro Glu Arg Arg Gly
    1520                1525                1530

Ala Gly Glu Glu Glu Gly Arg Asp Ile Ser Asn Gly Ala Leu Ala
    1535                1540                1545

Phe Thr Pro Leu Asp Thr Ala Asp Pro Ala Lys Ser Pro Lys Pro
    1550                1555                1560

Ser Asn Gly Ala Gly Val Pro Asn Gly Ala Leu Arg Glu Ser Gly
    1565                1570                1575

Gly Ser Gly Phe Arg Ser Pro His Leu Trp Lys Lys Ser Ser Thr
    1580                1585                1590

Leu Thr Ser Ser Arg Leu Ala Thr Gly Glu Glu Glu Gly Gly Gly
    1595                1600                1605

Ser Ser Ser Lys Arg Phe Leu Arg Ser Cys Ser Val Ser Cys Val
    1610                1615                1620

Pro His Gly Ala Lys Asp Thr Glu Trp Arg Ser Val Thr Leu Pro
    1625                1630                1635

Arg Asp Leu Gln Ser Thr Gly Arg Gln Phe Asp Ser Ser Thr Phe
    1640                1645                1650

Gly Gly His Lys Ser Glu Lys Pro Ala Leu Pro Arg Lys Arg Ala
    1655                1660                1665

Gly Glu Asn Arg Ser Asp Gln Val Thr Arg Gly Thr Val Thr Pro
    1670                1675                1680

Pro Pro Arg Leu Val Lys Lys Asn Glu Glu Ala Ala Asp Glu Val
    1685                1690                1695

Phe Lys Asp Ile Met Glu Ser Ser Pro Gly Ser Ser Pro Pro Asn
    1700                1705                1710

Leu Thr Pro Lys Pro Leu Arg Arg Gln Val Thr Val Ala Pro Ala
    1715                1720                1725

Ser Gly Leu Pro His Lys Glu Glu Ala Trp Lys Gly Ser Ala Leu
    1730                1735                1740

Gly Thr Pro Ala Ala Ala Glu Pro Val Thr Pro Thr Ser Lys Ala
    1745                1750                1755

Gly Ser Gly Ala Pro Arg Gly Thr Ser Lys Gly Pro Ala Glu Glu
    1760                1765                1770

Ser Arg Val Arg Arg His Lys His Ser Ser Glu Ser Pro Gly Arg
    1775                1780                1785

Asp Lys Gly Lys Leu Ser Lys Leu Lys Pro Ala Pro Pro Pro Pro
    1790                1795                1800

Pro Ala Ala Ser Ala Gly Lys Ala Gly Gly Lys Pro Ser Gln Arg
    1805                1810                1815

Pro Gly Gln Glu Ala Ala Gly Glu Ala Val Leu Gly Ala Lys Thr
    1820                1825                1830

Lys Ala Thr Ser Leu Val Asp Ala Val Asn Ser Asp Ala Ala Lys
    1835                1840                1845

Pro Ser Gln Pro Ala Glu Gly Leu Lys Lys Pro Val Leu Pro Ala
    1850                1855                1860

Thr Pro Lys Pro His Pro Ala Lys Pro Ser Gly Thr Pro Ile Ser
    1865                1870                1875

Pro Ala Pro Val Pro Leu Ser Thr Leu Pro Ser Ala Ser Ser Ala
    1880                1885                1890

Leu Ala Gly Asp Gln Pro Ser Ser Thr Ala Phe Ile Pro Leu Ile
    1895                1900                1905

Ser Thr Arg Val Ser Leu Arg Lys Thr Arg Gln Pro Pro Glu Arg
    1910                1915                1920

Ala Ser Gly Ala Ile Thr Lys Gly Val Val Leu Asp Ser Thr Glu
    1925                1930                1935

Ala Leu Cys Leu Ala Ile Ser Gly Asn Ser Glu Gln Met Ala Ser
    1940                1945                1950

His Ser Ala Val Leu Glu Ala Gly Lys Asn Leu Tyr Thr Phe Cys
    1955                1960                1965

Val Ser Tyr Val Asp Ser Ile Gln Gln Met Arg Asn Lys Phe Ala
    1970                1975                1980

Phe Arg Glu Ala Ile Asn Lys Leu Glu Asn Asn Leu Arg Glu Leu
    1985                1990                1995

Gln Ile Cys Pro Ala Ser Ala Gly Ser Gly Pro Ala Ala Thr Gln
    2000                2005                2010

Asp Phe Ser Lys Leu Leu Ser Ser Val Lys Glu Ile Ser Asp Ile
    2015                2020                2025

Val Gln Arg
    2030

<210>  7
<211>  2006
<212>  PRT
<213>  Homo sapiens

<400>  7

Met Val Asp Pro Val Gly Phe Ala Glu Ala Trp Lys Ala Gln Phe Pro
1               5                   10                  15

Asp Ser Glu Pro Pro Arg Met Glu Leu Arg Ser Val Gly Asp Ile Glu
            20                  25                  30

Gln Glu Leu Glu Arg Cys Lys Ala Ser Ile Arg Arg Leu Glu Gln Glu
        35                  40                  45

Val Asn Gln Glu Arg Phe Arg Met Ile Tyr Leu Gln Thr Leu Leu Ala
    50                  55                  60

Lys Glu Lys Lys Ser Tyr Asp Arg Gln Arg Trp Gly Phe Arg Arg Ala
65                  70                  75                  80

Ala Gln Ala Pro Asp Gly Ala Ser Glu Pro Arg Ala Ser Ala Ser Arg
                85                  90                  95

Pro Gln Pro Ala Pro Ala Asp Gly Ala Asp Pro Pro Pro Ala Glu Glu
            100                 105                 110

Pro Glu Ala Arg Pro Asp Gly Glu Gly Ser Pro Gly Lys Ala Arg Pro
        115                 120                 125

Gly Thr Ala Arg Arg Pro Gly Ala Ala Ala Ser Gly Glu Arg Asp Asp
    130                 135                 140

Arg Gly Pro Pro Ala Ser Val Ala Ala Leu Arg Ser Asn Phe Glu Arg
145                 150                 155                 160

Ile Arg Lys Gly His Gly Gln Pro Gly Ala Asp Ala Glu Lys Pro Phe
                165                 170                 175

Tyr Val Asn Val Glu Phe His His Glu Arg Gly Leu Val Lys Val Asn
            180                 185                 190

Asp Lys Glu Val Ser Asp Arg Ile Ser Ser Leu Gly Ser Gln Ala Met
        195                 200                 205

Gln Met Glu Arg Lys Lys Ser Gln His Gly Ala Gly Ser Ser Val Gly
    210                 215                 220

Asp Ala Ser Arg Pro Pro Tyr Arg Gly Arg Ser Ser Glu Ser Ser Cys
225                 230                 235                 240

Gly Val Asp Gly Asp Tyr Glu Asp Ala Glu Leu Asn Pro Arg Phe Leu
                245                 250                 255

Lys Asp Asn Leu Ile Asp Ala Asn Gly Gly Ser Arg Pro Pro Trp Pro
            260                 265                 270

Pro Leu Glu Tyr Gln Pro Tyr Gln Ser Ile Tyr Val Gly Gly Ile Met
        275                 280                 285

Glu Gly Glu Gly Lys Gly Pro Leu Leu Arg Ser Gln Ser Thr Ser Glu
    290                 295                 300

Gln Glu Lys Arg Leu Thr Trp Pro Arg Arg Ser Tyr Ser Pro Arg Ser
305                 310                 315                 320

Phe Glu Asp Cys Gly Gly Gly Tyr Thr Pro Asp Cys Ser Ser Asn Glu
                325                 330                 335

Asn Leu Thr Ser Ser Glu Glu Asp Phe Ser Ser Gly Gln Ser Ser Arg
            340                 345                 350

Val Ser Pro Ser Pro Thr Thr Tyr Arg Met Phe Arg Asp Lys Ser Arg
        355                 360                 365

Ser Pro Ser Gln Asn Ser Gln Gln Ser Phe Asp Ser Ser Ser Pro Pro
    370                 375                 380

Thr Pro Gln Cys His Lys Arg His Arg His Cys Pro Val Val Val Ser
385                 390                 395                 400

Glu Ala Thr Ile Val Gly Val Arg Lys Thr Gly Gln Ile Trp Pro Asn
                405                 410                 415

Asp Asp Glu Gly Ala Phe His Gly Asp Ala Asp Gly Ser Phe Gly Thr
            420                 425                 430

Pro Pro Gly Tyr Gly Cys Ala Ala Asp Arg Ala Glu Glu Gln Arg Arg
        435                 440                 445

His Gln Asp Gly Leu Pro Tyr Ile Asp Asp Ser Pro Ser Ser Ser Pro
    450                 455                 460

His Leu Ser Ser Lys Gly Arg Gly Ser Arg Asp Ala Leu Val Ser Gly
465                 470                 475                 480

Ala Leu Lys Ser Thr Lys Ala Ser Glu Leu Asp Leu Glu Lys Gly Leu
                485                 490                 495

Glu Met Arg Lys Trp Val Leu Ser Gly Ile Leu Ala Ser Glu Glu Thr
            500                 505                 510

Tyr Leu Ser His Leu Glu Ala Leu Leu Leu Pro Met Lys Pro Leu Lys
        515                 520                 525

Ala Ala Ala Thr Thr Ser Gln Pro Val Leu Thr Ser Gln Gln Ile Glu
    530                 535                 540

Thr Ile Phe Phe Lys Val Pro Glu Leu Tyr Glu Ile His Lys Glu Ser
545                 550                 555                 560

Tyr Asp Gly Leu Phe Pro Arg Val Gln Gln Trp Ser His Gln Gln Arg
                565                 570                 575

Val Gly Asp Leu Phe Gln Lys Leu Ala Ser Gln Leu Gly Val Tyr Arg
            580                 585                 590

Ala Phe Val Asp Asn Tyr Gly Val Ala Met Glu Met Ala Glu Lys Cys
        595                 600                 605

Cys Gln Ala Asn Ala Gln Phe Ala Glu Ile Ser Glu Asn Leu Arg Ala
    610                 615                 620

Arg Ser Asn Lys Asp Ala Lys Asp Pro Thr Thr Lys Asn Ser Leu Glu
625                 630                 635                 640

Thr Leu Leu Tyr Lys Pro Val Asp Arg Val Thr Arg Ser Thr Leu Val
                645                 650                 655

Leu His Asp Leu Leu Lys His Thr Pro Ala Ser His Pro Asp His Pro
            660                 665                 670

Leu Leu Gln Asp Ala Leu Arg Ile Ser Gln Asn Phe Leu Ser Ser Ile
        675                 680                 685

Asn Glu Glu Ile Thr Pro Arg Arg Gln Ser Met Thr Val Lys Lys Gly
    690                 695                 700

Glu His Arg Gln Leu Leu Lys Asp Ser Phe Met Val Glu Leu Val Glu
705                 710                 715                 720

Gly Ala Arg Lys Leu Arg His Val Phe Leu Phe Thr Asp Leu Leu Leu
                725                 730                 735

Cys Thr Lys Leu Lys Lys Gln Ser Gly Gly Lys Thr Gln Gln Tyr Asp
            740                 745                 750

Cys Lys Trp Tyr Ile Pro Leu Thr Asp Leu Ser Phe Gln Met Val Asp
        755                 760                 765

Glu Leu Glu Ala Val Pro Asn Ile Pro Leu Val Pro Asp Glu Glu Leu
    770                 775                 780

Asp Ala Leu Lys Ile Lys Ile Ser Gln Ile Lys Ser Asp Ile Gln Arg
785                 790                 795                 800

Glu Lys Arg Ala Asn Lys Gly Ser Lys Ala Thr Glu Arg Leu Lys Lys
                805                 810                 815

Lys Leu Ser Glu Gln Glu Ser Leu Leu Leu Leu Met Ser Pro Ser Met
            820                 825                 830

Ala Phe Arg Val His Ser Arg Asn Gly Lys Ser Tyr Thr Phe Leu Ile
        835                 840                 845

Ser Ser Asp Tyr Glu Arg Ala Glu Trp Arg Glu Asn Ile Arg Glu Gln
    850                 855                 860

Gln Lys Lys Cys Phe Arg Ser Phe Ser Leu Thr Ser Val Glu Leu Gln
865                 870                 875                 880

Met Leu Thr Asn Ser Cys Val Lys Leu Gln Thr Val His Ser Ile Pro
                885                 890                 895

Leu Thr Ile Asn Lys Glu Glu Ala Leu Gln Arg Pro Val Ala Ser Asp

900                 905                 910

Phe Glu Pro Gln Gly Leu Ser Glu Ala Ala Arg Trp Asn Ser Lys Glu
        915                 920                 925

Asn Leu Leu Ala Gly Pro Ser Glu Asn Asp Pro Asn Leu Phe Val Ala
    930                 935                 940

Leu Tyr Asp Phe Val Ala Ser Gly Asp Asn Thr Leu Ser Ile Thr Lys
945                 950                 955                 960

Gly Glu Lys Leu Arg Val Leu Gly Tyr Asn His Asn Gly Glu Trp Cys
                965                 970                 975

Glu Ala Gln Thr Lys Asn Gly Gln Gly Trp Val Pro Ser Asn Tyr Ile
            980                 985                 990

Thr Pro Val Asn Ser Leu Glu Lys  His Ser Trp Tyr His  Gly Pro Val
        995                 1000                1005

Ser Arg  Asn Ala Ala Glu Tyr  Pro Leu Ser Ser Gly  Ile Asn Gly
    1010                1015                1020

Ser Phe  Leu Val Arg Glu Ser  Glu Ser Ser Pro Ser  Gln Arg Ser
    1025                1030                1035

Ile Ser  Leu Arg Tyr Glu Gly  Arg Val Tyr His Tyr  Arg Ile Asn
    1040                1045                1050

Thr Ala  Ser Asp Gly Lys Leu  Tyr Val Ser Ser Glu  Ser Arg Phe
    1055                1060                1065

Asn Thr  Leu Ala Glu Leu Val  His His His Ser Thr  Val Ala Asp
    1070                1075                1080

Gly Leu  Ile Thr Thr Leu His  Tyr Pro Ala Pro Lys  Arg Asn Lys
    1085                1090                1095

Pro Thr  Val Tyr Gly Val Ser  Pro Asn Tyr Asp Lys  Trp Glu Met
    1100                1105                1110

Glu Arg  Thr Asp Ile Thr Met  Lys His Lys Leu Gly  Gly Gly Gln
    1115                1120                1125

Tyr Gly  Glu Val Tyr Glu Gly  Val Trp Lys Lys Tyr  Ser Leu Thr
    1130                1135                1140

Val Ala  Val Lys Thr Leu Lys  Glu Asp Thr Met Glu  Val Glu Glu
    1145                1150                1155

Phe Leu Lys Glu Ala Ala Val Met Lys Glu Ile Lys His Pro Asn
    1160                1165                1170

Leu Val Gln Leu Leu Gly Val Cys Thr Arg Glu Pro Pro Phe Tyr
    1175                1180                1185

Ile Ile Ile Glu Phe Met Thr Tyr Gly Asn Leu Leu Asp Tyr Leu
    1190                1195                1200

Arg Glu Cys Asn Arg Gln Glu Val Asn Ala Val Val Leu Leu Tyr
    1205                1210                1215

Met Ala Thr Gln Ile Ser Ser Ala Met Glu Tyr Leu Glu Lys Lys
    1220                1225                1230

Asn Phe Ile His Arg Asp Leu Ala Ala Arg Asn Cys Leu Val Gly
    1235                1240                1245

Glu Asn His Leu Val Lys Val Ala Asp Phe Gly Leu Ser Arg Leu
    1250                1255                1260

Met Thr Gly Asp Thr Tyr Thr Ala His Ala Gly Ala Lys Phe Pro
    1265                1270                1275

Ile Lys Trp Thr Ala Pro Glu Ser Leu Ala Tyr Asn Lys Phe Ser
    1280                1285                1290

Ile Lys Ser Asp Val Trp Ala Phe Gly Val Leu Leu Trp Glu Ile
    1295                1300                1305

Ala Thr Tyr Gly Met Ser Pro Tyr Pro Gly Ile Asp Arg Ser Gln
    1310                1315                1320

Val Tyr Glu Leu Leu Glu Lys Asp Tyr Arg Met Lys Arg Pro Glu
    1325                1330                1335

Gly Cys Pro Glu Lys Val Tyr Glu Leu Met Arg Ala Cys Trp Gln
    1340                1345                1350

Trp Asn Pro Ser Asp Arg Pro Ser Phe Ala Glu Ile His Gln Ala
    1355                1360                1365

Phe Glu Thr Met Phe Gln Glu Ser Ser Ile Ser Asp Glu Val Glu
    1370                1375                1380

Lys Glu Leu Gly Lys Gln Gly Val Arg Gly Ala Val Thr Thr Leu
    1385                1390                1395

Leu Gln Ala Pro Glu Leu Pro Thr Lys Thr Arg Thr Ser Arg Arg
    1400                1405                1410

Ala Ala Glu His Arg Asp Thr Thr Asp Val Pro Glu Met Pro His
    1415                1420                1425

Ser Lys Gly Gln Gly Glu Ser Asp Pro Leu Asp His Glu Pro Ala
    1430                1435                1440

Val Ser Pro Leu Leu Pro Arg Lys Glu Arg Gly Pro Pro Glu Gly
    1445                1450                1455

Gly Leu Asn Glu Asp Glu Arg Leu Leu Pro Lys Asp Lys Lys Thr
    1460                1465                1470

Asn Leu Phe Ser Ala Leu Ile Lys Lys Lys Lys Lys Thr Ala Pro
    1475                1480                1485

Thr Pro Pro Lys Arg Ser Ser Ser Phe Arg Glu Met Asp Gly Gln
    1490                1495                1500

Pro Glu Arg Arg Gly Ala Gly Glu Glu Glu Gly Arg Asp Ile Ser
    1505                1510                1515

Asn Gly Ala Leu Ala Phe Thr Pro Leu Asp Thr Ala Asp Pro Ala
    1520                1525                1530

Lys Ser Pro Lys Pro Ser Asn Gly Ala Gly Val Pro Asn Gly Ala
    1535                1540                1545

Leu Arg Glu Ser Gly Gly Ser Gly Phe Arg Ser Pro His Leu Trp
    1550                1555                1560

Lys Lys Ser Ser Thr Leu Thr Ser Ser Arg Leu Ala Thr Gly Glu
    1565                1570                1575

Glu Glu Gly Gly Gly Ser Ser Ser Lys Arg Phe Leu Arg Ser Cys
    1580                1585                1590

Ser Val Ser Cys Val Pro His Gly Ala Lys Asp Thr Glu Trp Arg
    1595                1600                1605

Ser Val Thr Leu Pro Arg Asp Leu Gln Ser Thr Gly Arg Gln Phe
    1610                1615                1620

Asp Ser Ser Thr Phe Gly Gly His Lys Ser Glu Lys Pro Ala Leu
    1625                1630                1635

Pro Arg Lys Arg Ala Gly Glu Asn Arg Ser Asp Gln Val Thr Arg
    1640                1645                1650

Gly Thr Val Thr Pro Pro Pro Arg Leu Val Lys Lys Asn Glu Glu
    1655                1660                1665

Ala Ala Asp Glu Val Phe Lys Asp Ile Met Glu Ser Ser Pro Gly
    1670                1675                1680

Ser Ser Pro Pro Asn Leu Thr Pro Lys Pro Leu Arg Arg Gln Val
    1685                1690                1695

Thr Val Ala Pro Ala Ser Gly Leu Pro His Lys Glu Glu Ala Trp
    1700                1705                1710

Lys Gly Ser Ala Leu Gly Thr Pro Ala Ala Ala Glu Pro Val Thr
    1715                1720                1725

Pro Thr Ser Lys Ala Gly Ser Gly Ala Pro Arg Gly Thr Ser Lys
    1730                1735                1740

Gly Pro Ala Glu Glu Ser Arg Val Arg Arg His Lys His Ser Ser
    1745                1750                1755

Glu Ser Pro Gly Arg Asp Lys Gly Lys Leu Ser Lys Leu Lys Pro
    1760                1765                1770

Ala Pro Pro Pro Pro Pro Ala Ala Ser Ala Gly Lys Ala Gly Gly
    1775                1780                1785

Lys Pro Ser Gln Arg Pro Gly Gln Glu Ala Ala Gly Glu Ala Val
    1790                1795                1800

Leu Gly Ala Lys Thr Lys Ala Thr Ser Leu Val Asp Ala Val Asn
    1805                1810                1815

Ser Asp Ala Ala Lys Pro Ser Gln Pro Ala Glu Gly Leu Lys Lys
    1820                1825                1830

Pro Val Leu Pro Ala Thr Pro Lys Pro His Pro Ala Lys Pro Ser
    1835                1840                1845

Gly Thr Pro Ile Ser Pro Ala Pro Val Pro Leu Ser Thr Leu Pro
    1850                1855                1860

Ser Ala Ser Ser Ala Leu Ala Gly Asp Gln Pro Ser Ser Thr Ala
    1865                1870                1875

Phe Ile Pro Leu Ile Ser Thr Arg Val Ser Leu Arg Lys Thr Arg

1880                1885                1890

Gln Pro  Pro Glu Arg Ala Ser  Gly Ala Ile Thr Lys  Gly Val Val
    1895                1900                1905

Leu Asp  Ser Thr Glu Ala Leu  Cys Leu Ala Ile Ser  Gly Asn Ser
    1910                1915                1920

Glu Gln  Met Ala Ser His Ser  Ala Val Leu Glu Ala  Gly Lys Asn
    1925                1930                1935

Leu Tyr  Thr Phe Cys Val Ser  Tyr Val Asp Ser Ile  Gln Gln Met
    1940                1945                1950

Arg Asn  Lys Phe Ala Phe Arg  Glu Ala Ile Asn Lys  Leu Glu Asn
    1955                1960                1965

Asn Leu  Arg Glu Leu Gln Ile  Cys Pro Ala Ser Ala  Gly Ser Gly
    1970                1975                1980

Pro Ala  Ala Thr Gln Asp Phe  Ser Lys Leu Leu Ser  Ser Val Lys
    1985                1990                1995

Glu Ile  Ser Asp Ile Val Gln  Arg
    2000                2005

<210>  8
<211>  1530
<212>  PRT
<213>  Homo sapiens

<400>  8

Met Val Asp Pro Val Gly Phe Ala Glu Ala Trp Lys Ala Gln Phe Pro
1               5                   10                  15

Asp Ser Glu Pro Pro Arg Met Glu Leu Arg Ser Val Gly Asp Ile Glu
            20                  25                  30

Gln Glu Leu Glu Arg Cys Lys Ala Ser Ile Arg Arg Leu Glu Gln Glu
        35                  40                  45

Val Asn Gln Glu Arg Phe Arg Met Ile Tyr Leu Gln Thr Leu Leu Ala
    50                  55                  60

Lys Glu Lys Lys Ser Tyr Asp Arg Gln Arg Trp Gly Phe Arg Arg Ala
65                  70                  75                  80

Ala Gln Ala Pro Asp Gly Ala Ser Glu Pro Arg Ala Ser Ala Ser Arg
                85                  90                  95

Pro Gln Pro Ala Pro Ala Asp Gly Ala Asp Pro Pro Pro Ala Glu Glu
        100                 105                 110

Pro Glu Ala Arg Pro Asp Gly Glu Gly Ser Pro Gly Lys Ala Arg Pro
        115                 120                 125

Gly Thr Ala Arg Arg Pro Gly Ala Ala Ala Ser Gly Glu Arg Asp Asp
    130                 135                 140

Arg Gly Pro Pro Ala Ser Val Ala Ala Leu Arg Ser Asn Phe Glu Arg
145                 150                 155                 160

Ile Arg Lys Gly His Gly Gln Pro Gly Ala Asp Ala Glu Lys Pro Phe
                165                 170                 175

Tyr Val Asn Val Glu Phe His His Glu Arg Gly Leu Val Lys Val Asn
            180                 185                 190

Asp Lys Glu Val Ser Asp Arg Ile Ser Ser Leu Gly Ser Gln Ala Met
        195                 200                 205

Gln Met Glu Arg Lys Lys Ser Gln His Gly Ala Gly Ser Ser Val Gly
    210                 215                 220

Asp Ala Ser Arg Pro Pro Tyr Arg Gly Arg Ser Ser Glu Ser Ser Cys
225                 230                 235                 240

Gly Val Asp Gly Asp Tyr Glu Asp Ala Glu Leu Asn Pro Arg Phe Leu
                245                 250                 255

Lys Asp Asn Leu Ile Asp Ala Asn Gly Gly Ser Arg Pro Pro Trp Pro
            260                 265                 270

Pro Leu Glu Tyr Gln Pro Tyr Gln Ser Ile Tyr Val Gly Gly Ile Met
        275                 280                 285

Glu Gly Glu Gly Lys Gly Pro Leu Leu Arg Ser Gln Ser Thr Ser Glu
    290                 295                 300

Gln Glu Lys Arg Leu Thr Trp Pro Arg Arg Ser Tyr Ser Pro Arg Ser
305                 310                 315                 320

Phe Glu Asp Cys Gly Gly Gly Tyr Thr Pro Asp Cys Ser Ser Asn Glu
                325                 330                 335

Asn Leu Thr Ser Ser Glu Glu Asp Phe Ser Ser Gly Gln Ser Ser Arg
            340                 345                 350

Val Ser Pro Ser Pro Thr Thr Tyr Arg Met Phe Arg Asp Lys Ser Arg
        355                 360                 365

Ser Pro Ser Gln Asn Ser Gln Gln Ser Phe Asp Ser Ser Ser Pro Pro
    370                 375                 380

Thr Pro Gln Cys His Lys Arg His Arg His Cys Pro Val Val Val Ser
385                 390                 395                 400

Glu Ala Thr Ile Val Gly Val Arg Lys Thr Gly Gln Ile Trp Pro Asn
                405                 410                 415

Asp Asp Glu Gly Ala Phe His Gly Asp Ala Glu Ala Leu Gln Arg Pro
            420                 425                 430

Val Ala Ser Asp Phe Glu Pro Gln Gly Leu Ser Glu Ala Ala Arg Trp
        435                 440                 445

Asn Ser Lys Glu Asn Leu Leu Ala Gly Pro Ser Glu Asn Asp Pro Asn
    450                 455                 460

Leu Phe Val Ala Leu Tyr Asp Phe Val Ala Ser Gly Asp Asn Thr Leu
465                 470                 475                 480

Ser Ile Thr Lys Gly Glu Lys Leu Arg Val Leu Gly Tyr Asn His Asn
                485                 490                 495

Gly Glu Trp Cys Glu Ala Gln Thr Lys Asn Gly Gln Gly Trp Val Pro
            500                 505                 510

Ser Asn Tyr Ile Thr Pro Val Asn Ser Leu Glu Lys His Ser Trp Tyr
        515                 520                 525

His Gly Pro Val Ser Arg Asn Ala Ala Glu Tyr Pro Leu Ser Ser Gly
    530                 535                 540

Ile Asn Gly Ser Phe Leu Val Arg Glu Ser Glu Ser Ser Pro Ser Gln
545                 550                 555                 560

Arg Ser Ile Ser Leu Arg Tyr Glu Gly Arg Val Tyr His Tyr Arg Ile
                565                 570                 575

Asn Thr Ala Ser Asp Gly Lys Leu Tyr Val Ser Ser Glu Ser Arg Phe
            580                 585                 590

Asn Thr Leu Ala Glu Leu Val His His His Ser Thr Val Ala Asp Gly
        595                 600                 605

Leu Ile Thr Thr Leu His Tyr Pro Ala Pro Lys Arg Asn Lys Pro Thr

610                 615                 620

Val Tyr Gly Val Ser Pro Asn Tyr Asp Lys Trp Glu Met Glu Arg Thr
625                 630                 635                 640

Asp Ile Thr Met Lys His Lys Leu Gly Gly Gly Gln Tyr Gly Glu Val
                645                 650                 655

Tyr Glu Gly Val Trp Lys Lys Tyr Ser Leu Thr Val Ala Val Lys Thr
            660                 665                 670

Leu Lys Glu Asp Thr Met Glu Val Glu Glu Phe Leu Lys Glu Ala Ala
        675                 680                 685

Val Met Lys Glu Ile Lys His Pro Asn Leu Val Gln Leu Leu Gly Val
    690                 695                 700

Cys Thr Arg Glu Pro Pro Phe Tyr Ile Ile Ile Glu Phe Met Thr Tyr
705                 710                 715                 720

Gly Asn Leu Leu Asp Tyr Leu Arg Glu Cys Asn Arg Gln Glu Val Asn
                725                 730                 735

Ala Val Val Leu Leu Tyr Met Ala Thr Gln Ile Ser Ser Ala Met Glu
            740                 745                 750

Tyr Leu Glu Lys Lys Asn Phe Ile His Arg Asp Leu Ala Ala Arg Asn
        755                 760                 765

Cys Leu Val Gly Glu Asn His Leu Val Lys Val Ala Asp Phe Gly Leu
    770                 775                 780

Ser Arg Leu Met Thr Gly Asp Thr Tyr Thr Ala His Ala Gly Ala Lys
785                 790                 795                 800

Phe Pro Ile Lys Trp Thr Ala Pro Glu Ser Leu Ala Tyr Asn Lys Phe
                805                 810                 815

Ser Ile Lys Ser Asp Val Trp Ala Phe Gly Val Leu Leu Trp Glu Ile
            820                 825                 830

Ala Thr Tyr Gly Met Ser Pro Tyr Pro Gly Ile Asp Arg Ser Gln Val
        835                 840                 845

Tyr Glu Leu Leu Glu Lys Asp Tyr Arg Met Lys Arg Pro Glu Gly Cys
    850                 855                 860

Pro Glu Lys Val Tyr Glu Leu Met Arg Ala Cys Trp Gln Trp Asn Pro
865                 870                 875                 880

Ser Asp Arg Pro Ser Phe Ala Glu Ile His Gln Ala Phe Glu Thr Met
                885                 890                 895

Phe Gln Glu Ser Ser Ile Ser Asp Glu Val Glu Lys Glu Leu Gly Lys
            900                 905                 910

Gln Gly Val Arg Gly Ala Val Thr Thr Leu Leu Gln Ala Pro Glu Leu
        915                 920                 925

Pro Thr Lys Thr Arg Thr Ser Arg Arg Ala Ala Glu His Arg Asp Thr
    930                 935                 940

Thr Asp Val Pro Glu Met Pro His Ser Lys Gly Gln Gly Glu Ser Asp
945                 950                 955                 960

Pro Leu Asp His Glu Pro Ala Val Ser Pro Leu Leu Pro Arg Lys Glu
                965                 970                 975

Arg Gly Pro Pro Glu Gly Gly Leu Asn Glu Asp Glu Arg Leu Leu Pro
            980                 985                 990

Lys Asp Lys Lys Thr Asn Leu Phe  Ser Ala Leu Ile Lys  Lys Lys Lys
        995                 1000                1005

Lys Thr  Ala Pro Thr Pro Pro  Lys Arg Ser Ser Ser  Phe Arg Glu
    1010                1015                1020

Met Asp  Gly Gln Pro Glu Arg  Arg Gly Ala Gly Glu  Glu Glu Gly
    1025                1030                1035

Arg Asp  Ile Ser Asn Gly Ala  Leu Ala Phe Thr Pro  Leu Asp Thr
    1040                1045                1050

Ala Asp  Pro Ala Lys Ser Pro  Lys Pro Ser Asn Gly  Ala Gly Val
    1055                1060                1065

Pro Asn  Gly Ala Leu Arg Glu  Ser Gly Gly Ser Gly  Phe Arg Ser
    1070                1075                1080

Pro His  Leu Trp Lys Lys Ser  Ser Thr Leu Thr Ser  Ser Arg Leu
    1085                1090                1095

Ala Thr  Gly Glu Glu Glu Gly  Gly Gly Ser Ser Ser  Lys Arg Phe
    1100                1105                1110

Leu Arg  Ser Cys Ser Val Ser  Cys Val Pro His Gly  Ala Lys Asp
    1115                1120                1125

Thr Glu Trp Arg Ser Val Thr Leu Pro Arg Asp Leu Gln Ser Thr
    1130                1135                1140

Gly Arg Gln Phe Asp Ser Ser Thr Phe Gly Gly His Lys Ser Glu
    1145                1150                1155

Lys Pro Ala Leu Pro Arg Lys Arg Ala Gly Glu Asn Arg Ser Asp
    1160                1165                1170

Gln Val Thr Arg Gly Thr Val Thr Pro Pro Pro Arg Leu Val Lys
    1175                1180                1185

Lys Asn Glu Glu Ala Ala Asp Glu Val Phe Lys Asp Ile Met Glu
    1190                1195                1200

Ser Ser Pro Gly Ser Ser Pro Pro Asn Leu Thr Pro Lys Pro Leu
    1205                1210                1215

Arg Arg Gln Val Thr Val Ala Pro Ala Ser Gly Leu Pro His Lys
    1220                1225                1230

Glu Glu Ala Trp Lys Gly Ser Ala Leu Gly Thr Pro Ala Ala Ala
    1235                1240                1245

Glu Pro Val Thr Pro Thr Ser Lys Ala Gly Ser Gly Ala Pro Arg
    1250                1255                1260

Gly Thr Ser Lys Gly Pro Ala Glu Glu Ser Arg Val Arg Arg His
    1265                1270                1275

Lys His Ser Ser Glu Ser Pro Gly Arg Asp Lys Gly Lys Leu Ser
    1280                1285                1290

Lys Leu Lys Pro Ala Pro Pro Pro Pro Pro Ala Ala Ser Ala Gly
    1295                1300                1305

Lys Ala Gly Gly Lys Pro Ser Gln Arg Pro Gly Gln Glu Ala Ala
    1310                1315                1320

Gly Glu Ala Val Leu Gly Ala Lys Thr Lys Ala Thr Ser Leu Val
    1325                1330                1335

Asp Ala Val Asn Ser Asp Ala Ala Lys Pro Ser Gln Pro Ala Glu
    1340                1345                1350

Gly Leu Lys Lys Pro Val Leu Pro Ala Thr Pro Lys Pro His Pro
    1355                1360                1365

Ala Lys Pro Ser Gly Thr Pro Ile Ser Pro Ala Pro Val Pro Leu
    1370                1375                1380

Ser Thr Leu Pro Ser Ala Ser Ser Ala Leu Ala Gly Asp Gln Pro
    1385                1390                1395

Ser Ser Thr Ala Phe Ile Pro Leu Ile Ser Thr Arg Val Ser Leu
    1400                1405                1410

Arg Lys Thr Arg Gln Pro Pro Glu Arg Ala Ser Gly Ala Ile Thr
    1415                1420                1425

Lys Gly Val Val Leu Asp Ser Thr Glu Ala Leu Cys Leu Ala Ile
    1430                1435                1440

Ser Gly Asn Ser Glu Gln Met Ala Ser His Ser Ala Val Leu Glu
    1445                1450                1455

Ala Gly Lys Asn Leu Tyr Thr Phe Cys Val Ser Tyr Val Asp Ser
    1460                1465                1470

Ile Gln Gln Met Arg Asn Lys Phe Ala Phe Arg Glu Ala Ile Asn
    1475                1480                1485

Lys Leu Glu Asn Asn Leu Arg Glu Leu Gln Ile Cys Pro Ala Ser
    1490                1495                1500

Ala Gly Ser Gly Pro Ala Ala Thr Gln Asp Phe Ser Lys Leu Leu
    1505                1510                1515

Ser Ser Val Lys Glu Ile Ser Asp Ile Val Gln Arg
    1520                1525                1530

<210>  9
<211>  676
<212>  PRT
<213>  Homo sapiens

<400>  9

Leu Gly Tyr Trp Lys Ile Lys Gly Leu Val Gln Pro Thr Arg Leu Leu
1               5                   10                  15

Leu Glu Tyr Leu Glu Glu Lys Tyr Glu Glu His Leu Tyr Glu Arg Asp
            20                  25                  30

Glu Gly Asp Lys Trp Arg Asn Lys Lys Phe Glu Leu Gly Leu Glu Phe
        35                  40                  45

Pro Asn Leu Pro Tyr Tyr Ile Asp Gly Asp Val Lys Leu Thr Gln Ser
    50                  55                  60

Met Ala Ile Ile Arg Tyr Ile Ala Asp Lys His Asn Met Leu Gly Gly
65                  70                  75                  80

Cys Pro Lys Glu Arg Ala Glu Ile Ser Met Leu Glu Gly Ala Val Asp
                85                  90                  95

Ile Arg Tyr Gly Val Ser Arg Ile Ala Tyr Ser Lys Asp Phe Glu Thr
            100                 105                 110

Leu Lys Val Asp Phe Leu Ser Lys Leu Pro Glu Met Leu Lys Met Phe
        115                 120                 125

Glu Asp Arg Leu Cys His Lys Thr Tyr Leu Asn Gly Asp His Val Thr
    130                 135                 140

His Pro Asp Phe Met Leu Tyr Asp Ala Leu Asp Val Val Leu Tyr Met
145                 150                 155                 160

Asp Pro Met Cys Leu Asp Ala Phe Pro Lys Leu Val Cys Phe Lys Lys
                165                 170                 175

Arg Ile Glu Ala Ile Pro Gln Ile Asp Lys Tyr Leu Lys Ser Ser Lys
            180                 185                 190

Tyr Ile Trp Pro Leu Gln Gly Trp Gln Ala Thr Phe Gly Gly Gly Asp
        195                 200                 205

His Pro Pro Lys Ser Asp Leu Val Pro Arg His Asn Gln Thr Ser Leu
    210                 215                 220

Tyr Lys Lys Ala Gly Ser Ala Ala Ala Val Leu Glu Glu Asn Leu Tyr
225                 230                 235                 240

Phe Gln Gly Thr Tyr Lys Tyr Leu Gln Lys Pro Met Tyr Glu Val Gln
                245                 250                 255

Trp Lys Val Val Glu Glu Ile Asn Gly Asn Asn Tyr Val Tyr Ile Asp
            260                 265                 270

Pro Thr Gln Leu Pro Tyr Asp His Lys Trp Glu Phe Pro Arg Asn Arg
        275                 280                 285

Leu Ser Phe Gly Lys Thr Leu Gly Ala Gly Ala Phe Gly Lys Val Val
    290                 295                 300

Glu Ala Thr Ala Tyr Gly Leu Ile Lys Ser Asp Ala Ala Met Thr Val
305                 310                 315                 320

Ala Val Lys Met Leu Lys Pro Ser Ala His Leu Thr Glu Arg Glu Ala
                325                 330                 335

Leu Met Ser Glu Leu Lys Val Leu Ser Tyr Leu Gly Asn His Met Asn
            340                 345                 350

Ile Val Asn Leu Leu Gly Ala Cys Thr Ile Gly Gly Pro Thr Leu Val
        355                 360                 365

Ile Thr Glu Tyr Cys Cys Tyr Gly Asp Leu Leu Asn Phe Leu Arg Arg
    370                 375                 380

Lys Arg Asp Ser Phe Ile Cys Ser Lys Gln Glu Asp His Ala Glu Ala
385                 390                 395                 400

Ala Leu Tyr Lys Asn Leu Leu His Ser Lys Glu Ser Ser Cys Ser Asp
                405                 410                 415

Ser Thr Asn Glu Tyr Met Asp Met Lys Pro Gly Val Ser Tyr Val Val
            420                 425                 430

Pro Thr Lys Ala Asp Lys Arg Arg Ser Val Arg Ile Gly Ser Tyr Ile
        435                 440                 445

Glu Arg Asp Val Thr Pro Ala Ile Met Glu Asp Asp Glu Leu Ala Leu
    450                 455                 460

Asp Leu Glu Asp Leu Leu Ser Phe Ser Tyr Gln Val Ala Lys Gly Met
465                 470                 475                 480

Ala Phe Leu Ala Ser Lys Asn Cys Ile His Arg Asp Leu Ala Ala Arg
                485                 490                 495

Asn Ile Leu Leu Thr His Gly Arg Ile Thr Lys Ile Cys Asp Phe Gly
            500                 505                 510

Leu Ala Arg Asp Ile Lys Asn Asp Ser Asn Tyr Val Val Lys Gly Asn
        515                 520                 525

Ala Arg Leu Pro Val Lys Trp Met Ala Pro Glu Ser Ile Phe Asn Cys
    530                 535                 540

Val Tyr Thr Phe Glu Ser Asp Val Trp Ser Tyr Gly Ile Phe Leu Trp
545                 550                 555                 560

Glu Leu Phe Ser Leu Gly Ser Ser Pro Tyr Pro Gly Met Pro Val Asp
                565                 570                 575

Ser Lys Phe Tyr Lys Met Ile Lys Glu Gly Phe Arg Met Leu Ser Pro
            580                 585                 590

Glu His Ala Pro Ala Glu Met Tyr Asp Ile Met Lys Thr Cys Trp Asp
        595                 600                 605

Ala Asp Pro Leu Lys Arg Pro Thr Phe Lys Gln Ile Val Gln Leu Ile
    610                 615                 620

Glu Lys Gln Ile Ser Glu Ser Thr Asn His Ile Tyr Ser Asn Leu Ala
625                 630                 635                 640

Asn Cys Ser Pro Asn Arg Gln Lys Pro Val Val Asp His Ser Val Arg
                645                 650                 655

Ile Asn Ser Val Gly Ser Thr Ala Ser Ser Ser Gln Pro Leu Leu Val
            660                 665                 670

His Asp Asp Val
        675

<210>  10
<211>  474
<212>  PRT
<213>  Homo sapiens

<400>  10

Met Ser Tyr Tyr His His His His His His Asp Tyr Asp Ile Pro Thr
1               5                   10                  15

Thr Glu Asn Leu Tyr Phe Gln Gly Ala Met Leu Val Pro Arg Gly Ser
            20                  25                  30

Pro Trp Ile Pro Phe Thr Met Lys Lys Arg Lys Gln Ile Lys Asp Leu
        35                  40                  45

Gly Ser Glu Leu Val Arg Tyr Asp Ala Arg Val His Thr Pro His Leu
    50                  55                  60

Asp Arg Leu Val Ser Ala Arg Ser Val Ser Pro Thr Thr Glu Met Val
65                  70                  75                  80

Ser Asn Glu Ser Val Asp Tyr Arg Ala Thr Phe Pro Glu Asp Gln Phe
                85                  90                  95

Pro Asn Ser Ser Gln Asn Gly Ser Cys Arg Gln Val Gln Tyr Pro Leu
            100                 105                 110

Thr Asp Met Ser Pro Ile Leu Thr Ser Gly Asp Ser Asp Ile Ser Ser
        115                 120                 125

Pro Leu Leu Gln Asn Thr Val His Ile Asp Leu Ser Ala Leu Asn Pro
    130                 135                 140

Glu Leu Val Gln Ala Val Gln His Val Val Ile Gly Pro Ser Ser Leu
145                 150                 155                 160

Ile Val His Phe Asn Glu Val Ile Gly Arg Gly His Phe Gly Cys Val
                165                 170                 175

Tyr His Gly Thr Leu Leu Asp Asn Asp Gly Lys Lys Ile His Cys Ala
            180                 185                 190

Val Lys Ser Leu Asn Arg Ile Thr Asp Ile Gly Glu Val Ser Gln Phe
        195                 200                 205

Leu Thr Glu Gly Ile Ile Met Lys Asp Phe Ser His Pro Asn Val Leu
    210                 215                 220

Ser Leu Leu Gly Ile Cys Leu Arg Ser Glu Gly Ser Pro Leu Val Val
225                 230                 235                 240

Leu Pro Tyr Met Lys His Gly Asp Leu Arg Asn Phe Ile Arg Asn Glu
                245                 250                 255

Thr His Asn Pro Thr Val Lys Asp Leu Ile Gly Phe Gly Leu Gln Val
            260                 265                 270

Ala Lys Gly Met Lys Tyr Leu Ala Ser Lys Lys Phe Val His Arg Asp
        275                 280                 285

Leu Ala Ala Arg Asn Cys Met Leu Asp Glu Lys Phe Thr Val Lys Val
    290                 295                 300

Ala Asp Phe Gly Leu Ala Arg Asp Met Tyr Asp Lys Glu Tyr Tyr Ser
305                 310                 315                 320

Val His Asn Lys Thr Gly Ala Lys Leu Pro Val Lys Trp Met Ala Leu
                325                 330                 335

Glu Ser Leu Gln Thr Gln Lys Phe Thr Thr Lys Ser Asp Val Trp Ser
            340                 345                 350

Phe Gly Val Leu Leu Trp Glu Leu Met Thr Arg Gly Ala Pro Pro Tyr
        355                 360                 365

Pro Asp Val Asn Thr Phe Asp Ile Thr Val Tyr Leu Leu Gln Gly Arg
    370                 375                 380

Arg Leu Leu Gln Pro Glu Tyr Cys Pro Asp Pro Leu Tyr Glu Val Met
385                 390                 395                 400

Leu Lys Cys Trp His Pro Lys Ala Glu Met Arg Pro Ser Phe Ser Glu
                405                 410                 415

Leu Val Ser Arg Ile Ser Ala Ile Phe Ser Thr Phe Ile Gly Glu His
            420                 425                 430

Tyr Val His Val Asn Ala Thr Tyr Val Asn Val Lys Cys Val Ala Pro
        435                 440                 445

Tyr Pro Ser Leu Leu Ser Ser Glu Asp Asn Ala Asp Asp Glu Val Asp
    450                 455                 460

Thr Arg Pro Ala Ser Phe Trp Glu Thr Ser
465                 470

<210>  11
<211>  307
<212>  PRT
<213>  Homo sapiens

<400>  11

Met Asp Arg Gly Ser His His His His His His Gly Ser Glu Asp Arg
1               5                   10                  15

Asn Arg Met Lys Thr Leu Gly Arg Arg Asp Ser Ser Asp Asp Trp Glu
            20                  25                  30

Ile Pro Asp Gly Gln Ile Thr Val Gly Gln Arg Ile Gly Ser Gly Ser
        35                  40                  45

Phe Gly Thr Val Tyr Lys Gly Lys Trp His Gly Asp Val Ala Val Lys
    50                  55                  60

Met Leu Asn Val Thr Ala Pro Thr Pro Gln Gln Leu Gln Ala Phe Lys
65                  70                  75                  80

Asn Glu Val Gly Val Leu Arg Lys Thr Arg His Val Asn Ile Leu Leu
                85                  90                  95

Phe Met Gly Tyr Ser Thr Lys Pro Gln Leu Ala Ile Val Thr Gln Trp
            100                 105                 110

Cys Glu Gly Ser Ser Leu Tyr His His Leu His Ile Ile Glu Thr Lys
        115                 120                 125

Phe Glu Met Ile Lys Leu Ile Asp Ile Ala Arg Gln Thr Ala Gln Gly

130                 135                 140

Met Asp Tyr Leu His Ala Lys Ser Ile Ile His Arg Asp Leu Lys Ser
145                 150                 155                 160

Asn Asn Ile Phe Leu His Glu Asp Leu Thr Val Lys Ile Gly Asp Phe
                165                 170                 175

Gly Leu Ala Thr Glu Lys Ser Arg Trp Ser Gly Ser His Gln Phe Glu
            180                 185                 190

Gln Leu Ser Gly Ser Ile Leu Trp Met Ala Pro Glu Val Ile Arg Met
        195                 200                 205

Gln Asp Lys Asn Pro Tyr Ser Phe Gln Ser Asp Val Tyr Ala Phe Gly
    210                 215                 220

Ile Val Leu Tyr Glu Leu Met Thr Gly Gln Leu Pro Tyr Ser Asn Ile
225                 230                 235                 240

Asn Asn Arg Asp Gln Ile Ile Phe Met Val Gly Arg Gly Tyr Leu Ser
                245                 250                 255

Pro Asp Leu Ser Lys Val Arg Ser Asn Cys Pro Lys Ala Met Lys Arg
            260                 265                 270

Leu Met Ala Glu Cys Leu Lys Lys Lys Arg Asp Glu Arg Pro Leu Phe
        275                 280                 285

Pro Gln Ile Leu Ala Ser Ile Glu Leu Leu Ala Arg Ser Leu Pro Lys
    290                 295                 300

Ile His Arg
305

<210>  12
<211>  333
<212>  PRT
<213>  Homo sapiens

<400>  12

Met Glu Leu Lys Asp Asp Asp Phe Glu Lys Ile Ser Glu Leu Gly Ala
1               5                   10                  15

Gly Asn Gly Gly Val Val Phe Lys Val Ser His Lys Pro Ser Gly Leu
            20                  25                  30

Val Met Ala Arg Lys Leu Ile His Leu Glu Ile Lys Pro Ala Ile Arg
        35                  40                  45

Asn Gln Ile Ile Arg Glu Leu Gln Val Leu His Glu Cys Asn Ser Pro
    50                  55                  60

Tyr Ile Val Gly Phe Tyr Gly Ala Phe Tyr Ser Asp Gly Glu Ile Ser
65                  70                  75                  80

Ile Cys Met Glu His Met Asp Gly Gly Ser Leu Asp Gln Val Leu Lys
                85                  90                  95

Lys Ala Gly Arg Ile Pro Glu Gln Ile Leu Gly Lys Val Ser Ile Ala
            100                 105                 110

Val Ile Lys Gly Leu Thr Tyr Leu Arg Glu Lys His Lys Ile Met His
        115                 120                 125

Arg Asp Val Lys Pro Ser Asn Ile Leu Val Asn Ser Arg Gly Glu Ile
    130                 135                 140

Lys Leu Cys Asp Phe Gly Val Ser Gly Gln Leu Ile Asp Ser Met Ala
145                 150                 155                 160

Asn Ser Phe Val Gly Thr Arg Ser Tyr Met Ser Pro Glu Arg Leu Gln
                165                 170                 175

Gly Thr His Tyr Ser Val Gln Ser Asp Ile Trp Ser Met Gly Leu Ser
            180                 185                 190

Leu Val Glu Met Ala Val Gly Arg Tyr Pro Ile Pro Pro Pro Asp Ala
        195                 200                 205

Lys Glu Leu Glu Leu Met Phe Gly Cys Gln Val Glu Gly Asp Ala Ala
    210                 215                 220

Glu Thr Pro Pro Arg Pro Arg Thr Pro Gly Arg Pro Leu Ser Ser Tyr
225                 230                 235                 240

Gly Met Asp Ser Arg Pro Pro Met Ala Ile Phe Glu Leu Leu Asp Tyr
                245                 250                 255

Ile Val Asn Glu Pro Pro Pro Lys Leu Pro Ser Gly Val Phe Ser Leu
            260                 265                 270

Glu Phe Gln Asp Phe Val Asn Lys Cys Leu Ile Lys Asn Pro Ala Glu
        275                 280                 285

Arg Ala Asp Leu Lys Gln Leu Met Val His Ala Phe Ile Lys Arg Ser
    290                 295                 300

Asp Ala Glu Glu Val Asp Phe Ala Gly Trp Leu Cys Ser Thr Ile Gly
305                 310                 315                 320

Leu Asn Gln Pro Ser Thr Pro Thr His Ala Ala Gly Val
                325                 330